# EUROPÄISCHE PATENTSCHRIFT

(11) **EP 2 872 590 B1**
(45) Veröffentlichungstag und Bekanntmachung des Hinweises auf die Patenterteilung: **14.11.2018**
(21) Anmeldenummer: 13730492.9
(22) Anmeldetag: 21.06.2013
(51) Int. Cl.: C09K 11/06, H05B 33/10, H01L 51/00, C07F 15/00

(54) **METALLKOMPLEXE**
METAL COMPLEXES
COMPLEXES METALLIQUES

(30) Priorität: 13.07.2012 EP 12005187
(43) Veröffentlichungstag der Anmeldung: 20.05.2015
(73) Patentinhaber: Merck Patent GmbH, 64293 Darmstadt (DE)
(72) Erfinder: STOESSEL, Philipp, 60487 Frankfurt am Main (DE); JOOSTEN, Dominik, 60487 Frankfurt am Main (DE); BREUNING, Esther, 64372 Ober-Ramstadt (DE); KAISER, Joachim, 64289 Darmstadt (DE); EHRENREICH, Christian, 64297 Darmstadt (DE)
(86) Internationale Anmeldenummer: PCT/EP2013/001844
(87) Internationale Veröffentlichungsnummer: WO 2014/008982

(56) Entgegenhaltungen:
- WO-A1-2011/157339
- DE-A1-102010 027 316

## Beschreibung

Die vorliegende Erfindung betrifft Metallkomplexe, welche sich für den Einsatz als Emitter in organischen Elektrolumineszenzvorrichtungen eignen.

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Dabei werden als emittierende Materialien zunehmend metallorganische Komplexe eingesetzt, die Phosphoreszenz statt Fluoreszenz zeigen (M. A. Baldo et al., Appl. Phys. Lett. 1999, 75, 4-6). Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, die Triplettemission zeigen, immer noch Verbesserungsbedarf, insbesondere im Hinblick auf Effizienz, Betriebsspannung und Lebensdauer. Dies gilt insbesondere für OLEDs, welche im kürzerwelligen Bereich, also grün und insbesondere blau, emittieren.

Gemäß dem Stand der Technik werden in phosphoreszierenden OLEDs als Triplettemitter insbesondere Iridium- und Platinkomplexe eingesetzt. Eine Verbesserung dieser OLEDs konnte dadurch erzielt werden, dass Metallkomplexe mit polypodalem Liganden bzw. Kryptate eingesetzt wurden, wodurch die Komplexe eine höhere thermische Stabilität aufweisen, was zu einer höheren Lebensdauer der OLEDs führt (WO 2004/081017, WO 2005/113563, WO 2006/008069). Es sind jedoch weitere Verbesserungen, insbesonderen in Hinblick auf die Effizienz und die Lebensdauer der Komplexe, wünschenswert.

Aus dem Stand der Technik sind weiterhin Iridiumkomplexe bekannt, welche als Liganden Imidazophenanthridin-Derivate bzw. Diimidazochinazolin-Derivate enthalten (WO 2007/095118). Diese Komplexe können bei Anwendung in organischen Elektrolumineszenzvorrichtungen, je nach genauer Struktur des Liganden, zu blauer Phosphoreszenz führen. Auch hier sind noch weitere Verbesserungen hinsichtlich Effizienz, Betriebsspannung und Lebensdauer, wünschenswert. Weiterhin besteht hier auch noch Verbesserungsbedarf in Bezug auf die Farbkoordinaten, um tiefblaue Emission erzielen zu können.

Aus WO 2010/086089 und WO 2011/157339 sind Metallkomplexe bekannt, welche als Liganden Imidazo-isochinolin-Derivate enthalten. Mit derartigen Komplexen wurden bereits gute Fortschritte in der Entwicklung blauer Triplettemitter erzielt. Jedoch sind auch hier noch weitere Verbesserungen hinsichtlich Effizienz, Betriebsspannung und Lebensdauer wünschenswert.

Aufgabe der vorliegenden Erfindung ist daher die Bereitstellung neuer Metallkomplexe, welche sich als Emitter für die Verwendung in OLEDs eignen. Insbesondere ist die Aufgabe, Emitter bereitzustellen, welche sich auch für blau oder grün phosphoreszierende OLEDs eignen, und welche dabei verbesserte Eigenschaften in Bezug auf Effizienz, Betriebsspannung, Lebensdauer, Farbkoordinaten und/oder Farbreinheit, d. h. Breite der Emissionsbande, zeigen.

Überraschend wurde gefunden, dass bestimmte, unten näher beschriebene Metallchelatkomplexe diese Aufgabe lösen und sich sehr gut für die Verwendung in einer organischen Elektrolumineszenzvorrichtung eignen. Diese Metallkomplexe und organische Elektrolumineszenzvorrichtungen, welche diese Komplexe enthalten, sind daher der Gegenstand der vorliegenden Erfindung.

Gegenstand der Erfindung ist somit eine Verbindung gemäß Formel (1),

M(L)ₙ(L')ₘ Formel (1)

welche eine Teilstruktur M(L)ₙ der Formel (2), Formel (3) oder Formel (4) enthält: 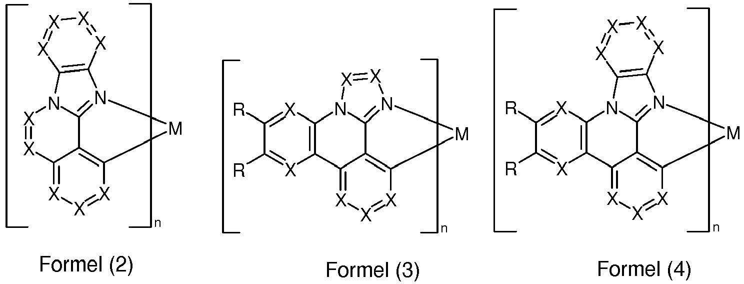 wobei für die verwendeten Symbole und Indizes gilt:
- M: ist Iridium oder Platin;
- X: ist bei jedem Auftreten gleich oder verschieden ausgewählt aus der Gruppe bestehend aus CR und N;
- R: ist bei jedem Auftreten gleich oder verschieden H, D, F, Cl, Br, I, N(R¹)₂, CN, NO₂, OH, COOH, C(=O)N(R¹)₂, Si(R¹)₃, B(OR¹)₂, C(=O)R¹, P(=O)(R¹)₂, S(=O)R¹, S(=O)₂R¹, OSO₂R¹, eine geradkettige Alkyl-, Alkoxy- oder Thioalkoxygruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkoxygruppe mit 3 bis 20 C-Atomen, die jeweils mit einem oder mehreren Resten R¹ substituiert sein kann, wobei eine oder mehrere nicht benachbarte CH₂-Gruppen durch R¹C=CR¹, C≡C, Si(R¹)₂, C=O, NR¹, O, S oder CONR¹ ersetzt sein können und wobei ein oder mehrere H-Atome durch D, F, Cl, Br, I oder CN ersetzt sein können, oder ein aromatisches oder heteroaromatisches Ringsystem mit 5 bis 60 aromatischen Ringatomen, das jeweils durch einen oder mehrere Reste R¹ substituiert sein kann, oder eine Aryloxy- oder Heteroaryloxygruppe mit 5 bis 40 aromatischen Ringatomen, die durch einen oder mehrere Reste R¹ substituiert sein kann, oder eine Aralkyl- oder Heteroaralkylgruppe mit 5 bis 40 aromatischen Ringatomen, die durch einen oder mehrere Reste R¹ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R¹ substituiert sein kann; dabei können zwei benachbarte Reste R auch miteinander ein mono- oder polycyclisches, aliphatisches, aromatisches oder heteroaromatisches Ringsystem bilden;
- R¹: ist bei jedem Auftreten gleich oder verschieden H, D, F, Cl, Br, I, N(R²)2, CN, NO₂, Si(R²)₃, B(OR²)₂, C(=O)R², P(=O)(R²)₂, S(=O)R², S(=O)₂R², OSO₂R², eine geradkettige Alkyl-, Alkoxy- oder Thioalkoxygruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkoxygruppe mit 3 bis 20 C-Atomen, die jeweils mit einem oder mehreren Resten R² substituiert sein kann, wobei eine oder mehrere nicht benachbarte CH₂-Gruppen durch R²C=CR², C≡C, Si(R²)₂, C=O, NR², O, S oder CONR² ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 40 aromatischen Ringatomen, die durch einen oder mehrere Reste R² substituiert sein kann, oder eine Aralkyl- oder Heteroaralkylgruppe mit 5 bis 40 aromatischen Ringatomen, die durch einen oder mehrere Reste R² substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R² substituiert sein kann; dabei können zwei oder mehrere benachbarte Reste R¹ miteinander oder R¹ mit R ein mono- oder polycyclisches, aliphatisches, aromatisches oder heteroaromatisches Ringsystem bilden;
- R²: ist bei jedem Auftreten gleich oder verschieden H, D, F oder ein aliphatischer, aromatischer und/oder heteroaromatischer Kohlenwasserstoffrest mit 1 bis 20 C-Atomen, in dem auch ein oder mehrere H-Atome durch F ersetzt sein können; dabei können zwei oder mehrere Substituenten R² auch miteinander ein mono- oder polycyclisches, aliphatisches Ringsystem bilden;
- L': ist gleich oder verschieden bei jedem Auftreten ein beliebiger Coligand;
- n: ist 1, 2 oder 3;
- m: ist 0, 1, 2, 3 oder 4;
dabei können auch mehrere Liganden L miteinander oder L mit L' über eine Einfachbindung oder eine bivalente oder trivalente Brücke verknüpft sein und so ein tridentates, tetradentates, pentadentates oder hexadentates Ligandensystem aufspannen;
dabei kann auch ein Substituent R zusätzlich an das Metall koordinieren;
dadurch gekennzeichnet, dass zwei benachbarte Gruppen X für CR stehen und die jeweiligen Reste R zusammen mit den C-Atomen einen Ring der folgenden Formel (5) oder Formel (6) aufspannen, 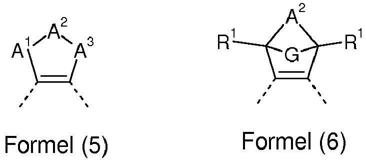 wobei R¹ und R² die oben genannten Bedeutungen aufweisen, die gestrichelten Bindungen die Verknüpfung der beiden Kohlenstoffatome im Liganden andeuten und weiterhin gilt:
- A¹, A³: ist gleich oder verschieden bei jedem Auftreten C(R³)₂, O, S, NR³ oder C(=O);
- A²: ist C(R¹)₂, O, S, NR³ oder C(=O);
- G: ist eine Alkylengruppe mit 1, 2 oder 3 C-Atomen, welche mit einem oder mehreren Resten R² substituiert sein kann, -CR²=CR²- oder eine ortho-verknüpfte Arylen- oder Heteroarylengruppe mit 5 bis 14 aromatischen Ringatomen, welche durch einen oder mehrere Reste R² substituiert sein kann;
- R³: ist gleich oder verschieden bei jedem Auftreten F, eine geradkettige Alkyl- oder Alkoxygruppe mit 1 bis 20 C-Atomen, eine verzweigte oder cyclische Alkyl- oder Alkoxygruppe mit 3 bis 20 C-Atomen, die jeweils mit einem oder mehreren Resten R² substituiert sein kann, wobei eine oder mehrere nicht benachbarte CH₂-Gruppen durch R²C=CR², C≡C, Si(R²)₂, C=O, NR², O, S oder CONR² ersetzt sein können und wobei ein oder mehrere H-Atome durch D oder F ersetzt sein können, oder ein aromatisches oder heteroaromatisches Ringsystem mit 5 bis 24 aromatischen Ringatomen, das jeweils durch einen oder mehrere Reste R² substituiert sein kann, oder eine Aryloxy- oder Heteroaryloxygruppe mit 5 bis 24 aromatischen Ringatomen, die durch einen oder mehrere Reste R² substituiert sein kann, oder eine Aralkyl- oder Heteroaralkylgruppe mit 5 bis 24 aromatischen Ringatomen, die durch einen oder mehrere Reste R² substituiert sein kann; dabei können zwei Reste R³, welche an dasselbe Kohlenstoffatom gebunden sind, miteinander ein aliphatisches oder aromatisches Ringsystem bilden und so ein Spirosystem aufspannen; weiterhin kann R³ mit einem benachbarten Rest R oder R¹ ein aliphatisches Ringsystem bilden;
mit der Maßgabe, dass in A¹-A²-A³ nicht zwei Heteroatome direkt aneinander gebunden sind.

Dabei ist die Anwesenheit einer Teilstruktur der Formel (5) oder Formel (6), also eines ankondensierten aliphatischen Fünfrings erfindungswesentlich. Wie aus der oben genannten Formel (5) hervorgeht, enthält der durch die beiden C-Atome, A¹, A² und A³ gebildete 5-Ring keine benzylischen Protonen, da R³, wenn diese für C(R³)₂ stehen, ungleich Wasserstoff ist. In den oben abgebildeten Strukturen der Formeln (5) und (6) sowie den weiteren als bevorzugt genannten Ausführungsformen dieser Strukturen wird formal eine Doppelbindung zwischen den zwei Kohlenstoffatomen abgebildet. Dies stellt eine Vereinfachung der chemischen Struktur dar, da diese beiden Kohlenstoffatome in ein aromatisches oder heteroaromatisches System eingebunden sind und somit die Bindung zwischen diesen beiden Kohlenstoffatomen formal zwischen dem Bindungsgrad einer Einfachbindung und dem einer Doppelbindung liegt. Das Einzeichnen der formalen Doppelbindung ist somit nicht limitierend für die Struktur auszulegen, sondern es ist dem Fachmann offensichtlich, dass hiermit eine aromatische Bindung gemeint ist.

Dabei bedeutet "benachbarte Gruppen X", dass die Gruppen X in der Struktur direkt aneinander gebunden sind.

Weiterhin bedeutet "benachbarte Reste" in der Definition der Reste, dass diese Reste an dasselbe oder an direkt aneinander gebundene C-Atome gebunden sind oder, wenn sie nicht an direkt gebundene C-Atome gebunden sind, dass es sich um die nächstmögliche Position handelt, in der ein Substituent gebunden sein kann. Dies wird anhand von einem spezifischen Liganden in der folgenden schematischen Darstellung nochmals erläutert:

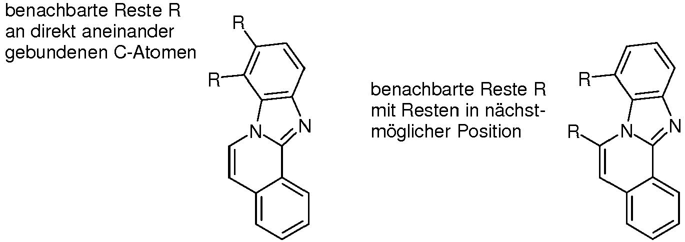

Dabei werden in den Komplexen der Formel (1) die Indizes n und m so gewählt, dass die Koordinationszahl am Metall M insgesamt, je nach Metall, der für dieses Metall üblichen Koordinationszahl entspricht. Dies ist für Übergangsmetalle je nach Metall üblicherweise die Koordinationszahl 4, 5 oder 6. Es ist generell bekannt, dass Metallkoordinationsverbindungen abhängig vom Metall und von der Oxidationsstufe des Metalls unterschiedliche Koordinationszahlen aufweisen, also eine unterschiedliche Anzahl von Liganden binden. Da die bevorzugten Koordinationszahlen von Metallen bzw. Metallionen in verschiedenen Oxidationsstufen zum allgemeinen Fachwissen des Fachmanns auf dem Gebiet der metallorganischen Chemie bzw. der Koordinationschemie gehören, ist es für den Fachmann ein Leichtes, je nach Metall und dessen Oxidationsstufe und je nach genauer Struktur des Liganden L eine geeignete Anzahl Liganden zu verwenden und somit die Indizes n und m geeignet zu wählen.

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Phenanthren, Chinolin, Isochinolin, etc., verstanden.

Ein aromatisches Ringsystem im Sinne dieser Erfindung enthält 6 bis 60 C-Atome im Ringsystem. Ein heteroaromatisches Ringsystem im Sinne dieser Erfindung enthält 1 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom oder eine Carbonylgruppe, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind. Weiterhin sollen Systeme, in denen zwei oder mehrere Aryl- oder Heteroarylgruppen direkt aneinander gebunden sind, wie z. B. Biphenyl oder Terphenyl, ebenfalls als aromatisches bzw. heteroaromatisches Ringsystem verstanden werden.

Unter einer cyclischen Alkyl-, Alkoxy- oder Thioalkoxygruppe im Sinne dieser Erfindung wird eine monocyclische, eine bicyclische oder eine polycyclische Gruppe verstanden.

Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluormethyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer C₁- bis C₄₀-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Unter einem aromatischen oder heteroaromatischen Ringsystem mit 5 - 60 aromatischen Ringatomen, welches noch jeweils mit den oben genannten Resten R substituiert sein kann und welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Bevorzugt sind Verbindungen gemäß Formel (1), dadurch gekennzeichnet, dass diese nicht geladen, d. h. elektrisch neutral, sind. Dies wird auf einfache Weise dadurch erreicht, dass die Ladung der Liganden L und L' so gewählt werden, dass sie die Ladung des komplexierten Metallatoms M kompensieren.

Bevorzugt sind weiterhin Verbindungen gemäß Formel (1), dadurch gekennzeichnet, dass die Summe der Valenzelektronen um das Metallatom in vierfach koordinierten Komplexen 16 und in sechsfach koordinierten Komplexen 18 beträgt. Diese Bevorzugung ist durch die besondere Stabilität dieser Metallkomplexe begründet.

In einer bevorzugten Ausführungsform der Erfindung steht M für ein Übergangsmetall, ausgewählt aus Ir(III) und Pt(II), insbesondere Ir(III).

In einer bevorzugten Ausführungsform der Erfindung ist M = Pt(II), also ein tetrakoordiniertes Metall, und der Index n steht für 1 oder 2. Wenn der Index n = 1 ist, sind noch ein bidentater oder zwei monodentate Liganden L', bevorzugt ein bidentater Ligand L', an das Metall M koordiniert. Wenn der Index n = 2 ist, ist der Index m = 0.

In einer weiteren bevorzugten Ausführungsform der Erfindung ist M = Ir(III), also ein hexakoordiniertes Metall, und der Index n steht für 1, 2 oder 3, bevorzugt für 2 oder 3. Wenn der Index n = 1 ist, sind noch vier monodentate oder zwei bidentate oder ein bidentater und zwei monodentate oder ein tridentater und ein monodentater oder ein tetradentater Ligand L', bevorzugt zwei bidentate Liganden L', an das Metall koordiniert. Wenn der Index n = 2 ist, sind noch ein bidentater oder zwei monodentate Liganden L', bevorzugt ein bidentater Ligand L', an das Metall koordiniert. Wenn der Index n = 3 ist, ist der Index m = 0.

Im Liganden L stehen bevorzugt keine, eine, zwei, drei oder vier Gruppen X, besonders bevorzugt keine, eine, zwei oder drei Gruppen X, ganz besonders bevorzugt keine, eine oder zwei Gruppen X für N.

Bevorzugte Ausführungsformen der Teilstrukturen der Formel (2) sind die Teilstrukturen der folgenden Formeln (2-A) bis (2-Q), 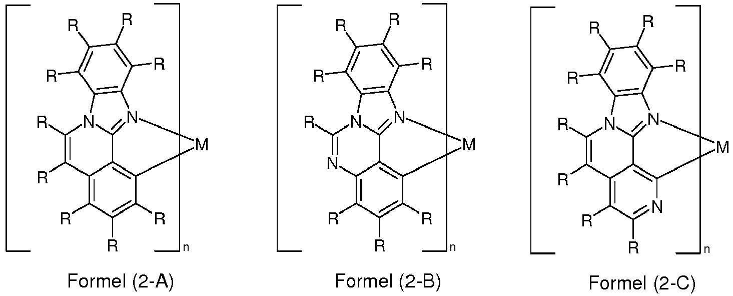 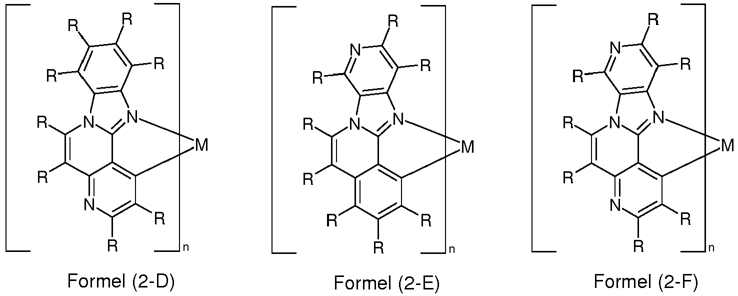 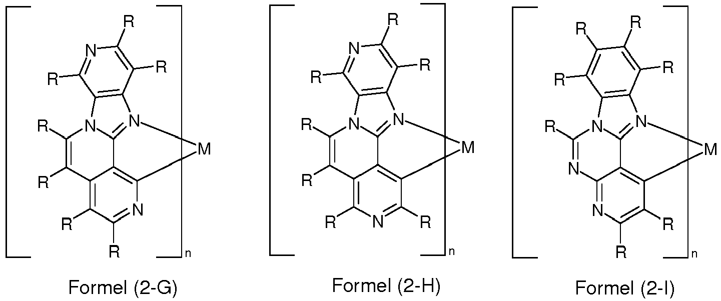 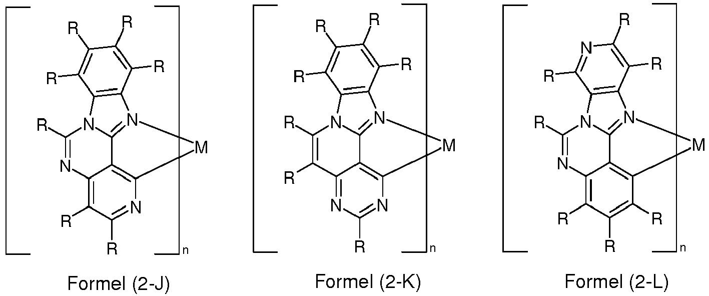 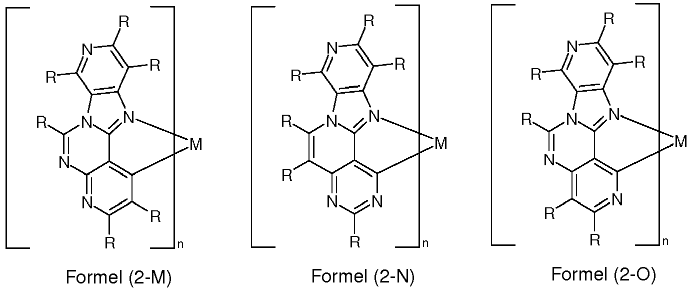 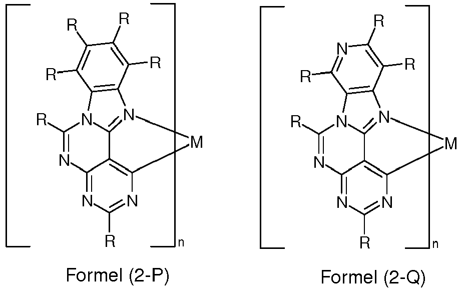 wobei die verwendeten Symbole und Indizes die oben genannten Bedeutungen aufweisen.

Bevorzugte Ausführungsformen der Teilstrukturen der Formel (3) sind die Teilstrukturen der folgenden Formeln (3-A) bis (3-F), 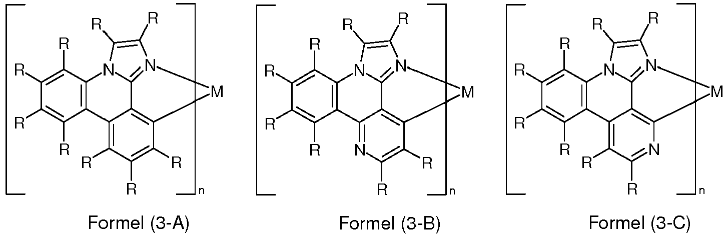 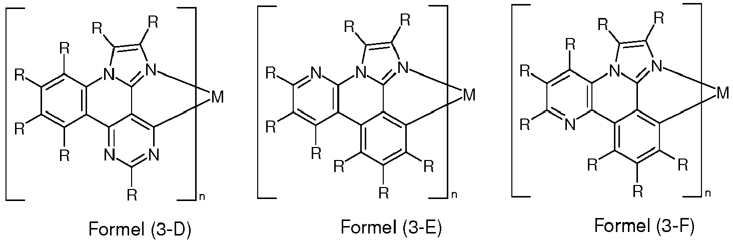 wobei die verwendeten Symbole und Indizes die oben genannten Bedeutungen aufweisen.

Bevorzugte Ausführungsformen der Teilstrukturen der Formel (4) sind die Teilstrukturen der folgenden Formeln (4-A) bis (4-F), 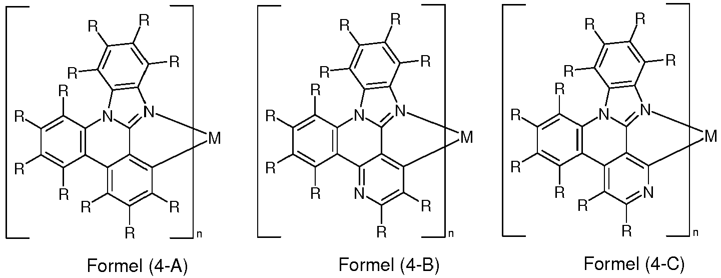 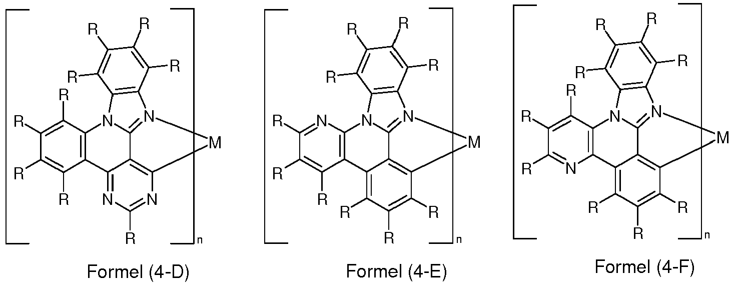 wobei die verwendeten Symbole und Indizes die oben genannten Bedeutungen aufweisen.

Wie oben beschrieben, ist es erfindungswesentlich, dass zwei benachbarte Gruppen X für CR stehen und die jeweiligen Reste R zusammen mit den C-Atomen, an die sie gebunden sind, einen Ring der oben genannten Formel (5) oder (6) aufspannen.

Bevorzugte Positionen für benachbarte Gruppen X, welche für CR stehen, wobei die jeweiligen Reste R zusammen mit den C-Atomen, an die sie gebunden sind, einen Ring der oben genannten Formel (5) oder (6) aufspannen, sind in den folgenden Formeln (2-1) bis (2-5), (3-1) bis (3-3) und (4-1) bis (4-4) abgebildet, 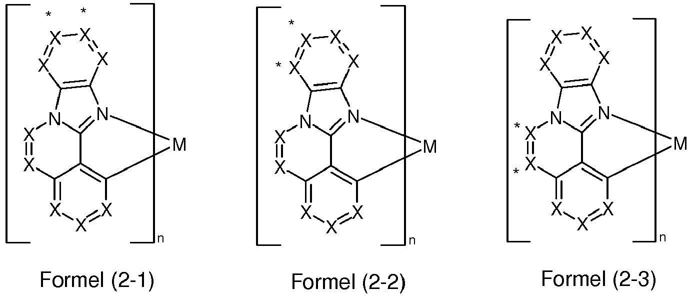 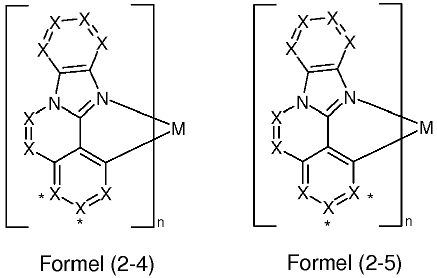 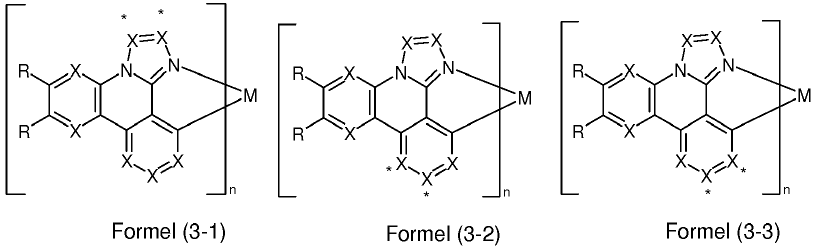 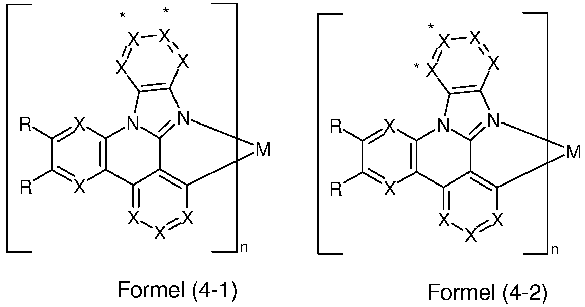 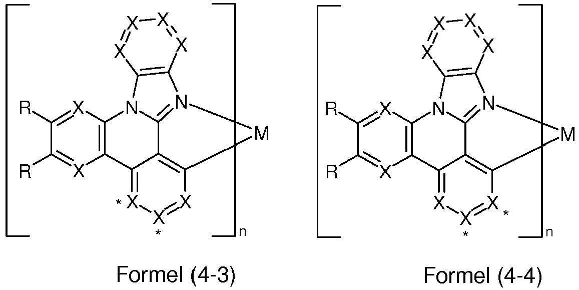 wobei die verwendeten Symbole und Indizes die oben genannten Bedeutungen aufweisen und * jeweils die Positionen kennzeichnet, die für CR stehen, wobei die jeweiligen Reste R zusammen mit den C-Atomen, an die sie gebunden sind, einen Ring der oben genannten Formel (5) oder (6) aufspannen.

Im Folgenden werden bevorzugte Ausführungsformen der Gruppen gemäß den Formeln (5) und (6) ausgeführt.

Wesentlich bei den Gruppen der Formeln (5) und (6) ist, dass diese keine aziden benzylischen Protonen aufweisen. Unter benzylischen Protonen werden Protonen verstanden, die an ein Kohlenstoffatom binden, welches direkt an den heteroaromatischen Liganden gebunden sind. Die Abwesenheit von aziden benzylischen Protonen wird in Formel (5) dadurch erreicht, dass A¹ und A³, wenn diese für C(R³)₂ stehen, so definiert sind, dass R³ ungleich Wasserstoff ist. Die Abwesenheit von aziden benzylischen Protonen ist in Formel (6) automatisch dadurch erreicht, dass es sich dabei um eine bicyclische Struktur handelt. Aufgrund der starren räumlichen Anordnung ist R¹, wenn es für H steht, deutlich weniger azide als benzylische Protonen, da das korrespondierende Anion der bicyclischen Struktur nicht mesomeriestabilisiert ist. Auch wenn R¹ in Formel (6) für H steht, handelt es sich dabei um ein nicht-azides Proton im Sinne der vorliegenden Anmeldung.

In einer bevorzugten Ausführungsform der Struktur gemäß Formel (5) steht maximal eine der Gruppen A¹, A² und A³ für ein Heteroatom, insbesondere für O oder NR³, und die anderen beiden Gruppen stehen für C(R³)₂ bzw. C(R¹)₂ oder A¹ und A³ stehen gleich oder verschieden bei jedem Auftreten für O oder NR³ und A² steht für C(R¹)₂. In einer besonders bevorzugten Ausführungsform der Erfindung stehen A¹ und A³ gleich oder verschieden bei jedem Auftreten für C(R³)₂ und A² steht für C(R¹)₂ und besonders bevorzugt für C(R³)₂. Bevorzugte Ausführungsformen der Formel (5) sind somit die Strukturen der Formel (5-A), (5-B), (5-C) und (5-D), und eine besonders bevorzugte Ausführungsform der Formel (5-A) ist die Struktur der Formel (5-E), 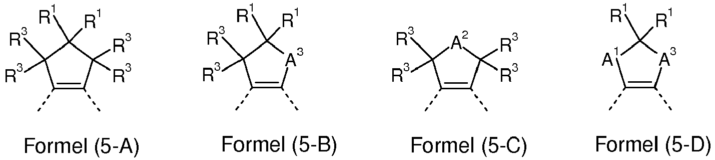 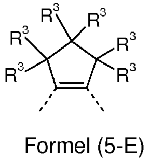 wobei R¹ und R³ die oben genannten Bedeutungen aufweisen und A¹, A² und A³ gleich oder verschieden bei jedem Auftreten für O oder NR³ steht.

In einer bevorzugten Ausführungsform der Struktur gemäß Formel (6) stehen die Reste R¹, die an den Brückenkopf gebunden sind, für H, D, F oder CH₃. Weiterhin bevorzugt steht A² für C(R¹)₂ oder O, und besonders bevorzugt für C(R³)₂. Bevorzugte Ausführungsformen der Formel (6) sind somit eine Strukturen der Formel (6-A) und (6-B), und eine besonders bevorzugte Ausführungsform der Formel (6-A) ist eine Struktur der Formel (6-C), 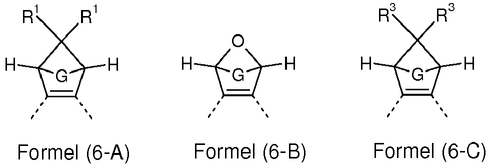 wobei die verwendeten Symbole die oben genannten Bedeutungen aufweisen.

Weiterhin bevorzugt steht die Gruppe G in den Formeln (6), (6-A), (6-B) und (6-C) für eine Ethylengruppe, welche mit einem oder mehreren Resten R² substituiert sein kann, wobei R² bevorzugt gleich oder verschieden bei jedem Auftreten für H oder eine Alkylgruppe mit 1 bis 4 C-Atomen steht, oder eine ortho-Arylengruppe mit 6 bis 10 C-Atomen, welche mit einem oder mehreren Resten R² substituiert sein kann, bevorzugt aber unsubstituiert ist, insbesondere eine ortho-Phenylengruppe, welche mit eniem oder mehreren Resten R² substituiert sein kann, bevorzugt aber unsubstituiert ist.

In einer weiteren bevorzugten Ausführungsform der Erfindung steht R³ in den Gruppen der Formel (5) und (6) und in den bevorzugten Ausführungsformen gleich oder verschieden bei jedem Auftreten für F, eine geradkettige Alkylgruppe mit 1 bis 10 C-Atomen oder eine verzweigte oder cyclische Alkylgruppe mit 3 bis 20 C-Atomen, wobei jeweils eine oder mehrere nicht benachbarte CH₂-Gruppen durch R²C=CR² ersetzt sein können und ein oder mehrere H-Atome durch D oder F ersetzt sein können, oder ein aromatisches oder heteroaromatisches Ringsystem mit 5 bis 14 aromatischen Ringatomen, das jeweils durch einen oder mehrere Reste R² substituiert sein kann; dabei können zwei Reste R³, welche an dasselbe Kohlenstoffatom gebunden sind, miteinander ein aliphatisches oder aromatisches Ringsystem bilden und so ein Spirosystem aufspannen; weiterhin kann R³ mit einem benachbarten Rest R oder R¹ ein aliphatisches Ringsystem bilden.

In einer besonders bevorzugten Ausführungsform der Erfindung steht R³ in den Gruppen der Formeln (5) und (6) und in den bevorzugten Ausführungsformen gleich oder verschieden bei jedem Auftreten für F, eine geradkettige Alkylgruppe mit 1 bis 3 C-Atomen, insbesondere Methyl, oder ein aromatisches oder heteroaromatisches Ringsystem mit 5 bis 12 aromatischen Ringatomen, das jeweils durch einen oder mehrere Reste R² substituiert sein kann, bevorzugt aber unsubstituiert ist; dabei können zwei Reste R³, welche an dasselbe Kohlenstoffatom gebunden sind, miteinander ein aliphatisches oder aromatisches Ringsystem bilden und so ein Spirosystem aufspannen; weiterhin kann R³ mit einem benachbarten Rest R oder R¹ ein aliphatisches Ringsystem bilden.

Beispiele für besonders geeignete Gruppen der Formel (5) sind die im Folgenden aufgeführten Gruppen (5-1) bis (5-69):

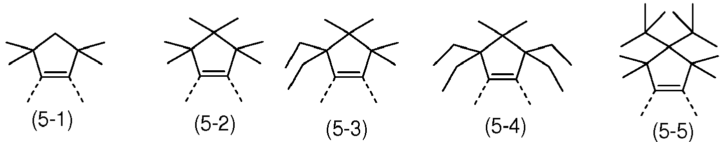

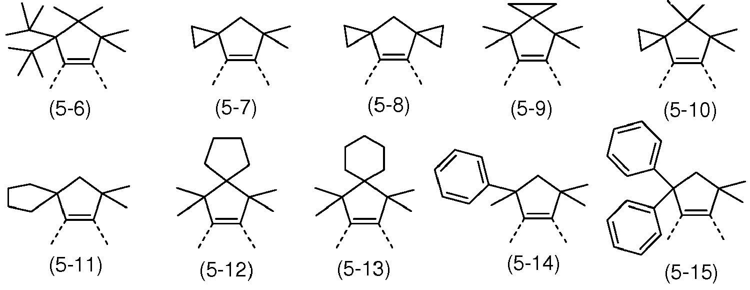

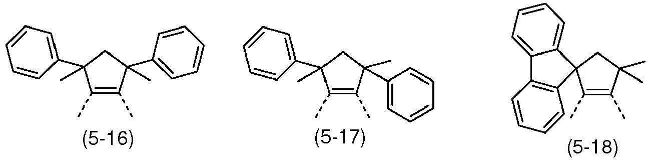

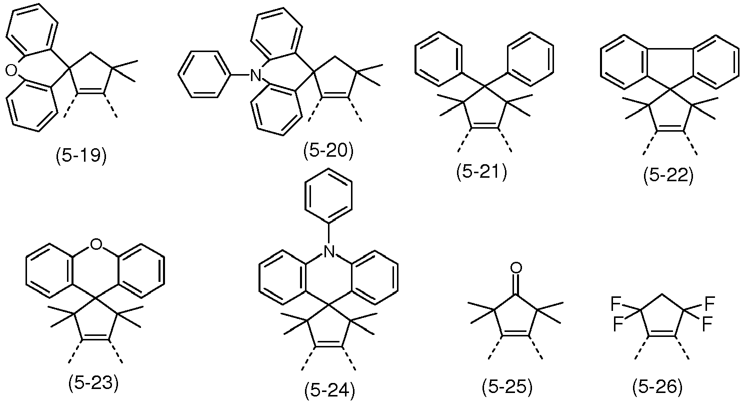

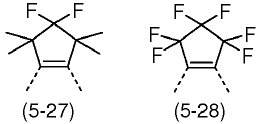

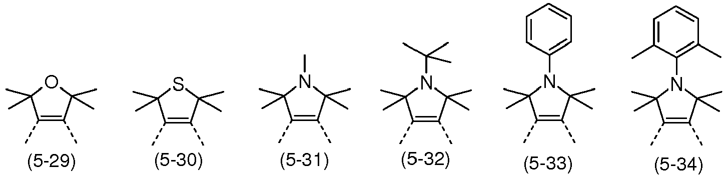

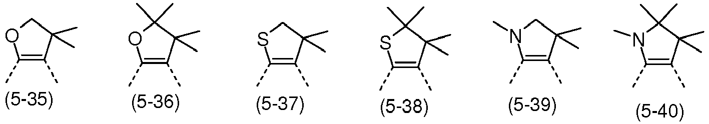

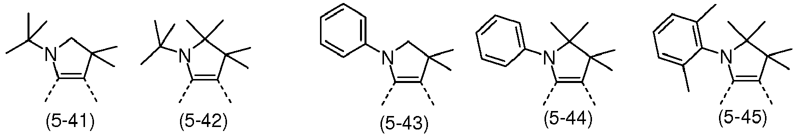

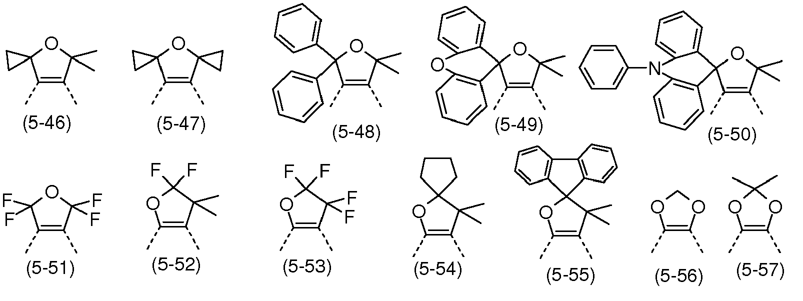

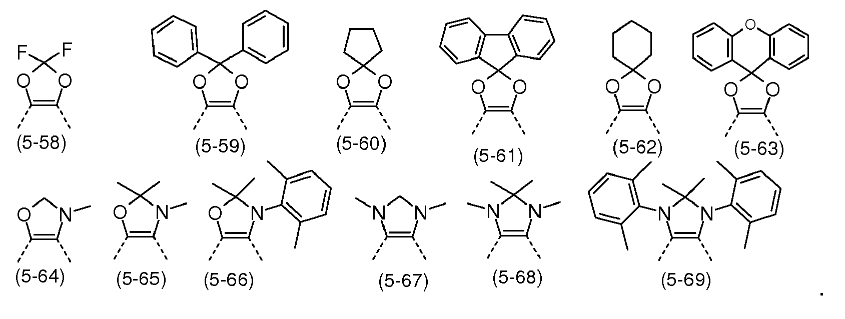

Beispiele für besonders geeignete Gruppen der Formel (6) sind die im Folgenden aufgeführten Gruppen (6-1) bis (6-22):

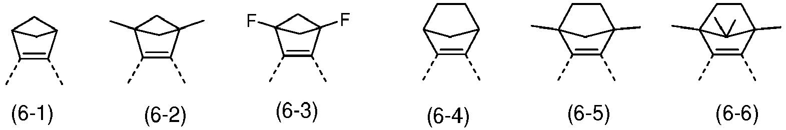

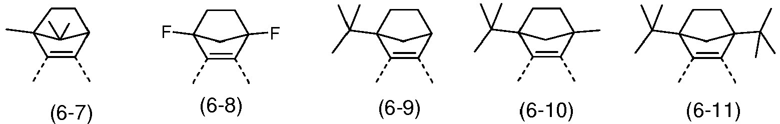

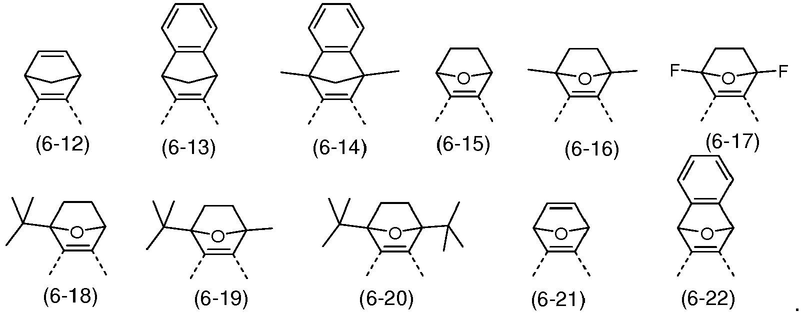

Wenn eine oder mehrere Gruppen X in den Teilstrukturen der Formel (2), (3) oder (4) für Stickstoff stehen, ist weiterhin in den erfindungsgemäßen Verbindungen bevorzugt, wenn benachbart zu diesem Stickstoffatom eine Gruppe R als Substituent gebunden ist, welche ungleich Wasserstoff oder Deuterium ist. Dabei ist dieses R bevorzugt eine Gruppe, ausgewählt aus CF₃, OCF₃, Alkyl- oder Alkoxygruppen mit 1 bis 10 C-Atomen, insbesondere verzweigten oder cyclischen Alkyl- oder Alkoxygruppen mit 3 bis 10 C-Atomen, aromatischen bzw. heteroaromatischen Ringsystemen oder Aralkyl- bzw. Heteroaralkylgruppen. Es handelt sich bei diesen Gruppen um sterisch anspruchsvolle Gruppen. Weiterhin bevorzugt kann dieser Rest R auch mit einem benachbarten Rest R einen Cyclus der Formel (5) oder (6) bilden.

Wenn der Rest R, der einem Stickstoffatom benachbart ist, für eine Alkylgruppe steht, dann weist diese Alkylgruppe bevorzugt 4 bis 10 C-Atome auf. Bevorzugt handelt es sich weiterhin um eine sekundäre oder tertiäre Alkylgruppe, bei der das sekundäre oder tertiäre C-Atom entweder direkt an den Liganden gebunden ist oder über eine CH₂-Gruppe an den Liganden gebunden ist. Besonders bevorzugt ist diese Alkylgruppe ausgewählt aus den Strukturen der folgenden Formeln (R-1) bis (R-33), wobei jeweils auch die Anknüpfung dieser Gruppen an den Liganden mit eingezeichnet ist: 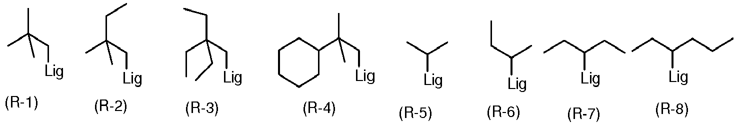 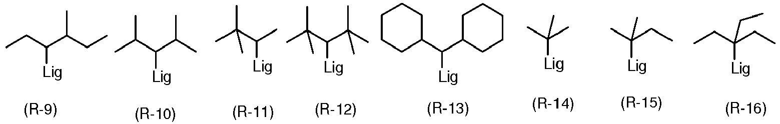 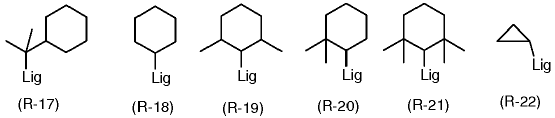 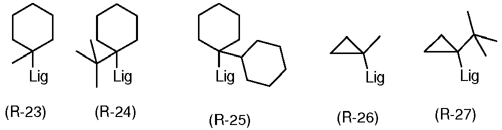 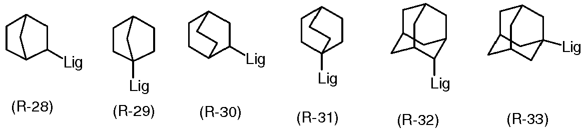 wobei Lig die Anknüpfung der Alkylgruppe an den Liganden kennzeichnet.

Wenn der Rest R, der einem Stickstoffatom benachbart ist, für eine Alkoxygruppe steht, dann weist diese Alkoxygruppe bevorzugt 3 bis 10 C-Atome auf. Bevorzugt ist diese Alkoxygruppe ausgewählt aus den Strukturen der folgenden Formeln (R-34) bis (R-47), wobei jeweils auch die Anknüpfung dieser Gruppen an den Liganden mit eingezeichnet ist: 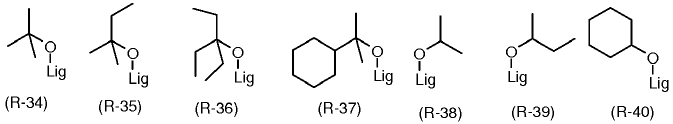 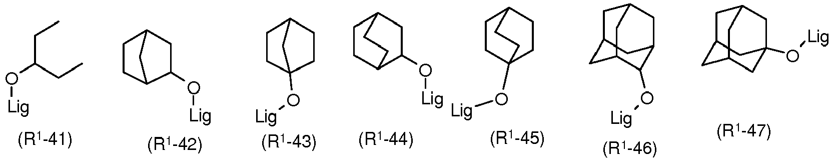 wobei Lig die Anknüpfung der Alkylgruppe an den Liganden kennzeichnet.

Wenn der Rest R, der einem Stickstoffatom benachbart ist, für eine Dialkylaminogruppe steht, dann weist jede dieser Alkylgruppen bevorzugt 1 bis 8 C-Atome auf, besonders bevorzugt 1 bis 6 C-Atome. Beispiele für geeignete Alkylgruppen sind Methyl, Ethyl oder die oben als Gruppen (R-1) bis (R-33) aufgeführten Strukturen. Besonders bevorzugt ist die Dialkylaminogruppe ausgewählt aus den Strukturen der folgenden Formeln (R-48) bis (R-55), wobei jeweils auch die Anknüpfung dieser Gruppen an den Liganden mit eingezeichnet ist: 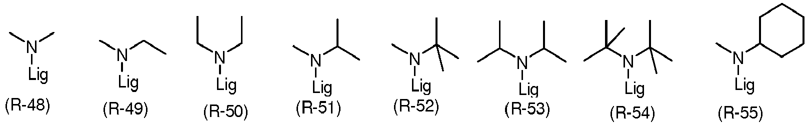 wobei Lig die Anknüpfung der Alkylgruppe an den Liganden kennzeichnet.

Wenn der Rest R, der einem Stickstoffatom benachbart ist, für eine Aralkylgruppe steht, dann ist diese Aralkylgruppe bevorzugt ausgewählt aus den Strukturen der folgenden Formeln (R-56) bis (R-69), wobei jeweils auch die Anknüpfung dieser Gruppen an den Liganden eingezeichnet ist: 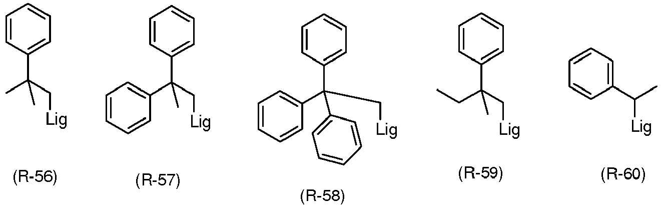 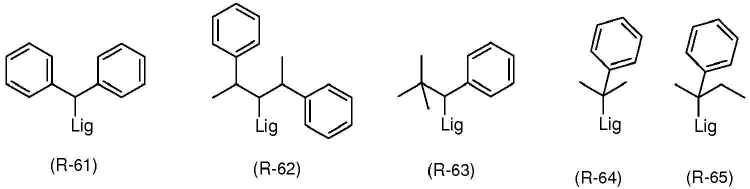 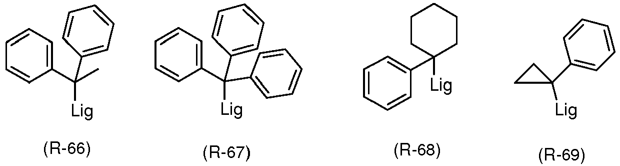 wobei Lig die Anknüpfung der Aralkylgruppe an den Liganden kennzeichnet und die Phenylgruppen jeweils durch einen oder mehrere Reste R¹ substituiert sein können.

Wenn der Rest R, der einem Stickstoffatom benachbart ist, für ein aromatisches bzw. heteroaromatisches Ringsystem steht, dann weist dieses aromatische bzw. heteroaromatische Ringsystem bevorzugt 5 bis 30 aromatische Ringatome auf, besonders bevorzugt 5 bis 24 aromatische Ringatome. Weiterhin enthält dieses aromatische bzw. heteroaromatische Ringsystem bevorzugt keine Aryl- bzw. Heteroarylgruppen, in denen mehr als zwei aromatische Sechsringe direkt aneinander kondensiert sind. Besonders bevorzugt enthält das aromatische bzw. heteroaromatische Ringsystem überhaupt keine kondensierten Aryl- bzw. Heteroarylgruppen, und ganz besonders bevorzugt enthält es nur Phenylgruppen. Dabei ist das aromatische Ringsystem bevorzugt ausgewählt aus den Strukturen der folgenden Formeln (R-70) bis (R-84), wobei jeweils auch die Anknüpfung dieser Gruppen an den Liganden eingezeichnet ist: 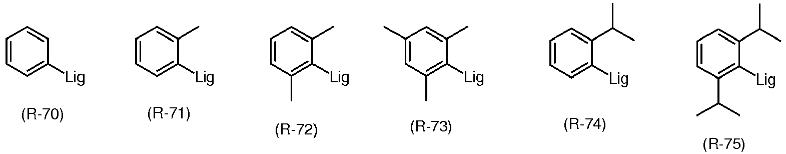 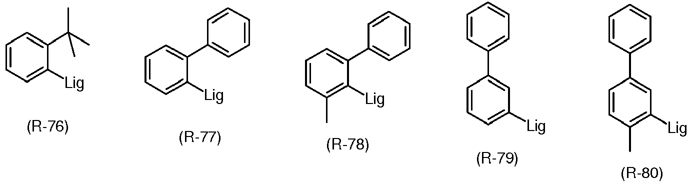 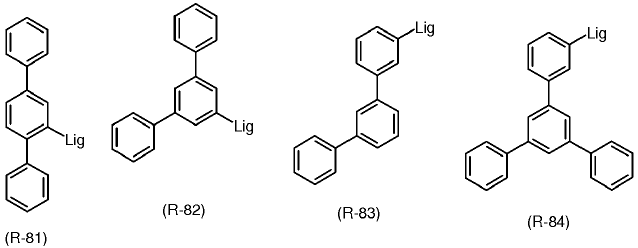 wobei Lig die Anknüpfung des aromatischen oder heteroaromatischen Ringsystems an den Liganden kennzeichnet und die Phenylgruppen jeweils durch einen oder mehrere Reste R¹ substituiert sein können.

Weiterhin ist das heteroaromatische Ringsystem bevorzugt ausgewählt aus den Strukturen der folgenden Formeln (R-85) bis (R-112), wobei jeweils auch die Anknüpfung dieser Gruppen an den Liganden eingezeichnet ist: 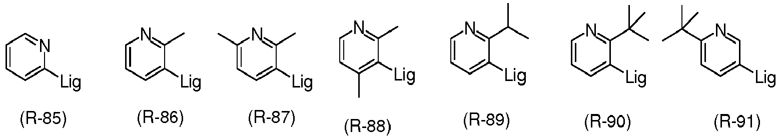 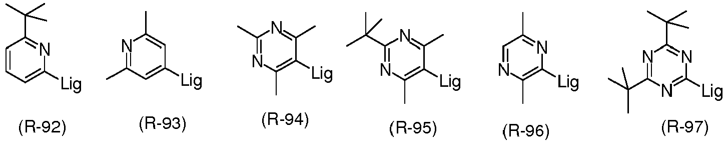 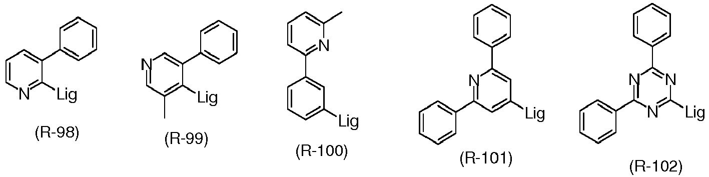 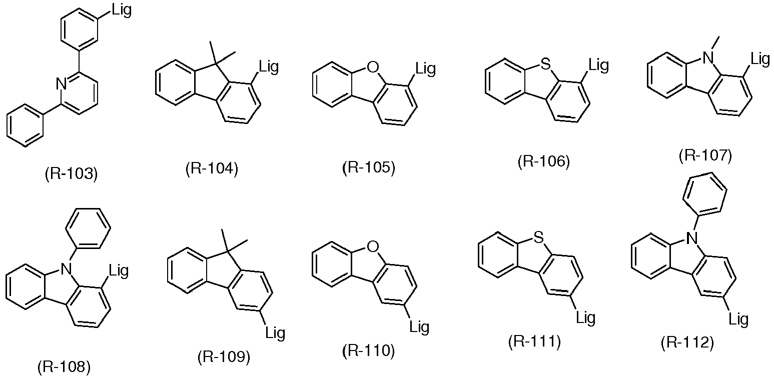 wobei Lig die Anknüpfung des aromatischen oder heteroaromatischen Ringsystems an den Liganden kennzeichnet und die aromatischen und heteroaromatischen Gruppen jeweils durch einen oder mehrere Reste R¹ substituiert sein können.

Wenn in der Teilstruktur der Formel (2), (3) oder (4) noch weitere bzw. andere Reste R gebunden sind, so sind diese Reste R bei jedem Auftreten gleich oder verschieden bevorzugt ausgewählt aus der Gruppe bestehend aus H, D, F, Br, I, N(R¹)₂, CN, Si(R¹)₃, B(OR¹)₂, C(=O)R¹, einer geradkettigen Alkylgruppe mit 1 bis 10 C-Atomen oder einer Alkenylgruppe mit 2 bis 10 C-Atomen oder einer verzweigten oder cyclischen Alkylgruppe mit 3 bis 10 C-Atomen, die jeweils mit einem oder mehreren Resten R¹ substituiert sein kann, wobei ein oder mehrere H-Atome durch D oder F ersetzt sein können, oder einem aromatischen oder heteroaromatischen Ringsystem mit 5 bis 30 aromatischen Ringatomen, das jeweils durch einen oder mehrere Reste R¹ substituiert sein kann; dabei können zwei benachbarte Rest R oder R mit R¹ auch miteinander ein mono- oder polycyclisches, aliphatisches oder aromatisches Ringsystem bilden. Besonders bevorzugt sind diese Reste R bei jedem Auftreten gleich oder verschieden ausgewählt aus der Gruppe bestehend aus H, D, F, N(R¹)₂, einer geradkettigen Alkylgruppe mit 1 bis 6 C-Atomen oder einer verzweigten oder cyclischen Alkylgruppe mit 3 bis 10 C-Atomen, wobei ein oder mehrere H-Atome durch D oder F ersetzt sein können, oder einem aromatischen oder heteroaromatischen Ringsystem mit 5 bis 24 aromatischen Ringatomen, das jeweils durch einen oder mehrere Reste R¹ substituiert sein kann; dabei können zwei benachbarte Reste R oder R mit R¹ auch miteinander ein mono- oder polycyclisches, aliphatisches oder aromatisches Ringsystem bilden.

Weiterhin ist es möglich, dass der Substituent R, der in der ortho-Position zur Metallkoordination gebunden ist, eine Gruppe darstellt, die ebenfalls an das Metall M koordiniert bzw. bindet. Bevorzugte koordinierende Gruppen R sind Aryl- bzw. Heteroarylgruppen, beispielsweise Phenyl oder Pyridyl, Aryl- oder Alkylcyanide, Aryl- oder Alkylisocyanide, Amine oder Amide, Alkohole oder Alkoholate, Thioalkohole oder Thioalkoholate, Phosphine, Phosphite, Carbonylfunktionen, Carboxylate, Carbamide oder Aryl- oder Alkylacetylide. Beispiele für Teilstrukturen ML der Formel (2) sind die Strukturen der folgenden Formeln (7) bis (12): 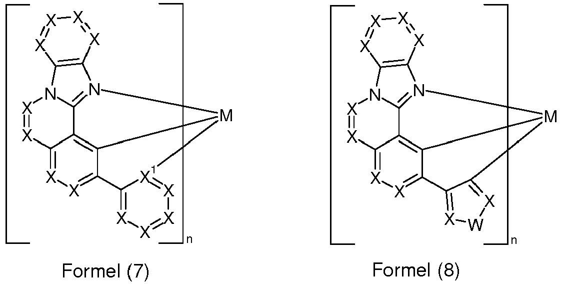 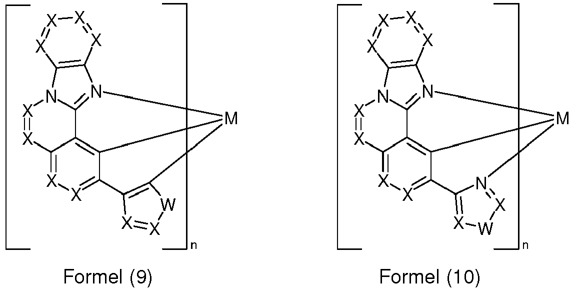 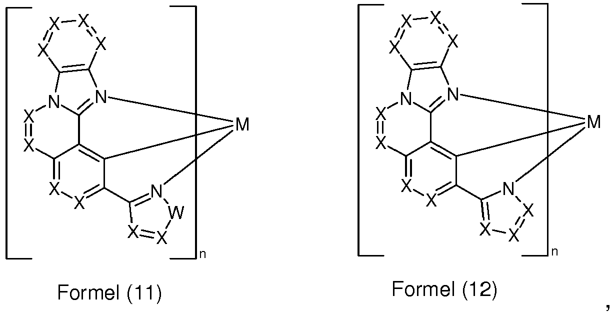 wobei die verwendeten Symbole und Indizes die gleichen Bedeutungen aufweisen, wie oben beschrieben, X¹ gleich oder verschieden bei jedem Auftreten für C oder N steht und W gleich oder verschieden bei jedem Auftreten für S, O oder NR¹ steht.

Die Formeln (7) bis (12) zeigen nur exemplarisch, wie der Substituent R zusätzlich an das Metall koordinieren kann. Ganz analog sind ohne weiteres erfinderisches Zutun auch andere an das Metall koordinierende Gruppen R zugänglich, beispielsweise auch Carbene. Ebenso sind ganz analog entsprechende Teilstrukturen ML möglich, die auf Formel (3) oder Formel (4) basieren.

Wie oben beschrieben, kann auch statt einem der Reste R eine verbrückende Einheit vorhanden sein, die diesen Liganden L mit einem oder mehreren weiteren Liganden L bzw. L' verknüpft. In einer bevorzugten Ausführungsform der Erfindung ist statt einem der Reste R, insbesondere statt der Reste R, die in ortho- oder meta-Position zum koordinierenden Atom stehen, eine verbrückende Einheit vorhanden, so dass die Liganden dreizähnigen oder mehrzähnigen oder polypodalen Charakter aufweisen. Es können auch zwei solcher verbrückenden Einheiten vorhanden sein. Dies führt zur Bildung makrocyclischer Liganden bzw. zur Bildung von Kryptaten.

Bevorzugte Strukturen mit mehrzähnigen Liganden bzw. mit polydentaten Liganden sind die Metallkomplexe der folgenden Formeln (13) bis (18), 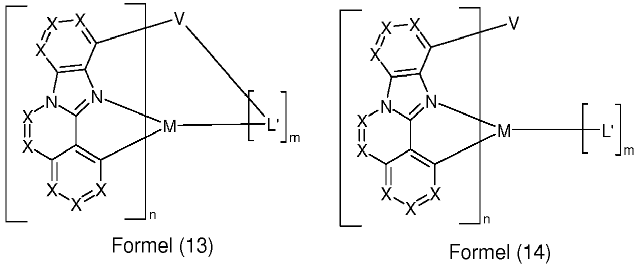 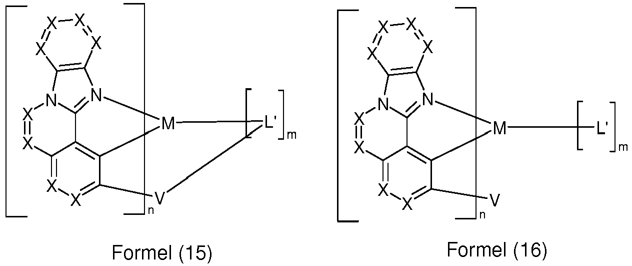 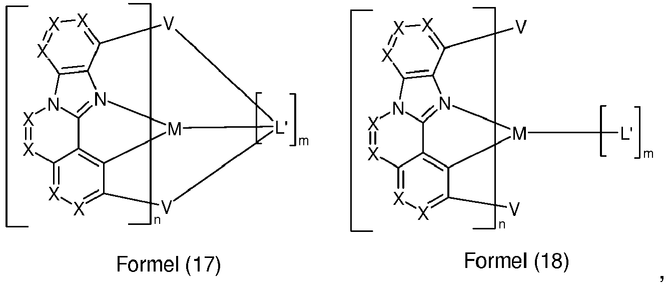 wobei die verwendeten Symbole und Indizes die oben genannten Bedeutungen aufweisen.

Strukturen mit Teileinheiten der Formel (3) bzw. Formel (4) sind ganz analog zugänglich.

Ebenso können die Liganden über die cyclische Gruppe der Formel (5) oder (6) miteinander verbrückt sein. Dies ist schematisch für einen Liganden der Formel (2) in der folgenden Formel (19) dargestellt: 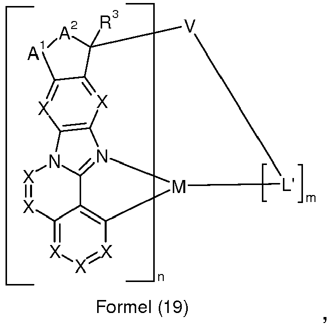 wobei die verwendeten Symbole und Indizes die oben genannten Bedeutungen aufweisen und V bevorzugt für CR¹, eine Cyclopropylgruppe, die mit einem oder mehreren Resten R¹ substituiert sein kann, oder eine Gruppe der Formel R¹-C(CH₂)₂ steht.

Analog zu Formel (19) kann die Verknüpfung mit Teileinheiten der Formel (3) oder Formel (4) erfolgen.

Dabei stellt V bevorzugt eine Einfachbindung oder eine verbrückende Einheit dar, enthaltend 1 bis 80 Atome aus der dritten, vierten, fünften und/oder sechsten Hauptgruppe (Gruppe 13, 14, 15 oder 16 gemäß IUPAC) oder einen 3- bis 6-gliedrigen Homo- oder Heterocyclus, die die Teilliganden L miteinander oder L mit L' miteinander kovalent verbindet. Dabei kann die verbrückende Einheit V auch unsymmetrisch aufgebaut sein, d. h. die Verknüpfung von V zu L bzw. L' muss nicht identisch sein. Die verbrückende Einheit V kann neutral, einfach, zweifach oder dreifach negativ oder einfach, zweifach oder dreifach positiv geladen sein. Bevorzugt ist V neutral oder einfach negativ oder einfach positiv geladen, besonders bevorzugt neutral. Dabei wird die Ladung von V bevorzugt so gewählt, dass insgesamt ein neutraler Komplex entsteht. Dabei gelten für die Liganden die oben für die Teilstruktur MLₙ genannten Bevorzugungen und n ist bevorzugt mindestens 2.

Die genaue Struktur und chemische Zusammensetzung der Gruppe V hat keinen wesentlichen Einfluss auf die elektronischen Eigenschaften des Komplexes, da die Aufgabe dieser Gruppe im Wesentlichen darin liegt, durch die Verbrückung von L miteinander bzw. mit L' die chemische und thermische Stabilität der Komplexe zu erhöhen.

Wenn V eine trivalente Gruppe ist, also drei Liganden L miteinander bzw. zwei Liganden L mit L' oder einen Liganden L mit zwei Liganden L' verbrückt, ist V bevorzugt gleich oder verschieden bei jedem Auftreten gewählt aus der Gruppe bestehend aus B, B(R¹)⁻, B(C(R¹)₂)₃, (R¹)B(C(R¹)₂)₃⁻, B(O)₃, (R¹)B(O)₃⁻, B(C(R¹)₂C(R¹)₂)₃, (R¹)B(C(R¹)₂C(R¹)₂)₃⁻, B(C(R¹)₂O)₃, (R¹)B(C(R¹)₂O)₃⁻, B(OC(R¹)₂)₃, (R¹)B(OC(R¹)₂)₃⁻, C(R¹), CO⁻, CN(R¹)₂, (R¹)C(C(R¹)₂)₃, (R¹)C(O)₃, (R¹)C(C(R¹)₂C(R¹)₂)₃, (R¹)C(C(R¹)₂O)₃, (R¹)C(OC(R¹)₂)₃, (R¹)C(Si(R¹)₂)₃, (R¹)C(Si(R¹)₂C(R¹)₂)₃, (R¹)C(C(R¹)₂Si(R¹)₂)₃, (R¹)C(Si(R¹)₂Si(R¹)₂)₃, Si(R¹), (R¹)Si(C(R¹)₂)₃, (R¹)Si(O)₃, (R¹)Si(C(R¹)₂C(R¹)₂)₃, (R¹)Si(OC(R¹)₂)₃, (R¹)Si(C(R¹)₂O)₃, (R¹)Si(Si(R¹)₂)₃, (R¹)Si(Si(R¹)₂C(R¹)₂)₃, (R¹)Si(C(R¹)₂Si(R¹)₂)₃, (R¹)Si(Si(R¹)₂Si(R¹)₂)₃, N, NO, N(R¹)⁺, N(C(R¹)₂)₃, (R¹)N(C(R¹)₂)₃⁺, N(C=O)₃, N(C(R¹)₂C(R¹)₂)₃, (R¹)N(C(R¹)₂C(R¹)₂)⁺, P, P(R¹)⁺, PO, PS, P(O)₃, PO(O)₃, P(OC(R¹)₂)₃, PO(OC(R¹)₂)₃, P(C(R¹)₂)₃, P(R¹)(C(R¹)₂)₃⁺, PO(C(R¹)₂)₃, P(C(R¹)₂C(R¹)₂)₃, P(R¹) (C(R¹)₂C(R¹)₂)₃⁺, PO(C(R¹)₂C(R¹)₂)₃, S⁺, S(C(R¹)₂)₃⁺, S(C(R¹)₂C(R¹)₂)₃⁺,
oder eine Einheit gemäß Formel (20), (21), (22), (23) oder (24), 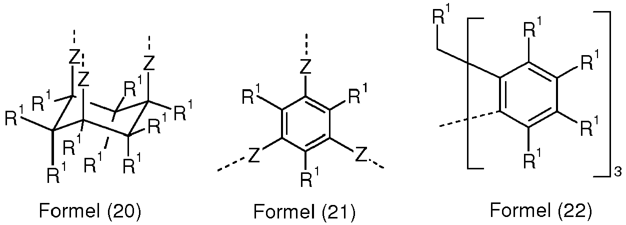 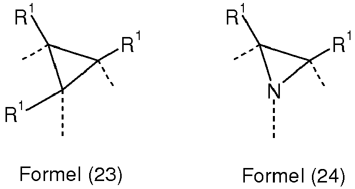 wobei die gestrichelten Bindungen jeweils die Bindung zu den Teilliganden L bzw. L' andeuten und Z gleich oder verschieden bei jedem Auftreten ausgewählt ist aus der Gruppe bestehend aus einer Einfachbindung, O, S, S(=O), S(=O)2, NR¹, PR¹, P(=O)R¹, C(R¹)₂, C(=O), C(=NR¹), C(=C(R¹)₂), Si(R¹)₂ oder BR¹. Die weiteren verwendeten Symbole haben die oben genannten Bedeutungen.

Wenn V für eine Gruppe CR₂ steht, so können die beiden Reste R auch miteinander verknüpft sein, so dass auch Strukturen wie zum Beispiele 9,9-Fluoren geeignete Gruppen V sind.

Wenn V eine bivalente Gruppe ist, also zwei Liganden L miteinander bzw. einen Liganden L mit L' verbrückt, ist V bevorzugt gleich oder verschieden bei jedem Auftreten gewählt aus der Gruppe bestehend aus BR¹, B(R¹)₂⁻, C(R¹)₂, C(=O), Si(R¹)₂, NR¹, PR¹, P(R¹)₂⁺, P(=O)(R¹), P(=S)(R¹), O, S, Se, oder eine Einheit gemäß Formel (25) bis (34), 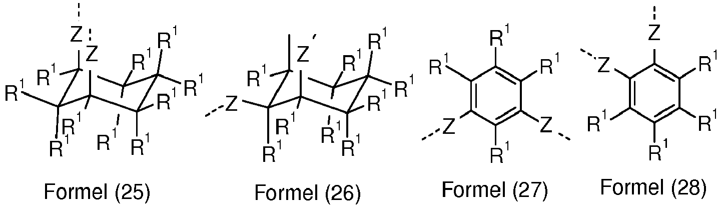 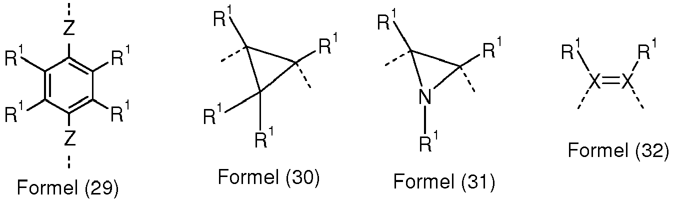 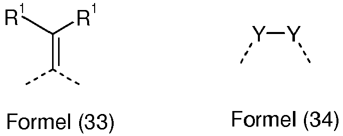 wobei die gestrichelten Bindungen jeweils die Bindung zu den Teilliganden L bzw. L' andeuten, Y bei jedem Auftreten gleich oder verschieden für C(R¹)₂, N(R¹), O oder S steht und die weiteren verwendeten Symbole jeweils die oben aufgeführten Bedeutungen haben.

Im Folgenden werden bevorzugte Liganden L' beschrieben, wie sie in Formel (1) vorkommen. Entsprechend können auch die Ligandengruppen L' gewählt sein, wenn diese über eine verbrückende Einheit V an L gebunden sind, wie in Formeln (13), (15) und (17) angedeutet.

Die Liganden L' sind bevorzugt neutrale, monoanionische, dianionische oder trianionische Liganden, besonders bevorzugt neutrale oder monoanionische Liganden. Sie können monodentat, bidentat, tridentat oder tetradentat sein und sind bevorzugt bidentat, weisen also bevorzugt zwei Koordinationsstellen auf. Wie oben beschrieben, können die Liganden L' auch über eine verbrückende Gruppe V an L gebunden sein.

Bevorzugte neutrale, monodentate Liganden L' sind ausgewählt aus der Gruppe bestehend aus Kohlenmonoxid, Stickstoffmonoxid, Alkylcyaniden, wie z. B. Acetonitril, Arylcyaniden, wie z. B. Benzonitril, Alkylisocyaniden, wie z. B. Methylisonitril, Arylisocyaniden, wie z. B. Benzoisonitril, Aminen, wie z. B. Trimethylamin, Triethylamin, Morpholin, Phosphinen, insbesondere Halogenphosphine, Trialkylphosphine, Triarylphosphine oder Alkylarylphosphine, wie z. B. Trifluorphosphin, Trimethylphosphin, Tricyclohexylphosphin, Tri-*tert*-butylphosphin, Triphenylphosphin, Tris(pentafluorphenyl)phosphin, Dimethylphenylphosphin, Methyldiphenylphosphin, Bis(tert-butyl)phenylphosphin, Phosphiten, wie z. B. Trimethylphosphit, Triethylphosphit, Arsinen, wie z. B. Trifluorarsin, Trimethylarsin, Tricyclohexylarsin, Tri-*tert*-butylarsin, Triphenylarsin, Tris(pentafluorphenyl)arsin, Stibinen, wie z. B. Trifluorstibin, Trimethylstibin, Tricyclohexylstibin, Tri-*tert-*butylstibin, Triphenylstibin, Tris(pentafluorphenyl)stibin, stickstoffhaltigen Heterocyclen, wie z. B. Pyridin, Pyridazin, Pyrazin, Pyrimidin, Triazin, und Carbenen, insbesondere Arduengo-Carbenen.

Bevorzugte monoanionische, monodentate Liganden L' sind ausgewählt aus Hydrid, Deuterid, den Halogeniden F⁻, Cl⁻, Br⁻ und I⁻, Alkylacetyliden, wie z. B. Methyl-C≡C⁻, tert-Butyl-C≡C⁻, Arylacetyliden, wie z. B. Phenyl-C≡C⁻, Cyanid, Cyanat, Isocyanat, Thiocyanat, Isothiocyanat, aliphatischen oder aromatischen Alkoholaten, wie z. B. Methanolat, Ethanolat, Propanolat, *iso*-Propanolat, *tert*-Butylat, Phenolat, aliphatischen oder aromatischen Thioalkoholaten, wie z. B. Methanthiolat, Ethanthiolat, Propanthiolat, *iso*-Propanthiolat, *tert*-Thiobutylat, Thiophenolat, Amiden, wie z. B. Dimethylamid, Diethylamid, Di-*iso-*propylamid, Morpholid, Carboxylaten, wie z. B. Acetat, Trifluoracetat, Propionat, Benzoat, Arylgruppen, wie z. B. Phenyl, Naphthyl, und anionischen, stickstoffhaltigen Heterocyclen, wie Pyrrolid, Imidazolid, Pyrazolid. Dabei sind die Alkylgruppen in diesen Gruppen bevorzugt C₁-C₂₀-Alkylgruppen, besonders bevorzugt C₁-C₁₀-Alkylgruppen, ganz besonders bevorzugt C₁-C₄-Alkylgruppen. Unter einer Arylgruppe werden auch Heteroarylgruppen verstanden. Diese Gruppen sind wie oben definiert.

Bevorzugte di- bzw. trianionische Liganden sind O²⁻, S²⁻, Carbide, welche zu einer Koordination der Form R-C≡M führen, und Nitrene, welche zu einer Koordination der Form R-N=M führen, wobei R allgemein für einen Substituenten steht, oder N³⁻.

Bevorzugte neutrale oder mono- oder dianionische, bidentate oder höherdentate Liganden L' sind ausgewählt aus Diaminen, wie z. B. Ethylendiamin, N,N,N',N'-Tetramethylethylendiamin, Propylendiamin, N,N,N',N'-Tetramethylpropylendiamin, cis- oder trans-Diaminocyclohexan, cis- oder trans-N,N,N',N'-Tetramethyldiaminocyclohexan, Iminen, wie z. B. 2-[1-(Phenylimino)ethyl]pyridin, 2-[1-(2-Methylphenylimino)ethyl]pyridin, 2-[1-(2,6-Di-*iso*-propylphenylimino)ethyl]pyridin, 2-[1-(Methylimino)ethyl]pyridin, 2-[1-(ethylimino)ethyl]pyridin, 2-[1-(*Iso*-Propylimino)ethyl]pyridin, 2-[1-(*Tert-*Butylimino)ethyl]pyridin, Diiminen, wie z. B. 1,2-Bis(methylimino)ethan, 1,2-Bis(ethylimino)ethan, 1,2-Bis(*iso*-propylimino)ethan, 1,2-Bis(*tert*-butyl-imino)ethan, 2,3-Bis(methylimino)butan, 2,3-Bis(ethylimino)butan, 2,3-Bis-(*iso*-propylimino)butan, 2,3-Bis(*tert*-butylimino)butan, 1,2-Bis(phenylimino)-ethan, 1,2-Bis(2-methylphenylimino)ethan, 1,2-Bis(2,6-di-*iso*-propylphenyl-imino)ethan, 1,2-Bis(2,6-di-*tert*-butylphenylimino)ethan, 2,3-Bis(phenyl-imino)butan, 2,3-Bis(2-methylphenylimino)butan, 2,3-Bis(2,6-di-*iso*-propyl-phenylimino)butan, 2,3-Bis(2,6-di-*tert*-butylphenylimino)butan, Heterocyclen enthaltend zwei Stickstoffatome, wie z. B. 2,2'-Bipyridin, o-Phenanthrolin, Diphosphinen, wie z. B. Bis(diphenylphosphino)methan, Bis(diphenylphosphino)ethan, Bis(diphenylphosphino)propan, Bis(diphenylphosphino)butan, Bis(dimethylphosphino)methan, Bis(dimethylphosphino)-ethan, Bis(dimethylphosphino)propan, Bis(diethylphosphino)methan, Bis-(diethylphosphino)ethan, Bis(diethylphosphino)propan, Bis(di-*tert*-butylphosphino)methan, Bis(di-*tert*-butylphosphino)ethan, Bis(*tert*-butylphosphino)propan, 1,3-Diketonaten abgeleitet von 1,3-Diketonen, wie z. B. Acetylaceton, Benzoylaceton, 1,5-Diphenylacetylaceton, Dibenzoylmethan, Bis(1,1,1-trifluoracetyl)methan, 3-Ketonaten abgeleitet von 3-Ketoestern, wie z. B. Acetessigsäureethylester, Carboxylate, abgeleitet von Aminocarbonsäuren, wie z. B. Pyridin-2-carbonsäure, Chinolin-2-carbonsäure, Glycin, N,N-Dimethylglycin, Alanin, N,N-Dimethylaminoalanin, Salicyliminaten abgeleitet von Salicyliminen, wie z. B. Methylsalicylimin, Ethylsalicylimin, Phenylsalicylimin, Dialkoholaten abgeleitet von Dialkoholen, wie z. B. Ethylenglykol, 1,3-Propylenglykol, Dithiolaten abgeleitet von Dithiolen, wie z. B. 1,2-Ethylendithiol, 1,3-Propylendithiol, Bis(pyrazolylboraten), Bis(imidazolyl)boraten, 3-(2-Pyridyl)-diazolen oder 3-(2-Pyridyl)-triazolen.

Bevorzugte tridentate Liganden sind Borate stickstoffhaltiger Heterocyclen, wie z. B. Tetrakis(1-imidazolyl)borat und Tetrakis(1-pyrazolyl)borat.

Bevorzugt sind weiterhin bidentate monoanionische, neutrale oder dianionische Liganden L', insbesondere monoanionische Liganden, welche mit dem Metall einen cyclometallierten Fünfring oder Sechsring mit mindestens einer Metall-Kohlenstoff-Bindung aufweisen, insbesondere einen cyclometallierten Fünfring. Dies sind insbesondere Liganden, wie sie allgemein im Gebiet der phosphoreszierenden Metallkomplexe für organische Elektrolumineszenzvorrichtungen verwendet werden, also Liganden vom Typ Phenylpyridin, Naphthylpyridin, Phenylchinolin, Phenylisochinolin, etc., welche jeweils durch einen oder mehrere Reste R substituiert sein können. Dem Fachmann auf dem Gebiet der phosphoreszierenden Elektrolumineszenzvorrichtungen ist eine Vielzahl derartiger Liganden bekannt, und er kann ohne erfinderisches Zutun weitere derartige Liganden als Ligand L' für Verbindungen gemäß Formel (1) auswählen. Generell eignet sich dafür besonders die Kombination aus zwei Gruppen, wie sie durch die folgenden Formeln (35) bis (66) dargestellt sind, wobei eine Gruppe bevorzugt über ein neutrales Stickstoffatom oder ein Carbenkohlenstoffatom bindet und die andere Gruppe bevorzugt über ein negativ geladenes Kohlenstoffatom oder ein negativ geladenes Stickstoffatom bindet. Der Ligand L' kann dann aus den Gruppen der Formeln (35) bis (66) gebildet werden, indem diese Gruppen jeweils an der durch # gekennzeichneten Position aneinander binden. Die Position, an der die Gruppen an das Metall koordinieren, sind durch * gekennzeichnet. Diese Gruppen können auch über eine oder zwei verbrückende Einheiten V an den Liganden L gebunden sein.

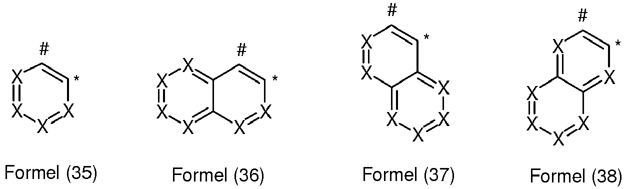

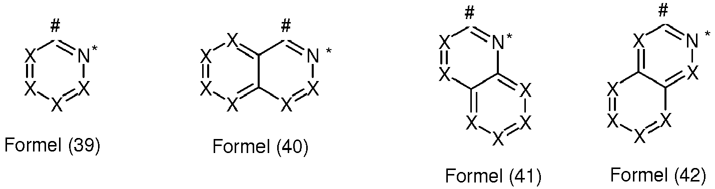

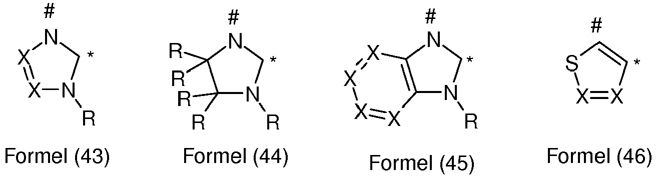

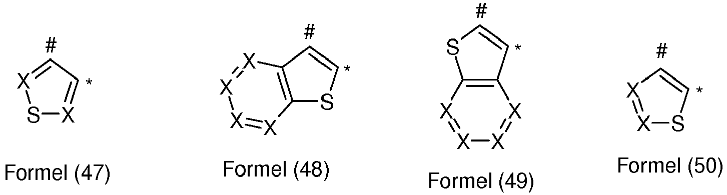

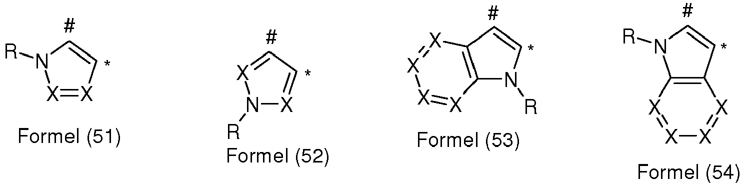

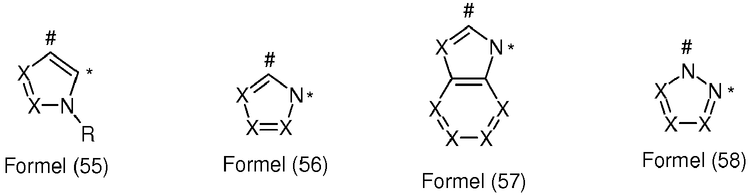

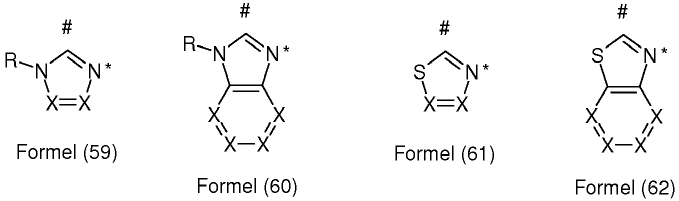

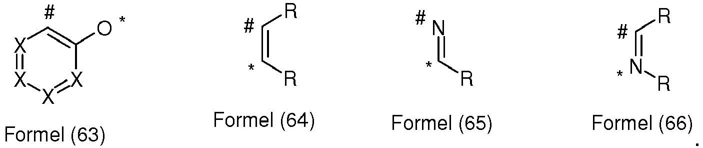

Dabei steht X bei jedem Auftreten gleich oder verschieden für CR oder N, wobei hier die oben genannte Limitierung, dass mindestens zwei benachbarte Gruppen X für CR stehen und die Reste R einen Ring der Formel (5) oder (6) bilden, nicht gilt; und R hat dieselbe Bedeutung wie oben beschrieben. Bevorzugt stehen maximal drei Symbole X in jeder Gruppe für N, besonders bevorzugt stehen maximal zwei Symbole X in jeder Gruppe für N, ganz besonders bevorzugt steht maximal ein Symbol X in jeder Gruppe für N. Insbesondere bevorzugt stehen alle Symbole X für CR.

Weiterhin können die Formeln (46) bis (50), (61) und (62) statt des Schwefels auch Sauerstoff enthalten.

Ebenfalls bevorzugte Liganden L' sind η⁵-Cyclopentadienyl, η⁵-Pentamethylcyclopentadienyl, η⁶-Benzol oder η⁷-Cycloheptatrienyl, welche jeweils durch einen oder mehrere Reste R substituiert sein können.

Ebenfalls bevorzugte Liganden L' sind 1,3,5-cis,cis-Cyclohexanderivate, insbesondere der Formel (67), 1,1,1-Tri(methylen)methanderivate, insbesondere der Formel (68) und 1,1,1 -trisubstituierte Methane, insbesondere der Formel (69) und (70), 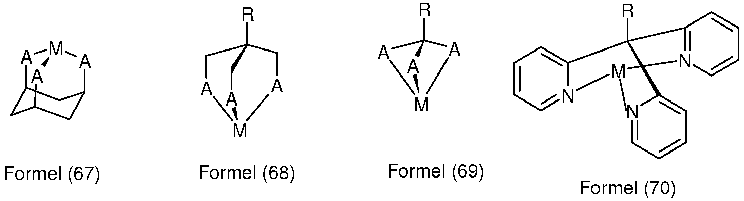 wobei in den Formeln jeweils die Koordination an das Metall M dargestellt ist, R die oben genannte Bedeutung hat und A, gleich oder verschieden bei jedem Auftreten, für O⁻, S⁻, COO⁻, PR₂ oder NR₂ steht.

Bevorzugte Reste R in den oben aufgeführten Strukturen sind bei jedem Auftreten gleich oder verschieden ausgewählt aus der Gruppe bestehend aus H, D, F, Br, N(R¹)₂, CN, B(OR¹)₂, C(=O)R¹, P(=O)(R¹)₂, einer geradkettigen Alkylgruppe mit 1 bis 10 C-Atomen oder einer Alkenyl- oder Alkinylgruppe mit 2 bis 10 C-Atomen oder einer verzweigten oder cyclischen Alkylgruppe mit 3 bis 10 C-Atomen, die jeweils mit einem oder mehreren Resten R¹ substituiert sein kann, wobei ein oder mehrere H-Atome durch D oder F ersetzt sein können, oder einem aromatischen oder heteroaromatischen Ringsystem mit 5 bis 14 aromatischen Ringatomen, das jeweils durch einen oder mehrere Reste R¹ substituiert sein kann; dabei können zwei oder mehrere benachbarte Reste R auch miteinander ein mono- oder polycyclisches, aliphatisches, aromatisches und/oder benzoannelliertes Ringsystem bilden. Besonders bevorzugte Reste R sind bei jedem Auftreten gleich oder verschieden ausgewählt aus der Gruppe bestehend aus H, D, F, Br, CN, B(OR¹)₂, einer geradkettigen Alkylgruppe mit 1 bis 5 C-Atomen, insbesondere Methyl, oder einer verzweigten oder cyclischen Alkylgruppe mit 3 bis 5 C-Atomen, insbesondere iso-Propyl oder tert-Butyl, wobei ein oder mehrere H-Atome durch D oder F ersetzt sein können, oder einem aromatischen oder heteroaromatischen Ringsystem mit 5 bis 12 aromatischen Ringatomen, das jeweils durch einen oder mehrere Reste R¹ substituiert sein kann; dabei können zwei oder mehrere Reste R auch miteinander ein mono- oder polycyclisches, aliphatisches, aromatisches und/oder benzoannelliertes Ringsystem bilden.

Die erfindungsgemäßen Komplexe können facial bzw. pseudofacial sein, oder sie können meridional bzw. pseudomeridional sein.

Die Liganden L können je nach Struktur auch chiral sein. Dies ist insbesondere dann der Fall, wenn sie eine bicyclische Gruppe der Formel (6) enthalten oder wenn sie Substituenten enthalten, beispielsweise Alkyl-, Alkoxy, Dialkylamino- oder Aralkylgruppen, welche ein oder mehrere Stereozentren aufweisen. Da es sich bei der Grundstruktur des Komplexes auch um eine chirale Struktur handeln kann, ist die Bildung von Diastereomeren und mehreren Enantiomerenpaaren möglich. Die erfindungsgemäßen Komplexe umfassen dann sowohl die Mischungen der verschiedenen Diastereomere bzw. die entsprechenden Racemate wie auch die einzelnen isolierten Diastereomere bzw. Enantiomere.

Die oben genannten bevorzugten Ausführungsformen sind beliebig miteinander kombinierbar. In einer besonders bevorzugten Ausführungsform der Erfindung gelten die oben genannten bevorzugten Ausführungsformen gleichzeitig.

Die erfindungsgemäßen Metallkomplexe sind prinzipiell durch verschiedene Verfahren darstellbar. Es haben sich jedoch die im Folgenden beschriebenen Verfahren als besonders geeignet herausgestellt.

Daher ist ein weiterer Gegenstand der vorliegenden Erfindung ein Verfahren zur Herstellung der Metallkomplex-Verbindungen gemäß Formel (1) durch Umsetzung der entsprechenden freien Liganden mit Metallalkoholaten der Formel (71), mit Metallketoketonaten der Formel (72), mit Metallhalogeniden der Formel (73), mit dimeren Metallkomplexen der Formel (74) oder mit Metallkomplexen der Formel (75), 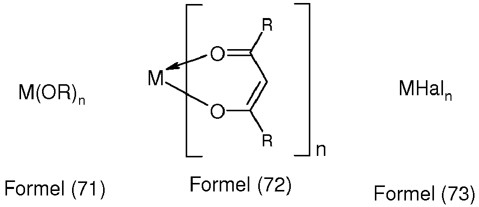 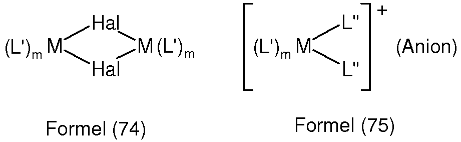 wobei die Symbole M, m, n und R die oben angegebenen Bedeutungen haben, Hal = F, Cl, Br oder I ist, L" für einen Alkohol, insbesondere für einen Alkohol mit 1 bis 4 C-Atomen oder ein Nitril, insbesondere Acetonitril oder Benzonitril, steht und (Anion) ein nicht-koordinierendes Anion ist, wie beispielsweise Triflat.

Es können ebenfalls Metallverbindungen, insbesondere Iridiumverbindungen, die sowohl Alkoholat- und/oder Halogenid- und/oder Hydroxy- wie auch Ketoketonatreste tragen, verwendet werden. Diese Verbindungen können auch geladen sein. Entsprechende Iridiumverbindungen, die als Edukte besonders geeignet sind, sind in WO 2004/085449 offenbart. Besonders geeignet sind [IrCl₂(acac)₂]⁻, beispielsweise Na[IrCl₂(acac)₂], Metallkomplexe mit Acetylacetonat-Derivaten als Ligand, beispielsweise Ir(acac)₃ oder Tris(2,2,6,6-Tetramethylheptan-3,5-dionato)iridium, und IrCl₃·xH₂O, wobei x üblicherweise für eine Zahl zwischen 2 und 4 steht.

Geeignete Platin-Edukte sind beispielsweise PtCl₂, K₂[PtCl₄], PtCl₂(DMSO)₂, Pt(Me)₂(DMSO)₂ oder PtCl₂(Benzonitril)₂.

Die Synthese der Komplexe wird bevorzugt durchgeführt wie in WO 2002/060910, WO 2004/085449 und WO 2007/065523 beschrieben. Heteroleptische Komplexe können beispielsweise auch gemäß WO 2005/042548 synthetisiert werden. Dabei kann die Synthese beispielsweise auch thermisch, photochemisch und/oder durch Mikrowellenstrahlung aktiviert werden. In einer bevorzugten Ausführungsform der Erfindung wird die Reaktion ohne die Verwendung eines zusätzlichen Lösemittels in der Schmelze durchgeführt. Dabei bedeutet "Schmelze", dass der Ligand geschmolzen vorliegt und die Metall-Vorstufe in dieser Schmelze gelöst oder suspendiert ist. Zur Aktivierung der Reaktion ist es weiterhin auch möglich, eine Lewis-Säure, beispielsweise ein Silbersalz oder AlCl₃, zuzugeben.

Durch diese Verfahren, gegebenenfalls gefolgt von Aufreinigung, wie z. B. Umkristallisation oder Sublimation, lassen sich die erfindungsgemäßen Verbindungen gemäß Formel (1) in hoher Reinheit, bevorzugt mehr als 99 % (bestimmt mittels ¹H-NMR und/oder HPLC) erhalten.

Die erfindungsgemäßen Verbindungen können auch durch geeignete Substitution, beispielsweise durch längere Alkylgruppen (ca. 4 bis 20 C-Atome), insbesondere verzweigte Alkylgruppen, oder gegebenenfalls substituierte Arylgruppen, beispielsweise Xylyl-, Mesityl- oder verzweigte Terphenyl- oder Quaterphenylgruppen, löslich gemacht werden. Solche Verbindungen sind dann in gängigen organischen Lösemitteln, wie beispielsweise Toluol oder Xylol bei Raumtemperatur in ausreichender Konzentration löslich, um die Komplexe aus Lösung verarbeiten zu können. Diese löslichen Verbindungen eignen sich besonders gut für die Verarbeitung aus Lösung, beispielsweise durch Druckverfahren.

Die erfindungsgemäßen Verbindungen können auch mit einem Polymer gemischt werden. Ebenso ist es möglich, diese Verbindungen kovalent in ein Polymer einzubauen. Dies ist insbesondere möglich mit Verbindungen, welche mit reaktiven Abgangsgruppen, wie Brom, lod, Chlor, Boronsäure oder Boronsäureester, oder mit reaktiven, polymerisierbaren Gruppen, wie Olefinen oder Oxetanen, substituiert sind. Diese können als Monomere zur Erzeugung entsprechender Polymere Verwendung finden. DiePolymerisation erfolgt dabei bevorzugt über die Halogenfunktionalität bzw. die Boronsäurefunktionalität bzw. über die polymerisierbare Gruppe. Es ist weiterhin möglich, die Polymere über derartige Gruppen zu vernetzen. Die erfindungsgemäßen Verbindungen und Polymere können als vernetzte oder unvernetzte Schicht eingesetzt werden.

Weiterer Gegenstand der Erfindung sind daher Polymere enthaltend eine oder mehrere der oben aufgeführten erfindungsgemäßen Verbindungen, wobei ein oder mehrere Bindungen der erfindungsgemäßen Verbindung zum Polymer vorhanden sind. Je nach Verknüpfung der erfindungsgemäßen Verbindung bildet diese daher eine Seitenkette des Polymers oder ist in der Hauptkette verknüpft. Die Polymere können konjugiert, teilkonjugiert oder nicht-konjugiert sein. Die Polymere können linear, verzweigt oder dendritisch sein. Für die Wiederholeinheiten der erfindungsgemäßen Verbindungen in Polymeren gelten dieselben Bevorzugungen, wie oben beschrieben.

Zur Herstellung der Polymere werden die erfindungsgemäßen Monomere homopolymerisiert oder mit weiteren Monomeren copolymerisiert. Bevorzugt sind Copolymere, wobei die Einheiten gemäß Formel (1) bzw. die oben ausgeführten bevorzugten Ausführungsformen zu 0.01 bis 99.9 mol%, bevorzugt 5 bis 90 mol%, besonders bevorzugt 20 bis 80 mol% vorhanden sind. Geeignete und bevorzugte Comonomere, welche das Polymergrundgerüst bilden, sind gewählt aus Fluorenen (z. B. gemäß EP 842208 oder WO 2000/022026), Spirobifluorenen (z. B. gemäß EP 707020, EP 894107 oder WO 2006/061181), Para-phenylenen (z. B. gemäß WO 92/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere können noch weitere Einheiten enthalten, beispielsweise Lochtransporteinheiten, insbesondere solche basierend auf Triarylaminen, und/oder Elektronentransporteinheiten.

Nochmals ein weiterer Gegenstand der vorliegenden Erfindung ist eine Formulierung, enthaltend eine erfindungsgemäße Verbindung bzw. ein erfindungsgemäßes Polymer und mindestens eine weitere Verbindung. Die weitere Verbindung kann beispielsweise ein Lösemittel sein. Die weitere Verbindung kann aber auch eine weitere organische oder anorganische Verbindung sein, die ebenfalls in der elektronischen Vorrichtung eingesetzt wird, beispielsweise ein Matrixmaterial. Diese weitere Verbindung kann auch polymer sein.

Die oben beschriebenen Komplexe gemäß Formel (1) bzw. die oben aufgeführten bevorzugten Ausführungsformen können in der elektronischen Vorrichtung als aktive Komponente verwendet werden. Unter einer elektronischen Vorrichtung wird eine Vorrichtung verstanden, welche Anode, Kathode und mindestens eine Schicht enthält, wobei diese Schicht mindestens eine organische bzw. metallorganische Verbindung enthält. Die erfindungsgemäße elektronische Vorrichtung enthält also Anode, Kathode und mindestens eine Schicht, welche mindestens eine Verbindung der oben aufgeführten Formel (1) enthält. Dabei sind bevorzugte elektronische Vorrichtungen ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), lichtemittierenden elektrochemischen Zellen (LECs) oder organischen Laserdioden (O-Laser), enthaltend in mindestens einer Schicht mindestens eine Verbindung gemäß der oben aufgeführten Formel (1). Besonders bevorzugt sind organische Elektrolumineszenzvorrichtungen. Aktive Komponenten sind generell die organischen oder anorganischen Materialien, welche zwischen Anode und Kathode eingebracht sind, beispielsweise Ladungsinjektions-, Ladungstransport- oder Ladungsblockiermaterialien, insbesondere aber Emissionsmaterialien und Matrixmaterialien. Die erfindungsgemäßen Verbindungen zeigen besonders gute Eigenschaften als Emissionsmaterial in organischen Elektrolumineszenzvorrichtungen. Eine bevorzugte Ausführungsform der Erfindung sind daher organische Elektrolumineszenzvorrichtungen. Weiterhin können die erfindungsgemäßen Verbindungen zur Erzeugung von Singulett-Sauerstoff oder in der Photokatalyse eingesetzt werden.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten, Ladungserzeugungsschichten und/oder organische oder anorganische p/n-Übergänge. Dabei ist es möglich, dass eine oder mehrere Lochtransportschichten p-dotiert sind, beispielsweise mit Metalloxiden, wie MoO₃ oder WO₃ oder mit (per)fluorierten elektronenarmen Aromaten, und/oder dass eine oder mehrere Elektronentransportschichten n-dotiert sind. Ebenso können zwischen zwei emittierende Schichten Interlayers eingebracht sein, welche beispielsweise eine Exzitonen-blockierende Funktion aufweisen und/oder die Ladungsbalance in der Elektrolumineszenzvorrichtung steuern. Es sei aber darauf hingewiesen, dass nicht notwendigerweise jede dieser Schichten vorhanden sein muss.

Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Dreischichtsysteme, wobei die drei Schichten blaue, grüne und orange oder rote Emission zeigen (für den prinzipiellen Aufbau siehe z. B. WO 2005/011013) bzw. Systeme, welche mehr als drei emittierende Schichten aufweisen. Es kann sich auch um ein Hybrid-System handeln, wobei eine oder mehrere Schichten fluoreszieren und eine oder mehrere andere Schichten phosphoreszieren.

In einer bevorzugten Ausführungsform der Erfindung enthält die organische Elektrolumineszenzvorrichtung die Verbindung gemäß Formel (1) bzw. die oben aufgeführten bevorzugten Ausführungsformen als emittierende Verbindung in einer oder mehreren emittierenden Schichten.

Wenn die Verbindung gemäß Formel (1) als emittierende Verbindung in einer emittierenden Schicht eingesetzt wird, wird sie bevorzugt in Kombination mit einem oder mehreren Matrixmaterialien eingesetzt. Die Mischung aus der Verbindung gemäß Formel (1) und dem Matrixmaterial enthält zwischen 0.1 und 99 Vol.-%, vorzugsweise zwischen 1 und 90 Vol.-%, besonders bevorzugt zwischen 3 und 40 Vol.-%, insbesondere zwischen 5 und 15 Vol.-% der Verbindung gemäß Formel (1) bezogen auf die Gesamtmischung aus Emitter und Matrixmaterial. Entsprechend enthält die Mischung zwischen 99.9 und 1 Vol.-%, vorzugsweise zwischen 99 und 10 Vol.-%, besonders bevorzugt zwischen 97 und 60 Vol.-%, insbesondere zwischen 95 und 85 Vol.-% des Matrixmaterials bezogen auf die Gesamtmischung aus Emitter und Matrixmaterial.

Als Matrixmaterial können generell alle Materialien eingesetzt werden, die gemäß dem Stand der Technik hierfür bekannt sind. Bevorzugt ist das Triplett-Niveau des Matrixmaterials höher als das Triplett-Niveau des Emitters.

Geeignete Matrixmaterialien für die erfindungsgemäßen Verbindungen sind Ketone, Phosphinoxide, Sulfoxide und Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl), m-CBP oder die in WO 2005/039246, US 2005/0069729, JP 2004/288381, EP 1205527, WO 2008/086851 oder US 2009/0134784 offenbarten Carbazolderivate, Indolocarbazolderivate, z. B. gemäß WO 2007/063754 oder WO 2008/056746, Indenocarbazolderivate, z. B. gemäß WO 2010/136109 oder WO 2011/000455, Azacarbazole, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Diazasilolderivate, z. B. gemäß WO 2010/054729, Diazaphospholderivate, z. B. gemäß WO 2010/054730, Triazinderivate, z. B. gemäß WO 2010/015306, WO 2007/063754 oder WO 2008/056746, Zinkkomplexe, z. B. gemäß EP 652273 oder WO 2009/062578, Dibenzofuranderivate, z. B. gemäß WO 2009/148015, oder verbrückte Carbazolderivate, z. B. gemäß US 2009/0136779, WO 2010/050778, WO 2011 /042107 oder WO 2011/088877.

Es kann auch bevorzugt sein, mehrere verschiedene Matrixmaterialien als Mischung einzusetzen, insbesondere mindestens ein elektronenleitendes Matrixmaterial und mindestens ein lochleitendes Matrixmaterial. Eine bevorzugte Kombination ist beispielsweise die Verwendung eines aromatischen Ketons, eines Triazin-Derivats oder eines Phosphinoxid-Derivats mit einem Triarylamin-Derivat oder einem Carbazol-Derivat als gemischte Matrix für den erfindungsgemäßen Metallkomplex. Ebenso bevorzugt ist die Verwendung einer Mischung aus einem ladungstransportierenden Matrixmaterial und einem elektrisch inerten Matrixmaterial, welches nicht bzw. nicht in wesentlichem Maße am Ladungstransport beteiligt ist, wie z. B. in WO 2010/108579 beschrieben.

Weiterhin bevorzugt ist es, eine Mischung aus zwei oder mehr Triplett-Emittern zusammen mit einer Matrix einzusetzen. Dabei dient der Triplett-Emitter mit dem kürzerwelligen Emissionsspektrum als Co-Matrix für den Triplett-Emitter mit dem längerwelligen Emissionsspektrum. So können beispielsweise die erfindungsgemäßen Komplexe gemäß Formel (1) als Co-Matrix für längerwellig emittierende Triplettemitter, beispielsweise für grün oder rot emittierende Triplettemitter, eingesetzt werden.

Die erfindungsgemäßen Verbindungen lassen sich auch in anderen Funktionen in der elektronischen Vorrichtung einsetzen, beispielsweise als Lochtransportmaterial in einer Lochinjektions- oder -transportschicht, als Ladungserzeugungsmaterial oder als Elektronenblockiermaterial. Ebenso lassen sich die erfindungsgemäßen Komplexe als Matrixmaterial für andere phosphoreszierende Metallkomplexe in einer emittierenden Schicht einsetzen.

Als Kathode sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag, wobei dann in der Regel Kombinationen der Metalle, wie beispielsweise Mg/Ag, Ca/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Ebenso kommen hierfür organische Alkalimetallkomplexe in Frage, z. B. Liq (Lithiumchinolinat). Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (O-SC) oder die Auskopplung von Licht (OLED/PLED, O-LASER) zu ermöglichen. Bevorzugte Anodenmaterialien sind hier leitfähige gemischte Metalloxide. Besonders bevorzugt sind Indium-Zinn-Oxid (ITO) oder Indium-Zink-Oxid (IZO). Bevorzugt sind weiterhin leitfähige, dotierte organische Materialien, insbesondere leitfähige dotierte Polymere, z. B. PEDOT, PANI oder Derivate dieser Polymere. Bevorzugt ist weiterhin, wenn auf die Anode ein p-dotiertes Lochtransportmaterial als Lochinjektionsschicht aufgebracht wird, wobei sich als p-Dotanden Metalloxide, beispielsweise MoO₃ oder WO₃, oder (per)fluorierte elektronenarme Aromaten eignen. Weitere geeignete p-Dotanden sind HAT-CN (Hexacyano-hexaazatriphenylen) oder die Verbindung NPD9 von Novaled. Eine solche Schicht vereinfacht die Lochinjektion in Materialien mit einem tiefen HOMO, also einem betragsmäßig großen HOMO.

In den weiteren Schichten können generell alle Materialien verwendet werden, wie sie gemäß dem Stand der Technik für die Schichten verwendet werden, und der Fachmann kann ohne erfinderisches Zutun jedes dieser Materialien in einer elektronischen Vorrichtung mit den erfindungsgemäßen Materialien kombinieren.

Die Vorrichtung wird entsprechend (je nach Anwendung) strukturiert, kontaktiert und schließlich hermetisch versiegelt, da sich die Lebensdauer derartiger Vorrichtungen bei Anwesenheit von Wasser und/oder Luft drastisch verkürzt.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck von üblicherweise kleiner 10⁻⁵ mbar, bevorzugt kleiner 10⁻⁶ mbar aufgedampft. Es ist auch möglich, dass der Anfangsdruck noch geringer oder noch höher ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck oder Nozzle-Printing, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig, welche beispielsweise durch geeignete Substitution erhalten werden.

Die organische Elektrolumineszenzvorrichtung kann auch als Hybrid-system hergestellt werden, indem eine oder mehrere Schichten aus Lösung aufgebracht werden und eine oder mehrere andere Schichten aufgedampft werden. So ist es beispielsweise möglich, eine emittierende Schicht enthaltend eine Verbindung gemäß Formel (1) und ein Matrixmaterial aus Lösung aufzubringen und darauf eine Lochblockierschicht und/oder eine Elektronentransportschicht im Vakuum aufzudampfen.

Diese Verfahren sind dem Fachmann generell bekannt und können von ihm ohne Probleme auf organische Elektrolumineszenzvorrichtungen enthaltend Verbindungen gemäß Formel (1) bzw. die oben aufgeführten bevorzugten Ausführungsformen angewandt werden.

Die erfindungsgemäßen elektronischen Vorrichtungen, insbesondere organische Elektrolumineszenzvorrichtungen, zeichnen sich durch einen oder mehrere der folgenden überraschenden Vorteile gegenüber dem Stand der Technik aus:
1. Die erfindungsgemäßen Verbindungen eignen sich sehr gut für den Einsatz in elektronischen Vorrichtungen, insbesondere in organischen Elektrolumineszenzvorrichtungen und führen dort zu sehr guten Eigenschaften.
2. Organische Elektrolumineszenzvorrichtungen enthaltend Verbindungen gemäß Formel (1) als emittierende Materialien weisen eine sehr gute Lebensdauer auf.
3. Organische Elektrolumineszenzvorrichtungen enthaltend Verbindungen gemäß Formel (1) als emittierende Materialien weisen eine hervorragende Effizienz auf. Insbesondere ist die Effizienz deutlich höher gegenüber analogen Verbindungen, die keine Struktureinheit gemäß Formel (5) oder Formel (6) enthalten.
4. Die erfindungsgemäßen Metallkomplexe weisen teilweise ein sehr schmales Emissionsspektrum auf, was zu einer hohen Farbreinheit der Emission führt, wie sie insbesondere für Displayanwendungen wünschenswert ist.

Diese oben genannten Vorteile gehen nicht mit einer Verschlechterung der weiteren elektronischen Eigenschaften einher.

Die Erfindung wird durch die nachfolgenden Beispiele näher erläutert, ohne sie dadurch einschränken zu wollen. Der Fachmann kann aus den Schilderungen ohne erfinderisches Zutun weitere erfindungsgemäße elektronische Vorrichtungen herstellen und somit die Erfindung im gesamten beanspruchten Bereich ausführen.

### Beispiele:

Die nachfolgenden Synthesen werden, sofern nicht anders angegeben, unter einer Schutzgasatmosphäre in getrockneten Lösungsmitteln durchgeführt. Die Metallkomplexe werden zusätzlich unter Ausschluss von Licht bzw. unter Gelblicht gehandhabt. Die Lösungsmittel und Reagenzien können z. B. von Sigma-ALDRICH bzw. ABCR bezogen werden. Die jeweiligen Angaben in eckigen Klammern bzw. die zu einzelnen Verbindungen angegebenen Nummern beziehen sich auf die CAS-Nummern der literaturbekannten Verbindungen.

### A: Synthese der Synthone S:

### Beispiel S1: 1,1,2,2,3,3-Hexamethyl-indan-d18, S1

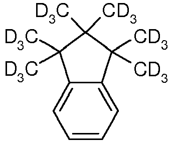

Darstellung analog zu J. Baran, et al., J. Org. Chem. 1988, 53, 19, 4626. Ein auf -78°C gekühltes Gemisch aus 160.7 g (1 mol) 2-Chlor-2-phenyl-propan-d6 [53102-26-4], 230.8 g (2,4 mol) 2,3-Dimethylbut-2-en-d12 [69165-86-2] und 2500 ml wasserfreiem Dichlormethan wird tropfenweise unter gutem Rühren mit 18.7 ml (170 mmol) Titantetrachlorid versetzt und 2 h nachgerührt. Man gießt die kalte Reaktionsmischung unter gutem Rühren in 1500 ml 3N Salzsäure ein, rührt 20 min. nach, trennt die org. Phase ab, wäscht diese zweimal mit je 1000 ml Wasser, einmal mit 500 ml ges. Natriumcarbonat-Lösung, einmal mit 500 ml ges. Kochsalzlösung, trocknet über Magnesiumsulfat, filtriert vom Trockenmittel ab, befreit das Filtrat im Vakuum vom Dichlormethan und destilliert den Rückstand fraktioniert (Kernfraktion 60-65 °C, ca. 0.5 mbar). Ausbeute: 163.1 g (740 mmol), 74 %; Reinheit: ca. 95 %ig nach NMR.

Analog können folgende Verbindungen dargestellt werden:

| Bsp. | Edukte | Produkt | Ausbeute |
|---|---|---|---|
| S2 | 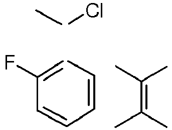 | 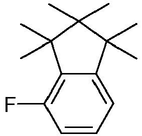 | 68 % |
| | 1716-38-7 / 563-79-1 | S2 | |
| S3 | 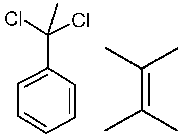 | 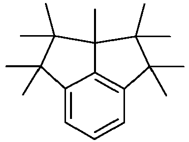 | 49 % |
| | 934-53-2 / 563-79-1 | S3 | |
| | Verwendung von 4.4 mol 2,3-Dimethylbut-2-en | | |

### Beispiel S4: 6-Brom-1,1,3,3-tetramethyl-indan-5-ylamin, S4

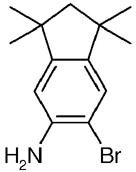

Ein Gemisch aus 166.0 g (500 mmol) 5,6-Dibrom-1,1,3,3-tetramethyl-indan S16a, 83.9 ml (500 mmol) Benzhydrylidenamin [1013-88-3], 52.9 g (550 mmol) Natrium-tert-butylat und 500 ml Toluol wird mit 6.23 g (10 mmol) rac-BINAP und dann mit 2.24 g (10 mmol) Palladium(II)acetat versetzt, und anschließend 16 h unter Rückfluss erhitzt. Nach Erkalten fügt man 500 ml Wasser zu, trennt die org. Phase ab, wäscht diese zweimal mit je 500 ml ges. Kochsalz-Lösung, rotiert das Toluol ab, nimmt den Rückstand in 1000 ml THF auf, fügt 250 ml 2 N Salzsäure zu und erhitzt die Reaktionsmischung 16 h unter Rückfluss. Man entfernt das Lösungsmittel im Vakuum, nimmt den Rückstand in 1000 ml Ethylacetat auf, wäscht die org. Phase mit ges. Natriumhyrdogencarbonat-Lösung bis pH = 7 erreicht ist, tocknet die org. Phase über Magnesiumsulfat, filtriert vom Trockenmittel ab, setzt dem Filtrat 500 g Kieselgel zu und entfernt das Lösungsmittel im Vakuum. Man plaziert das beladene Kieselgel auf einer Kieselgel-Säule (1500 g, aufgeschlämmt in n-Heptan : Ethylacetat, 95:5 vv), eluiert mit n-Heptan : Ethylacetat (95:5 vv) zunächst das Benzophenon, stellt dann auf Ethylacetat um und eluiert das Produkt. Ausbeute: 85.8 g (320 mmol), 64 %; Reinheit: ca. 95 %ig nach ¹H-NMR.

Analog können folgende Verbindungen dargestellt werden:

| Bsp. | Edukt | Produkt | Ausbeute |
|---|---|---|---|
| S5 | 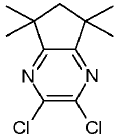 | 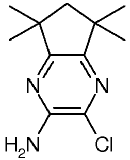 | 58 % |
| | 1311465-45-8 | S5 | |
| S6 | 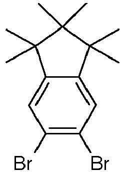 | 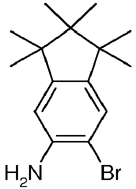 | 67 % |
| | S24 | S6 | |
| | Variante A, Stufe A | | |
| S7 | 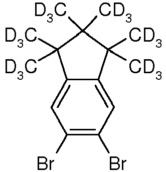 | 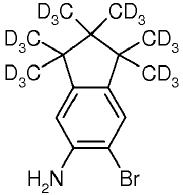 | 66 % |
| | S25 | S7 | |
| | Variante A, Stufe A | | |
| S8 | 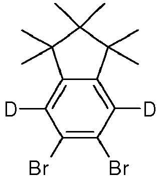 | 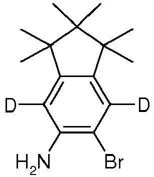 | 65 % |
| | S26 | S8 | |
| | Variante A, Stufe A | | |
| S9 | 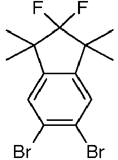 | 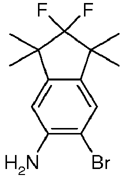 | 59 % |
| | S29 | S9 | |
| | Variante A, Stufe A | | |
| S10 | 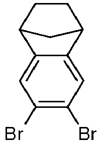 | 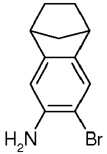 | 61 % |
| | 42810-32-2 | S10 | |
| S11 | 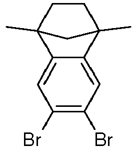 | 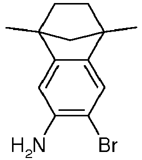 | 63 % |
| | S36 | S11 | |
| | Variante A, Stufe A | | |
| S12 | 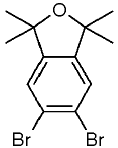 | 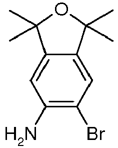 | 58 % |
| | S39 | S12 | |
| | Variante A, Stufe A | | |
| S13 | 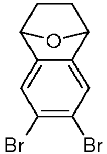 | 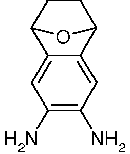 | 55% |
| | 749859-07-2 | S13 | |
| S14 | 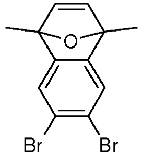 | 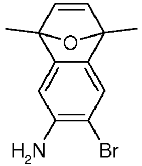 | 36 % |
| | 197245-07-1 | S14 | |
| S15 | 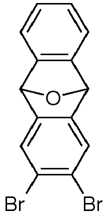 | 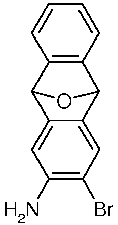 | 71 % |
| | 128726-58-9 | S15 | |

### Beispiel S16: 1,1,3,3-Tetramethyl-indan-5,6-diamin, [83721-95-3], S16

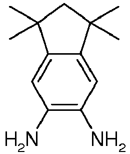

### Variante A:

### A: 5,6-Dibrom-1,1,3,3-tetramethyl-indan, S16a

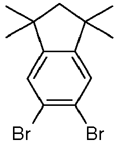

Eine Lösung von 87.2 g (500 mmol) 1,1,3,3-Tetramethyl-indan [4834-33-7] in 2000 ml Dichlormethan wird mit 1.3 g wasserfreiem Eisen(III)chlorid und dann unter Lichtausschluss tropfenweise mit einer Mischung von 64.0 ml (1.25 mol) Brom und 300 ml Dichlormethan so versetzt, dass die Temperatur 25 °C nicht übersteigt, gegebenenfalls wird mit einem Kaltwasserbad gegengekühlt. Man rührt die Reaktionsmischung 16 h bei Raumtemperatur nach, versetzt dann langsam mit 500 ml ges. Natriumsulfit-Lösung, trennt die wässrige Phase ab, wäscht die organische dreimal mit je 1000 ml Wasser, trocknet über Natriumsulfat, filtriert über eine kurze Säule aus Kieselgel und zieht dann das Lösungsmittel ab. Abschießend wird der Feststoff einmal aus wenig (ca. 100 ml) Ethanol umkristallisiert. Ausbeute: 121.2 g (365 mmol), 73 %; Reinheit: ca. 95 %ig nach ¹H NMR.

### B: 1,1,3,3-Tetramethyl-indan-5,6-diamin, S16

Ein Gemisch aus 121.2 g (365 mmol) 5,6-Dibrom-1,1,3,3-tetramethyl-indan, 153.2 ml (913 mmol) Benzhydrylidenamin [1013-88-3], 96.1 g (1.0 mol) Natrium-tert-butylat und 1000 ml Toluol wird mit 9.34 g (15 mmol) rac-BINAP und dann mit 3.36 g (15 mmol) Palladium(II)acetat versetzt, und anschließend 16 h unter Rückfluss erhitzt. Nach Erkalten fügt man 500 ml Wasser zu, trennt die org. Phase ab, wäscht diese zweimal mit je 500 ml ges. Kochsalz-Lösung, rotiert das Toluol ab, nimmt den Rückstand in 500 ml THF auf, fügt 200 ml 2 N Salzsäure zu und erhitzt die Reaktionsmischung 16 h unter Rückfluss. Man entfernt das Lösungsmittel im Vakuum, nimmt den Rückstand in 1000 ml Ethylacetat auf, wäscht die org. Phase Natriumhyrdogencarbonat-Lösung bis pH = 7 erreicht ist, tocknet die org. Phase über Magnesiumsulfat, filtriert vom Trockenmittel ab, setzt dem Filtrat 500 g Kieselgel zu und entfernt das Lösungsmittel im Vakuum. Man plaziert das beladene Kieselgel auf einer Kieselgel-Säule (1500 g, aufgeschlämmt in n-Heptan : Ethylacetat, 95:5 vv), eluiert mit n-Heptan : Ethylacetat (95:5 vv) zunächst das Benzophenon, stellt dann auf Ethylacetat um und eluiert das Produkt. Ausbeute: 56.8 g (278 mmol), 76 %; Reinheit: ca. 95 % nach ¹H-NMR.

### Variante B:

### A: 5,6-Dinitro-1,1,3,3-tetramethyl-indan, S16b

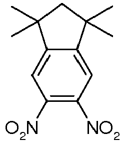

Zu einer gut gerührten, auf 0°C gekühlten Mischung aus 87.2 g (500 mmol) 1,1,3,3-Tetramethyl-indan [4834-33-7] und 350 ml 95 Gew.-%iger Schwefelsäure tropft man langsam 350 ml 100 Gew.-%ige Salpetersäure so zu, dass die Temperatur + 5°C nicht übersteigt. Anschließend lässt man während 2 - 3 h langsam auf Raumtemperatur erwärmen und gießt die Reaktionsmischung dann in ein gut gerührtes Gemisch aus 6 kg Eis und 2 kg Wasser. Man stellt durch Zugabe von 40 Gew.-%iger NaOH auf pH = 8-9 ein, extrahiert dreimal mit je 1000 ml Ethylacetat, wäscht die vereinigten org. Phasen zweimal mit je 1000 ml Wasser, trocknet über Magnesiumsulfat, entfernt dann das Ethylacetat im Vakuum fast vollständig, bis zur beginnenden Kristallisation, und vervollständigt die Kristallisation durch Zusatz von 500 ml Heptan. Man saugt von den so erhaltenen beigefarbenen Kristallen ab und trocknet diese im Vakuum. Ausbeute: 121.6 g (460 mmol), 92 %; Reinheit: ca. 94 %ig nach ¹H-NMR, Rest ca. 4 % 4,6-Dinitro-1,1,3,3-tetramethyl-indan. Aus der ML können ca. 3 % 4,5-Dinitro-1,1,3,3-tetramethyl-indan isoliert werden.

In einigen Fällen - insbesondere bei den bicyclichen Edukten - fällt neben dem 4,6-Isomeren auch das 4,5-Isomere in Anteilen bis zu ca. 15 % an (s. H. Tanida, J. Am. Chem. Soc. 1965, 87, 21, 4794). Dieses kann durch Umkristallisation bzw. chromatographisch abgetrennt werden, dann ebenfalls hydriert und weiter in der Ligandensynthese verwendet werden. Das ebenfalls entstehende 4,6-Isomere lässt sich durch Umkristallistion oder chromatographisch abtrennen, Anteile von eingen % stören aber in der weiteren Darstellung der Liganden nicht, da die m-Stellung der Amniofunktionen eine Cyclisierung zu kondensierten Ligandensysten nicht zulässt.

### B: 1,1,3,3-Tetramethyl-indan-5,6-diamin, S16

126.9 g (480 mmol) 5,6-Dinitro-1,1,3,3-tetramethyl-indan, S16b werden bei Raumtemperatur in 1200 ml Ethanol an 10 g Palladium/Kohle bei 3 bar Wasserstoffdruck während 24 h hydriert. Die Reaktionsmischung wird zweimal über ein Celite-Bett filtriert, der nach Entferen des Ethanols erhaltene braune Feststoff wird Kugelrohr-destilliert (T ca. 160 °C, p ca. 10⁻⁴ mbar). Ausbeute: 90.3 g (442 mmol), 92 %; Reinheit: ca. 95 % nach ¹H-NMR.
1,1,3,3-Tetramethyl-indan-5,6-diamin-di-hydrochlorid, S16 x 2HCl kann aus S16 durch lösen in Dichlormethan und einleiten von gasförmiger HCl bis zur Sättigung und anschließendes Entfernen des Dichlormethans erhalten werden.

Analog können folgende Verbindungen dargestellt werden:

| **Bsp.** | **Edukt** | **Produkt** | **Variante Ausbeute Stufe A+B** |
|---|---|---|---|
| S17 | 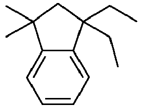 | 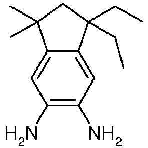 | B |
| | | | 70 % |
| | 72977-53-8 | S17 | |
| S18 | 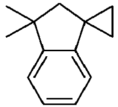 | 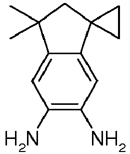 | A |
| | | | 47 % |
| | 59770-92-2 | S18 | |
| S19 | 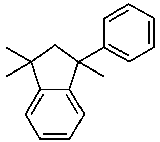 | 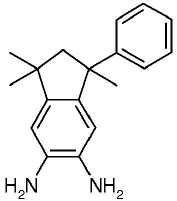 | A |
| | | | 17% |
| | 3910-35-8 | S19 | |
| S20 | 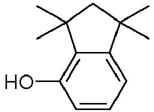 | 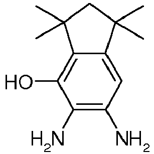 | A |
| | | | 21 % |
| | 53718-36-8 | S20 | |
| S21 | 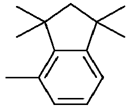 | 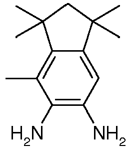 | B |
| | | | 68 % |
| | 479070-73-0 | S21 | |
| S22 | 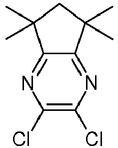 | 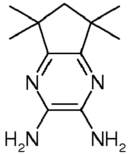 | A nur Stufe B |
| | | | 64 % |
| | 1311465-45-8 | S22 | |
| S23 | 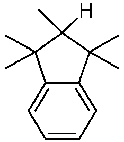 | 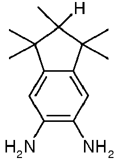 | B |
| | | | 76 % |
| | 1203-17-4 | S23 | |
| S24 | 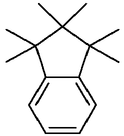 | 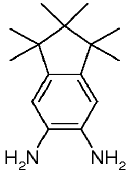 | A |
| | | | 63 % |
| | | | B |
| | 91324-94-6 | S24 | 78 % |
| S25 | 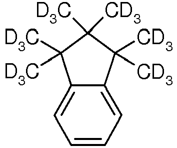 | 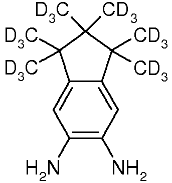 | B |
| | | | 80% |
| | S1 | S25 | |
| S26 | 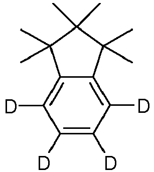 | 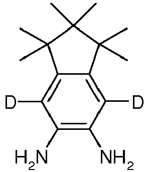 | B |
| | | | 76% |
| | 142076-41-3 | S26 | |
| S27 | 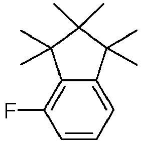 | 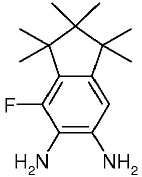 | B |
| | | | 60 % |
| | S2 | S27 | |
| S28 | 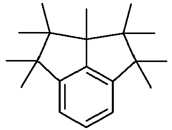 | 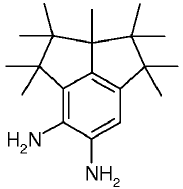 | B |
| | | | 73 % |
| | S3 | S28 | |
| S29 | 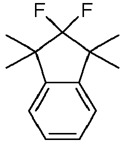 | 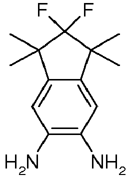 | B |
| | | | 77 % |
| | 59508-28-0 | S29 | |
| S30 | 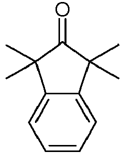 | 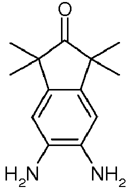 | A |
| | | | 56 % |
| | 5689-12-3 | 81864-09-7 | |
| | | S30 | |
| S31 | 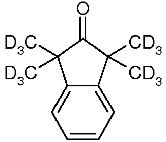 | 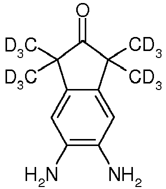 | A |
| | | | 54% |
| | 153735-62-7 | S31 | |
| S32 | 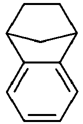 | 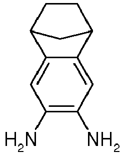 | B |
| | | | 71 % |
| | 4486-29-7 | 124639-03-8 S32 | |
| S32 | 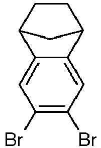 | 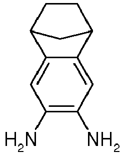 | A nur Stufe B |
| | | | 70 % |
| | 42810-32-2 | 124639-03-8 S32 | |
| S33 | 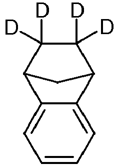 | 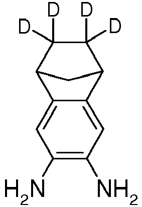 | B |
| | | | 75 % |
| | 15087-73-7 | S33 | |
| S34 | 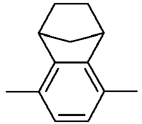 | 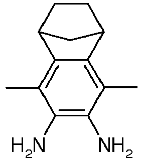 | B |
| | | | 83 % |
| | 215725-23-8 | S34 | |
| S35 | 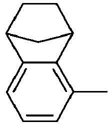 | 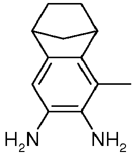 | B |
| | | | 58 % |
| | 215725-16-9 | S35 | |
| S36 | 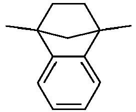 | 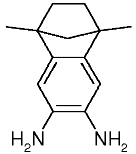 | B |
| | | | 70 % |
| | 66684-45-5 | S36 | |
| S37 | 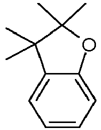 | 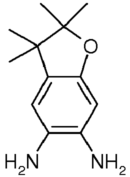 | A |
| | | | 61 % |
| | 124797-68-8 | S37 | |
| S38 | 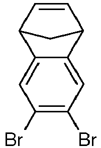 | 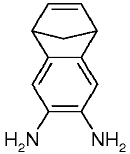 | A nur Stufe B |
| | | | 36 % |
| | 137495-57-9 | S38 | |
| S39 | 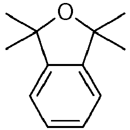 | 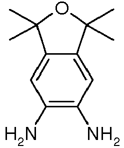 | B |
| | | | 68 % |
| | 113710-83-1 | S39 | |
| S40 | 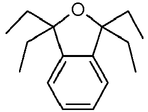 | 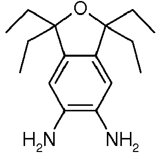 | B |
| | | | 68 % |
| | 65089-09-0 | S40 | |
| S41 | 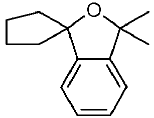 | 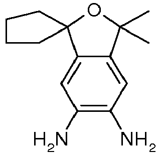 | B |
| | | | 64 % |
| | 1020726-74-2 | S41 | |
| S42 | 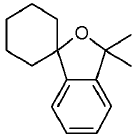 | 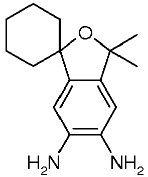 | B |
| | | | 66 % |
| | 59043-55-9 | S42 | |
| S43 | 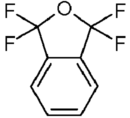 | 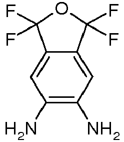 | A |
| | | | 76 % |
| | 651-39-8 | S43 | |
| S44 | 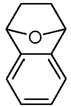 | 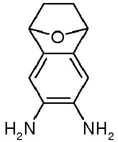 | B |
| | | | 57 % |
| | 35185-96-7 | S44 | |
| S44 | 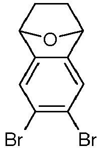 | 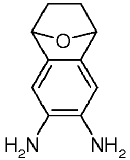 | A nur Stufe B |
| | | | 71 % |
| | 749859-07-2 | S44 | |
| S45 | 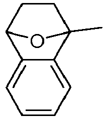 | 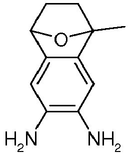 | B |
| | | | 74 % |
| | 61200-07-5 | S45 | |
| S46 | 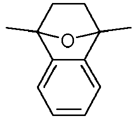 | 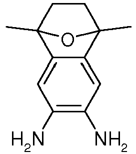 | B |
| | | | 77 % |
| | 61200-08-6 | S46 | |
| S47 | 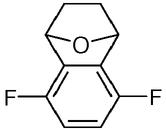 | 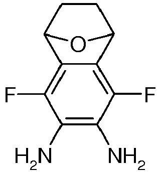 | B |
| | 773-70-6 | S47 | 70 % |
| S48 | 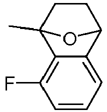 | 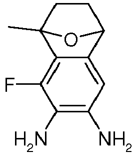 | B |
| | | | 59 % |
| | 78998-40-0 | S48 | |
| S49 | 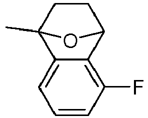 | 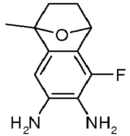 | A |
| | | | 62 % |
| | 78998-39-7 | S49 | |
| S50 | 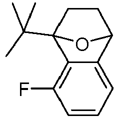 | 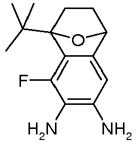 | A |
| | | | 60 % |
| | 121223-61-8 | S50 | |
| S51 | 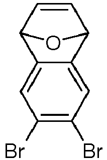 | 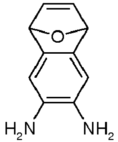 | A nur Stufe B |
| | | | 69 % |
| | 106750-88-3 | S51 | |
| S52 | 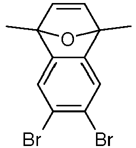 | 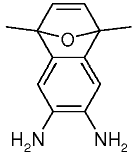 | A nur Stufe B |
| | | | 74 % |
| | 197245-07-1 | S52 | |
| S53 | 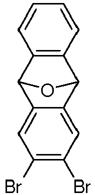 | 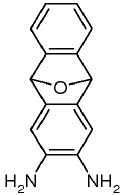 | A nur Stufe B |
| | | | 67 % |
| | 128726-58-9 | S53 | |
| S54 | 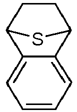 | 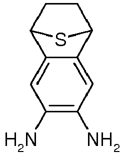 | A |
| | | | 32 % |
| | 300692-59-5 | S54 | |
| S55 | 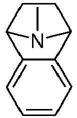 | 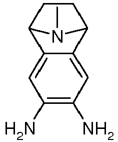 | A |
| | | | 26 % |
| | 55257-99-3 | S55 | |
| S56 | 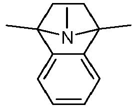 | 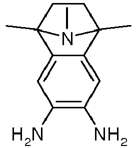 | A |
| | | | 28 % |
| | 169384-31-0 | S56 | |
| S57 | 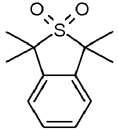 | 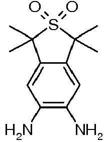 | B |
| | | | 57 % |
| | 135050-18-9 | S57 | |
| S58 | 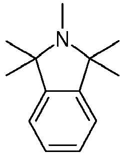 | 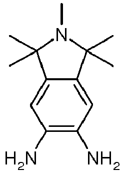 | A |
| | | | 33 % |
| | 3723-85-1 | S58 | |
| S59 | 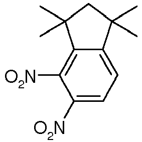 | 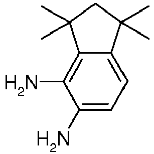 | B Schritt B |
| | | | 2 % |
| | Nebenprodukt aus S16 | S59 | |
| S60 | 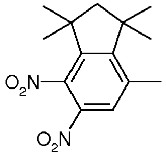 | 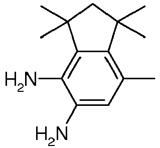 | B Schritt B |
| | Nebenprodukt aus S21 | S60 | 5 % |
| S61 | 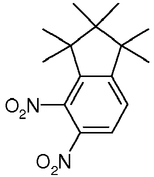 | 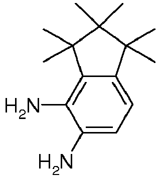 | B Schritt B |
| | | | 5 % |
| | Nebenprodukt aus S24 | S61 | |
| S62 | 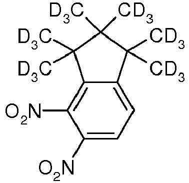 | 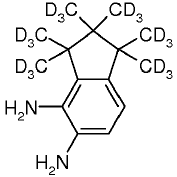 | B Schritt B |
| | | | 4% |
| | Nebenprodukt aus S25 | S62 | |
| S63 | 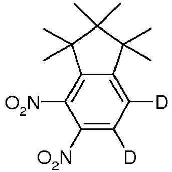 | 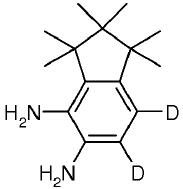 | B Schritt B |
| | | | 4% |
| | Nebenprodukt aus S26 | S63 | |
| S64 | 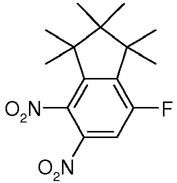 | 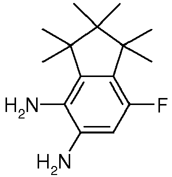 | B Schritt B |
| | | | 7% |
| | Nebenprodukt aus S27 | S64 | |
| S65 | 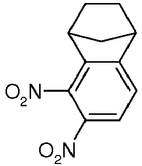 | 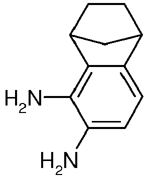 | B Schritt B |
| | | | 91 % |
| | 4228-35-7 | S65 | |
| S66 | 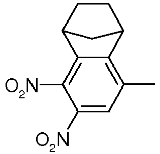 | 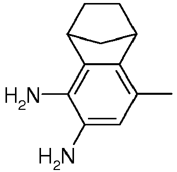 | B Schritt B |
| | | | 16 % |
| | Nebenprodukt aus S35 | S66 | |

### Beispiel S67: 7-Brom-1,2,3,4-tetrahydro-1,4-methano-naphthalin-6-carbaldehyd, S67

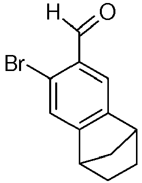

Durchführung analog J. Organomet. Chem., L. S. Chen et al., 1980, 193, 283-292. Eine auf -110 °C gekühlte Lösung von 30.2 g (100 mmol) 6,7-Dibrom-1,2,3,4-tetrahydro-1,4-methano-naphthalin [42810-32-2] in einem Gemisch aus 1000 ml THF und 1000 ml Diethylether wird so mit 40 ml (100 mmol) n-BuLi, 2.5 M in Hexan, vorgekühlt auf -110 °C, versetzt, dass die Temperatur - 105 °C nicht übersteigt. Man rührt 30 min. nach, versetzt dann trofenweise mit einem auf - 110 °C vo rgekühlten Gemisch aus 9.2 ml (120 mmol) DMF und 100 ml Diethylether, rührt dann 2 h nach, lässt auf -10 °C erwärmen, fügt 1000 ml 2 N HCl zu und rührt 2 h bei Raumtemperatur nach. Man trennt die org. Phase ab, wäscht diese einmal mit 500 ml Wasser, einmal mit 500 ml ges. Kochsalzlösung, trocknet über Magnesiumsulfat, entfernt das Lösungsmittel im Vakuum und unterwirft den Rückstand einer Kugelrohrdestillation (T ca. 90 °C, p ca. 10 ⁻⁴ mbar). Ausbeute: 15.8 g (63 mmol), 63 %; Reinheit: ca. 95 % nach ¹H-NMR.

Analog kann 6-Brom-7-formyl-1,2,3,4-tetrahydro-1,4-epoxynaphthalin, S68, dargestellt werden.

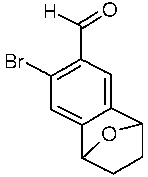

Einsatz von 30.4 g (100 mmol) 6,7-Dibrom-1,2,3,4-tetrahydro-1,4-epoxynaphthalin [749859-07-2] . Ausbeute: 14.4 g (54 mmol), 54 %; Reinheit: > 95 % nach ¹H-NMR.

### Beispiel S69: 2-(N-Pivaloylamido)-benzaldehyd, S69

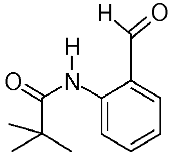

Ein Gemisch aus 18.5 g (100 mmol) 2-Brom-benzaldehyd [6630-33-7], 14.2 g (140 mmol) Pivalinsäureamid [754-10-9], 81.5 g (250 mmol) Cäsiumcarbonat, 1.7 g (3 mmol) 9,9-Dimethyl-4,5-bis(diphenylphosphino)-xanthen und 630 mg (2.8 mmol) Palladium(II)acetat in 400 ml Dioxan wird 4 h bei 100 °C gerührt. Nach Erkalten wird das Lösu ngsmittel im Vakuum entfernt, der Rückstand wird in 1000 ml Ethylacetat aufgenommen, die org. Phase wird dreimal mit je 300 ml Wasser und einmal mit 300 ml ges. Kochsalzlösung gewaschen und über eine kurze Kieselgel-Säule filtriert. Die nach Abziehen des Lösungsmittels im Vakuum erhaltenen Feststoffe werden weiter umgesetzt. Ausbeute: 19.3 g (94 mmol), 94 %. Reinheit: > 95 % nach ¹H-NMR.

Analog können folgende Derivate dargestellt werden:

| **Bsp.** | **Bromarylaldeyd** | **Amid** | **Produkt** | **Ausbeute** |
|---|---|---|---|---|
| S70 | 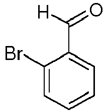 | 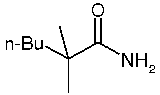 | 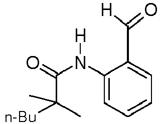 | 89 % |
| | 6630-33-7 | 20923-67-5 | S70 | |
| S71 | 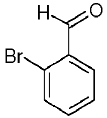 | 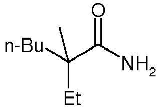 | 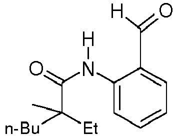 | 87 % |
| | 6630-33-7 | 13146-40-2 | S71 | |
| S72 | 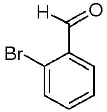 | 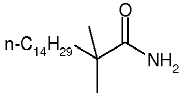 | 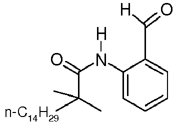 | 92 % |
| | 6630-33-7 | 62641-207 | S72 | |
| S73 | 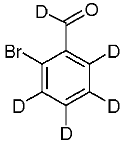 | 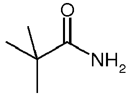 | 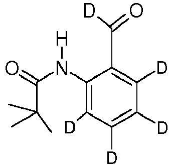 | 95 % |
| | 229948-71-4 | 754-10-9 | S73 | |
| S74 | 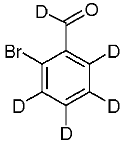 | 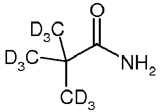 | 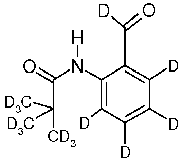 | 94 % |
| | 229948-71-4 | 6045-07-4 | S74 | |
| S75 | 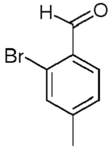 | 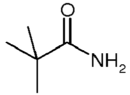 | 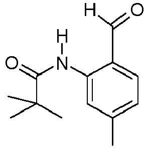 | 93 % |
| | 824-54-4 | 754-10-9 | S75 | |
| S76 | 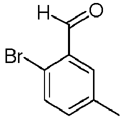 | 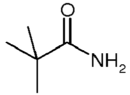 | 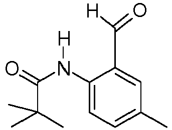 | 94 % |
| | 90221-55-9 | 754-10-9 | S76 | |
| S77 | 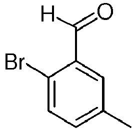 | 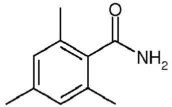 | 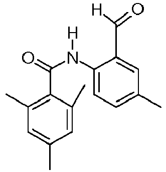 | 88 % |
| | 90221-55-9 | 4380-68-1 | S77 | |
| S78 | 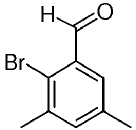 | 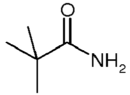 | 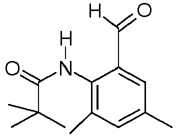 | 81 % |
| | 1000990-16-8 | 754-10-9 | S78 | |
| S79 | 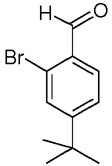 | 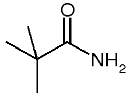 | 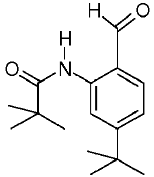 | 90 % |
| | 246139-77-5 | 754-10-9 | S79 | |
| S80 | 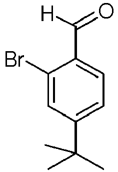 | 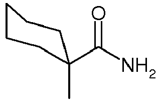 | 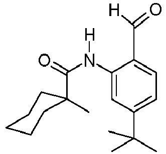 | 91 % |
| | 246139-77-5 | 1123-24-6 | S80 | |
| S81 | 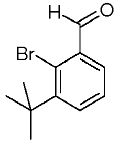 | 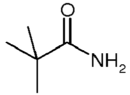 | 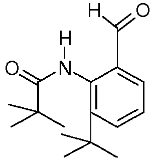 | 21 % |
| | 1289049-94-0 | 754-10-9 | S81 | |
| | | | Chromatographische Reinigung | |
| S82 | 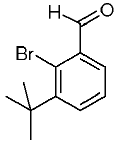 | 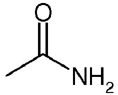 | 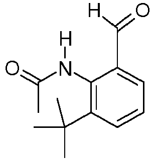 | 27 % |
| | 1289049-94-0 | 60-35-5 | S82 | |
| | | | Chromatographische Reinigung | |
| S83 | 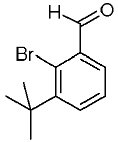 | 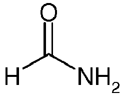 | 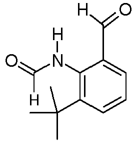 | 33 % |
| | 1289049-94-0 | 75-12-7 | S83 | |
| | | | Chromatographische Reinigung | |
| S84 | 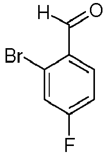 | 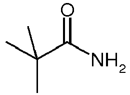 | 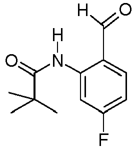 | 93 % |
| | 59142-68-6 | 754-10-9 | S84 | |
| S85 | 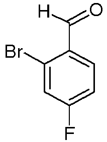 | 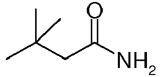 | 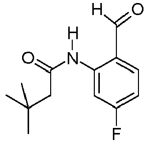 | 95 % |
| | 59142-68-6 | 926-04-5 | S85 | |
| S86 | 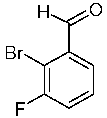 | 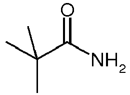 | 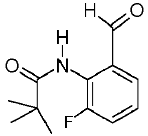 | 73 % |
| | 891180-59-9 | 754-10-9 | S86 | |
| S87 | 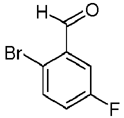 | 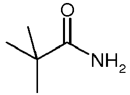 | 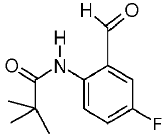 | 87 % |
| | 94569-84-3 | 754-10-9 | S87 | |
| S88 | 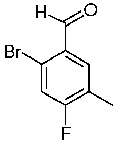 | 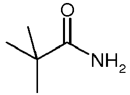 | 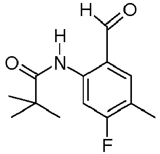 | 90 % |
| | 916792-17-1 | 754-10-9 | S88 | |
| S89 | 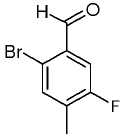 | 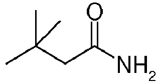 | 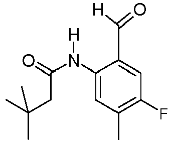 | 90 % |
| | 916792-21-7 | 926-04-5 | S89 | |
| S90 | 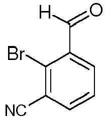 | 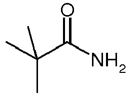 | 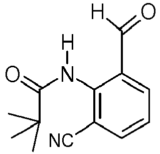 | 67 % |
| | 446864-55-7 | 754-10-9 | S90 | |
| S91 | 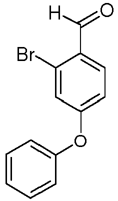 | 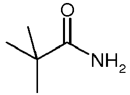 | 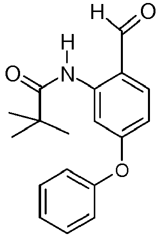 | 90 % |
| | 69240-52-4 | 754-10-9 | S91 | |
| S92 | 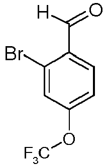 | 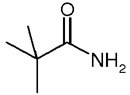 | 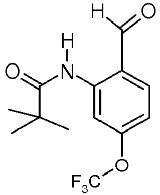 | 92 % |
| | 1114808-87-5 | 754-10-9 | S92 | |
| S93 | 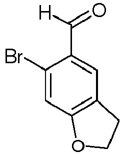 | 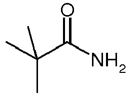 | 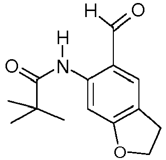 | 88 % |
| | 1221160-68-4 | 754-10-9 | S93 | |
| S94 | 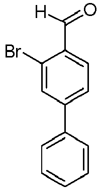 | 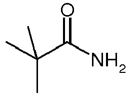 | 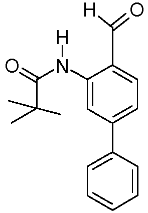 | 94 % |
| | 1237125-81-3 | 754-10-9 | S94 | |
| S95 | 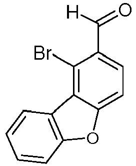 | 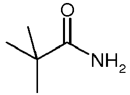 | 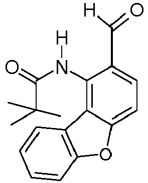 | 93 % |
| | 1062569-66-7 | 754-10-9 | S95 | |
| S96 | 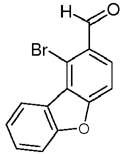 | 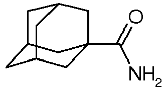 | 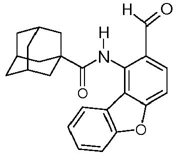 | 96 % |
| | 1062569-66-7 | 5511-18-2 | S96 | |
| S97 | 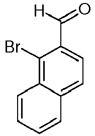 | 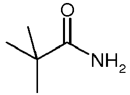 | 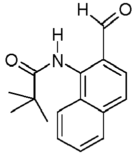 | 89 % |
| | 3378-82-3 | 754-10-9 | S97 | |
| S98 | 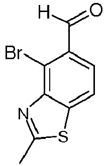 | 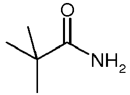 | 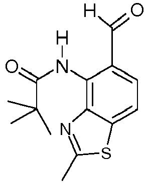 | 63 % |
| | 1312684-59-5 | 754-10-9 | S98 | |
| S99 | 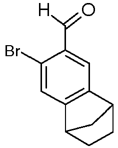 | 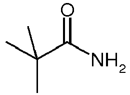 | 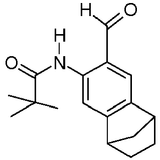 | 90 % |
| | S67 | 754-10-9 | S99 | |
| S100 | 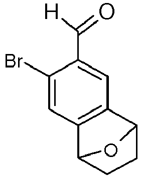 | 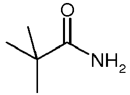 | 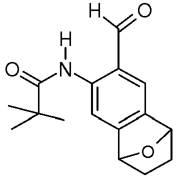 | 92 % |
| | S68 | 754-10-9 | S100 | |
| S101 | 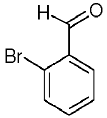 | 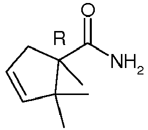 | 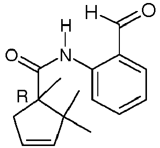 | 90 % |
| | 6630-33-7 | 173411-22-8 | S101 | |
| S102 | 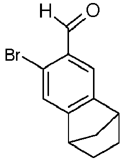 | 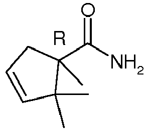 | 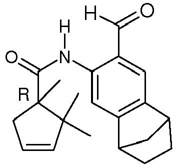 | 83 % |
| | S67 | 173411-22-8 | S102 | |

### Beispiel S103: 7-(3,3-Dimethyl-but-1-ynyl)-1,2,3,4-tetrahydro-1,4-methano-naphthalin-6-carbaldehyd, S103

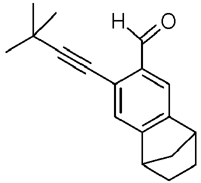

Eine Lösung von 25.1 g (100 mmol) 7-Brom-1,2,3,4-tetrahydro-1,4-methano-naphthalin-6-carbaldehyd, S67 in einem Gemisch aus 200 ml DMF und 100 ml Triethylamin wird konsekutiv mit 1.6 g (6 mmol) Triphenylphosphin, 674 mg (3 mmol) Palladium(II)acetat, 571 mg (30 mmol) Kupfer(I)iodid und 15.6 g (190 mmol) tert-Butylacetylen [917-92-0] versetzt und 4 h bei 65 °C gerührt. Nach Erkalte n wird vom ausgefallenen Triethylammonium-hydrochlorid abgesaugt, dieses wird mit 30 ml DMF nachgewaschen. Das Filtrat wird im Vakuum von den Lösungsmitteln befreit. Der ölige Rückstand wird in 300 ml Ethylacetat aufgenommen, die Lösung wird fünfmal mit je 100 ml Wasser und einmal mit 100 ml gesättigter Kochsalzlösung gewaschen, und die organische Phase wird über Magnesiumsulfat getrocknet. Nach Entfernen des Ethylacetats im Vakuum wird der schwarze ölige Rückstand einer Kugelrohrdestillation unterzogen (p ca. 10⁻⁴ mbar, T = 100 - 120 °C). Ausbeute: 19.2 g (76 mmol), 76 %; Reinheit: > 96 % nach ¹H-NMR.

Analog können folgende Derivate dargestellt werden:

| **Bsp.** | **Bromarylaldeyd** | **Alkin** | **Produkt** | **Ausbeute** |
|---|---|---|---|---|
| S104 | 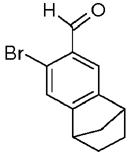 | 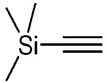 | 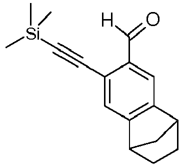 | 61 % |
| | S67 | 1066-54-2 | S104 | |
| S105 | 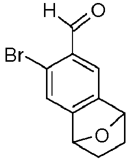 | 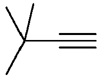 | 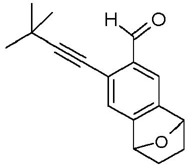 | 82 % |
| | S68 | 917-92-0 | S105 | |
| S106 | 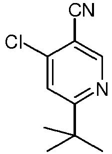 | 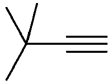 | 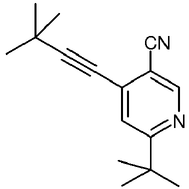 | 78 % |
| | 1352329-26-0 | 917-92-0 | S106 | |
| S107 | 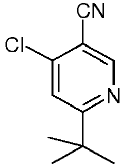 | 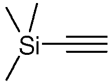 | 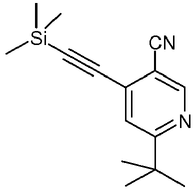 | 54 % |
| | 1352329-26-0 | 1066-54-2 | S107 | |

### Beispiel S108: 2-tert-Butyl-4-(3,3-dimethyl-but-1-ynyl)-pyridin-5-carboxaldehyd, S108

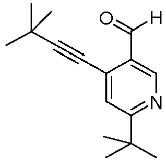

Eine auf -78 °C gekühlte Lösung von 72.1 g (300 mmol) 2-tert-Butyl-4-(3,3-dimethyl-but-1-ynyl)-5-cyano-pyridin, S106 in 1500 ml Dichlormethan wird tropfenweise so mit 315 ml (315 mmol) Di-iso-butylaluminiumhydrid, 1 M in Toluol versetzt, dass die Temperatur -65 °C nicht ü bersteigt. Nach beendeter Zugabe wird weitere 2 h bei -78 °C gerührt, dann lässt man die Reaktionsmischung langsam auf Raumtemperatur erwärmen und rührt 12 h nach. Nach erneutem Abkühlen auf -10 °C setzt man 300 ml THF und dann unter gutem Rühren 230 ml 2 N Schwefelsäure (exotherm!) zu und rührt 12 h bei Raumtemperatur nach. Nach erneuten Abkühlen auf -10 °C gibt man eine Lösung von 70 g NaOH in 300 ml Wasser zu, trennt die wässrige Phase ab, wäscht die organische Phase dreimal mit je 1000 ml Wasser, einmal mit 500 ml gesättigter Kochsalzlösung, trocknet über Magnesiumsulfat und entfernt das Lösungsmittel im Vakuum.
Ausbeute: 69.6 g (286 mmol), 95 %. Reinheit: > 95 % nach ¹H-NMR.

Analog wird S107 zu 2-tert-Butyl-4-(2-trimethylsilyl-eth-1-ynyl)-5-cyano-pyridin, S109 umgesetzt. Ausbeute: 68.7 g (268 mmol), 89 %; Reinheit: > 95 % nach ¹H-NMR.

### Beispiel S110: 2-(2-tert-Butyl-pyrimidin-5-yl)-5,5-7,7-tetramethyl-1,5,6,7-tetrahydro-indeno[5,6-d]imidazol

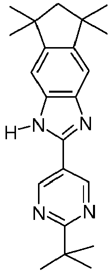

Eine Lösung von 16.4 g (100 mmol) 2-tert-Butyl-pyrimidin-5-carboxaldehyd [104461-06-5] und 22.5 g (110 mmol) 1,1,3,3-Tetramethyl-indan-5,6-diamin, [83721-95-3], S16 in einem Gemisch aus 100 ml DMF und 3 ml Wasser wird bei 10 °C unter Rühren portionsweise mit 16.9 g (55 mmol) Oxone [70693-62-8] versetzt und anschließend bei Raumtemperatur bis zum vollständigen Umsatz des Aldehyds gerührt (ca. 2 h). Man rührt die Reaktionsmischung in eine Lösung von 40 g Kaliumcarbonat-Lösung in 1000 ml Wasser ein, rührt 15 min. nach, saugt vom gebildeten Feststoff ab, wäscht diesen dreimal mit je 100 ml Wasser und trocknet im Vakuum. Das Rohprodukt wird aus Ethylacetat/Cyclohexan umkristallisiert. Ausbeute: 20.2 g (58 mmol), 58 %. Reinheit: > 95 % nach ¹H-NMR.

Analog können folgende Derivate dargestellt werden:

| **Bsp.** | **Pyrimidin-5-catboxaldehyd** | **1,2-Diaminobenzol** | **Produkt** | **Ausbeute** |
|---|---|---|---|---|
| S111 | 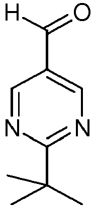 | 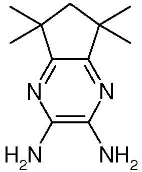 | 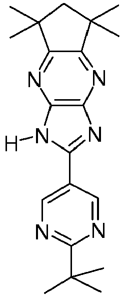 | 31 % |
| | 104461-06-5 | S22 | S111 | |
| S112 | 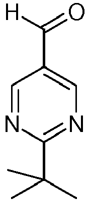 | 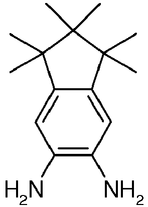 | 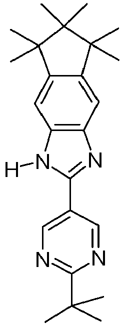 | 48 % |
| | 104461-06-5 | S24 | S112 | |
| S113 | 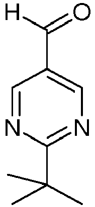 | 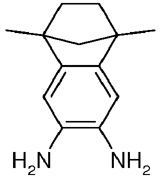 | 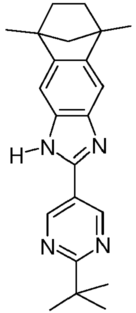 | 45 % |
| | 104461-06-5 | S36 | S113 | |

### Beispiel S114: 2-tert-Butyl-3-(3,3-dimethyl-but-1-inyl)-pyridin-4-carboxaldehyd, S114

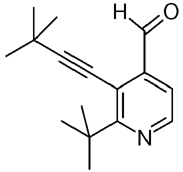

Darstellung analog zu 7-(3,3-Dimethyl-but-1-ynyl)-1,2,3,4-tetrahydro-1,4-methano-naphthalin-6-carbaldehyd, S103. Anstelle von 7-Brom-1,2,3,4-tetrahydro-1,4-methano-naphthalin-6-carbaldehyd, S67 wird 24.2 g (100 mmol) 2-tert-Butyl-3-brom-pyridin-4-carboxaldehyd [1289119-19-2] eingesetzt. Ausbeute: 15.3 g (63 mmol), 63 %. Reinheit: > 95 % nach ¹H-NMR.
Analog werden 24.2 g (100 mmol) 2-tert-Butyl-3-brom-pyridin-4-carboxaldehyd [1289119-19-2] und 18.7 g (190 mmol) Trimethylsilylacetylen [1066-54-2] zu 2-tert-Butyl-3-(2-trimethylsilyl-but-1-inyl)-pyridin-4-carboxaldehyd, S115 umgesetzt. Ausbeute: 14.5 g (56 mmol), 56 %; Reinheit: > 95 % nach ¹H-NMR.

### Beispiel S116: N-(2-tert-Butyl-4-formyl-pyridin-3-yl)-acetamid, S116

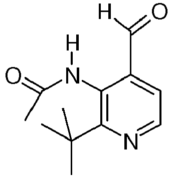

Eine Lösung von 17.8 g (100 mmol) 2-tert-Butyl-3-amino-pyridin-4-yl-carbaldehyd [1289036-95-8] in 100 ml Dioxan wird tropfenweise mit 7.5 ml (105 mmol) Acetylchlorid versetzt. Die Reaktionsmischung wird 30 min. unter Rückfluss erhitzt, nach Erkalten auf 500 ml Eiswasser gegeben und mit Natriumhydrogencarbonat neutral gestellt. Der ausgefallene Feststoff wird abgesaugt, zweimal mit je 50 ml Wasser gewaschen, mit Vakuum getrocknet, und dann einmal aus DMF/EtOH umkristallisiert.
Ausbeute: 18.7 g (85 mmol), 85 %. Reinheit: > 95 % nach ¹H-NMR.

### Beispiel S117: 1,1,3,3-Tetramethyl-2,3-dihydro-1H-cyclopenta[c]iso-chromen-5-on, S117

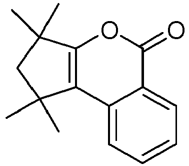

Darstellung analog zu A. C. Tadd et al., Chem. Commun. 2009, 6744. Ein Gemisch aus 14.0 g (100 mmol) 2,2,4,4-Tetramethylcyclopentanon [4694-11-5], 28.3 g (100 mmol) 1-Brom-2-iodbenzol [583-55-1], 97.7 g (300 mmol) Cäsiumcarbonat, 200 g Glaskugeln (3 mm Durchmesser), 2.9 g (5 mmol) Xanthphos, 1.1 g (5 mmol) Palladium(II)acetat und 500 ml Toluol wird 24 h bei 80 °C gerührt. Dann wird ein schwache r Kohlenmonoxidstrom durch die Reaktionsmischung geleitet, und die Temperatur wird auf 110 °C erhöht, so dass sich ein schwacher Rückfluss einstellt. Nach 16 h lässt man die Reaktionsmischung erkalten, saugt von den Salzen über eine Celite-Schicht ab, wäscht diese mit 1000 ml Toluol nach und befreit das Filtrat im Vakuum von Toluol. Der Rückstand wird zweimal aus Ethylacetat / Ethanol umkristallisiert. Ausbeute: 8.7 g (36 mmol) 36 %. Reinheit: ca. 95 % ig nach ¹H-NMR.

### Beispiel S118: 1,1,3,3-Tetramethyl-2,3-dihydro-1H-4-oxa-9-aza-cyclopenta[a]naphthalin-5-on, S118

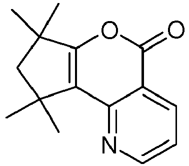

Darstellung analog S117, wobei anstatt 1-Brom-2-iodbenzol 28.4 g (100 mmol) 2-lod-3-brom-pyridin [408502-43-2] eingesetzt werden. Ausbeute: 7.3 g (30 mmol), 30 %. Reinheit: ca. 95 % ig nach ¹H-NMR.

### B: Synthese der Liganden L:

### 1) Liganden vom Benzo[4,5]imidazo[2,1-a]isochinolin-Typ Allgemeine Ligandensynthese Variante A: Aus 1-Chlorisochinolinen und 2-Halogen-anilinen:

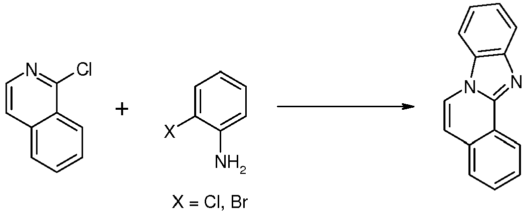

Eine gut gerührte Mischung von 500 mmol des 1-Chlor-isochinolin-Derivats, 600 mmol des 2-Halogen-anilins, 1250 mmol Kaliumcarbonat, 200 g Glaskugeln (3 mm Durchmesser) 10 mmol Triphenylphosphin und 2 mmol Palladium(II)acetat in 1500 ml o-Xylol wird 3 - 48 h unter Rückfluss erhitzt, bis das 1-Chlor-isochinolin-Derivat verbraucht ist. Nach Erkalten wird über ein Celite-Bett abfiltriert, mit 2000 ml THF nachgewaschen und das Filtrat zur Trockene eingeengt. Der Rückstand wird in 100 ml Essigsäureethylester in der Siedehitze gelöst und langsam mit 800 ml n-Heptan oder Cyclohexan versetzt. Nach Erkalten wird vom auskristallisierten FS abgesaugt, dieser wird zweimal mit je 100 ml n-Heptan gewaschen und im Vakuum getrocknet. Nicht kristallisierende Öle werden zur Reinigung chromatographiert. Die so erhaltenen Feststoffe bzw. Öle werden durch fraktionierte Kugelrohrdestillation oder Sublimation (p ca. 10⁻⁴- 10⁻⁵ mbar, T ca. 160 - 240 °C) von Leichtsiedern und nicht flüchtigen Nebenkomponenten befreit. Verbindungen mit aliphatischen Resten, die mehr als 6 C-Atome aufweisen, bzw. solche mit Aralkylgruppen, die mehr als 9 C-Atome aufweisen, werden typischerweise chromatographisch gereinigt und dann im Vakuum getrocknet, um Leichtsieder zu entfernen. Reinheit nach ¹H-NMR typischerweise > 99.5 % ig.

### Beispiel L1: L1

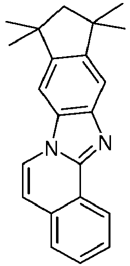

Einsatz von 81.8 g (500 mmol) 1-Chlor-isochinolin [19493-44-8] und 160.9 g (600 mmol) 6-Brom-1,1,3,3-tetramethyl-indan-5-ylamin, S4, Sublimation des Produktes bei T ca. 180 °C, p ca. 10⁻⁴ mbar. Ausbeute: 100.6 g (320 mmol), 64 %; Reinheit: ca. 99.5 % ig nach ¹H-NMR.

Analog können folgende Verbindungen dargestellt werden:

| **Bsp.** | **1-Chlor-isochinolin-Derivat** | **2-Halogenanilin** | **Produkt** | **Ausbeute** |
|---|---|---|---|---|
| L2 | 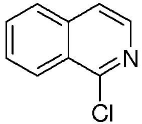 | 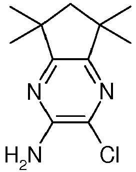 | 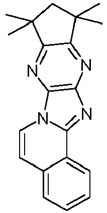 | 58 % |
| | 19493-44-8 | S5 | | |
| L3 | 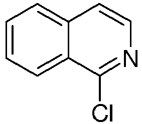 | 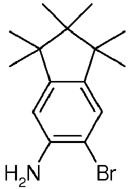 | 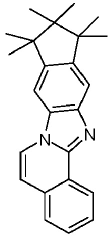 | 68 % |
| | 19493-44-8 | S6 | | |
| L4 | 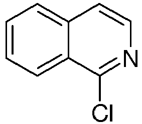 | 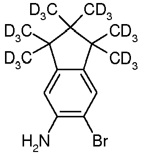 | 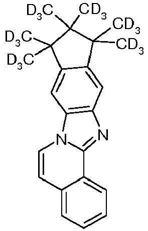 | 69 % |
| | 19493-44-8 | S7 | | |
| L5 | 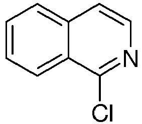 | 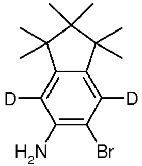 | 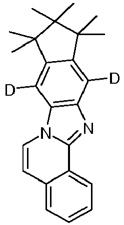 | 68 % |
| | 19493-44-8 | S8 | | |
| L6 | 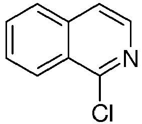 | 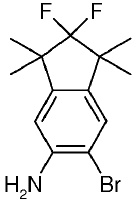 | 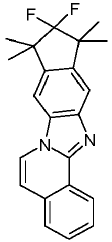 | 56 % |
| | 19493-44-8 | S9 | | |
| L7 | 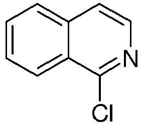 | 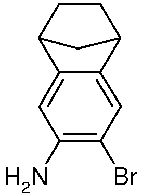 | 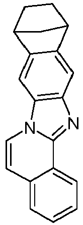 | 69 % |
| | 19493-44-8 | S10 | | |
| L8 | 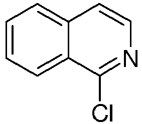 | 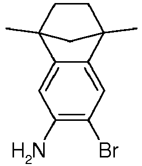 | 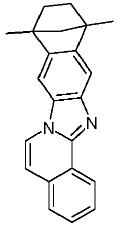 | 64 % |
| | 19493-44-8 | S11 | | |
| L9 | 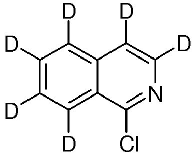 | 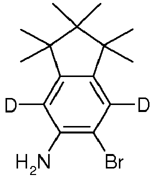 | 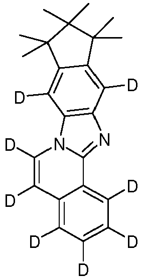 | 64 % |
| | 1003195-34-3 | S8 | | |
| L10 | 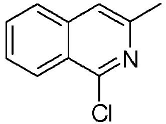 | 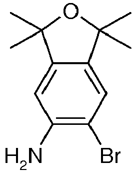 | 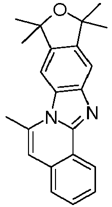 | 59 % |
| | 7115-16-4 | S12 | | |
| L11 | 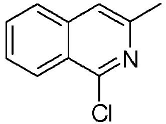 | 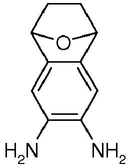 | 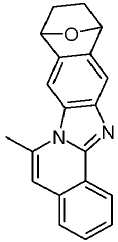 | 61 % |
| | 7115-16-4 | S13 | | |
| L12 | 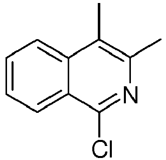 | 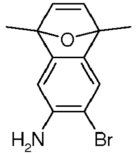 | 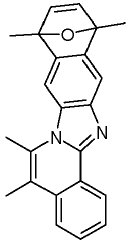 | 34 % |
| | 15787-20-9 | S14 | | |
| L13 | 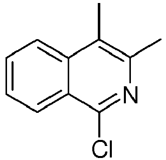 | 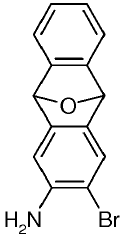 | 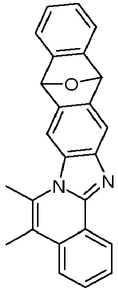 | 67 % |
| | 15787-20-9 | S15 | | |
| L14 | 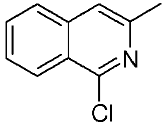 | 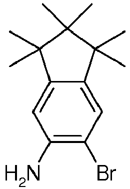 | 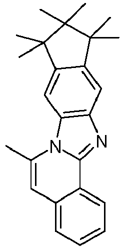 | 65 % |
| | 7115-16-4 | S6 | | |
| L15 | 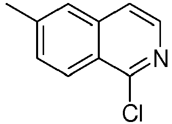 | 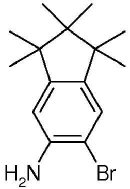 | 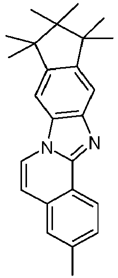 | 60 % |
| | 209286-73-7 | S6 | | |
| L16 | 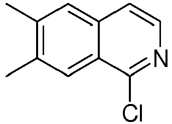 | 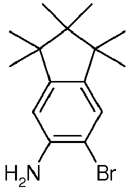 | 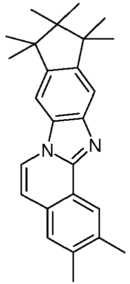 | 61 % |
| | 1368924-44-0 | S6 | | |
| L17 | 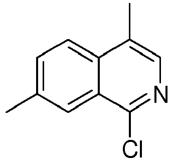 | 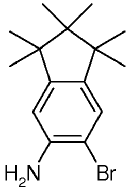 | 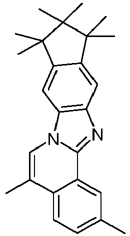 | 65 % |
| | 1206973-73-0 | S6 | | |
| L18 | 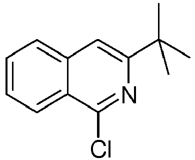 | 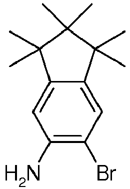 | 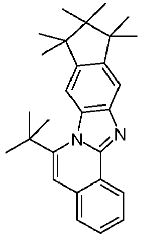 | 67 % |
| | 1198271-38-3 | S6 | | |
| L19 | 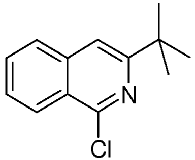 | 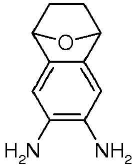 | 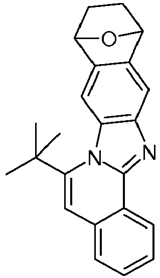 | 59 % |
| | 1198271-38-3 | S13 | | |
| L20 | 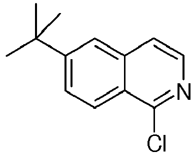 | 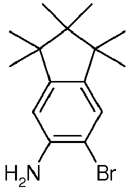 | 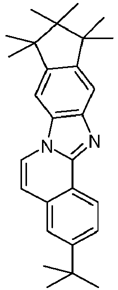 | 60 % |
| | 630422-60-5 | S6 | | |
| L21 | 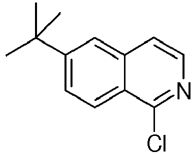 | 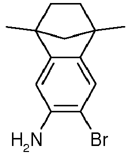 | 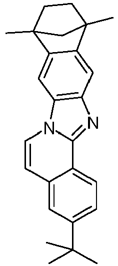 | 63 % |
| | 630422-60-5 | S11 | | |
| L22 | 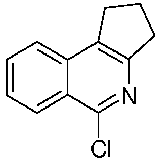 | 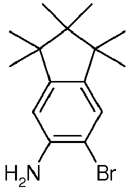 | 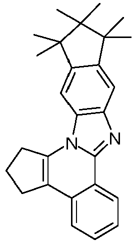 | 43 % |
| | 630419-61-3 | S6 | | |
| L23 | 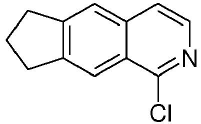 | 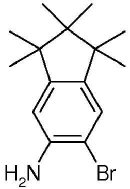 | 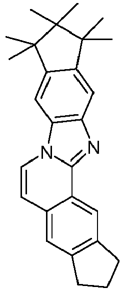 | 38 % |
| | 1368753-27-8 | S6 | | |
| L24 | 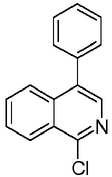 | 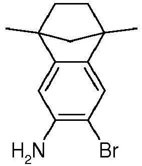 | 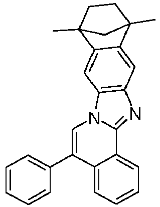 | 55 % |
| | 65810-96-0 | S11 | | |
| L25 | 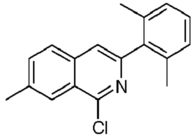 | 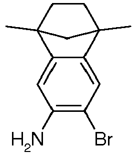 | 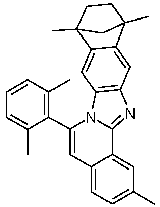 | 65 % |
| | 1248622-18-5 | S11 | | |
| L26 | 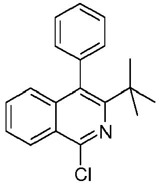 | 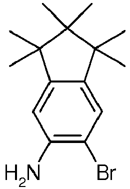 | 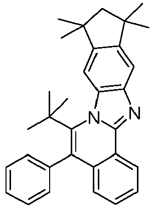 | 63 % |
| | 55792-01-3 | S6 | | |
| L27 | 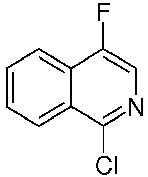 | 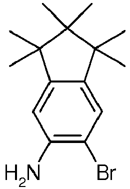 | 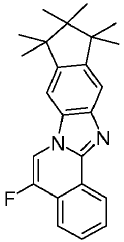 | 64 % |
| | 435278-06-1 | S6 | | |
| L28 | 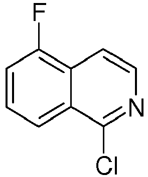 | 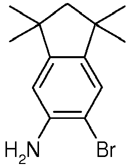 | 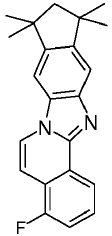 | 66 % |
| | 435278-02-7 | S4 | | |
| L29 | 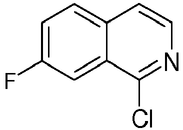 | 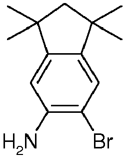 | 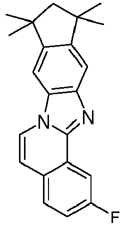 | 67 % |
| | 630422-89-8 | S4 | | |
| L30 | 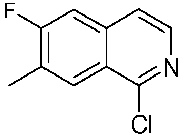 | 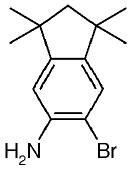 | 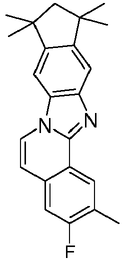 | 64 % |
| | 1369071-11-3 | S4 | | |
| L31 | 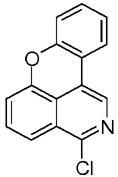 | 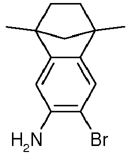 | 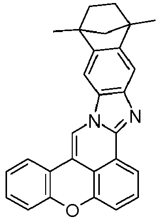 | 70% |
| | 59500-31-2 | S11 | | |
| L32 | 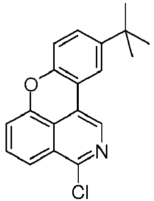 | 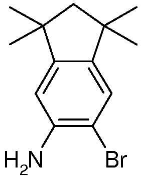 | 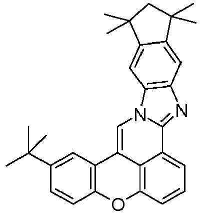 | 69 % |
| | 86761-09-3 | S4 | | |
| L33 | 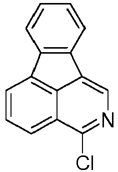 | 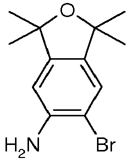 | 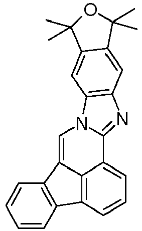 | 54 % |
| | 33279-84-4 | S12 | | |
| L34 | 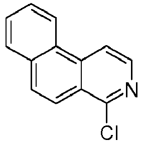 | 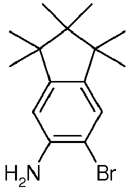 | 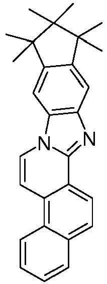 | 67 % |
| | 32081-28-0 | S6 | | |
| L35 | 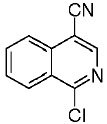 | 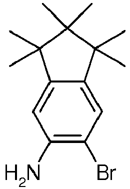 | 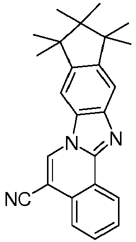 | 45 % |
| | 53491-80-8 | S6 | | |
| L36 | 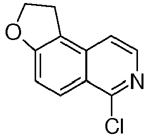 | 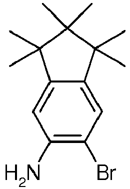 | 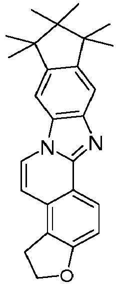 | 31 % |
| | 630423-15-3 | S6 | | |
| L37 | 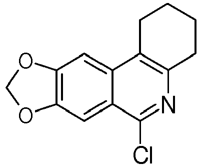 | 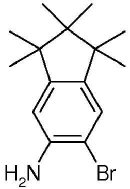 | 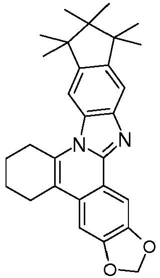 | 27 % |
| | 61877-29-0 | S6 | | |
| L38 | 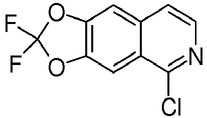 | 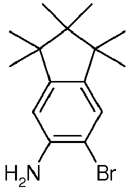 | 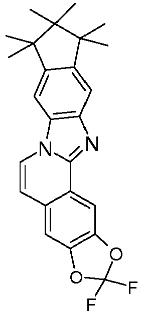 | 42 % |
| | 630423-54-0 | S6 | | |
| L39 | 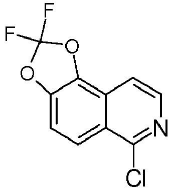 | 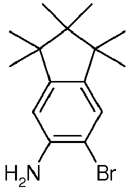 | 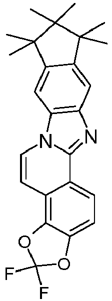 | 37 % |
| | 630423-52-8 | S6 | | |
| L40 | 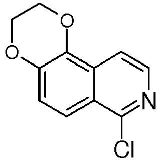 | 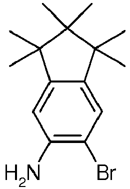 | 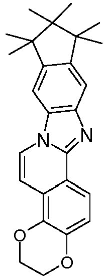 | 57 % |
| | 630423-50-6 | S6 | | |

### Allgemeine Ligandensynthese Variante B: Aus 2-Alkinyl-aryl-aldehyden und 1,2-Diamino-benzolen:

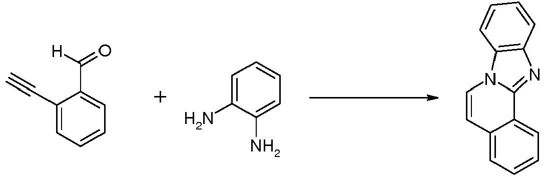

Eine Lösung von 500 mmol des 2-Alkinyl-aryl-aldehyds und 550 mmol des 1,2-Diaminobenzols in 1000 ml Nitrobenzol wird in einer Apparatur bestehend aus einem 2000 ml Einhalskolben mit Hahnstück und aufgesetzter Destillationsbrücke platziert und unter Rühren langsam auf 200 °C (Ölbadtemperatur) erhitzt, wobei das gebildete Wasser abdestilliert. Man rührt 2 h bei 200 °C nach, steigert dann die Temperatur auf ca. 230 °C und destilliert das Nitrobenzol im Argonstrom ab. Gegen Ende der Destillation legt man ein Vakuum von ca. 100 mbar an, um letzte Reste von Nitrobenzol zu entfernen, dann lässt man die Reaktionsmischung erkalten. Fällt das Rohprodukt glasartig an, wird das Glas mechanisch zerkleinert, Öle versetzt man direkt mit 200 - 400 ml Methanol oder Acetonitril und erhitzt die Mischung unter Rückfluss, wobei sich das Glas bzw. das Öl löst und das Produkt auskristallisiert. Die so erhaltenen Rohprodukte weisen schon eine hohe Reinheit auf (¹H-NMR typischerweise 97 - 99 %ig). Gegebenenfalls werden sie erneut umkristallisiert und dann durch fraktionierte Kugelrohrdestillation oder Sublimation (p ca. 10⁻⁴ - 10 ⁻⁵ mbar, T ca. 160 - 240 °C) von Leichtsiedern und nicht flüchtigen Nebenkomponenten befreit. Verbindungen mit aliphatischen Resten, die mehr als 6 C-Atome aufweisen, bzw. solche mit Aralkylgruppen, die mehr als 9 C-Atome aufweisen, werden typischerweise chromatographisch gereinigt und dann im Vakuum getrocknet, um Leichtsieder zu entfernen. Reinheit nach ¹H-NMR typischerweise > 99.5 % ig.

### Beispiel L41:

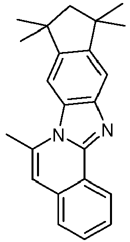

Einsatz von 72.1 g (500 mmol) 2-(1-Propin-1-yl)-benzaldehyd [176910-65-9] und 112.4 g (550 mmol) 1,1,3,3-Tetramethyl-indan-5,6-diamin [83721-95-3], S16, Aufnehmen des Rohprodukts in Acetonitril, Umkristallisation aus Ethylacetat/Methanol, Sublimation des Produktes bei T ca. 210 °C, p ca. 10 ⁻⁴ mbar. Ausbeute: 99.9 g (304 mmol), 61 %; Reinheit: ca. 99.5 % ig nach ¹H-NMR.

Analog können folgende Verbindungen dargestellt werden:

| **Bsp.** | **2-Alkinyl-arylaldehyd** | **1,2-Diaminobenzol** | **Produkt** | **Ausbeute** |
|---|---|---|---|---|
| L42 | 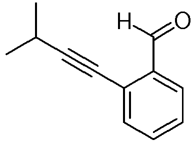 | 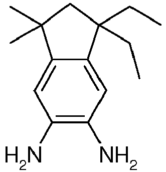 | 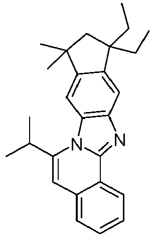 | 54 % |
| | 220649-62-7 | S17 | Chromatographische Abtrennung des Regioisomeren | |
| L43 | 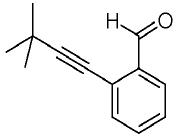 | 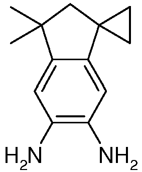 | 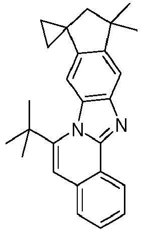 | 55 % |
| | 183312-34-7 | S18 | Chromatographische Abtrennung des Regioisomeren | |
| L44 | 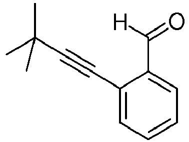 | 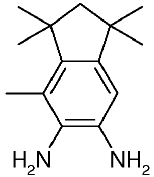 | 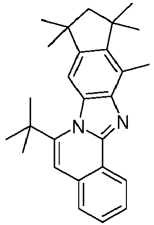 | 62 % |
| | 183312-34-7 | S21 | Chromatographische Abtrennung des Regioisomeren | |
| L45 | 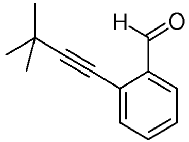 | 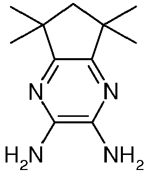 | 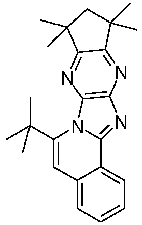 | 46 % |
| | 183312-34-7 | S22 | | |
| L46 | 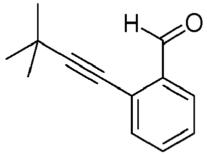 | 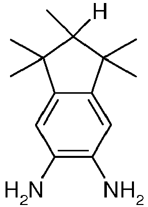 | 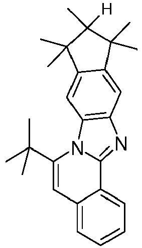 | 48 % |
| | 183312-34-7 | S23 | | |
| L47 | 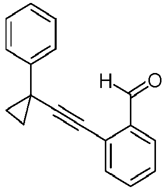 | 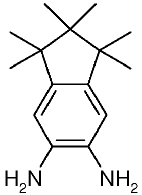 | 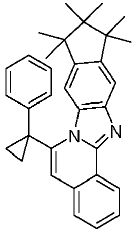 | 40 % |
| | 1189356-64-6 | S24 | | |
| L48 | 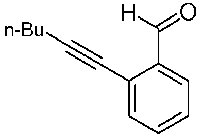 | 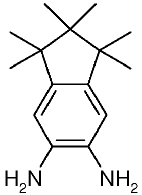 | 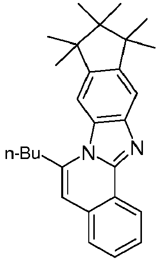 | 51 % |
| | 872471-05-1 | S24 | | |
| L49 | 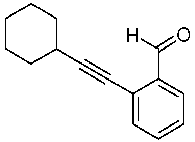 | 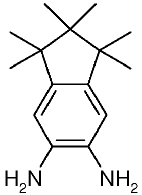 | 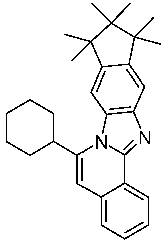 | 43 % |
| | 396717-17-2 | S24 | | |
| L50 | 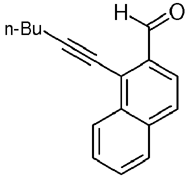 | 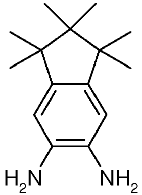 | 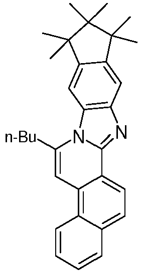 | 52 % |
| | 1199262-45-7 | S24 | | |
| L51 | 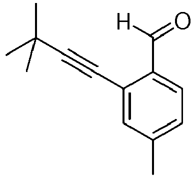 | 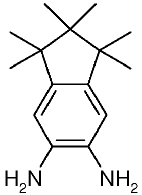 | 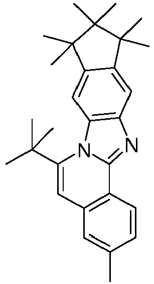 | 59 % |
| | 1378013-34-3 | S24 | | |
| L52 | 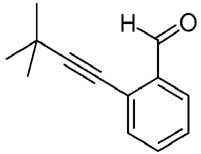 | 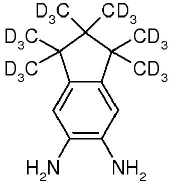 | 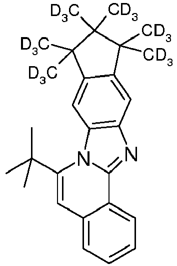 | 60 % |
| | 183312-34-7 | S25 | | |
| L53 | 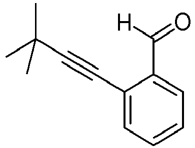 | 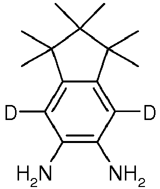 | 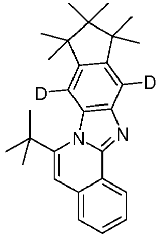 | 60 % |
| | 183312-34-7 | S26 | | |
| L54 | 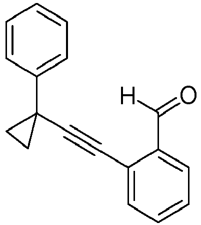 | 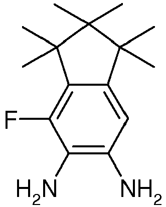 | 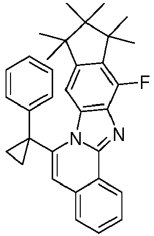 | 38 % |
| | 1189356-64-6 | S27 | Chromatographische Abtrennung des Regioisomeren | |
| L55 | 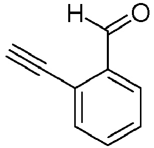 | 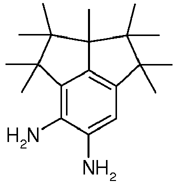 | 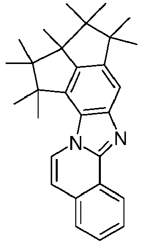 | 27% |
| | 38846-64-9 | S28 | Chromatographische Abtrennung des Regioisomeren | |
| L56 | 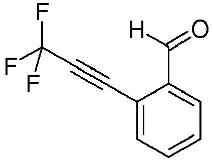 | 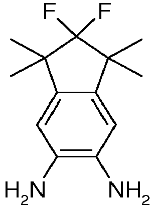 | 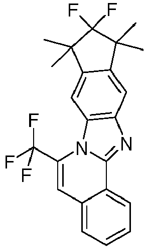 | 58 % |
| | 275386-61-3 | S29 | | |
| L57 | 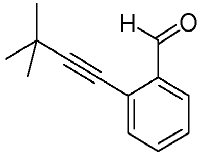 | 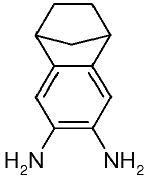 | 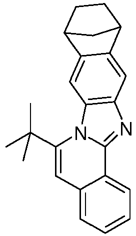 | 56 % |
| | 183312-34-7 | 124639-03-8 S32 | | |
| L58 | 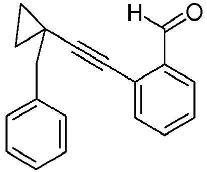 | 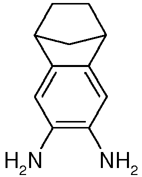 | 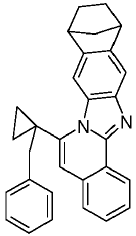 | 43 % |
| | 1189356-65-7 | 124639-03-8 S32 | | |
| L59 | 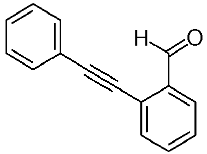 | 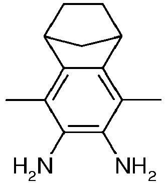 | 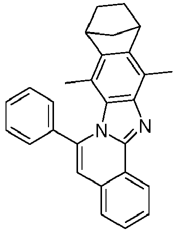 | 54 % |
| | 59046-72-9 | S34 | | |
| L60 | 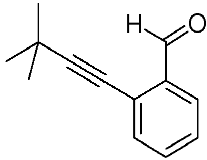 | 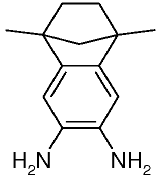 | 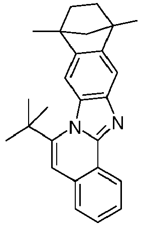 | 55 % |
| | 183312-34-7 | S36 | | |
| L61 | 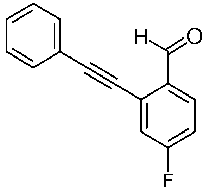 | 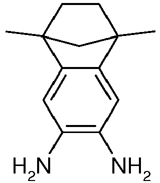 | 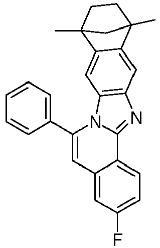 | 51 % |
| | 1189207-30-4 | S36 | | |
| L62 | 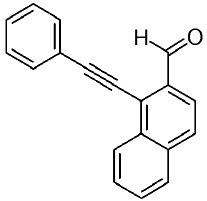 | 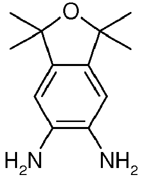 | 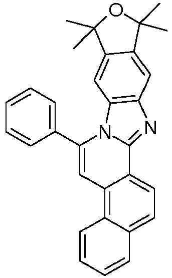 | 57 % |
| | 712278-61-0 | S39 | | |
| L63 | 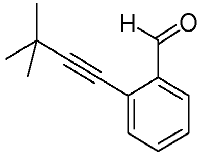 | 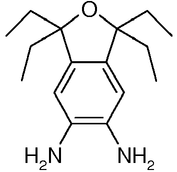 | 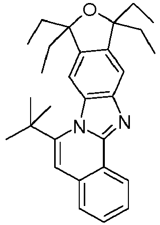 | 49 % |
| | 183312-34-7 | S40 | | |
| L64 | 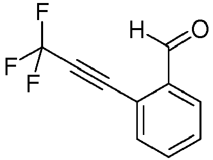 | 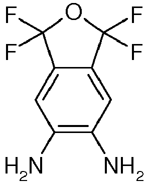 | 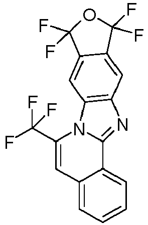 | 40 % |
| | 275386-61-3 | S43 | | |
| L65 | 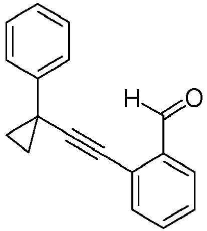 | 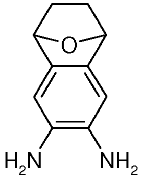 | 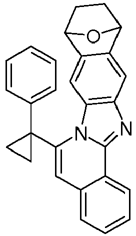 | 38 % |
| | 1189356-64-6 | S44 | | |
| L66 | 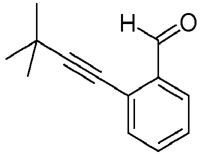 | 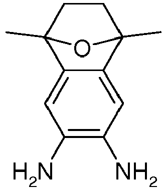 | 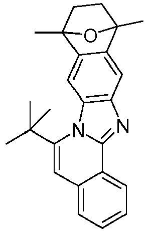 | 52 % |
| | 183312-34-7 | S46 | | |
| L67 | 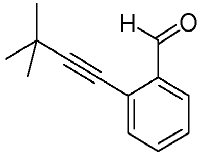 | 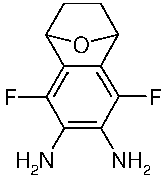 | 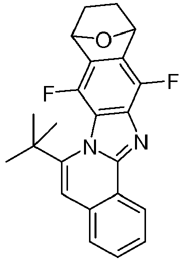 | 53 % |
| | 183312-34-7 | S47 | | |
| L68 | 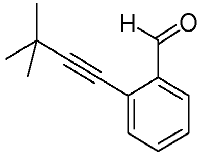 | 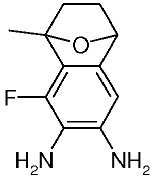 | 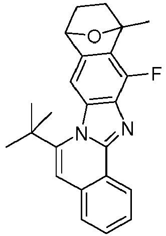 | 26 % |
| | 183312-34-7 | S48 | Chromatographische Abtrennung des Regioisomeren | |
| L69 | 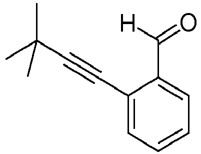 | 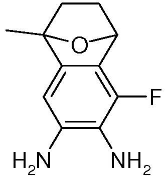 | 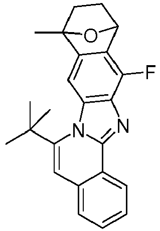 | 28 % |
| | 183312-34-7 | S49 | Chromatographische Abtrennung des Regioisomeren | |
| L70 | 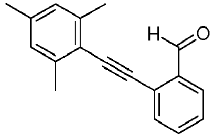 | 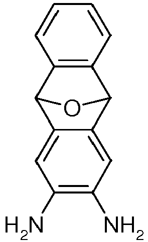 | 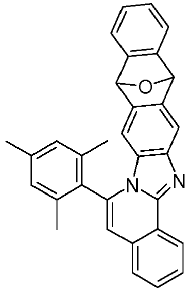 | 61 % |
| | 1338698-45-5 | S53 | | |
| L71 | 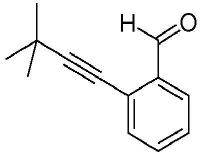 | 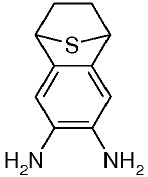 | 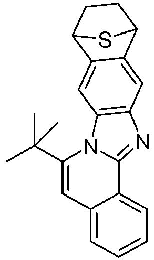 | 46 % |
| | 183312-34-7 | S54 | | |
| L72 | 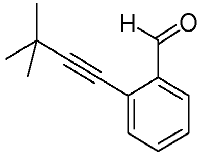 | 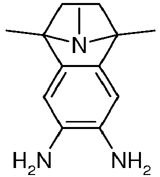 | 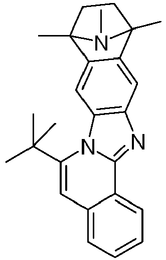 | 20 % |
| | 183312-34-7 | S56 | | |
| L73 | 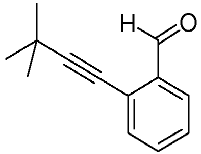 | 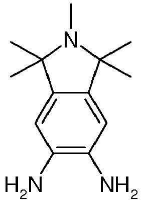 | 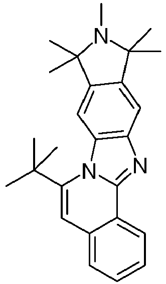 | 23 % |
| | 183312-34-7 | S58 | | |
| L74 | 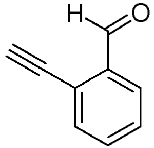 | 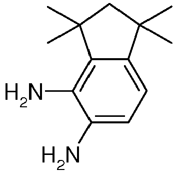 | 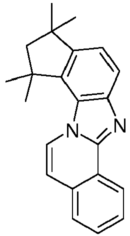 | 21 % |
| | 38846-64-9 | S59 | Chromatographische Abtrennung des Regioisomeren | |
| L75 | 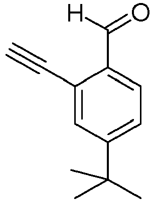 | 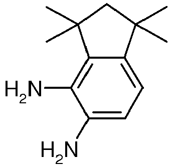 | 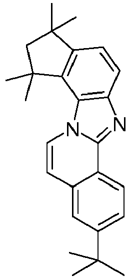 | 23 % |
| | 1010447-02-5 | S59 | Chromatographische Abtrennung des Regioisomeren | |
| L76 | 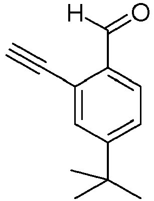 | 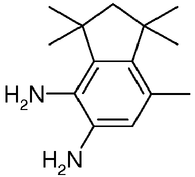 | 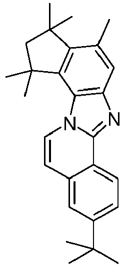 | 26 % |
| | 1010447-02-5 | S60 | Chromatographische Abtrennung des Regioisomeren | |
| L77 | 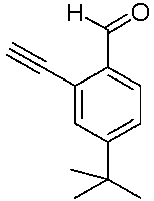 | 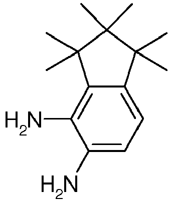 | 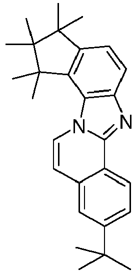 | 20 % |
| | 1010447-02-5 | S61 | Chromatographische Abtrennung des Regioisomeren | |
| L78 | 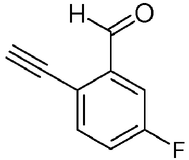 | 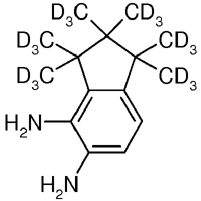 | 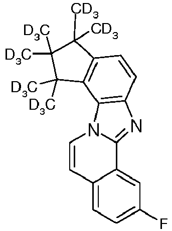 | 18 % |
| | 749874-24-6 | S62 | Chromatographische Abtrennung des Regioisomeren | |
| L79 | 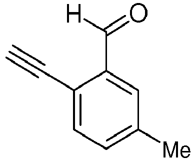 | 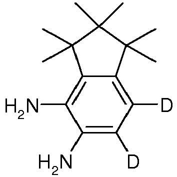 | 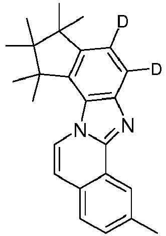 | 20 % |
| | 1309565-96-5 | S63 | Chromatographische Abtrennung des Regioisomeren | |
| L80 | 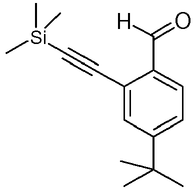 | 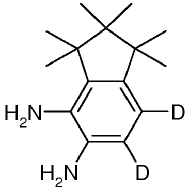 | 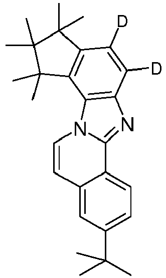 | 22 % |
| | 1010446-99-7 | S63 | Chromatographische Abtrennung des Regioisomeren | |
| L81 | 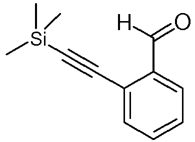 | 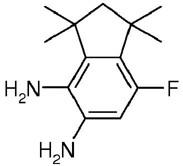 | 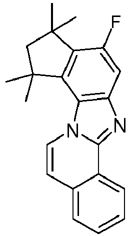 | 24 % |
| | 1010446-99-7 | S64 | Chromatographische Abtrennung des Regioisomeren | |
| L82 | 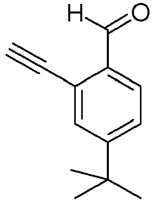 | 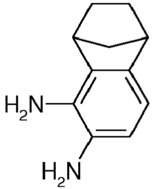 | 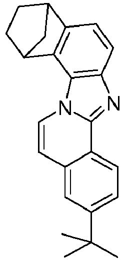 | 23 % |
| | 1010447-02-5 | S65 | Chromatographische Abtrennung des Regioisomeren | |
| L83 | 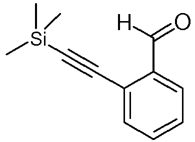 | 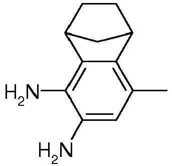 | 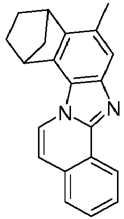 | 24 % |
| | 1010446-99-7 | S66 | Chromatographische Abtrennung des Regioisomeren | |
| L84 | 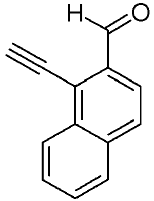 | 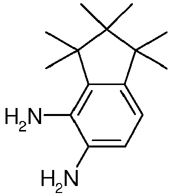 | 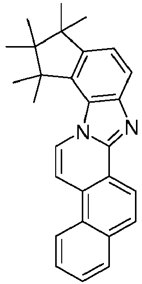 | 26 % |
| | 1010447-02-5 | S61 | Chromatographische Abtrennung des Regioisomeren | |
| L85 | 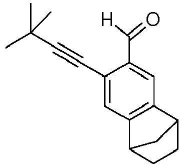 | 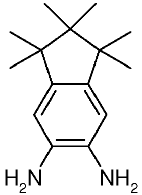 | 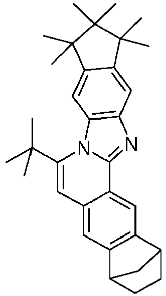 | 58 % |
| | S103 | S24 | | |
| L86 | 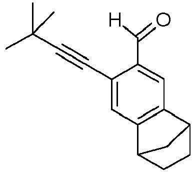 | 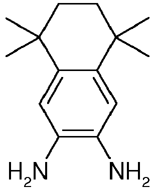 | 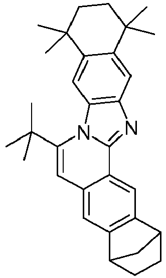 | 54 % |
| | S103 | 81864-05-3 | | |
| L87 | 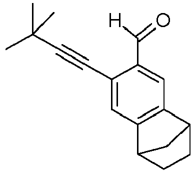 | 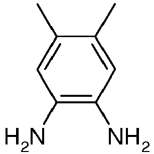 | 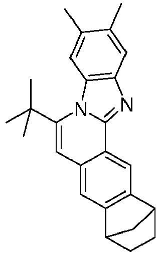 | 59 % |
| | S103 | 3171-45-7 | | |
| L88 | 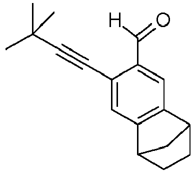 | 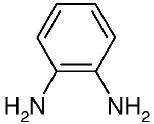 | 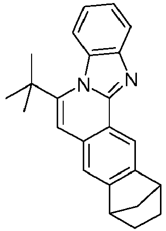 | 59 % |
| | S103 | 95-54-5 | | |
| L89 | 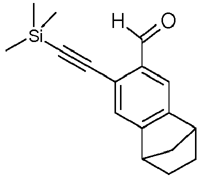 | 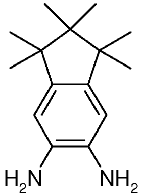 | 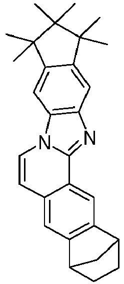 | 56 % |
| | S104 | S24 | | |
| L90 | 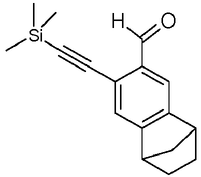 | 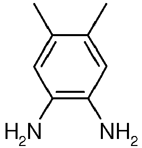 | 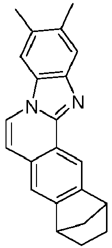 | 61 % |
| | S104 | 3171-45-7 | | |
| L91 | 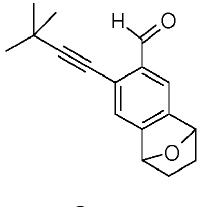 | 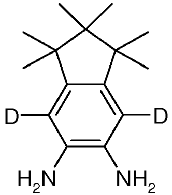 | 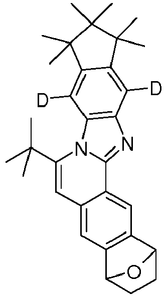 | 60 % |
| | S105 | S26 | | |
| L92 | 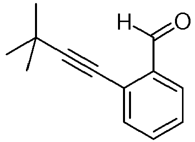 | 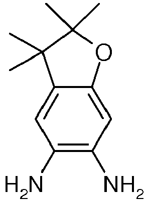 | 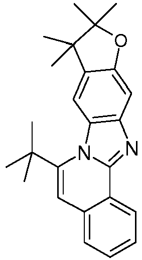 | 23 % |
| | 183312-34-7 | S37 | Chromatographische Abtrennung des Regioisomeren | |
| L93 | 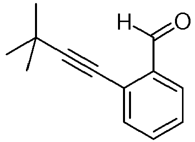 | 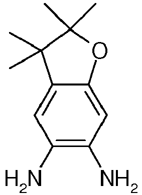 | 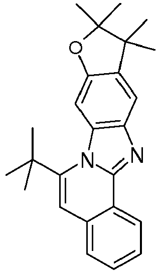 | 20 % |
| | 183312-34-7 | S37 | Chromatographische Abtrennung des Regioisomeren | |
| L94 | 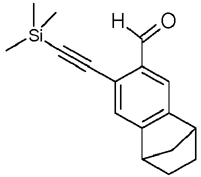 | 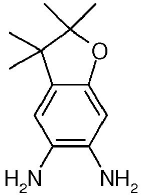 | 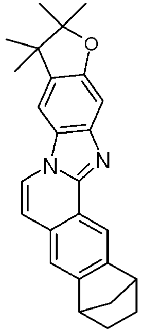 | 24 % |
| | S104 | S37 | Chromatographische Abtrennung des Regioisomeren | |
| L95 | 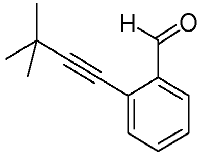 | 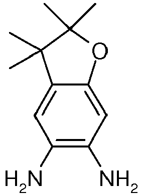 | 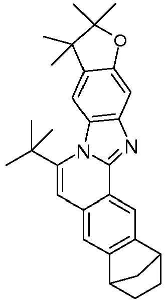 | 23 % |
| | 183312-34-7 | S37 | Chromatographische Abtrennung des Regioisomeren | |

### Allgemeine Ligandensynthese Variante C:

### Aus 1-Amino-isochinolinen und 1,2-Dihalogenbenzolen:

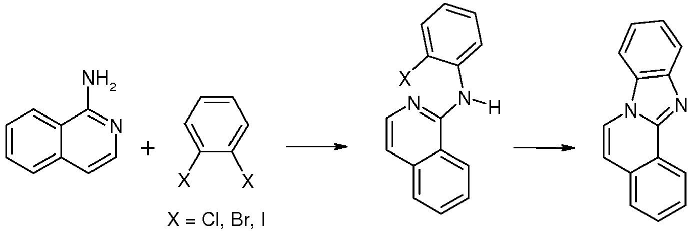

Eine gut gerührte Mischung von 100 mmol des 1,2-Dihalogenbenzols, 120 mmol 1-Amino-isochinolin, 300 mmol Lithium-bis(trimethylsilyl)amid, 100 g Glaskugeln (3 mm Durchmesser), 5 mmol Xanthphos und 5 mmol Palladium(II)acetat in 600 ml trockenem 1,4-Dioxan wird 72 h unter Rückfluss erhitzt, bis das 1-Amino-isochinolin verbraucht ist. Zur Zyklisierung des sekundären Amins werden nach Erkalten der Reaktionsmischung 10 mmol Kupfer(I)iodid, 20 mmol N,N'-Ethylendiamin und 230 mmol Kaliumcarbonat zugegeben. Das Reaktionsgemisch wird erneut für 2-4 Stunden refluxiert, bis das sekundäre Amin verbraucht ist. Nach Erkalten wird über ein Celite-Bett abfiltriert, mit 1500 ml Dioxan nachgewaschen und das Filtrat zur Trockene eingeengt. Der Rückstand wird in 200 ml Dichlormethan gelöst und über ein Kieselgelbett filtriert. Es wird mit einem Gemisch aus 2000 ml Dichlormethan und 150 ml Ethylacetat nachgewaschen und das Filtrat zur Trockene eingeengt. Der Rückstand wird umkristallisiert und im Vakuum getrocknet. Das so erhaltene Benzo[4,5]-imidazo[2,1-a]isochinolin wird durch Kugelrohrdestillation oder fraktionierte Sublimation (p ca. 10⁻⁴ - 10 ⁻⁵ mbar, T ca. 160 - 240 °C) von Leichtsiedern und nicht flüchtigen Nebenkomponenten befreit. Verbindungen mit aliphatischen Resten, die mehr als 6 C-Atome aufweisen, bzw. solche mit Aralkylgruppen, die mehr als 9 C-Atome aufweisen, werden typischerweise chromatographisch gereinigt und dann im Vakuum getrocknet, um Leichtsieder zu entfernen. Reinheit nach ¹H-NMR typischerweise > 99.5 % ig.

### Beispiel L3:

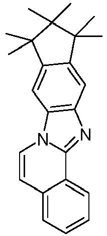

Einsatz von 36.0 g (100 mmol) 5,6-Dibromo-1,1,2,2,3,3-hexamethylindan, S24, Variante A, Stufe A, 17.4 g (120 mmol) 1-Amino-isochinolin [1532-84-9], 50.2 g (300 mmol) Lithium bis(trimethylsilyl)amid, 2.9 g (5 mmol) Xanthphos, 1.1 g (5 mmol) Palladium(II)acetat und dann 1.9 g (10 mmol) Kupfer(I)iodid, 1.75 g (20 mmol) N,N'-Ethylendiamin und 31.8 g (230 mmol) Kaliumcarbonat. Das Rohprodukt wird aus Cyclohexan (ca. 15 ml/g) umkristallisiert und im Vakuum sublimiert (p = 10⁻⁵ mbar, T = 220 °C). Ausbeute: 20.9 g (61 mmol), 61 %; Reinheit: ca. 99.5 % ig nach ¹H-NMR.

Analog werden folgende Verbindungen dargestellt:

| **Bsp.** | **1-Amino-isochinolin-Derivat** | **1,2-Dihalogenbenzol** | **Produkt** | **Ausbeute** |
|---|---|---|---|---|
| L4 | 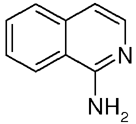 | 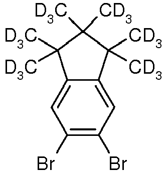 | 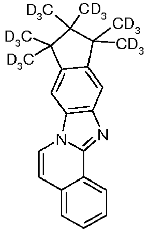 | 63 % |
| | 1532-84-9 | S25 Variante A, Stufe A | | |
| L7 | 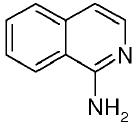 | 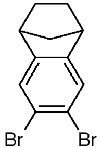 | 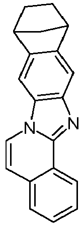 | 66 % |
| | 1532-84-9 | 42810-32-2 | | |

### Allgemeine Ligandensynthese Variante D:

### Aus Isocumarinen und 1,2-Diamino-benzolen:

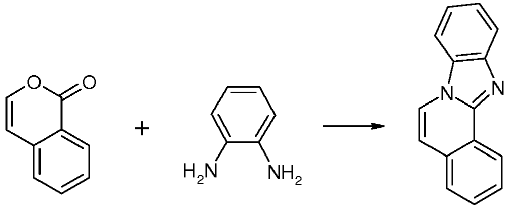

Darstellung analog V. K. Pandey et al., Ind. J. Chem., Section B: 1999, 38B(12), 138.
Ein Gemisch aus 100 mmol des Isocumarin-Derivats, 110 mmol des 1,2-Diaminobenzols, 5 mmol 4-(N,N-Dimethylamino)pyridin und 200 ml trockenem Pyridin werden am Wasserabscheider gekocht, wobei von Zeit zu Zeit Pyridin abgelassen wird, bis das Pyridin weitgehend abdestilliert ist. Gegen Ende legt man ein schwaches Vakuum an, um Pyridinreste zu entfernen. Nach Erkalten wird der zähe bis glasartige Rückstand in 200 ml Methanol aufgenommen und in der Hitze gelöst, wobei das Produkt zu kristallisieren beginnt. Nach Erkalten saugt man vom Produkt ab und wäscht mit wenig Methanol. Nach Umkristallisation (Methanol, Ethanol, Aceton, Dioxan, DMF, etc.) des so erhaltenen Benzo[4,5]imidazo[2,1-a]-isochinolins wird dieses durch Kugelrohrdestillation oder fraktionierte Sublimation (p ca. 10⁻⁴ - 10 ⁻⁵ mbar, T ca. 160 - 240 °C) von Leichtsiedern und nicht flüchtigen Nebenkomponenten befreit. Verbindungen mit aliphatischen Resten, die mehr als 6 C-Atome aufweisen, bzw. solche mit Aralkylgruppen, die mehr als 9 C-Atome aufweisen, werden typischerweise chromatographisch gereinigt und dann im Vakuum getrocknet, um Leichtsieder zu entfernen. Reinheit nach ¹H-NMR typischerweise > 99.5 % ig.

### Beispiel L276:

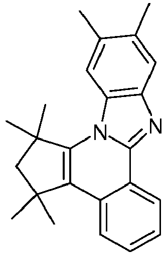

Einsatz von 24.4 g (100 mmol) 1,1,3,3-Tetramethyl-2,3-dihydro-1H-cyclopenta[c]iso-chromen-5-on, S117, 15.0 g (110 mmol) 4,5-Dimethyl-1,2-diaminobenzol [3171-45-7], 611 mg (5 mmol) 4-(N,N-Dimethyl-amino)pyridin. Das Rohprodukt wird aus Cyclohexan (ca. 15 ml/g) umkristallisiert und im Vakuum sublimiert (p = 10⁻⁵ mbar, T = 220 °C). Ausbeute: 16.4 g (48 mmol), 48 %; Reinheit: ca. 99.5 % ig nach ¹H-NMR.

Analog werden folgende Verbindungen dargestellt:

| **Bsp.** | **Isocumarin** | **1,2-Diaminobenzol** | **Produkt** | **Ausbeute** |
|---|---|---|---|---|
| L277 | 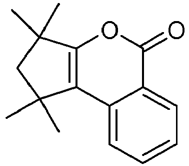 | 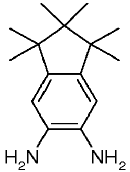 | 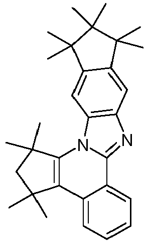 | 45 % |
| | S117 | S24 | | |
| L278 | 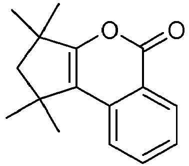 | 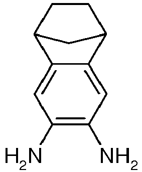 | 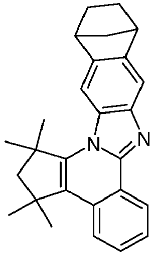 | 43 % |
| | S117 | 124639-03-8 S32 | | |

### 2) Liganden vom Benzo[4,5]imidazo[2,1-c]chinazolin-Typ Allgemeine Ligandensynthese Variante A:

### Aus 2-Amido-aryl-aldehyden und 1,2-Diamino-benzolen:

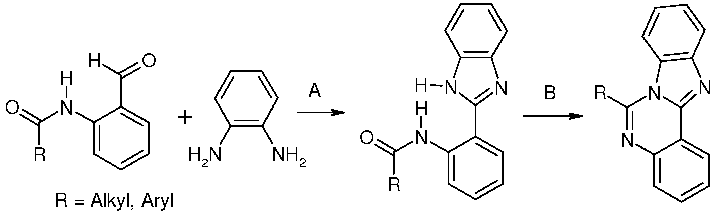

### Schritt A:

Eine Lösung von 100 mmol des 2-Amido-arylaldehyds und 110 mmol des 1,2-Diaminobenzols in 70 ml Ethanol wird in einem 500 ml Rundkolben mit Wasserabscheider platziert und 30 min. bei 50 °C ge rührt. Dann gibt man 70 ml Nitrobenzol zu und steigert die Temperatur schrittweise bis zum schwachen Rückfluss des Nitrobenzols, wobei man beim Hochheizen das Ethanol und gebildetes Wasser abdestilliert. Nach 4 h unter schwachem Rückfluss lässt man auf 50 °C erkalten, gibt 40 ml Methanol zu, lässt dann unter Rühren ganz erkalten, rührt 2 h bei Raumtemperatur nach, saugt dann von den gebildeten Kristallen des 2-(2-Amido-phenyl)-benzimidazols ab, wäscht diese zweimal mit je 20 ml Methanol und trocknet im Vakuum. Falls das 2-(2-Amido-phenyl)-benzimidazol nicht auskristallisiert, entfernt man das Lösungsmittel im Vakuum und setzt den Rückstand in Schritt B ein.

### Schritt B:

### Variante A:

Eine gut gerührte Mischung (KPG-Rührer) aus 100 mmol des 2-(2-Amido-phenyl)-benzimidazols und 150 ml Dioxan oder Diethylenglykoldimethylether wird mit 350 mmol des entsprechenden Carbonsäurechlorids und 50 mmol der entsprechenden Carbonsäure versetzt und so lange (typischerweise 4 - 48 h) unter Rückfluss erhitzt, bis das 2-(2-Amido-phenyl)-benzimidazol umgesetzt ist. Entsprechende Carbonsäurechloride und Carbonsäuren sind die, die den jeweiligen Amidrest bilden.
Nach Erkalten wird die Reaktionsmischung unter gutem Rühren in ein Gemisch aus 1000 g Eis und 300 ml wässrigem konz. Ammoniak eingerührt. Fällt das Produkt als Feststoff an, wird dieser abgesaugt, mit Wasser gewaschen und trockengesaugt. Fällt das Produkt als Öl an, wird dieses mit drei Portionen zu je 300 ml Ethylacetat oder Dichlormethan extrahiert. Die organische Phase wird abgetrennt, mit 500 ml Wasser gewaschen und im Vakuum eingeengt. Das Rohprodukt wird in Ethylacetat oder Dichlormethan aufgenommen, über eine kurze Säule aus Alox, basisch, Aktivitätsstufe 1 oder Kieselgel filtriert, um braune Verunreinigungen zu entfernen. Nach Umkristallisation (Methanol, Ethanol, Aceton, Dioxan, DMF, etc.) des so erhaltenen Benzo[4,5]-imidazo[2,1-c]-chinazolins wird dieses, durch Kugelrohrdestillation oder fraktionierte Sublimation (p ca. 10⁻⁴ - 10⁻⁵ mbar, T ca. 160 - 240 °C) von Leichtsiedern und nicht flüchtigen Nebenkomponenten befreit. Verbindungen mit aliphatischen Resten, die mehr als 6 C-Atome aufweisen, bzw. solche mit Aralkylgruppen, die mehr als 9 C-Atome aufweisen, werden typischerweise chromatographisch gereinigt und dann im Vakuum getrocknet, um Leichtsieder zu entfernen. Reinheit nach ¹H-NMR typischerweise > 99.5 % ig.

### Variante B:

Analoge Durchführung zu Variante A, jedoch werden anstatt der Carbonsäure 50 mmol Wasser zugesetzt.

### Variante C:

Analoge Durchführung zu Variante A, jedoch wird keine Carbonsäure zugesetzt.

### Beispiel L96:

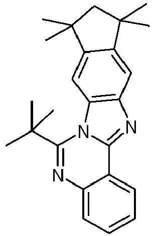

### Schritt A:

Einsatz von 20.5 g (100 mmol) S69 und 22.5 g (110 mmol) S16.
Das 2,2-Dimethyl-N-[2-(5,5,7,7-tetramethyl-1,5,6,6-tetrahydro-indeno[5,6-d]imidazol-2-yl)-phenyl]-propionamid kristallisiert aus, Ausbeute 31.6 g (81 mmol) 81 %; Reinheit: 97 % nach ¹H-NMR.

### Schritt B, Variante A:

Einsatz von 31.6 g (81 mmol) 2,2-Dimethyl-N-[2-(5,5,7,7-tetramethyl-1,5,6,6-tetrahydro-indeno[5,6-d]imidazol-2-yl)-phenyl]-propionamid (Schritt A), 120 ml Dioxan, 33.8 g (280 mmol) Pivalinsäurechlorid [3282-30-2] und 4.1 g (40 mmol) Pivalinsäure [75-98-9], Reaktionszeit 16 h, das Rohprodukt fällt beim Neutralisieren als Feststoff an, Umkristallisation aus DMF/Ethanol, zweimalige fraktionierte Sublimation des Produktes bei T ca. 170 °C, p ca. 10⁻⁴ mbar. Ausbeute: 19.3 g (52 mmol), 64 %; Reinheit: ca. 99.5 % ig nach ¹H-NMR.

### Allgemeine Ligandensynthese Variante B:

### Aus 2-Formamido-aryl-aldehyden und 1,2-Diamino-benzolen:

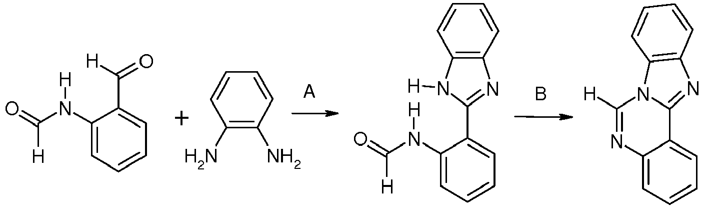

### Schritt A:

Analog zu Schritt A der Umsetzung von 2-Amido-aryl-aldehyden und 1,2 Diamino-benzolen.

### Schritt B:

100 mmol des 2-(2-Formamido-phenyl)-benzimidazols wird in 100 ml Dioxan suspendiert. Nach Zugabe von 1 ml Pyridin wird die Reaktionsmischung tropfenweise mit 500 mmol Thionylchlorid versetzt und bis zum vollständigen Umsatz (typischerweise 24 h) bei Raumtemperatur gerührt. Die weitere Aufarbeitung erfolgt wie unter Schritt B der Umsetzung von 2-Amido-aryl-aldehyden und 1,2 Diamino-benzolen beschrieben.

Analog können folgende Verbindungen dargestellt werden:

| **Bsp.** | **2-Amido-arylaldehyd** | **1,2-Diaminobenzol** | **Produkt** | **Ausbeute** |
|---|---|---|---|---|
| L97 | 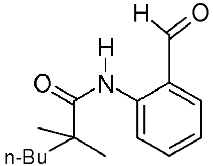 | 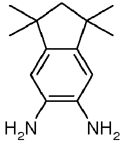 | 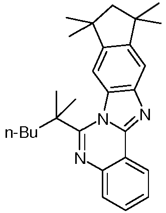 | 55 % |
| | S70 | S16 | | |
| L98 | 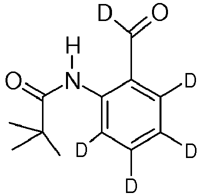 | 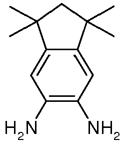 | 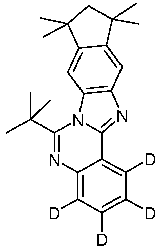 | 59 % |
| | S73 | S16 | | |
| L99 | 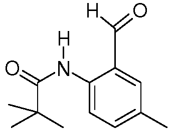 | 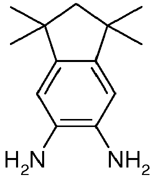 | 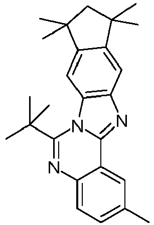 | 61 % |
| | S76 | | | |
| | | S16 | | |
| L100 | 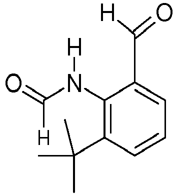 | 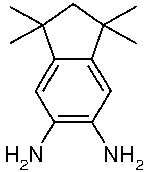 | 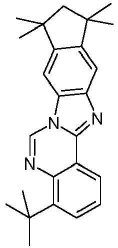 | 60 % |
| | S83 | S16 | | |
| L101 | 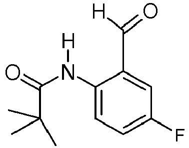 | 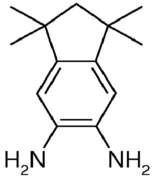 | 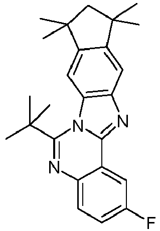 | 58 % |
| | S87 | S16 | | |
| L102 | 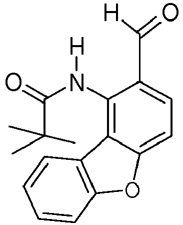 | 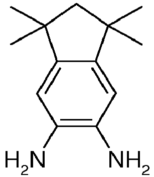 | 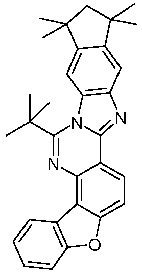 | 57 % |
| | S95 | S16 | | |
| L103 | 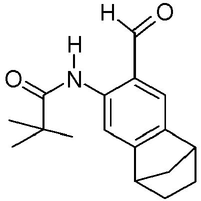 | 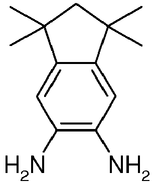 | 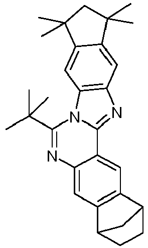 | 58 % |
| | S99 | S16 | | |
| L104 | 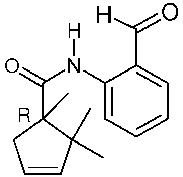 | 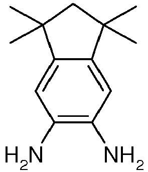 | 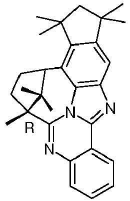 | 14 % |
| | S101 | S16 | Allg. Ligandensynthese Variante B | |
| L105 | 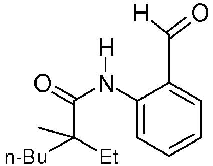 | 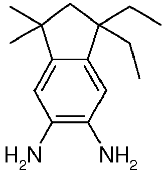 | 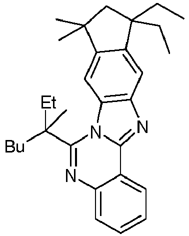 | 23 % |
| | S71 | S17 | Chromatographische Abtrennung des Regioisomeren | |
| L106 | 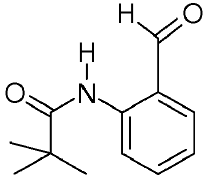 | 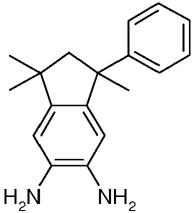 | 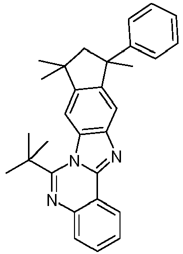 | 15 % |
| | S69 | S19 | Chromatographische Abtrennung des Regioisomeren | |
| L107 | 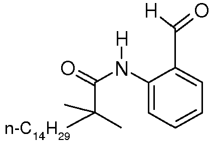 | 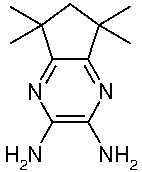 | 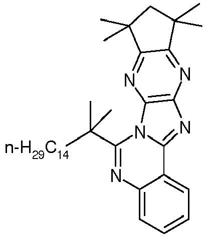 | 43 % |
| | S72 | S22 | | |
| L108 | 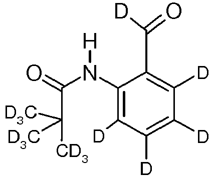 | 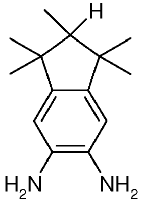 | 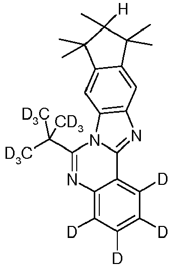 | 57 % |
| | S74 | S23 | | |
| L109 | 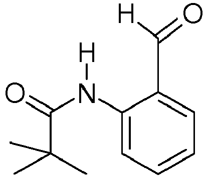 | 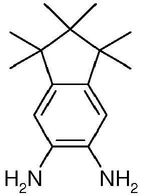 | 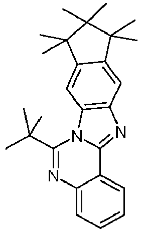 | 55 % |
| | S69 | S24 | | |
| L110 | 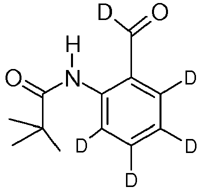 | 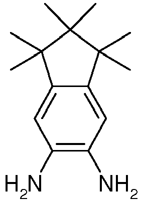 | 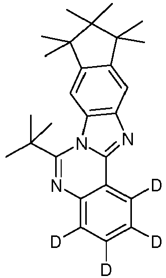 | 56 % |
| | S73 | S24 | | |
| L111 | 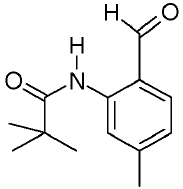 | 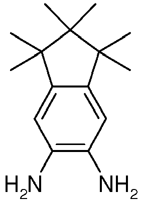 | 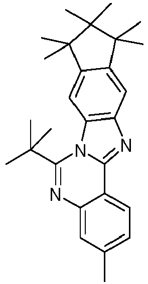 | 50 % |
| | S75 | S24 | | |
| L112 | 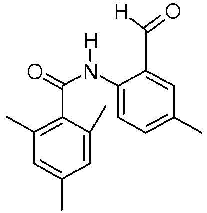 | 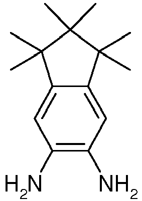 | 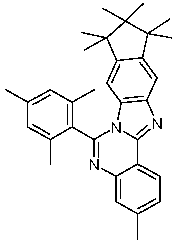 | 58 % |
| | S77 | S24 | | |
| L113 | 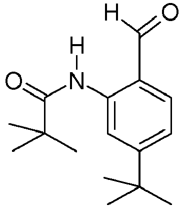 | 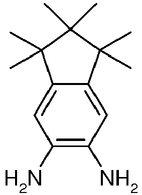 | 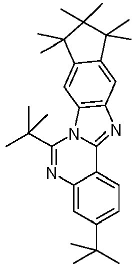 | 58 % |
| | S79 | S24 | | |
| L114 | 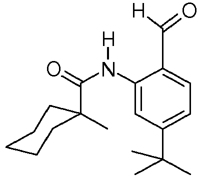 | 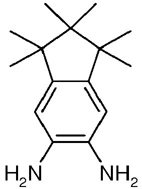 | 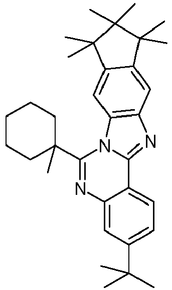 | 47 % |
| | S80 | S24 | | |
| L115 | 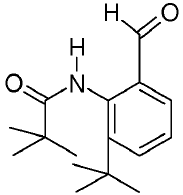 | 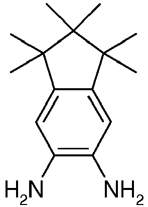 | 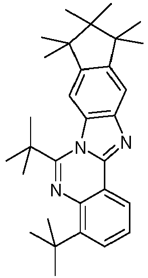 | 44 % |
| | S81 | S24 | | |
| L116 | 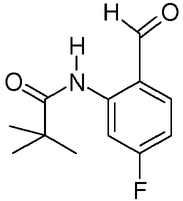 | 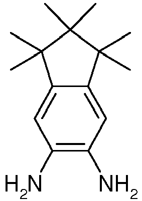 | 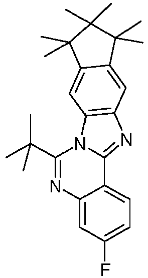 | 53 % |
| | S84 | S24 | | |
| L117 | 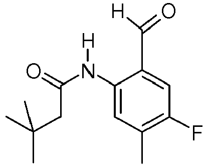 | 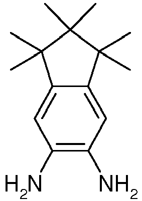 | 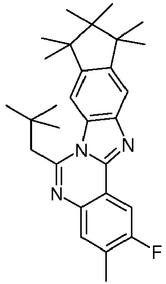 | 49 % |
| | S89 | S24 | | |
| L118 | 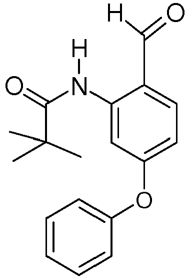 | 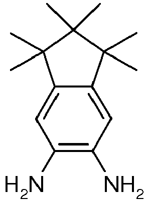 | 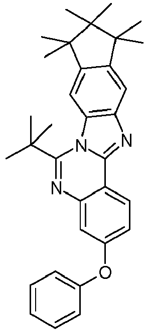 | 39 % |
| | S91 | S24 | | |
| L119 | 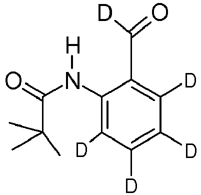 | 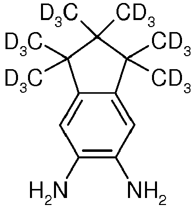 | 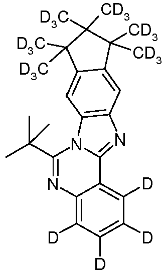 | 50 % |
| | S73 | S25 | | |
| L120 | 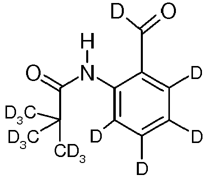 | 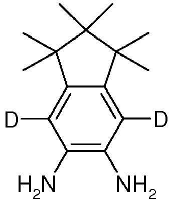 | 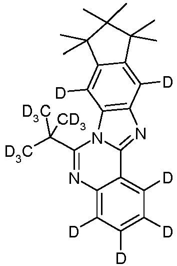 | 54 % |
| | S74 | S26 | | |
| L121 | 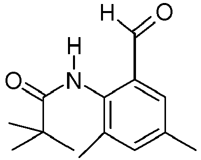 | 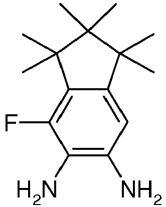 | 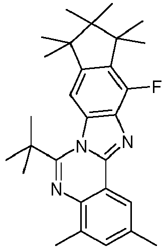 | 26 % |
| | S78 | S27 | Chromatographische Abtrennung des Regioisomeren | |
| L122 | 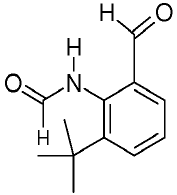 | 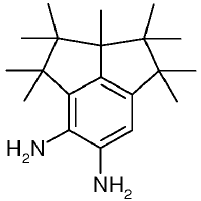 | 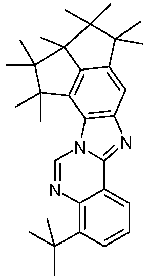 | 20 % |
| | S83 | S28 | Chromatographische Abtrennung des Regioisomeren | |
| L123 | 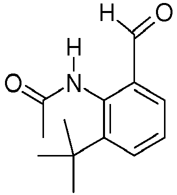 | 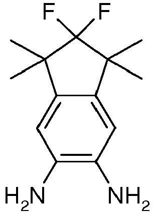 | 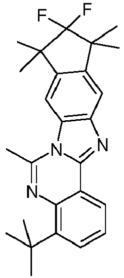 | 51 % |
| | S82 | S29 | | |
| L124 | 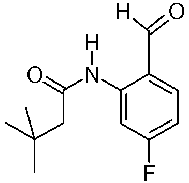 | 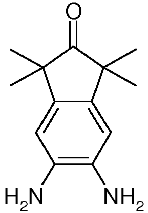 | 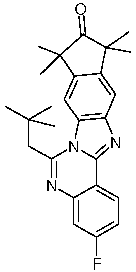 | 33 % |
| | S85 | S30 | | |
| L125 | 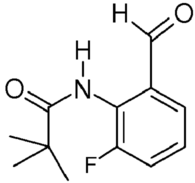 | 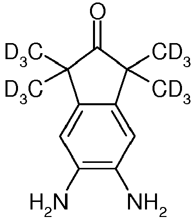 | 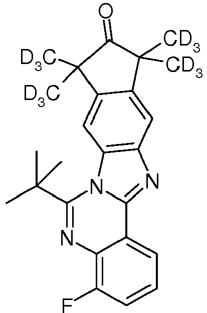 | 30 % |
| | S86 | S31 | | |
| L126 | 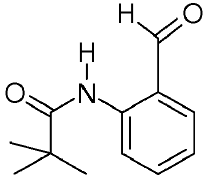 | 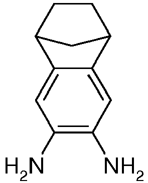 | 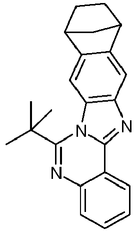 | 56 % |
| | S69 | S32 | | |
| L127 | 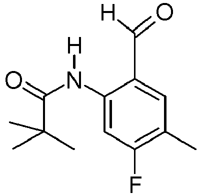 | 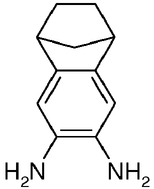 | 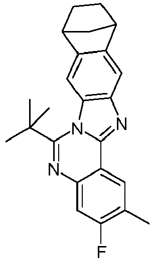 | 53 % |
| | S88 | S32 | | |
| L128 | 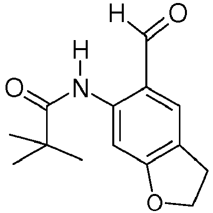 | 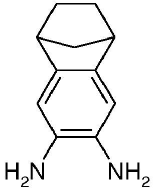 | 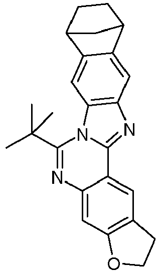 | 41 % |
| | S93 | S32 | | |
| L129 | 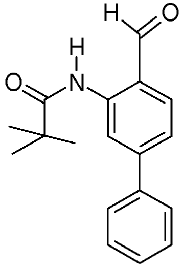 | 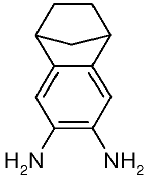 | 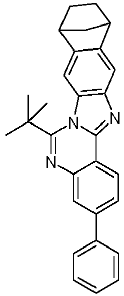 | 53 % |
| | S94 | S32 | | |
| L130 | 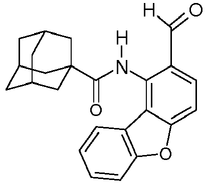 | 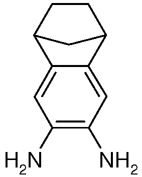 | 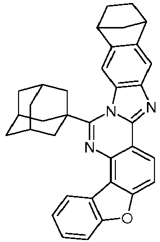 | 46 % |
| | S96 | S32 | | |
| L131 | 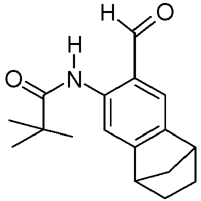 | 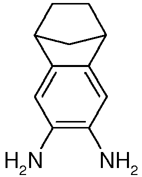 | 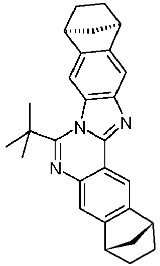 | 18 % |
| | S99 | S32 | Chromatographische Trennung der Diastereomeren | |
| L132 | 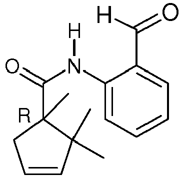 | 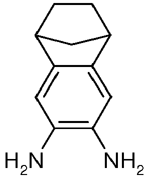 | 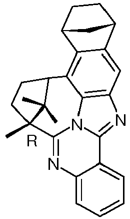 | 20 % |
| | S101 | S32 | Allg. Ligandensynthese Variante B, T = 100 °C Chromatographische Trennung der Diastereomeren | |
| L133 | 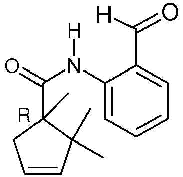 | 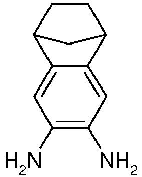 | 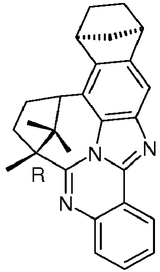 | 13 % |
| | S101 | S32 | Allg. Ligandensynthese Variante B, T =100 °C Chromatographische Trennung der Diastereomeren | |
| L134 | 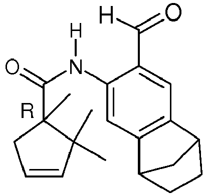 | 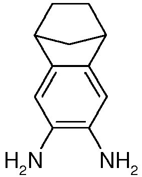 | 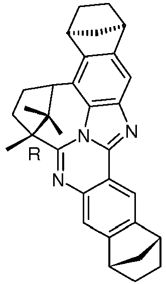 | 14 % |
| | S102 | S32 | Allg. Ligandensynthese Variante B, T = 100 °C Chromatographische Trennung der Diastereomeren | |
| L135 | 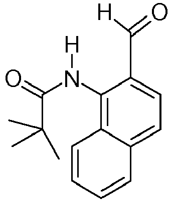 | 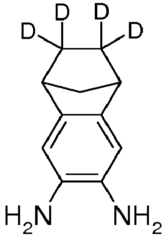 | 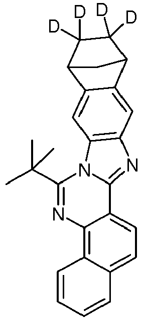 | 56 % |
| | S97 | S33 | | |
| L136 | 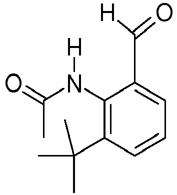 | 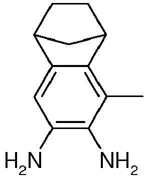 | 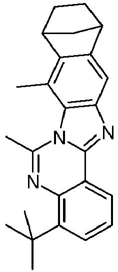 | 24 % |
| | S82 | S35 | Chromatographische Abtrennung des Regioisomeren | |
| L137 | 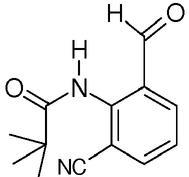 | 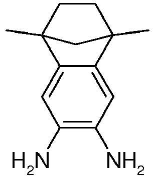 | 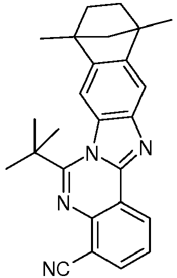 | 22 % |
| | S90 | S36 | | |
| L138 | 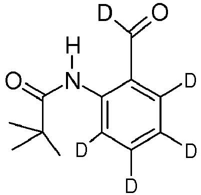 | 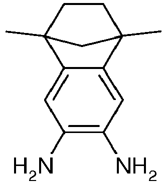 | 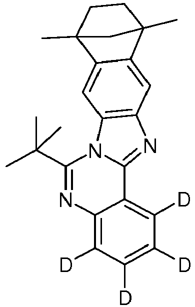 | 55 % |
| | S73 | S36 | | |
| L139 | 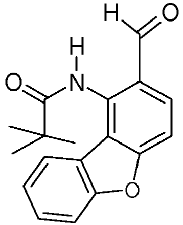 | 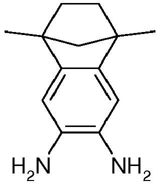 | 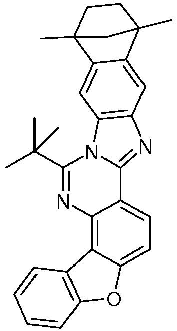 | 47 % |
| | S95 | S36 | | |
| L140 | 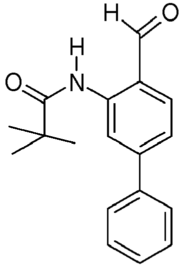 | 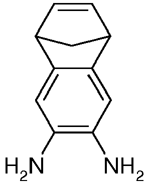 | 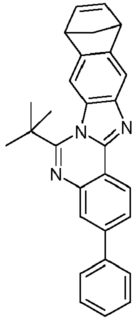 | 30 % |
| | S94 | S38 | | |
| L141 | 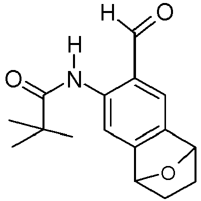 | 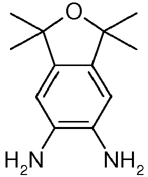 | 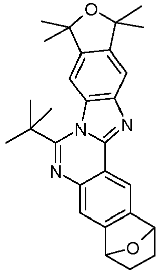 | 48 % |
| | S100 | S39 | | |
| L142 | 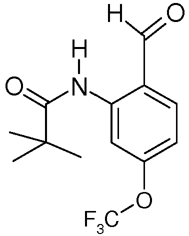 | 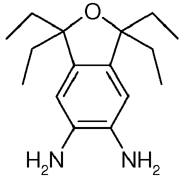 | 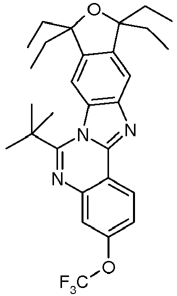 | 44 % |
| | S92 | S40 | | |
| L143 | 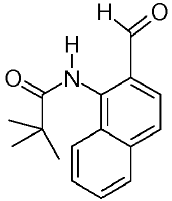 | 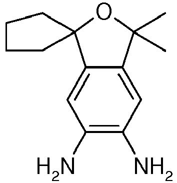 | 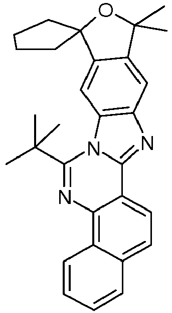 | 25 % |
| | S97 | S41 | Chromatographische Abtrennung des Regioisomeren | |
| L144 | 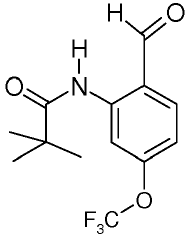 | 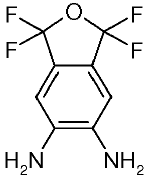 | 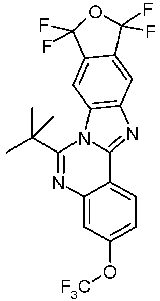 | 41 % |
| | S92 | S43 | | |
| L145 | 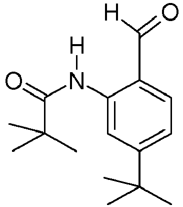 | 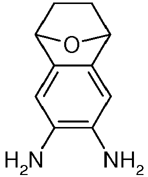 | 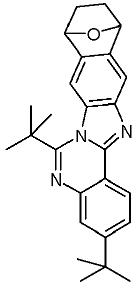 | 54 % |
| | S79 | S44 | | |
| L146 | 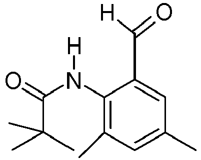 | 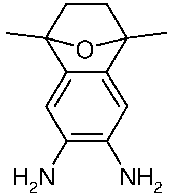 | 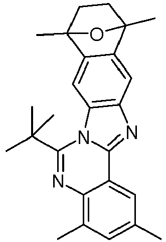 | 56 % |
| | S78 | S46 | | |
| L147 | 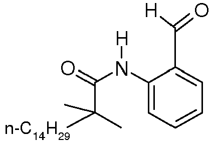 | 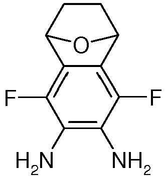 | 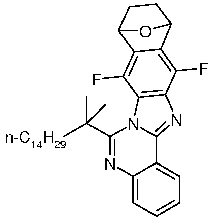 | 28 % |
| | S72 | S47 | | |
| L148 | 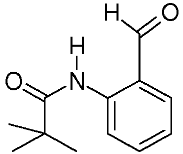 | 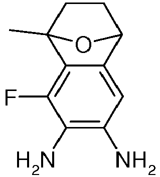 | 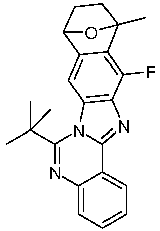 | 17 % |
| | S69 | S48 | Chromatographische Abtrennung des Regioisomeren | |
| L149 | 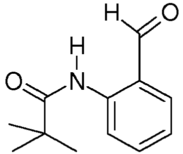 | 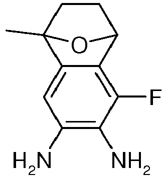 | 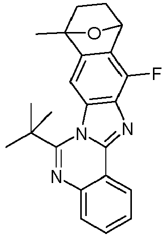 | 22 % |
| | S69 | S49 | Chromatographische Abtrennung des Regioisomeren | |
| L150 | 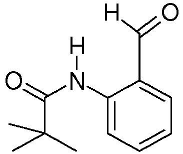 | 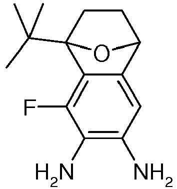 | 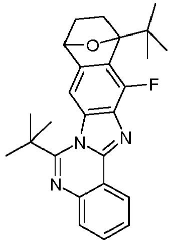 | 18 % |
| | S69 | S50 | Chromatographische Abtrennung des Regioisomeren | |
| L151 | 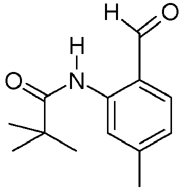 | 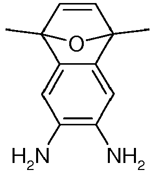 | 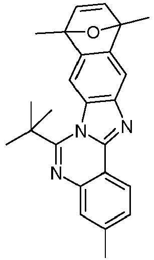 | 32 % |
| | S75 | S52 | | |
| L152 | 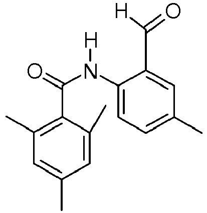 | 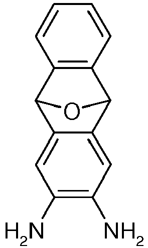 | 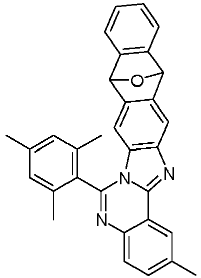 | 51 % |
| | S77 | S53 | | |
| L153 | 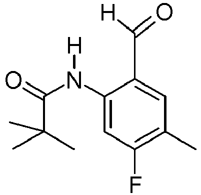 | 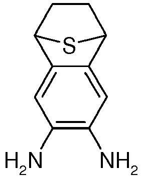 | 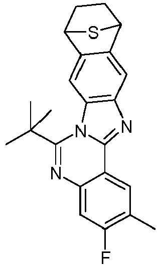 | 46 % |
| | S88 | S54 | | |
| L154 | 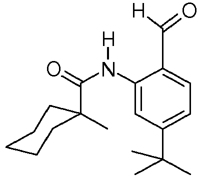 | 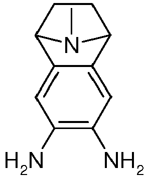 | 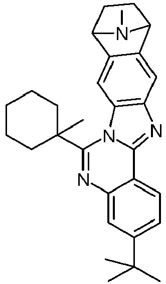 | 21 % |
| | S80 | S55 | | |
| L155 | 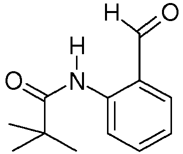 | 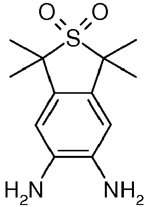 | 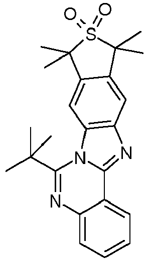 | 26 % |
| | S69 | S57 | | |
| L156 | 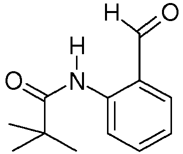 | 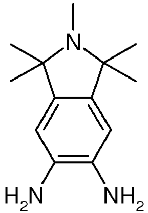 | 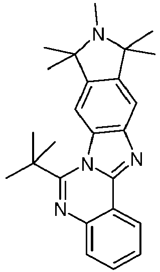 | 20 % |
| | S69 | S58 | | |
| L157 | 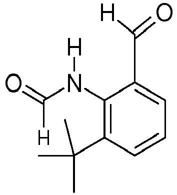 | 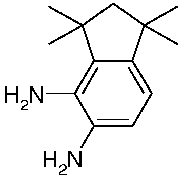 | 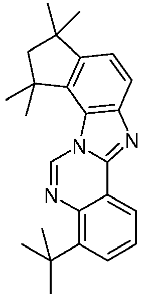 | 16 % |
| | S83 | S59 | Chromatographische Abtrennung des Regioisomeren | |
| L158 | 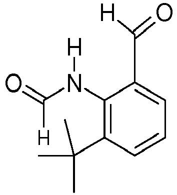 | 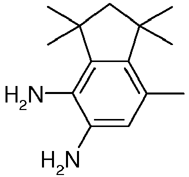 | 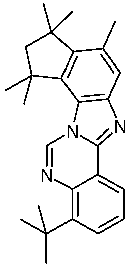 | 18 % |
| | S83 | S60 | Chromatographische Abtrennung des Regioisomeren | |
| L159 | 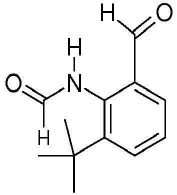 | 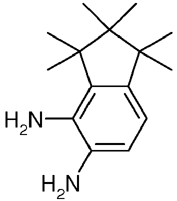 | 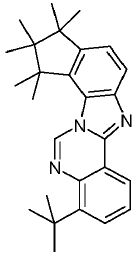 | 23 % |
| | S83 | S61 | Chromatographische Abtrennung des Regioisomeren | |
| L160 | 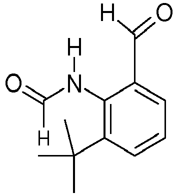 | 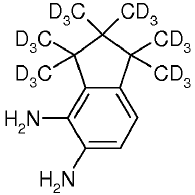 | 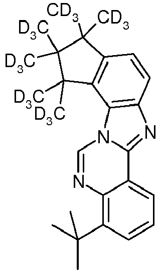 | 20 % |
| | S83 | S62 | Chromatographische Abtrennung des Regioisomeren | |
| L161 | 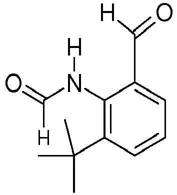 | 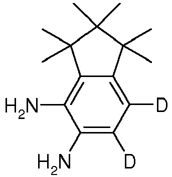 | 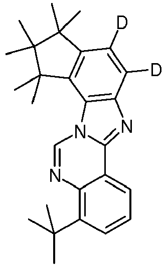 | 21 % |
| | S83 | S63 | Chromatographische Abtrennung des Regioisomeren | |
| L162 | 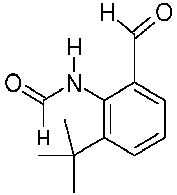 | 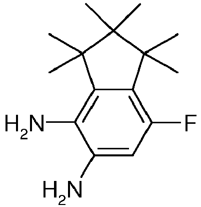 | 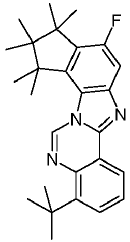 | 24 % |
| | S83 | S64 | Chromatographische Abtrennung des Regioisomeren | |
| L163 | 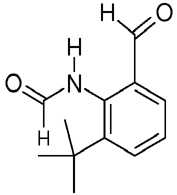 | 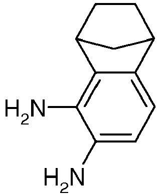 | 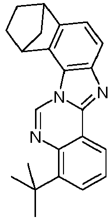 | 25 % |
| | S83 | S65 | Chromatographische Abtrennung des Regioisomeren | |
| L164 | 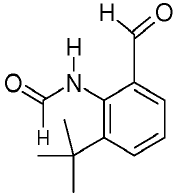 | 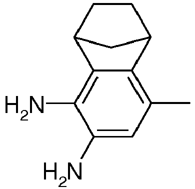 | 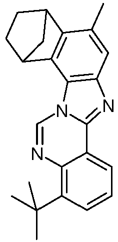 | 24 % |
| | S83 | S66 | Chromatographische Abtrennung des Regioisomeren | |
| L165 | 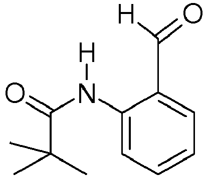 | 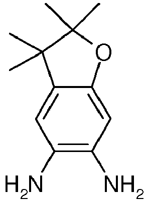 | 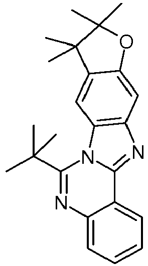 | 19 % |
| | S69 | S37 | Chromatographische Abtrennung des Regioisomeren | |
| L166 | 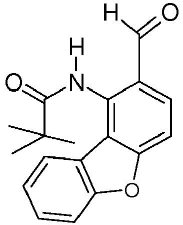 | 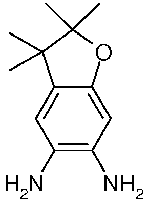 | 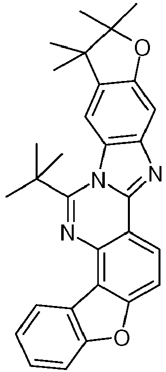 | 222 % |
| | S95 | S37 | Chromatographische Abtrennung des Regioisomeren | |

### 3) Liganden vom 2,6a,11-Triaza-benzo[a]fluoren-Typ Allgemeine Ligandensynthese Variante A:

### Aus 4-Alkinyl-3-formyl-pyridinen und 1,2-Diamino-benzolen:

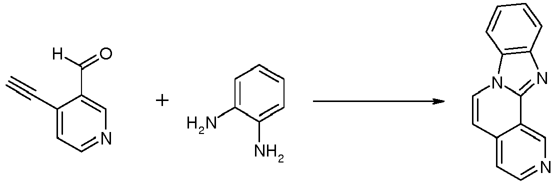

Eine Lösung von 500 mmol des 4-Alkinyl-3-formyl-pyridins und 550 mmol des 1,2-Diaminobenzols in 1000 ml Nitrobenzol wird in einer Apparatur bestehend aus einem 2000 ml Einhalskolben mit Hahnstück und aufgesetzter Destillationsbrücke platziert und unter Rühren langsam auf 200 °C (Ölbadtemperatur) erhitzt, wobei das gebildete Wasser abdestilliert. Man rührt 2 h bei 200 °C nach, steigert dann die Temperatur auf ca. 230 °C und destilliert das Nitrobenzol im Argonstrom ab. Gegen Ende der Destillation legt man ein Vakuum von ca. 100 mbar an, um letzte Reste von Nitrobenzol zu entfernen, dann lässt man die Reaktionsmischung erkalten. Fällt das Rohprodukt glasartig an, wird das Glas mechanisch zerkleinert, Öle versetzt man direkt mit 200 - 400 ml Methanol oder Acetonitril und erhitzt die Mischung unter Rückfluss, wobei sich das Glas bzw. das Öl löst und das Produkt auskristallisiert. Die so erhaltenen Rohprodukte weisen häufig schon eine hohe Reinheit auf (¹H-NMR typischerweise 97 - 99 %ig). Gegebenenfalls werden sie erneut umkristallisiert und dann durch fraktionierte Kugelrohrdestillation oder Sublimation (p ca. 10⁻⁴ - 10⁻⁵ mbar, T ca. 160 - 250 °C) von Leichtsiedern und nicht flüchtigen Nebenkomponenten befreit. Verbindungen mit aliphatischen Resten, die mehr als 6 C-Atome aufweisen, bzw. solche mit Aralkylgruppen, die mehr als 9 C-Atome aufweisen, werden typischerweise chromatographisch gereinigt und dann im Vakuum getrocknet, um Leichtsieder zu entfernen. Reinheit nach ¹H-NMR typischerweise > 99.5 % ig.

### Beispiel L167:

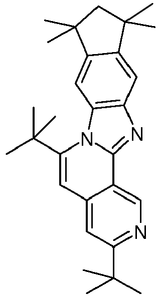

Einsatz von 121.7 g (500 mmol) S108 und 112.4 g (550 mmol) S16. Einmalige Umkristallisation des Rohprodukts aus Dioxan/EtOH, zweimalige fraktionierte Sublimation des Produktes bei T ca. 190 °C, p ca. 10⁻⁴ mbar. Ausbeute: 124.0 g (290 mmol), 58 %; Reinheit: > 99.5 % ig nach ¹H-NMR.

Analog können folgende Verbindungen dargestellt werden:

| **Bsp.** | **4-Alkinyl-3-formyl-pyridin** | **1,2-Diaminobenzol** | **Produkt** | **Ausbeute** |
|---|---|---|---|---|
| L168 | 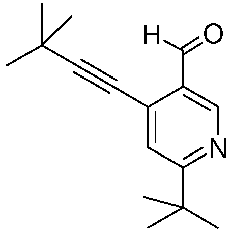 | 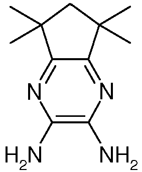 | 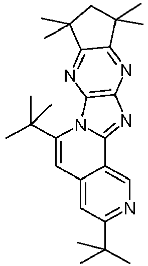 | 55 % |
| | S108 | S22 | | |
| L169 | 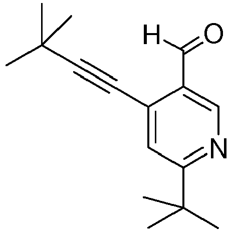 | 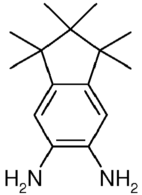 | 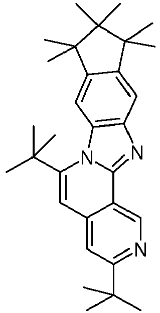 | 56 % |
| | S108 | S24 | | |
| L170 | 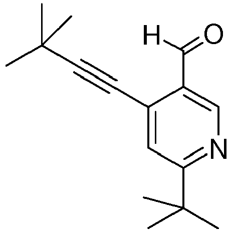 | 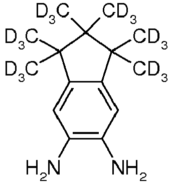 | 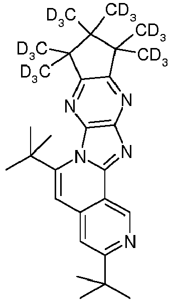 | 56 % |
| | S108 | S25 | | |
| L171 | 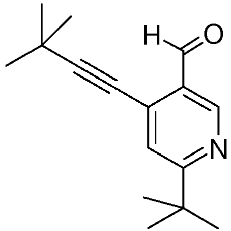 | 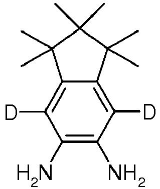 | 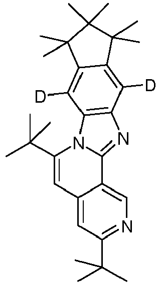 | 58 % |
| | S108 | S26 | | |
| L172 | 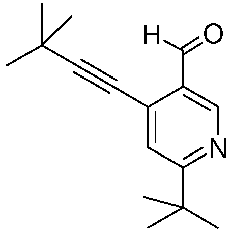 | 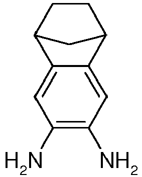 | 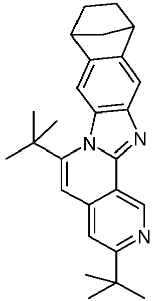 | 54 % |
| | S108 | S32 | | |
| L173 | 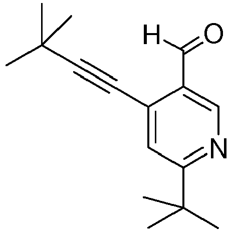 | 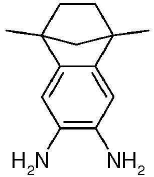 | 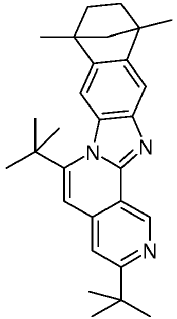 | 55 % |
| | S108 | S36 | | |
| L174 | 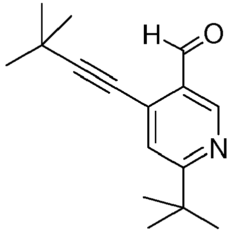 | 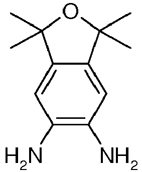 | 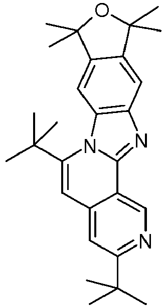 | 55 % |
| | S108 | S39 | | |
| L175 | 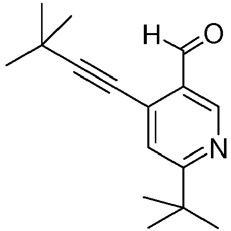 | 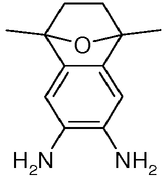 | 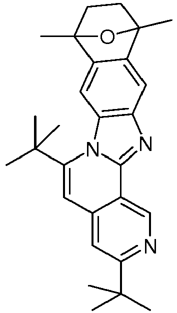 | 51 % |
| | S108 | S46 | | |
| L176 | 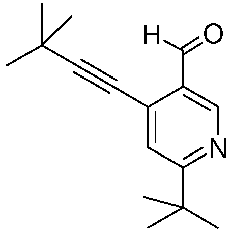 | 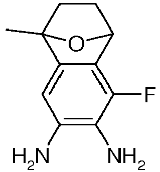 | 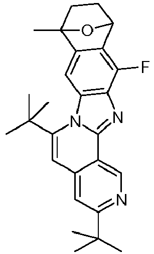 | 24 % |
| | S108 | S49 | Chromatographische Abtrennung des Regioisomeren | |
| L177 | 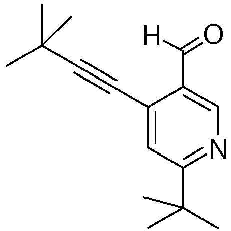 | 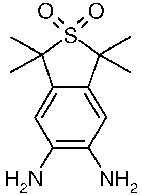 | 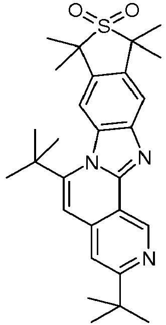 | 33 % |
| | S108 | S57 | | |
| L178 | 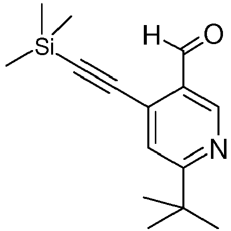 | 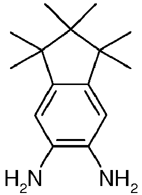 | 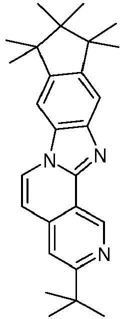 | 59 % |
| | S109 | S24 | | |
| L179 | 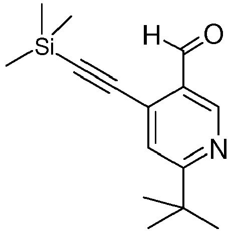 | 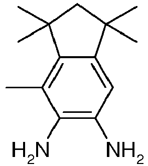 | 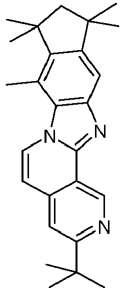 | 25 % |
| | S109 | S21 | Chromatographische Abtrennung des Regioisomeren | |
| L180 | 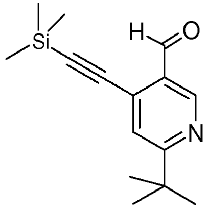 | 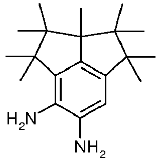 | 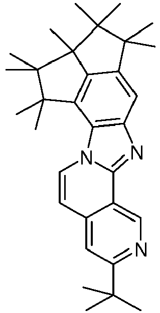 | 19 % |
| | S109 | S28 | Chromatographische Abtrennung des Regioisomeren | |
| L181 | 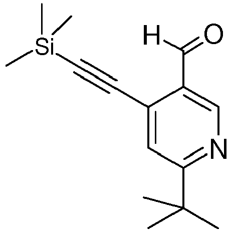 | 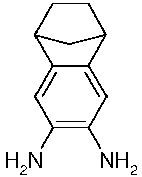 | 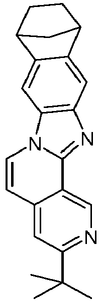 | 50 % |
| | S109 | 124639-03-8 S32 | | |
| L182 | 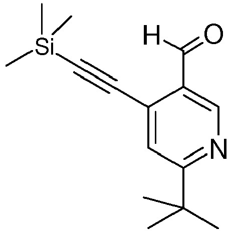 | 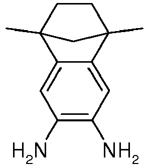 | 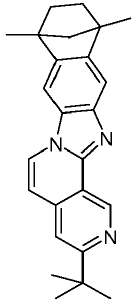 | 53 % |
| | S109 | S36 | | |
| L183 | 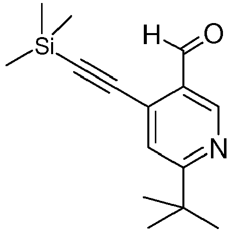 | 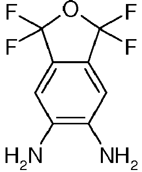 | 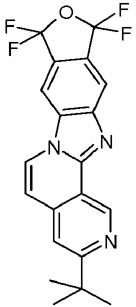 | 55 % |
| | S109 | S43 | | |
| L184 | 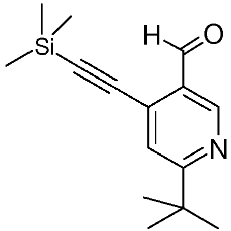 | 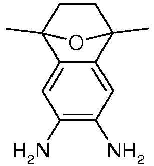 | 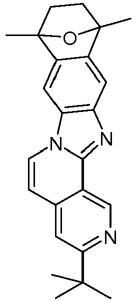 | 49 % |
| | S109 | S46 | | |
| L185 | 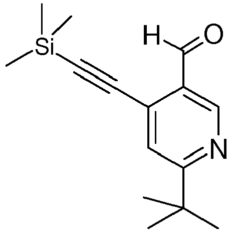 | 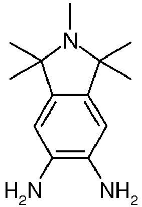 | 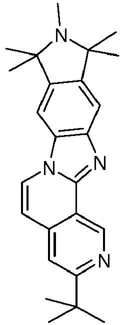 | 26 % |
| | S109 | S58 | | |
| L186 | 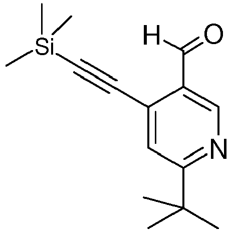 | 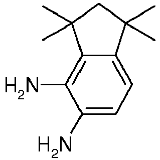 | 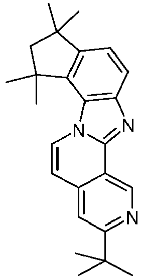 | 18 % |
| | S109 | S59 | Chromatographische Abtrennung des Regioisomeren | |
| L187 | 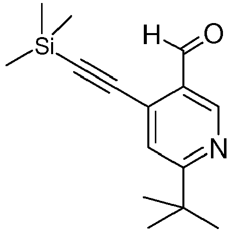 | 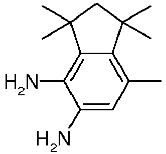 | 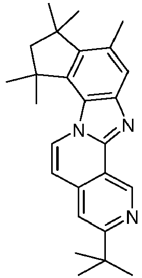 | 20 % |
| | S109 | S60 | Chromatographische Abtrennung des Regioisomeren | |
| L188 | 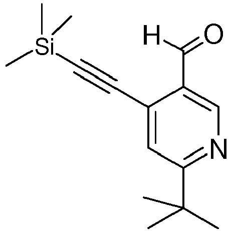 | 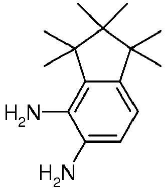 | 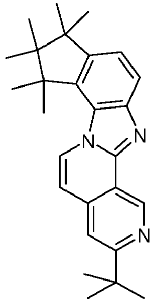 | 21 % |
| | S109 | S61 | Chromatographische Abtrennung des Regioisomeren | |
| L189 | 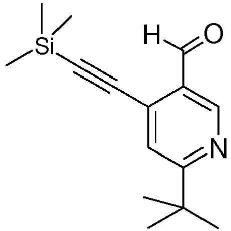 | 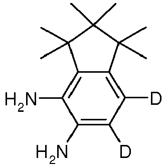 | 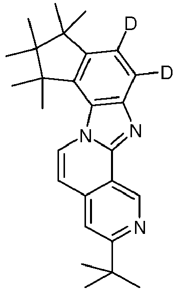 | 18 % |
| | S109 | S63 | Chromatographische Abtrennung des Regioisomeren | |
| L190 | 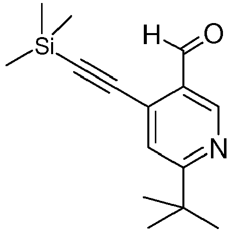 | 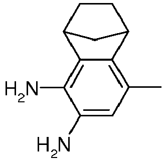 | 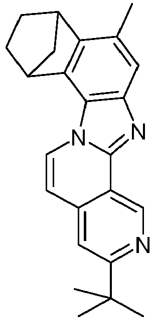 | 19 % |
| | S109 | S66 | Chromatographische Abtrennung des Regioisomeren | |
| L191 | 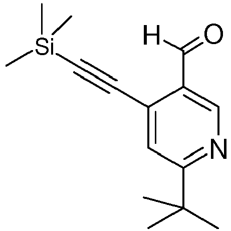 | 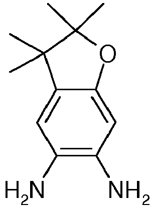 | 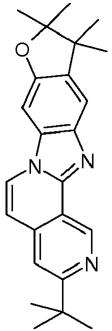 | 17 % |
| | S109 | S37 | Chromatographische Abtrennung des Regioisomeren | |

### Allgemeine Ligandensynthese Variante B:

### [2,7]-Naphthyridin-1-ylaminen und 1,2-Dihalogen-benzolen:

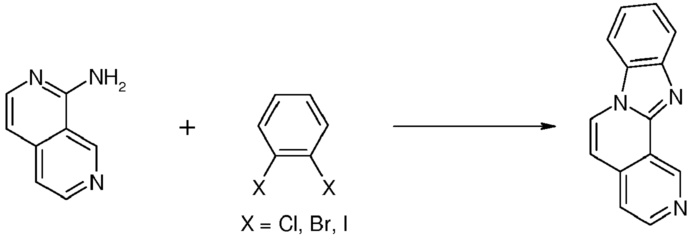

Eine gut gerührte Mischung von 100 mmol des 1,2-Dihalogenbenzols, 120 mmol des [2,7]-Naphthyridin-1-ylamins, 300 mmol Lithium-bis(trimethylsilyl)amid, 100 g Glaskugeln (3 mm Durchmesser), 5 mmol Xanthphos und 5 mmol Palladium(II)acetat in 600 ml trockenem 1,4-Dioxan wird 72 h unter Rückfluss erhitzt, bis das 1-Amino-isochinolin verbraucht ist. Nach Erkalten wird über ein Celite-Bett abfiltriert, mit 1500 ml Dioxan nachgewaschen und das Filtrat zur Trockene eingeengt. Der Rückstand wird in 400 ml Dichlormethan gelöst und über ein Kieselgelbett filtriert. Es wird mit einem Gemisch aus 2000 ml Dichlormethan und 150 ml Ethylacetat nachgewaschen und das Filtrat zur Trockene eingeengt. Der Rückstand wird umkristallisiert und im Vakuum getrocknet. Das so erhaltene 2,6a,11-Triaza-benzo[a]fluoren wird durch Kugelrohrdestillation oder fraktionierte Sublimation (p ca. 10⁻⁴ - 10⁻⁵ mbar, T ca. 160 - 240 °C) von Leichtsiedern und nicht flüchtigen Ne benkomponenten befreit. Verbindungen mit aliphatischen Resten, die mehr als 6 C-Atome aufweisen, bzw. solche mit Aralkylgruppen, die mehr als 9 C-Atome aufweisen, werden typischerweise chromatographisch gereinigt und dann im Vakuum getrocknet, um Leichtsieder zu entfernen. Reinheit nach ¹H-NMR typischerweise > 99.5 % ig.

### Beispiel L178:

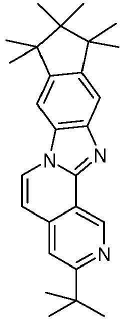

Einsatz von 36.0 g (100 mmol) 5,6-Dibrom-1,1,2,2,3,3-hexamethyl-indan, S24, Variante A, Stufe A, 24.3 g (120 mmol) 6-tert-Butyl-[2,7]naphthyridin-1-ylamin [1352329-35-1], 50.2 g (300 mmol) Lithium-bis(trimethylsilyl)-amid, 2.9 g (5 mmol) Xanthphos und 1.1 g (5 mmol) Palladium(II)acetat. Das Rohprodukt wird aus Ethanol (ca. 7 ml/g) umkristallisiert und im Vakuum sublimiert (p = 10⁻⁵ mbar, T = 230 °C). Ausbeute: 27.2 g (68 mmol), 68 %; Reinheit: ca. 99.5 % ig nach ¹H-NMR.

Analog können folgende Verbindungen dargestellt werden:

| **Bsp.** | **[2,71-Naph-thyridin-1-ylamin** | **1,2-Halogen-benzol** | **Produkt** | **Ausbeute** |
|---|---|---|---|---|
| L181 | 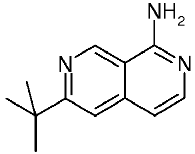 | 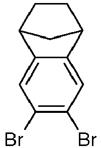 | 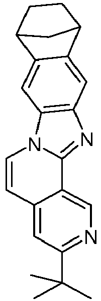 | 66 % |
| | 1352329-35-1 | 42810-32-2 | | |

### 4) Liganden vom 4,6a,11-Triaza-benzo[a]fluoren-Typ Allgemeine Ligandensynthese Variante A:

### Aus 2-Alkinyl-3-formyl-pyridinen und 1,2-Diamino-benzolen:

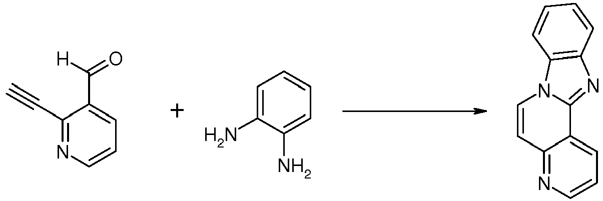

Darstellung analog zu 3) Variante A, Liganden vom 2,6a,11-Triaza-benzo[a]fluoren-Typ.

### Beispiel L192:

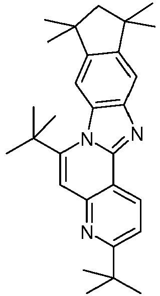

Einsatz von 121.7 g (500 mmol) 2-(3,3-Dimethyl-butin-1-yl)-6-(1,1-dimethylethyl)-pyridin-3-carboxaldehyd [1352329-59-9] und 112.4 g (550 mmol) S16. Einmalige Umkristallisation des Rohprodukts aus Dioxan/EtOH, zweimalige fraktionierte Sublimation des Produktes bei T ca. 190 °C, p ca. 10⁻⁴ mbar. Ausbeute: 121.0 g (283 mmol), 56 %; Reinheit: > 99.5 % ig nach ¹H-NMR.

Analog können folgende Derivate dargestellt werden:

| **Bsp.** | **2-Alkinyl-3-formyl-pyridin** | **1,2-Diaminobenzol** | **Produkt** | **Ausbeute** |
|---|---|---|---|---|
| L193 | 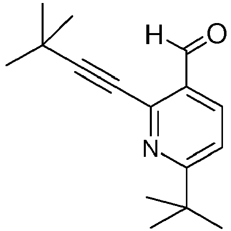 | 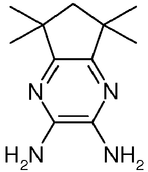 | 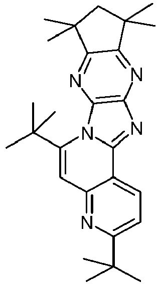 | 47 % |
| | 1352329-59-9 | S22 | | |
| L194 | 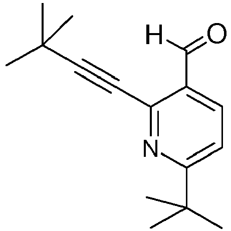 | 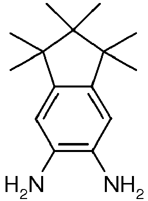 | 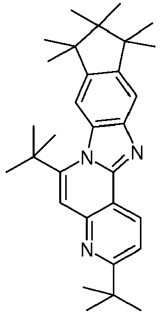 | 55 % |
| | 1352329-59-9 | S24 | | |
| L195 | 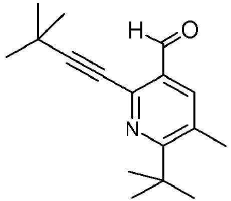 | 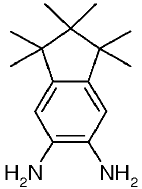 | 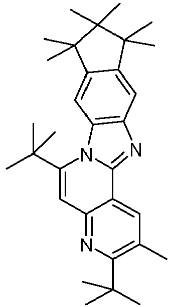 | 53 % |
| | 1352331-90-8 | S24 | | |
| L196 | 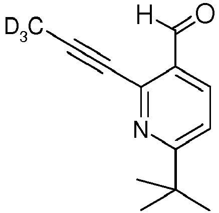 | 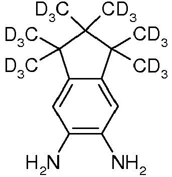 | 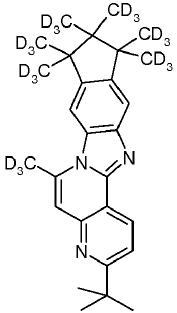 | 53 % |
| | 1352329-62-4 | S25 | | |
| L197 | 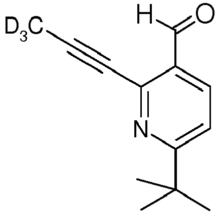 | 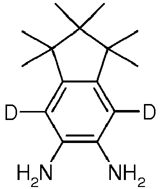 | 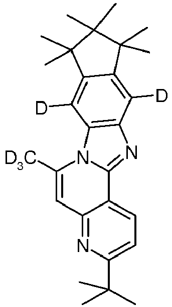 | 54 % |
| | 1352329-62-4 | S26 | | |
| L198 | 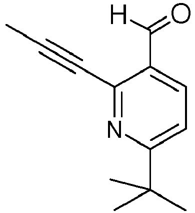 | 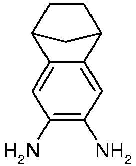 | 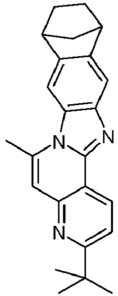 | 51 % |
| | 1352329-61-3 | S32 | | |
| L199 | 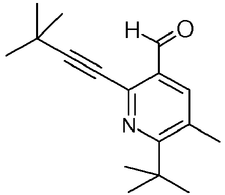 | 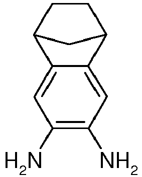 | 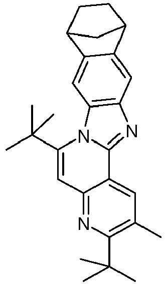 | 49 % |
| | 1352331-90-8 | S32 | | |
| L200 | 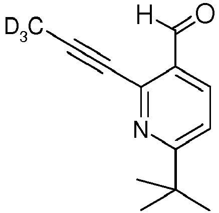 | 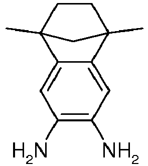 | 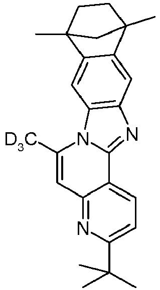 | 52 % |
| | 1352329-62-4 | S36 | | |
| L201 | 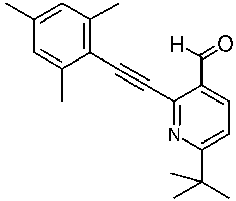 | 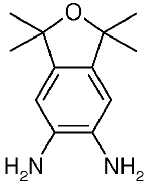 | 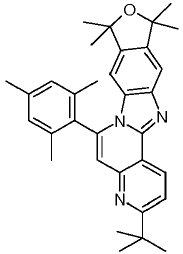 | 55 % |
| | 1352329-64-6 | S39 | | |
| L202 | 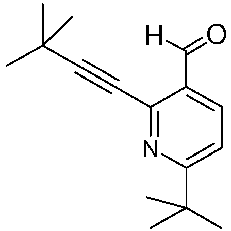 | 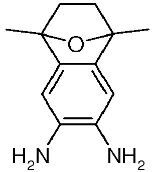 | 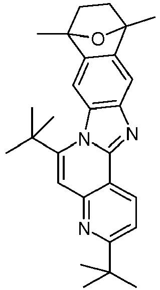 | 54 % |
| | 1352329-59-9 | S46 | | |
| L203 | 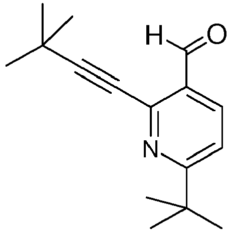 | 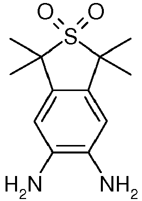 | 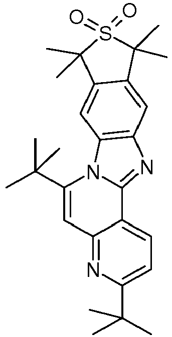 | 38 % |
| | 1352329-59-9 | S57 | | |
| L204 | 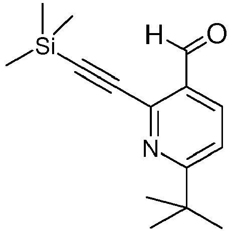 | 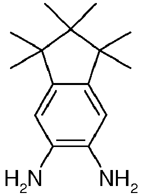 | 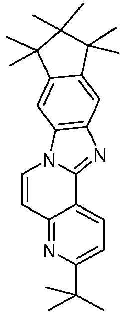 | 54 % |
| | 1352329-60-2 | S24 | | |
| L205 | 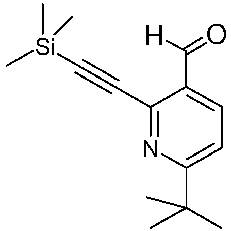 | 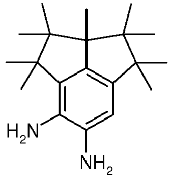 | 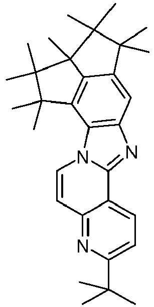 | 19 % |
| | 1352329-60-2 | S28 | Chromatographische Abtrennung des Regioisomeren | |
| L206 | 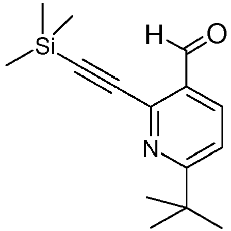 | 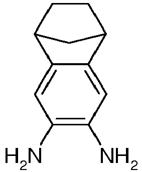 | 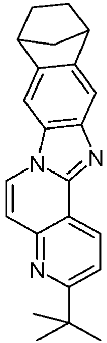 | 50 % |
| | 1352329-60-2 | 124639-03-8 | | |
| | | S32 | | |
| L207 | 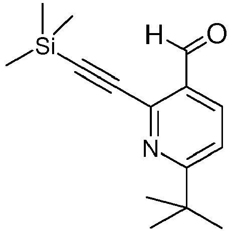 | 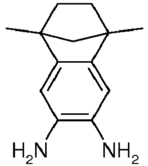 | 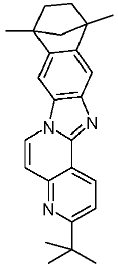 | 51 % |
| | 1352329-60-2 | S36 | | |
| L208 | 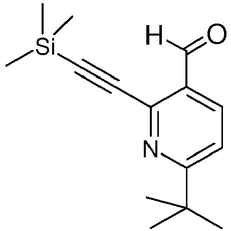 | 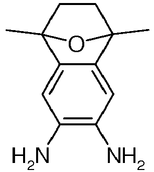 | 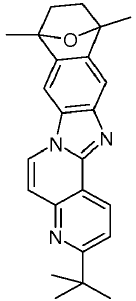 | 48 % |
| | 1352329-60-2 | S46 | | |
| L209 | 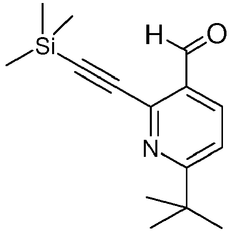 | 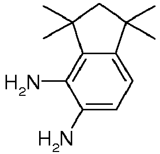 | 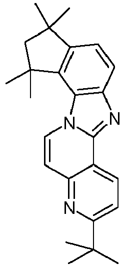 | 20 % |
| | 1352329-60-2 | S59 | Chromatographische Abtrennung des Regioisomeren | |
| L210 | 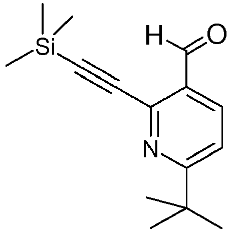 | 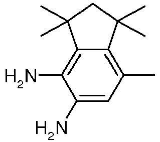 | 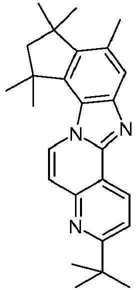 | 19 % |
| | 1352329-60-2 | S60 | Chromatographische Abtrennung des Regioisomeren | |
| L211 | 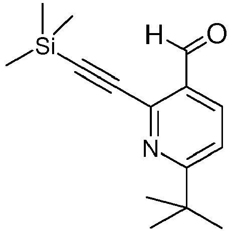 | 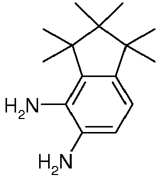 | 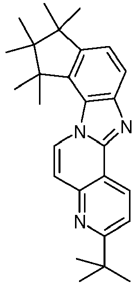 | 20 % |
| | 1352329-60-2 | S61 | Chromatographische Abtrennung des Regioisomeren | |
| L212 | 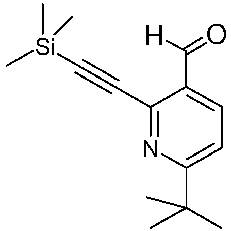 | 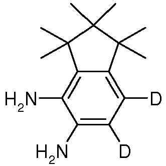 | 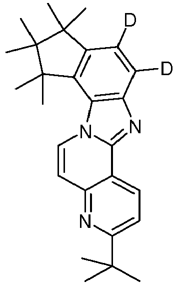 | 20 % |
| | 1352329-60-2 | S63 | Chromatographische Abtrennung des Regioisomeren | |
| L213 | 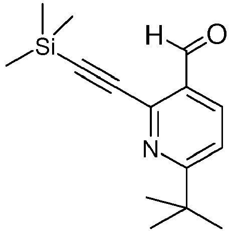 | 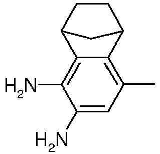 | 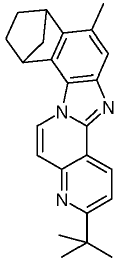 | 18 % |
| | 1352329-60-2 | S66 | Chromatographische Abtrennung des Regioisomeren | |
| L214 | 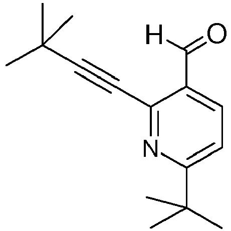 | 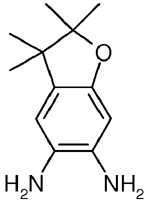 | 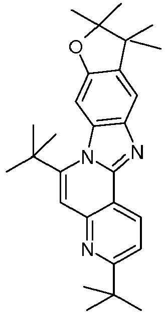 | 17 % |
| | 1352329-59-9 | S37 | Chromatographische Abtrennung des Regioisomeren | |

### Allgemeine Ligandensynthese Variante B:

### [1,6]-Naphthyridin-5-ylaminen und 1,2-Dihalogen-benzolen:

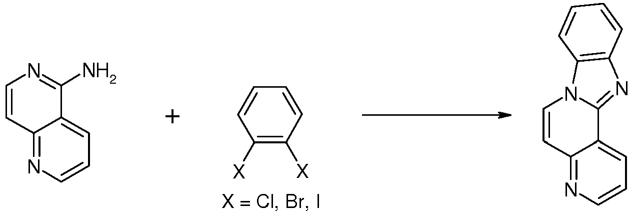

Darstellung analog zu 3) Variante B, Liganden vom 2,6a,11-Triazabenzo[a]fluoren-Typ.

### Beispiel L204:

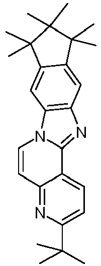

Einsatz von 36.0 g (100 mmol) 5,6-Dibromo-1,1,2,2,3,3-hexamethylindan, S24, Variante A, Stufe A, 24.3 g (120 mmol) 2-tert-Butyl-[1,6]naphthyridin-5-ylamin [1352329-32-8], 50.2 g (300 mmol) Lithium-bis(trimethylsilyl)-amid, 2.9 g (5 mmol) Xanthphos und 1.1 g (5 mmol) Palladium(II)acetat. Das Rohprodukt wird aus Ethanol (ca. 7 ml/g) umkristallisiert und im Vakuum sublimiert (p = 10⁻⁵ mbar, T = 230 °C). Ausbeute: 26.0 g (65 mmol), 65 %; Reinheit: ca. 99.5 % ig nach ¹H-NMR.

### Allgemeine Ligandensynthese Variante C:

### Aus Pyrano[4,3-b]pyridin-5-onen und 1,2-Amino-benzolen:

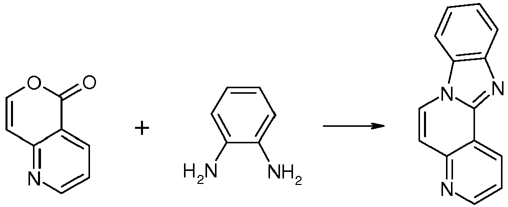

Darstellung analog V. K. Pandey et al., Ind. J. Chem., Section B: 1999, 38B(12), 138.
Ein Gemisch aus 100 mmol des Pyrano[3,2-b]pyridin-2-ons, 110 mmol des 1,2-Diaminobenzols, 5 mmol 4-(N,N-Dimethylamino)pyridin und 200 ml trockenem Pyridin werden am Wasserabscheider gekocht, wobei von Zeit zu Zeit Pyridin abgelassen wird, bis das Pyridin weitgehend abdestilliert ist. Gegen Ende legt man ein schwaches Vakuum an, um Pyridinreste zu entfernen. Nach Erkalten wird der zähe bis glasartige Rückstand in 200 ml Methanol aufgenommen und in der Hitze gelöst, wobei das Produkt zu kristallisieren beginnt. Nach Erkalten saugt man vom Produkt ab und wäscht mit wenig Methanol. Nach Umkristallisation (Methanol, Ethanol, Aceton, Dioxan, DMF, etc.) des so erhaltenen 4,6a,11-Triaza-benzo[a]-fluoren wird dieses durch Kugelrohrdestillation oder fraktionierte Sublimation (p ca. 10⁻⁴ - 10 ⁻⁵ mbar, T ca. 160 - 240 °C) von Leichtsiedern und nicht flüchtigen Nebenkomponenten befreit. Verbindungen mit aliphatischen Resten, die mehr als 6 C-Atome aufweisen, bzw. solche mit Aralkylgruppen, die mehr als 9 C-Atome aufweisen, werden typischerweise chromatographisch gereinigt und dann im Vakuum getrocknet, um Leichtsieder zu entfernen. Reinheit nach ¹H-NMR typischerweise > 99.5 % ig.

### Beispiel L279:

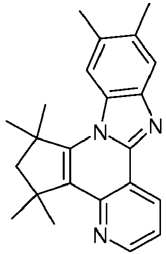

Einsatz von 24.3 g (100 mmol) 1,1,3,3-Tetramethyl-2,3-dihydro-1H-4-oxa-9-aza-cyclopenta[a]naphthalin-5-on, S118, 15.0 g (110 mmol) 4,5-Dimethyl-1,2-diaminobenzol [3171-45-7], 611 mg (5 mmol) 4-(N,N-Dimethylamino)pyridin. Das Rohprodukt wird aus Methanol / Ethylacetat umkristallisiert und im Vakuum sublimiert (p = 10⁻⁵ mbar, T = 220 °C). Ausbeute: 14.7 g (43 mmol), 43 %; Reinheit: ca. 99.5 % ig nach ¹H-NMR.

Analog können folgende Verbindungen dargestellt werden:

| **Bsp.** | **Pyrano[4,3-b]pyridin-5-onen** | **1,2-Diaminobenzol** | **Produkt** | **Ausbeute** |
|---|---|---|---|---|
| L280 | 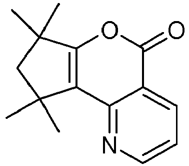 | 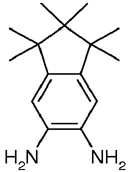 | 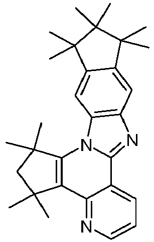 | 40 % |
| | S118 | S24 | | |
| L281 | 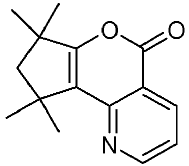 | 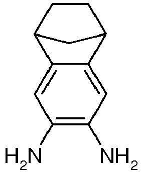 | 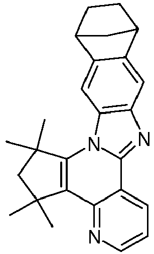 | 39 % |
| | S118 | 124639-03-8 S32 | | |
| L282 | 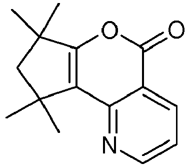 | 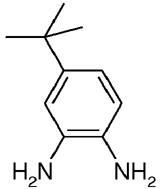 | 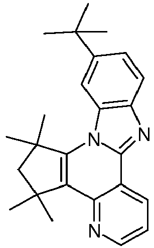 | 19 % |
| | S118 | [68176-57-8] | Chromatographische Abtrennung des Regioisomeren | |

### 5) Liganden vom 3,6a,11-Triaza-benzo[a]fluoren-Typ Allgemeine Ligandensynthese:

### Aus 3-Alkinyl-4-formyl-pyridinen und 1,2-Diamino-benzolen:

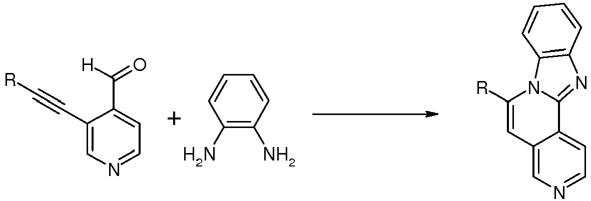

Darstellung analog zu 3) Variante A, Liganden vom 2,6a,11-Triazabenzo[a]fluoren-Typ.

### Beispiel L215:

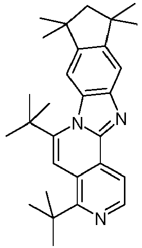

Einsatz von 121.7 g (500 mmol) 2-tert-Butyl-3-(3,3-dimethyl-but-1-inyl)-pyridin-4-carboxaldehyd, S114 und 112.4 g (550 mmol) S16. Einmalige Umkristallisation des Rohprodukts aus Dioxan/EtOH, zweimalige fraktionierte Sublimation des Produktes bei T ca. 190 °C, p ca. 10⁻⁴ mbar. Ausbeute: 113.3 g (265 mmol), 53 %; Reinheit: > 99.5 % ig nach ¹H-NMR.

Analog können folgende Derivate dargestellt werden:

| **Bsp.** | **3-Alkinyl-4-formyl-pyridin** | **1,2-Diaminobenzol** | **Produkt** | **Ausbeute** |
|---|---|---|---|---|
| L216 | 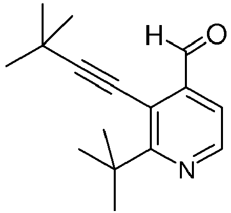 | 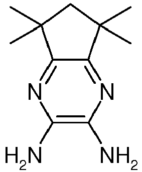 | 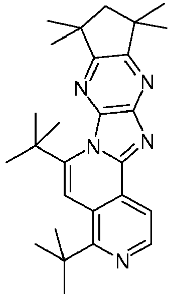 | 54 % |
| | S114 | S22 | | |
| L217 | 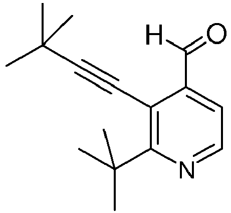 | 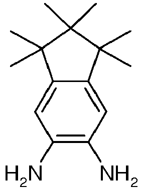 | 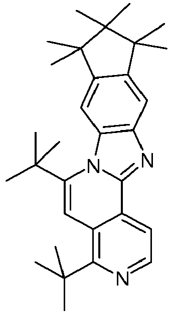 | 49 % |
| | S114 | S24 | | |
| L218 | 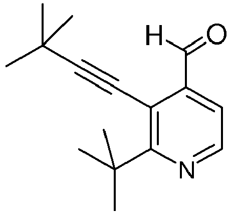 | 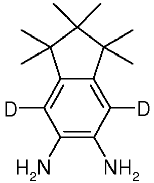 | 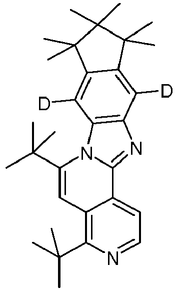 | 50 % |
| | S114 | S26 | | |
| L219 | 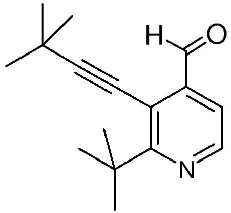 | 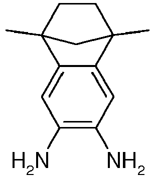 | 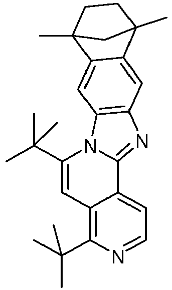 | 52 % |
| | S114 | S36 | | |
| L220 | 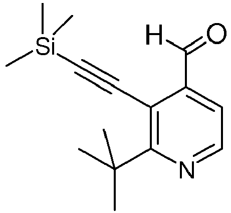 | 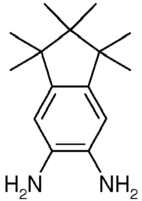 | 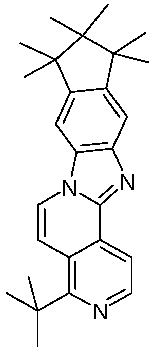 | 53 % |
| | S115 | S24 | | |
| L221 | 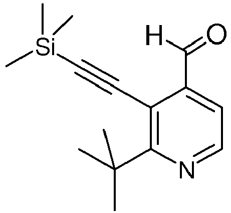 | 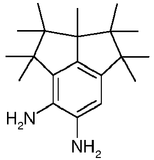 | 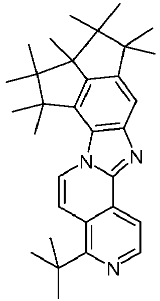 | 19 % |
| | S115 | S28 | Chromatographische Abtrennung des Regioisomeren | |
| L222 | 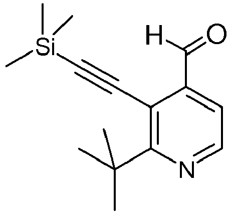 | 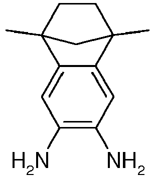 | 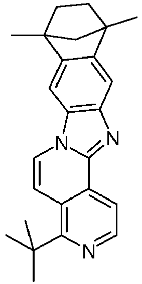 | 50 % |
| | S115 | S36 | | |
| L223 | 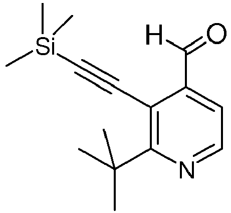 | 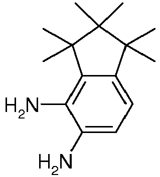 | 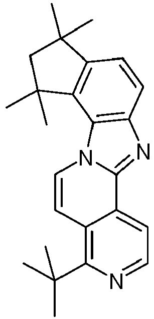 | 19 % |
| | S115 | S61 | Chromatographische Abtrennung des Regioisomeren | |
| L224 | 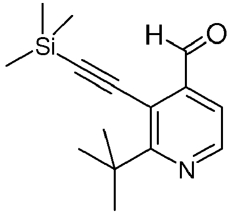 | 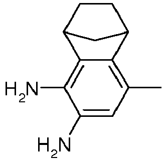 | 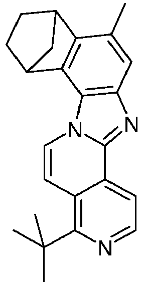 | 18 % |
| | S115 | S66 | Chromatographische Abtrennung des Regioisomeren | |
| L225 | 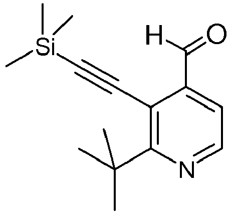 | 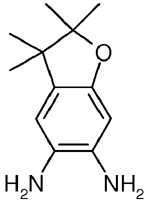 | 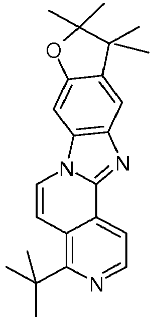 | 20 % |
| | S115 | S37 | Chromatographische Abtrennung des Regioisomeren | |

### 6) Liganden vom 6a,7,11- und vom 6a,10,11-Triaazabenzo[a]fluoren-Typ Allgemeine Ligandensynthese:

### Aus 2-Alkinyl-aryl-aldehyden und 2,3-Diamino-pyridinen:

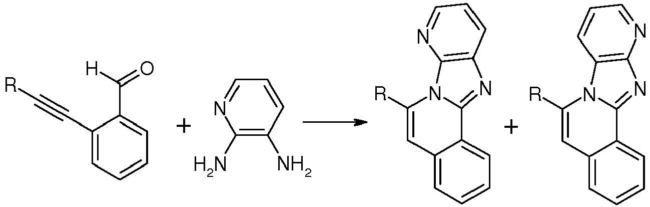

Darstellung analog zu 3) Liganden vom 2,6a,11-Triaza-benzo[a]fluoren-Typ.

### Beispiel L226 und L227:

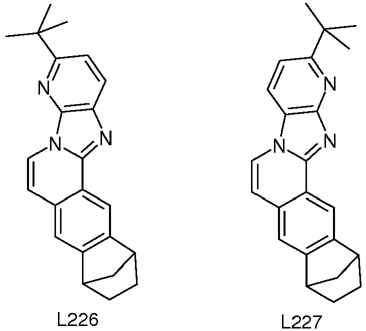

Einsatz von 134.2 g (500 mmol) S104 und 90.9 g (550 mmol) 2,3-Diamino-6-tert-butylpyridin [893444-20-7]. Chromatographische Trennung der Regioisomeren an Kieselgel (Heptan:Ethylacetat, 10:1 vv), zweimalige fraktionierte Sublimation der Produkte bei T ca. 180 °C, p ca. 10⁻⁴ mbar. Ausbeute L226: 41.7 g (112 mmol), 22 %; Reinheit: > 99.5 % ig nach ¹H-NMR.
Ausbeute L227: 44.4 g (130 mmol), 26 %; Reinheit: > 99.5 % ig nach ¹H-NMR.

Analog können folgende Derivate dargestellt werden:

| **Bsp.** | **2-Alkinyl-aryl-aldehyd** | **2,3-Diaminopyridin** | **Produkt** | **Ausbeute** |
|---|---|---|---|---|
| L228 | 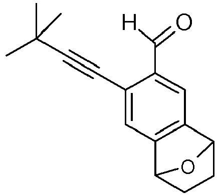 | 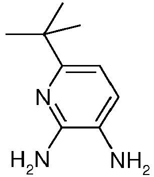 | 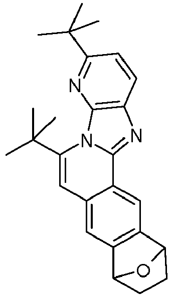 | 18 % |
| | S105 | 893444-20-7 | | |
| L229 | 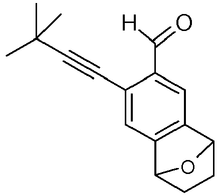 | 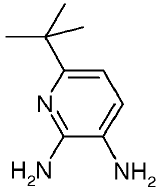 | 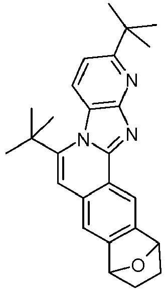 | 21 % |
| | S105 | 893444-20-7 | | |

### 7) Liganden vom 6a,8,11- und vom 6a,9,11-Triaaza-benzo[a] fluoren -Typ Allgemeine Ligandensynthese:

### Aus 2-Alkinyl-aryl-aldehyden und 3,4-Diamino-pyridinen:

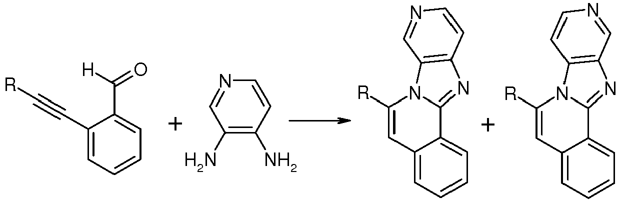

Darstellung analog zu 3) Liganden vom 2,6a,11-Triaza-benzo[a]fluoren-Typ.

### Beispiel L230 und L231:

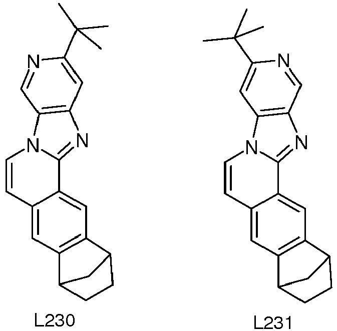

Einsatz von 134.2 g (500 mmol) S104 und 90.9 g (550 mmol) 3,4-Diamino-6-tert-butylpyridin [1237537-50-6]. Chromatographische Trennung der Regioisomeren an Kieselgel (Heptan:Ethylacetat, 10:1 vv), zweimalige fraktionierte Sublimation der Produkte bei T ca. 180 °C, p ca. 10⁻⁴ mbar. Ausbeute L230: 48.8 g (143 mmol), 28 %; Reinheit: > 99.5 % ig nach ¹H-NMR.
Ausbeute L231: 33.5 g (98 mmol), 19 %; Reinheit: > 99.5 % ig nach ¹H-NMR.

Analog können folgende Derivate dargestellt werden:

| **Bsp.** | **2-Alkinyl-aryl-aldehyd** | **3,4-Diamino-pyridin** | **Produkt** | **Ausbeute** |
|---|---|---|---|---|
| L232 | 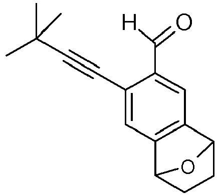 | 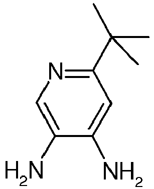 | 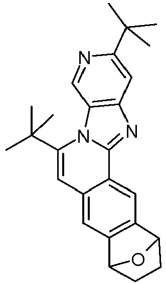 | 22 % |
| | S105 | 1237537-50-6 | | |
| L233 | 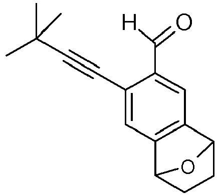 | 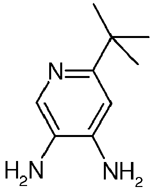 | 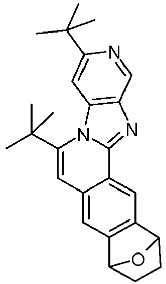 | 19 % |
| | S105 | 1237537-50-6 | | |

### 8) Liganden vom 2,5,6a,11-Tetraaza-benzo[a]fluoren-Typ Allgemeine Ligandensynthese:

### Aus 4-Amido-3-cyano-pyridinen und 1,2-Diamino-benzolen, Variante A:

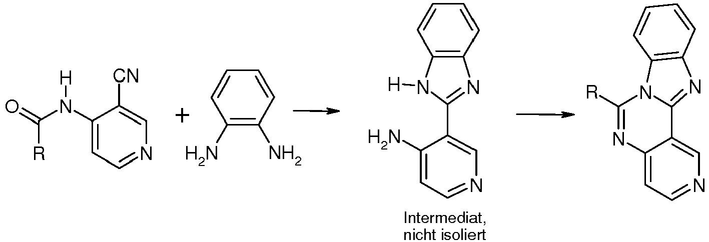

Ein Gemisch von 100 mmol des 4-Amido-3-cyano-pyridins, 210 mmol des 1,2-Diaminobenzols und 100 ml Nitrobenzol wird schrittweise bis zum schwachen Rückfluss erhitzt und gerührt bis das 4-Amido-3-cyano-pyridin verbraucht ist (typischerweise 6-16 h). Dann wird das Nitrobenzol zunächst im Argonstrom und gegen Ende durch Anlegen eines Vakuums (ca. 100 mbar) abdestilliert. Das so erhaltene Rohprodukt enthält neben dem 3-(1H-Benzimidazol-2-yl)-pyridin-4-ylamin noch 1 eq 2-R-¹H-Benzimidazol. Dieses Gemisch wird ohne weitere Reinigung umgesetzt. Nach Erkalten unter Argon zerkleinert man glasartige Rückstände mechanisch, Öle nimmt man ohne weitere Behandlung in 250 ml Dioxan oder Diethylenglykoldimethylether auf, versetzt mit 500 mmol des entsprechenden Carbonsäurechlorids und 50 mmol der entsprechenden Carbonsäure und erhitzt unter gutem Rühren (KPG-Rührer) unter Rückfluss bis um vollständigen Umsatz (typischerweise 4 - 48 h). Entsprechende Carbonsäurechloride und Carbonsäuren sind die, die den jeweiligen Amidrest bilden. Nach Erkalten wird die Reaktionsmischung unter gutem Rühren in ein Gemisch aus 1000 g Eis und 300 ml wässrigem konz. Ammoniak eingerührt. Fällt das Produkt als Feststoff an, wird dieser abgesaugt, mit Wasser gewaschen und trocken gesaugt. Fällt das Produkt als Öl an, wird dieses mit drei Portionen zu je 300 ml Ethylacetat oder Dichlormethan extrahiert. Die organische Phase wird abgetrennt, mit 500 ml Wasser gewaschen und im Vakuum eingeengt. Das Rohprodukt wird in Ethylacetat oder Dichlormethan aufgenommen, über eine kurze Säule aus Alox, basisch, Aktivitätsstufe 1 oder Kieselgel filtriert, um braune Verunreinigungen zu entfernen. Nach Umkristallisation (Methanol, Ethanol, Aceton, Dioxan, DMF, etc.) zur Entfernung des 1R-C(O)-2-R-Benzimidazols, wird das so erhaltene 2,5,6a,11-Tetraaza-benzo[a]fluoren durch Kugelrohrdestillation oder fraktionierte Sublimation (p ca. 1 x 10⁻⁵ mbar, T ca. 150 - 230 °C) von Leichtsiedern und ni cht flüchtigen Nebenkomponenten befreit. Verbindungen mit aliphatischen Resten, die mehr als 6 C-Atome aufweisen, bzw. solche mit Aralkylgruppen, die mehr als 9 C-Atome aufweisen, werden typischerweise chromatographisch gereinigt und dann im Vakuum getrocknet, um Leichtsieder zu entfernen. Reinheit nach ¹H-NMR typischerweise > 99.5 % ig.

### Aus 4-Amido-3-cyano-pyridinen und 1,2-Diamino-benzol-di-hydrochloriden, Variante B:

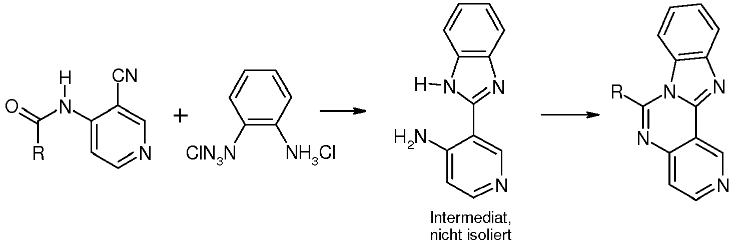

Ein im Mörser homogenisiertes Gemisch von 100 mmol des 4-Amido-3-cyano-pyridins und 300 mmol des 1,2-Diaminobenzol-Dihydrochlorids wird in ein auf 240 °C vorgeheiztes Ölbad eingestellt un d 3.5 h bei dieser Temperatur belassen. Nach Erkalten wird die tiefblaue Schmelze in einem Gemisch aus 150 ml Ethanol und 300 ml Wasser in der Wärme gelöst und dann tropfenweise unter gutem Rühren mit einer Lösung von 40 g Natriumcarbonat in 200 ml Wasser versetzt (Achtung: Schäumen, Kohlendioxidentwicklung). Nach beendeter Zugabe rührt man noch 30 min. nach, saugt dann vom grauen FS ab, wäscht diesen dreimal mit je 100 ml Wasser und trocknet im Vakuum. Es fällt ein 1:1 Gemisch aus dem dem 3-(1H-Benzimidazol-2-yl)-pyridin-4-ylamin und dem 2-R-¹H-Benzimidazol an, das ohne weitere Reinigung wie unter A beschrieben cyclisiert und anschließend gereinigt wird.

### Beispiel L234, Variante A:

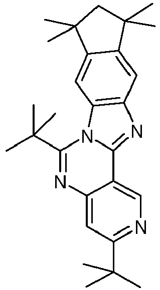

Einsatz von 25.9 g (100 mmol) N-(2-tert-Butyl-5-cyano-pyridin-4-yl)-2,2-dimethylpropionamid [1352329-37-3] und 42.9 g (210 mmol) S16, 60.3 g (500 mmol) Pivalinsäurechlorid [3282-30-2], 5.1 g (50 mmol) Pivalinsäure [75-98-9], 250 ml Diethylenglykoldimethylether, Reaktionszeit 8 h, das Rohprodukt fällt beim Neutralisieren als Feststoff an, Umkristallisation aus DMF/Ethanol, zweimalige fraktionierte Sublimation des Produktes bei T ca. 180 °C, p ca. 10⁻⁴ mbar. Ausbeute: 21.1 g (51 mmol), 51 %; Reinheit: ca. 99.5 % ig nach ¹H-NMR.

### Beispiel L234, Variante B:

Einsatz von 25.9 g (100 mmol) N-(2-tert-Butyl-5-cyano-pyridin-4-yl)-2,2-dimethylpropionamid [1352329-37-3] und 83.2 g (300 mmol) S16 x 2 HCl, 60.3 g (500 mmol) Pivalinsäurechlorid [3282-30-2], 5.1 g (50 mmol) Pivalinsäure [75-98-9], 250 ml Diethylenglykoldimethylether, Reaktionszeit 8 h, das Rohprodukt fällt beim Neutralisieren als Feststoff an, Umkristallisation aus DMF/Ethanol, zweimalige fraktionierte Sublimation des Produktes bei T ca. 180 °C, p ca. 10⁻⁴ mbar. Ausbeute: 32.5 g (76 mmol), 76 %; Reinheit: ca. 99.5 % ig nach ¹H-NMR.

Analog können folgende Derivate dargestellt werden:

| **Bsp.** | **4-Amido-3-cyano-pyridin** | **1,2-Diamino-benzol** | **Produkt Variante** | **Ausbeute** |
|---|---|---|---|---|
| L235 | 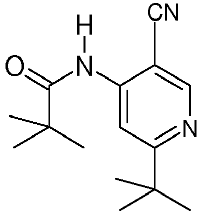 | 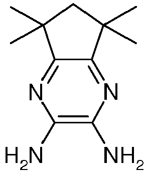 | 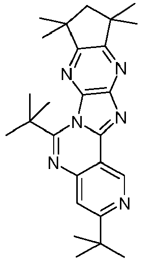 | 36 % |
| | 1352329-37-3 | S22 | A | |
| L236 | 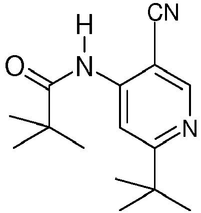 | 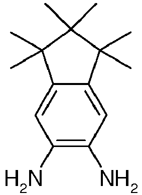 | 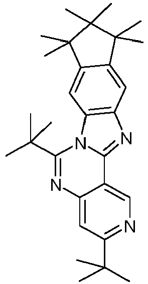 | 68 % |
| | 1352329-37-3 | S24 x 2HCl | B | |
| L237 | 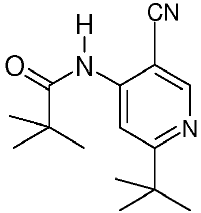 | 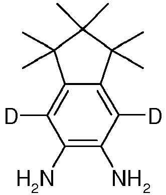 | 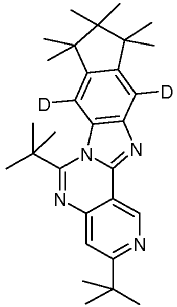 | 65 % |
| | 1352329-37-3 | S26 x 2 HCl | B | |
| L238 | 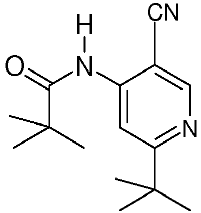 | 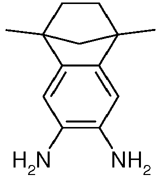 | 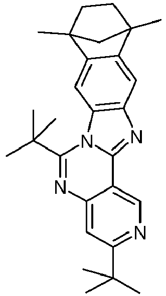 | 67 % |
| | 1352329-37-3 | S36 x 2 HCl | B | |
| L239 | 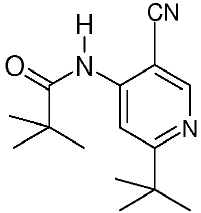 | 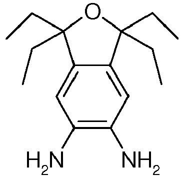 | 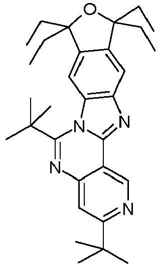 | 47 % |
| | 1352329-37-3 | S40 | A | |
| L240 | 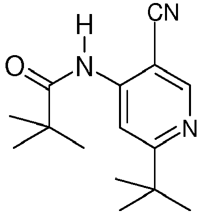 | 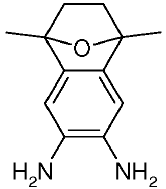 | 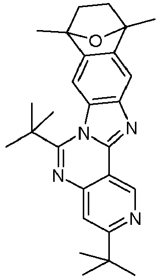 | 45 % |
| | 1352329-37-3 | S46 x 2 HCl | A | |
| L241 | 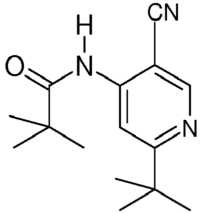 | 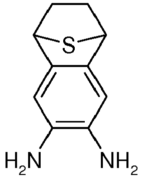 | 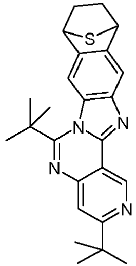 | 27% |
| | 1352329-37-3 | S54 x 2 HCl | B | |
| L242 | 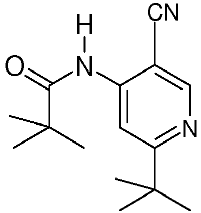 | 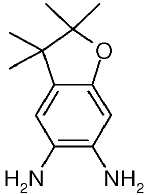 | 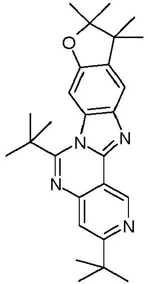 | 20 % |
| | 1352329-37-3 | S37 x 2 HCl | B | |
| | | | Chromatographische Abtrennung des Regioisomeren | |

### 9) Liganden vom 4,5,6a,11-Tetraaza-benzo[a]fluoren-Typ Allgemeine Ligandensynthese:

### Aus 2-Amido-3-cyano-pyridinen und 1,2-Diamino-benzolen:

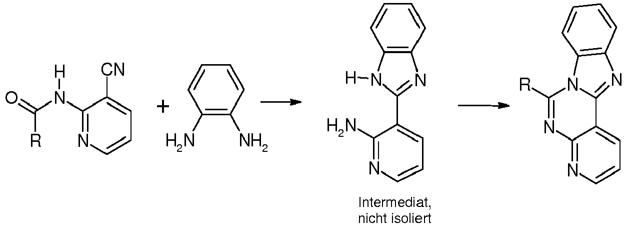

### Aus 2-Amido-3-cyano-pyridinen und 1,2-Diamino-benzol-di-hydrochloriden, Variante B:

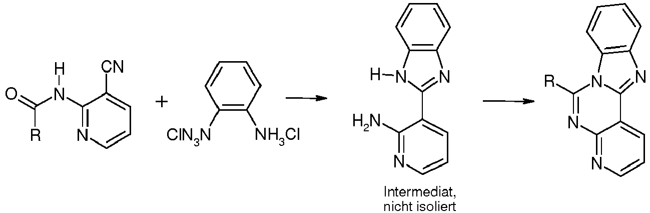

Darstellung analog 8) Liganden vom 2,5,6a,11-Tetraaza-benzo[a]fluoren-Typ.

### Beispiel L243, Variante A:

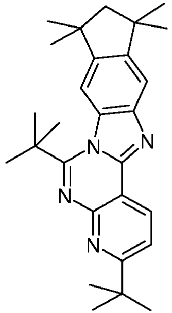

Einsatz von 25.9 g (100 mmol) N-(6-tert-Butyl-3-cyano-pyridin-2-yl)-2,2-dimethylpropionamid [1352329-36-2] und 42.9 g (210 mmol) S16, 60.3 g (500 mmol) Pivalinsäurechlorid [3282-30-2], 4.1 g (40 mmol) Pivalinsäure [75-98-9], 250 ml Diethylenglykoldimethylether, Reaktionszeit 8 h, das Rohprodukt fällt beim Neutralisieren als Feststoff an, Umkristallisation aus Dioxan/Ethanol, zweimalige fraktionierte Sublimation des Produktes bei T ca. 180 °C, p ca. 10⁻⁴ mbar. Ausbeute: 20.1 g (47 mmol), 47 %; Reinheit: ca. 99.5 % ig nach ¹H-NMR.

Analog können folgende Derivate dargestellt werden:

| **Bsp.** | **2-Amido-3-cyano-pyridin** | **1,2-Diaminobenzol** | **Produkt** | **Aus-beute** |
|---|---|---|---|---|
| L244 | 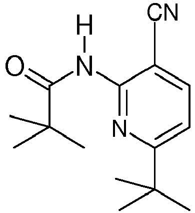 | 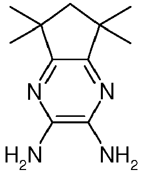 | 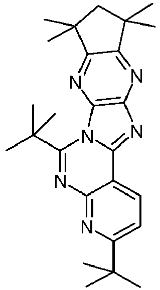 | 46 % |
| | 1352329-36-2 | S22 | A | |
| L245 | 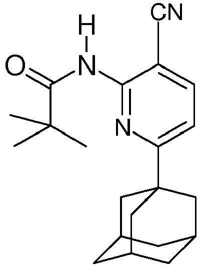 | 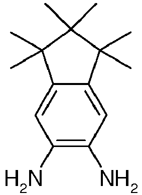 | 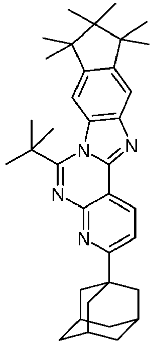 | 40 % |
| | 111678-77-4 | S24 x 2 HCl | B | |
| L246 | 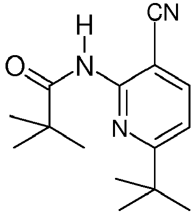 | 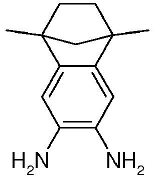 | 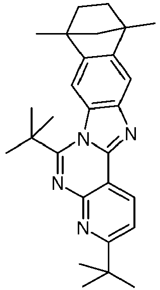 | 48 % |
| | 1352329-36-2 | S36 x 2 HCl | B | |
| L247 | 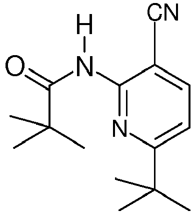 | 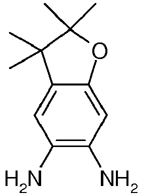 | 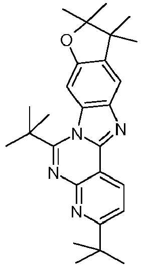 | 17% |
| | 1352329-36-2 | S37 x 2 HCl | B | |
| | | | Chromatographische Abtrennung des Regioisomeren | |

### 10) Liganden vom 2,4,6a,11-Tetraaza-benzo[a]fluoren-Typ Allgemeine Ligandensynthese:

### Aus Pyrimidin-5-yl-benzimidazolen und Alkinen:

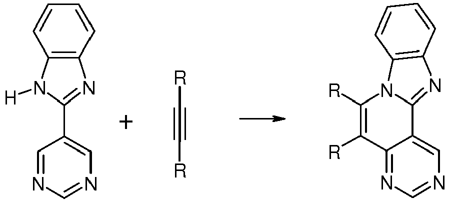

In einem Druckschlenkrohr wird eine Lösung von 100 mmol des Pyrimidin-5-yl-benzimidazols und 110 mmol des Alkins in 400 ml DMF vorgelegt, mit 1.5 g (4 mmol) Tetraphenylcyclopentadien, 547 mg (1 mmol) Pentamethylcyclopentadienyl-rhodium-chlorid-dimer und 21.0 g (105 mmol) Kupfer(II)-acetat-monohydrat versetzt und 18 h bei 100 °C vers chlossen gerührt. Nach Erkalten wird das DMF im Vakuum entfernt, der Rückstand wird in 1000 ml THF aufgenommen und über eine kurze Kieselgel-Säule filtriert. Nach Entfernen des THF im Vakuum wird der ölige Rückstand umkristallisiert oder chormotographiert und anschließend durch Kugelrohrdestillation oder fraktionierte Sublimation (p ca. 1 x 10⁻⁵ mbar, T ca. 150 - 230 °C) von Leichtsiedern und nicht flüchtige n Nebenkomponenten befreit. Verbindungen mit aliphatischen Resten, die mehr als 6 C-Atome aufweisen, bzw. solche mit Aralkylgruppen, die mehr als 9 C-Atome aufweisen, werden typischerweise chromatographisch gereinigt und dann im Vakuum getrocknet, um Leichtsieder zu entfernen. Reinheit nach ¹H-NMR typischerweise > 99.5 % ig.

### Beispiel L248:

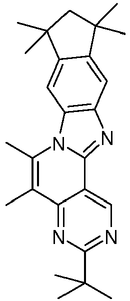

Einsatz von 34.9 g (100 mmol) 2-(2-tert-Butyl-pyrimidin-5-yl)-5,5-7,7-tetramethyl-1,5,6,7-tetrahydro-indeno[5,6-d]imidazol, S110 und 6.0 g (110 mmol) But-2-in. Umkristallisation aus Dioxan/Ethanol, zweimalige fraktionierte Sublimation des Produktes bei T ca. 200 °C, p ca. 10⁻⁴ mbar. Ausbeute: 10.8 g (27 mmol), 27 %; Reinheit: ca. 99.5 % ig nach ¹H-NMR.

Analog können folgende Derivate dargestellt werden:

| **Bsp.** | **2-(2-tert-Butyl-pyrimidin-5-yl)-benzimidazol** | **Alkin** | **Produkt** | **Aus-beute** |
|---|---|---|---|---|
| L249 | 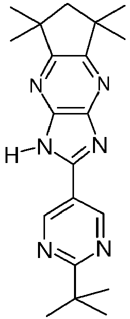 | 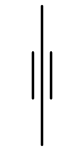 | 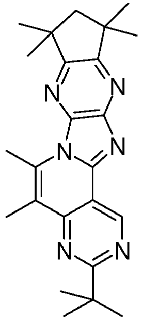 | 31 % |
| | S111 | 503-17-3 | | |
| L250 | 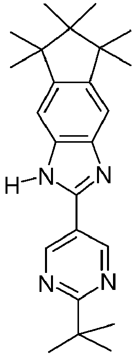 | 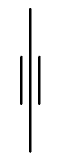 | 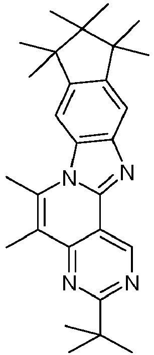 | 26 % |
| | S112 | 503-17-3 | | |
| L251 | 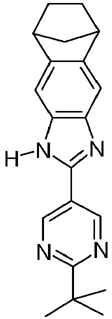 | 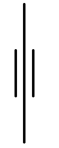 | 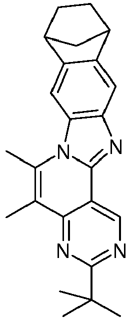 | 33 % |
| | S113 | 503-17-3 | | |

### 11) Liganden vom 3,5,6a,11-Tetraaza-benzo[a]fluoren-Typ Allgemeine Ligandensynthese:

### Aus 3-Amido-4-formyl-pyridinen und 1,2-Diamino-benzolen:

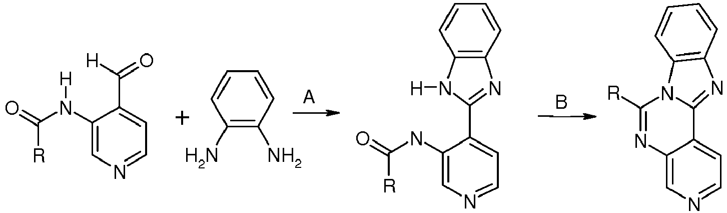

Darstellung analog 2) Liganden vom Benzo[4,5]imidazo[2,1-c]chinazolin-Typ.

### Beispiel L252, Variante A:

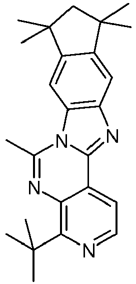

### Schritt A:

Einsatz von 22.0 g (100 mmol) S116 und 22.5 g (110 mmol) S16. Das N-[2-tert-Butyl-4-(5,5,7,7-tetramethyl-1,5,6,7-tetrahydro-indeno[5,6-d]imidazol-2-yl)-pyridin-3-yl]acetamid kristallisiert aus, Ausbeute 33.6 g (83 mmol) 83 %; Reinheit: 97 % nach ¹H-NMR.

### Schritt B, Variante A:

Einsatz von 33.6 g (83 mmol) N-[2-tert-Butyl-4-(5,5,7,7-tetramethyl-1,5,6,7-tetrahydro-indeno[5,6-d]imidazol-2-yl)-pyridin-3-yl]acetamid (Schritt A), 100 ml Dioxan, 20.0 ml (280 mmol) Acetylchlorid [75-36-5] und 2.3 ml (40 mmol) Essigsäure [64-19-7], Reaktionszeit 16 h, das Rohprodukt fällt beim Neutralisieren als Feststoff an, Umkristallisation aus Dioxan/Ethanol, zweimalige fraktionierte Sublimation des Produktes bei T ca. 180 °C, p ca. 10⁻⁴ mbar. Ausbeute: 21.6 g (56 mmol), 68 %; Reinheit: ca. 99.5 % ig nach ¹H-NMR.

Analog können folgende Derivate dargestellt werden:

| **Bsp.** | **3-Amido-4-formyl-pyridin** | **1,2-Diaminobenzol** | **Produkt Variante** | **Ausbeute** |
|---|---|---|---|---|
| L253 | 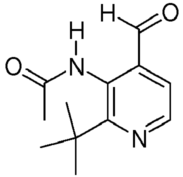 | 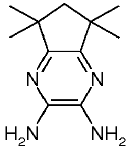 | 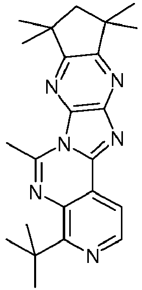 | 52 % |
| | S116 | S22 | A | |
| L254 | 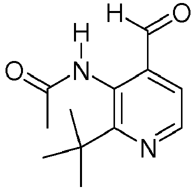 | 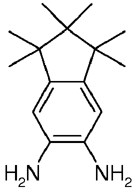 | 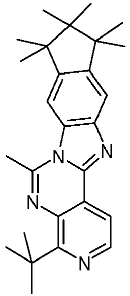 | 55 % |
| | S116 | S24 | | |
| | | | A | |
| L255 | 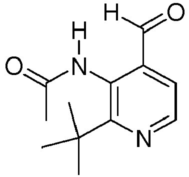 | 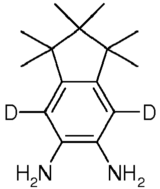 | 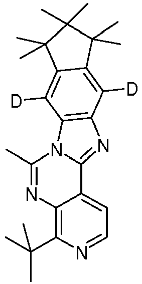 | 52 % |
| | S116 | S26 | A | |
| L256 | 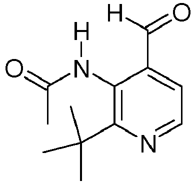 | 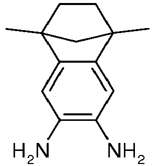 | 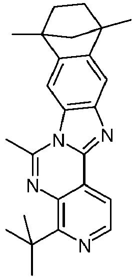 | 48 % |
| | S116 | S36 | A | |
| L257 | 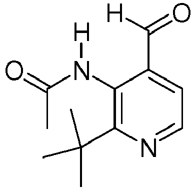 S116 | 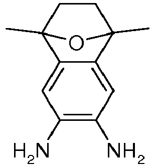 S46 | 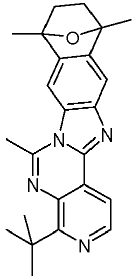 | 44 % |
| | | | A | |
| L258 | 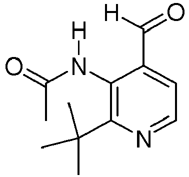 | 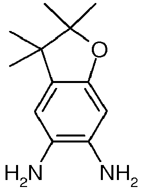 | 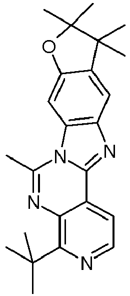 | 18 % |
| | S116 | S37 | A | |
| | | | Chromatographische Trennung der Regioisomeren | |

### 12) Liganden vom 5,6a,7,11- und 5,6a,10,11-Tetraaza-benzo[a]fluoren- Typ Allgemeine Ligandensynthese Variante A:

### Aus 2-Amido-arylaldehyden und 2,3-Diaminopyridinen:

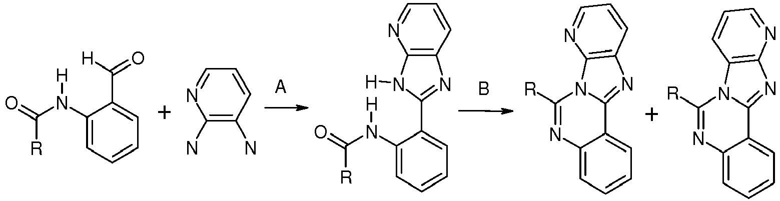

Darstellung analog 2) Liganden vom Benzo[4,5]imidazo[2,1-c]chinazolin-Typ.

### Beispiel L259 und L260, Variante A:

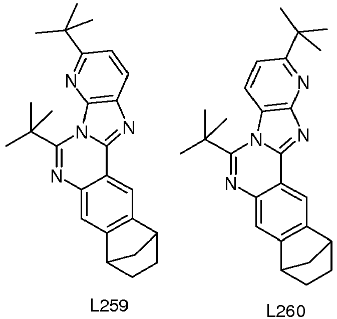

### Schritt A:

Einsatz von 27.1 g (100 mmol) S99 und 18.2 g (110 mmol) 2,3-Diamino-6-tert-butyl-pyridin [893444-20-7]. Das N-[7-(5-tert-Butyl-3H-imidazo[4,5-b]pyridin-2-yl)1,2,3,4-tetrahydro-1,4-methano-naphthalin-6-yl]-2,2-dimethylpropanamid kristallisiert aus. Ausbeute: 32.1 g (77 mmol) 77 %; Reinheit: 97 % nach ¹H-NMR.

### Schritt B, Variante A:

Einsatz von 32.1 g (77 mmol) N-[7-(5-tert-Butyl-3H-imidazo[4,5-b]pyridin-2-yl)1,2,3,4-tetrahydro-1,4-methano-naphthalin-6-yl]-2,2-dimethylpropanamid (Schritt A), 100 ml Dioxan, 31.4 g (260 mmol) Pivalinsäurechlorid [3282-30-2] und 3.8 g (37 mmol) Pivalinsäure [75-98-9], Reaktionszeit 18 h, das Rohprodukt fällt beim Neutralisieren als Feststoff an. Chromatographische Trennung der Regioisomeren an Kieselgel (Heptan:Ethylacetat, 10:1 vv), zweimalige fraktionierte Sublimation der Produkte bei T ca. 180 °C, p ca. 10⁻⁴ mbar.

Ausbeute L259: 9.1 g (23 mmol), 30 %; Reinheit: > 99.5 % ig nach ¹H-NMR.
Ausbeute L260: 7.2 g (18 mmol), 23 %; Reinheit: > 99.5 % ig nach ¹H-NMR.

Analog können folgende Derivate dargestellt werden:

| **Bsp.** | **2-Amido-Aryl-aldehyd** | **2,3-Diaminopyridin** | **Produkt Variante** | **Ausbeute** |
|---|---|---|---|---|
| L261 | 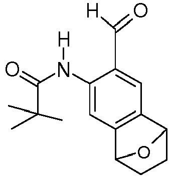 | 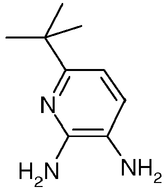 | 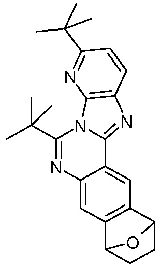 | 25 % |
| | S100 | 893444-20-7 | A | |
| L262 | 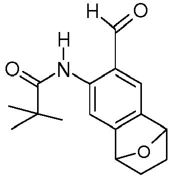 | 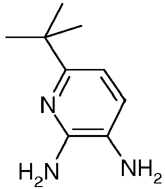 | 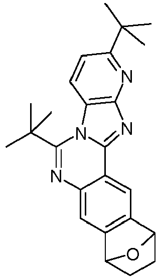 | 21 % |
| | S100 | 893444-20-7 | A | |

### 13) Liganden vom 5,6a,8,11- und 5,6a,9,11-Tetraaza-benzo[a]fluoren-Typ Allgemeine Ligandensynthese Variante A:

### Aus 2-Amido-arylaldehyden und 3,4-Diaminopyridinen:

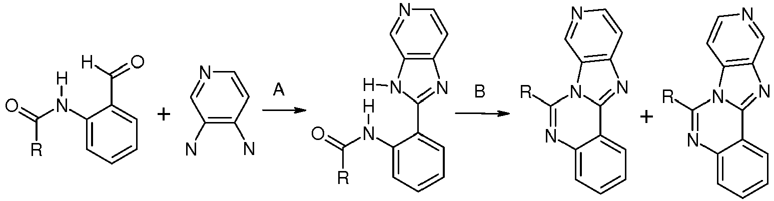

Darstellung analog 2) Liganden vom Benzo[4,5]imidazo[2,1-c]chinazolin- Typ.

### Beispiel L263 und L264, Variante A:

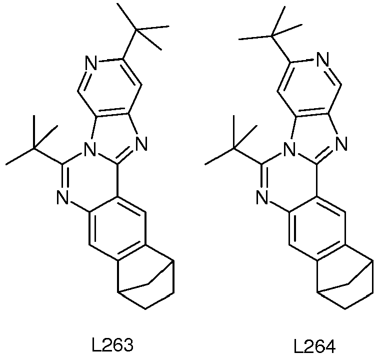

### Schritt A:

Einsatz von 27.1 g (100 mmol) S99 und 18.2 g (110 mmol) 3,4-Diamino-6-tert-butyl-pyridin [1237537-50-6]. Das N-[7-(6-tert-Butyl-3H-imidazo[4,5-c]pyridin-2-yl)1,2,3,4-tetrahydro-1,4-methano-naphthalin-6-yl]-2,2-dimethylpropanamid kristallisiert aus. Ausbeute: 28.8 g (69 mmol) 77 %; Reinheit: 97 % nach ¹H-NMR.

### Schritt B, Variante A:

Einsatz von 28.8 g (69 mmol) N-[7-(6-tert-Butyl-3H-imidazo[4,5-c]pyridin-2-yl)1,2,3,4-tetrahydro-1,4-methano-naphthalin-6-yl]-2,2-dimethylpropanamid (Schritt A), 100 ml Dioxan, 27.9 g (230 mmol) Pivalinsäurechlorid [3282-30-2] und 3.4 g (33 mmol) Pivalinsäure [75-98-9], Reaktionszeit 18 h, das Rohprodukt fällt beim Neutralisieren als Feststoff an. Chromatographische Trennung der Regioisomeren an Kieselgel (Heptan:Ethylacetat, 10:1 vv), zweimalige fraktionierte Sublimation der Produkte bei T ca. 180 °C, p ca. 10⁻⁴ mbar.
Ausbeute L263: 7.2 g (18 mmol), 26 %; Reinheit: > 99.5 % ig nach ¹H-NMR.
Ausbeute L264: 6.4 g (16 mmol), 23 %; Reinheit: > 99.5 % ig nach ¹H-NMR.

Analog können folgende Derivate dargestellt werden:

| **Bsp.** | **2-Amido-Aryl-aldehyd** | **3,4-Diaminopyridin** | **Produkt Variante** | **Aus-beute** |
|---|---|---|---|---|
| L265 | 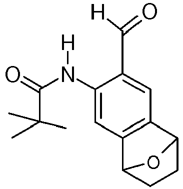 | 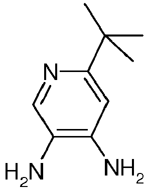 | 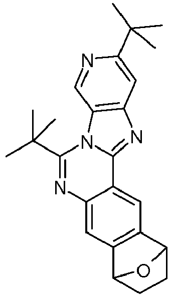 | 25 % |
| | S100 | 1237537-50-6 | A | |
| L266 | 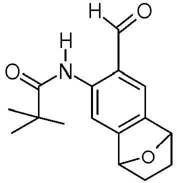 | 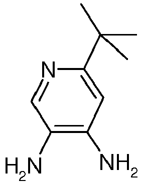 | 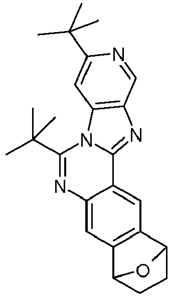 | 16 % |
| | S100 | 1237537-50-6 | A | |

### 14) Liganden vom 6a,7,9,11- und 6a,8,10,11-Tetraaza-benzo[a]fluoren- Typ Allgemeine Ligandensynthese:

### Aus 2-Amido-arylaldehyden und 5,6-Diaminopyrimidinen:

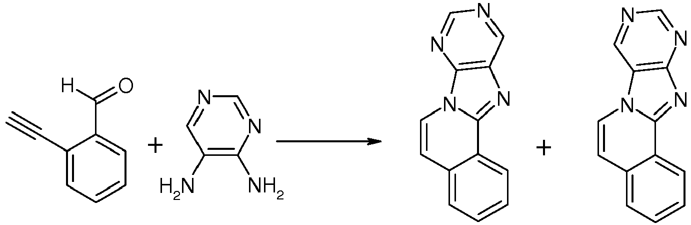

Darstellung analog zu 3) Liganden vom 2,6a,11-Triaza-benzo[a]fluoren-Typ

### Beispiel L267 und L268:

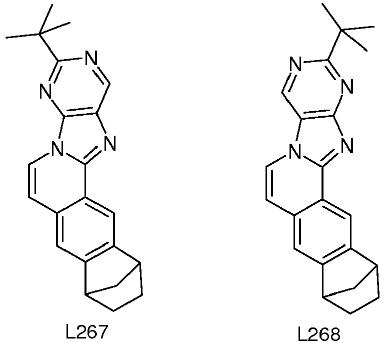

Einsatz von 134.2 g (500 mmol) S104 und 90.9 g (550 mmol) 2-tert-Butyl - 5,6-diamino-pyrimidin [18202-78-3]. Chromatographische Trennung der Regioisomeren an Kieselgel (Heptan:Ethylacetat, 10:1 vv), zweimalige fraktionierte Sublimation der Produkte bei T ca. 180 °C, p ca. 10⁻⁴ mbar. Ausbeute L267: 53.4 g (156 mmol), 31 %; Reinheit: > 99.5 % ig nach ¹H-NMR.
Ausbeute L268: 34.6 g (101 mmol), 20 %; Reinheit: > 99.5 % ig nach ¹H-NMR.

Analog können folgende Derivate dargestellt werden:

| **Bsp.** | **2-Alkinyl-aryl-aldehyd** | **5,6-Diaminopyrimidin** | **Produkt** | **Aus-beute** |
|---|---|---|---|---|
| L269 | 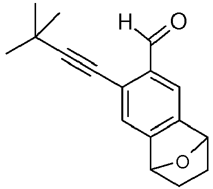 | 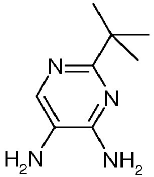 | 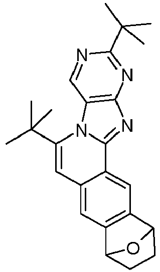 | 26 % |
| | S105 | 18202-78-3 | | |
| L270 | 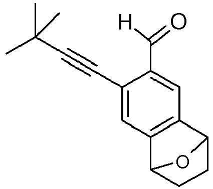 | 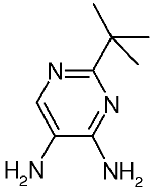 | 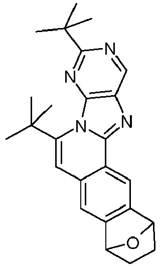 | 23 % |
| | S105 | 18202-78-3 | | |

### 15) Liganden vom 2,4,5,6a,11-Pentaaza-benzo[a]fluoren-Typ Allgemeine Ligandensynthese:

### Aus 4-Amido-5-methoxycarbonyl-pyrimidinen und 1,2-Diamino-benzolen-di-hydrochlorioden:

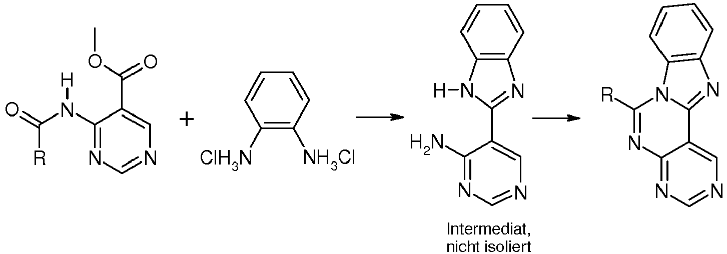

Darstellung analog 8) Variante B Liganden vom 2,5,6a,11-Tetraazabenzo[a]fluoren-Typ, wobei anstatt der 4-Amido-3-cyano-pyridine die 4-Amido-5-methoxycarbonyl-pyrimidine eingesetzt werden.

### Beispiel L271:

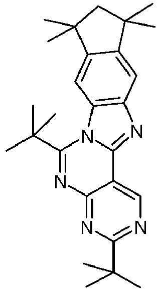

Einsatz von 29.3 g (100 mmol) 2-tert-Butyl-4-(2,2-dimethyl-propylamido)-pyrimidin-5-carbonsäuremethylester [1352329-45-3] und 83.2 g (300 mmol) S16 x 2 HCl, 60.3 g (500 mmol) Pivalinsäurechlorid [3282-30-2], 5.1 g (50 mmol) Pivalinsäure [75-98-9], 250 ml Diethylenglykoldimethylether, Reaktionszeit 8 h, das Rohprodukt fällt beim Neutralisieren als Feststoff an, Umkristallisation aus DMF/Ethanol, zweimalige fraktionierte Sublimation des Produktes bei T ca. 190 °C, p ca. 10⁻⁴ mbar. Ausbeute: 28.4 g (66 mmol), 66 %; Reinheit: ca. 99.5 % ig nach ¹H-NMR.

Analog können folgende Derivate dargestellt werden:

| **Bsp.** | **4-Amido-5-methoxycarbonylpyrimidin** | **1,2-Diaminobenzol-dihydrochlorid** | **Produkt** | **Aus-beute** |
|---|---|---|---|---|
| L272 | 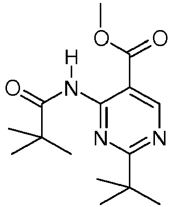 | 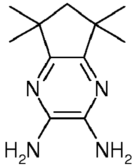 | 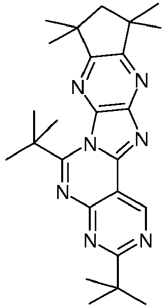 | 63 % |
| | 1352329-45-3 | S22 x 2HCl | | |
| L273 | 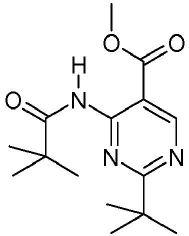 | 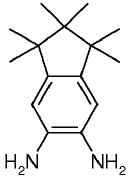 | 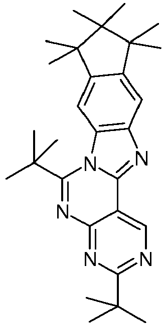 | 58 % |
| | 1352329-45-3 | S24 x 2 HCl | | |
| L274 | 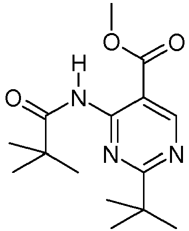 | 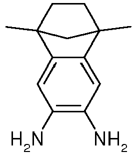 | 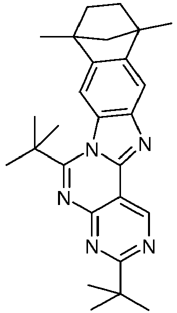 | 55 % |
| | 1352329-45-3 | S36 x 2 HCl | | |
| L275 | 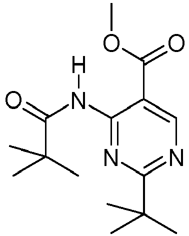 | 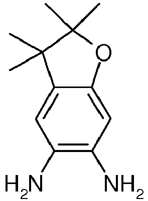 | 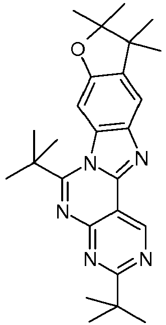 | 19 % |
| | 1352329-45-3 | S37 x 2 HCl | Chromatographische Abtrennung des Regioisomeren | |

### 16) Liganden vom Imidazo[1,2-f]phenanthridin-Typ Allgemeine Ligandensynthese Variante A:

### Aus 6-Amino-phenanthridinen und 2-Halogenketonen:

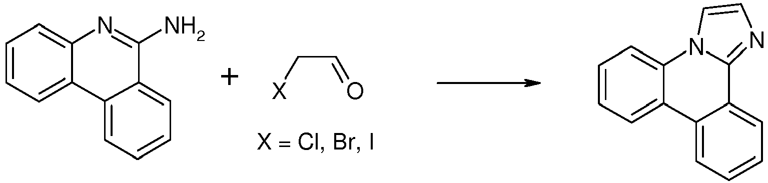

Ein Gemisch aus 100 mmol des 6-Amino-phenanthridin-Derivats, 300 mmol des Halogenketons, 300 mmol Natriumhydrogencarbonat, 300 ml Ethylenglycol und 30 ml Wasser wird 24 h bei 130 °C gerührt. Dann gibt man weitere 300 mmol des 2-Halogenketons und 300 mmol Natriumhydrogencarbonat zu, und rührt weitere 24 h bei 130 °C. Nach Erkalten verdünnt man die Reaktionsmischung mit 1000 ml Wasser, extrahiert dreimal mit je 300 ml Ethylacetat oder Dichlormethan, wäscht die vereinigten organischen Phasen mit 500 ml Wasser, mit 500 ml gesättigte Kochsalzlösung, engt die org. Phase im Vakuum ein und kristallisiert den Rückstand um oder chromatographiert an Kieselgel. Die so erhaltenen Feststoffe bzw. Öle werden durch fraktionierte Kugelrohrdestillation oder Sublimation (p ca. 10⁻⁴ - 10⁻⁵ mbar, T ca. 160 - 240 °C) von Leichtsiedern und nicht flüchtigen Nebenkomponenten befreit. Verbindungen mit aliphatischen Resten, die mehr als 6 C-Atome aufweisen, bzw. solche mit Aralkylgruppen, die mehr als 9 C-Atome aufweisen, werden typischerweise chromatographisch gereinigt und dann im Vakuum getrocknet, um Leichtsieder zu entfernen. Reinheit nach ¹H-NMR typischerweise > 99.5 % ig.

### Allgemeine Ligandensynthese Variante B:

### Aus 6-Amino-phenanthridinen und 1,2-Dihalogenolefinen:

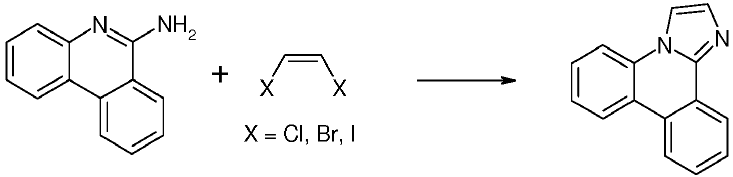

Ein Gemisch aus 100 mmol des 6-Amino-phenanthridin-Derivats, 200 mmol des 1,2-Dihalogenolefins, 300 mmol Kaliumcarbonat, 5 mmol Paladium(II)acetat, 30 mmol Triphenylphosphin, 100 g Glaskugeln (3 mm Durchmesser) und 200 ml o-Xylol wird unter gutem Rühren so lange unter Rückfluss (typischerweise 6 - 24 h) erhitzt, bis das 6-Amino-phenanthridin verbraucht ist. Nach Abkühlen auf 80 °C saugt man von den Salzen und den Glaskugeln über ein Celite-Bett ab, wäscht diese mit 300 heißem o-Xylol nach, engt das Filtrat im Vakuum zur Trockene ein und Kristallisiert den Rückstand um oder chromatographiert an Kieselgel. Die so erhaltenen Feststoffe bzw. Öle werden durch fraktionierte Kugelrohrdestillation oder Sublimation (p ca. 10⁻⁴ - 10⁻⁵ mbar, T ca. 160 - 240 °C) von Leichtsiedern und nicht flüchtigen Nebenkomponenten befreit. Reinheit nach ¹H-NMR typischerweise > 99.5 % ig.

### Beispiel L500:

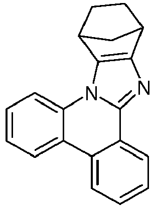

### Variante A:

Einsatz von 19.4 g (100 mmol) 6-Amino-phenanthridin [832-68-8] und 43.4 g (300 mmol) 3-Chlor-(1R,3R,4S)-bicyclo[2.2.1]heptan-2-on [10464-71-8], Chromatographie an Kieselgel (Ethylacetat: Dichlormethan 80 : 20 vv), dann Umkristallisation aus DMF/Ethanol, zweimalige fraktionierte Sublimation des Produktes bei T ca. 190 °C, p ca. 10⁻⁴ mbar. Ausbeute: 5.4 g (19 mmol), 19 %; Reinheit: ca. 99.5 % ig nach ¹H-NMR.

### Variante B:

Einsatz von 19.4 g (100 mmol) 6-Amino-phenanthridin [832-68-8] und 50.4 g (200 mmol) 2,3-Dibrom-bicyclo[2.2.1]hept-2-en [75267-72-0], Chromatographie an Kieselgel (Ethylacetat: Dichlormethan 80 : 20 vv), dann Umkristallisation aus DMF/Ethanol, zweimalige fraktionierte Sublimation des Produktes bei T ca. 190 °C, p ca. 10⁻⁴ mbar. Ausbeute: 10.8 g (38 mmol), 38 %; Reinheit: ca. 99.5 % ig nach ¹H-NMR.

Analog können folgende Derivate dargestellt werden:

| **Bsp.** | **6-Amino-phenanthridin** | **2-Halogenketon-bzw. 1,2-Dihalogen-**olefin | **Produkt** | **Aus-beute** |
|---|---|---|---|---|
| L501 | 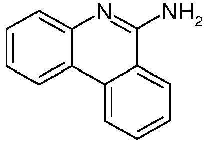 | 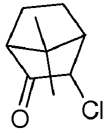 | 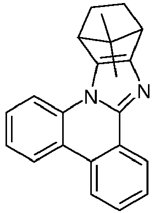 | 13 % |
| | 832-68-8 | 17215-81-5 | | |
| L502 | 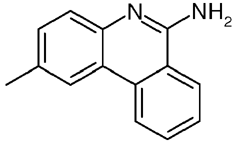 | 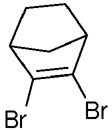 | 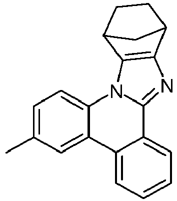 | 34 % |
| | 855829-20-8 | 75267-72-0 | | |
| L503 | 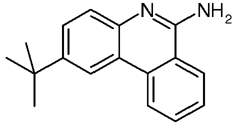 | 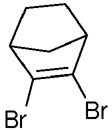 | 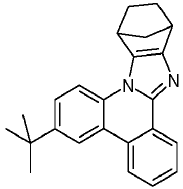 | 28 % |
| | 946147-22-4 | 75267-72-0 | | |
| L504 | 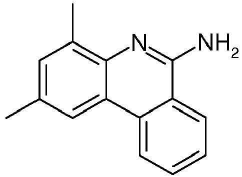 | 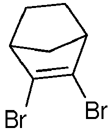 | 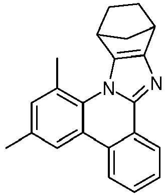 | 29 % |
| | 855830-85-2 | 75267-72-0 | | |
| L505 | 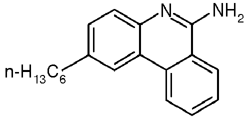 | 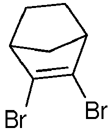 | 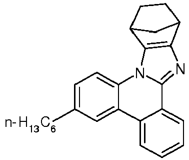 | 35 % |
| | | 75267-72-0 | | |
| L506 | 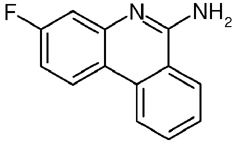 | 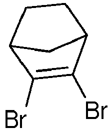 | 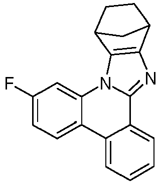 | 34 % |
| | 714243-31-9 | 75267-72-0 | | |
| L507 | 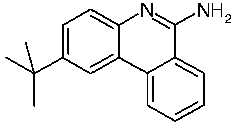 | 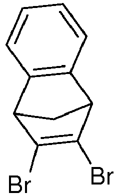 | 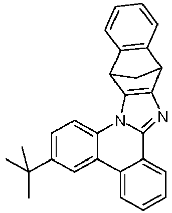 | 37 % |
| | 946147-22-4 | 194086-05-0 | | |
| L508 | 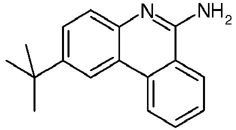 | 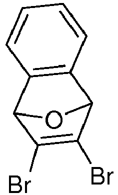 | 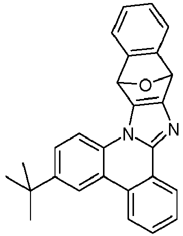 | 35 % |
| | 946147-22-4 | 301829-08-3 | | |
| L509 | 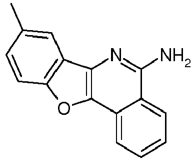 | 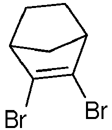 | 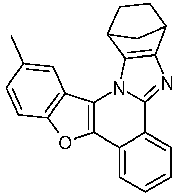 | 32 % |
| | 1293961-03-1 | 75267-72-0 | | |
| L510 | 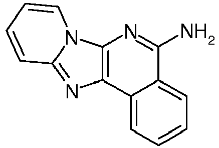 | 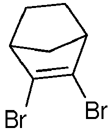 | 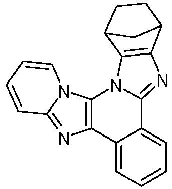 | 27% |
| | 94718-73-7 | 75267-72-0 | | |

### 17) Liganden vom 8b,13-Diaza-indeno[1,2-I]phenanthren-Typ Allgemeine Ligandensynthese Variante A:

### Aus 6-Amino-phenanthridinen und 1,2-Dihalogenbenzolen:

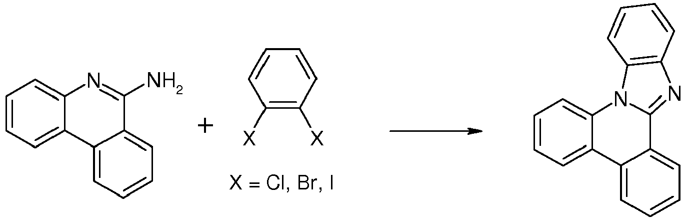

Eine gut gerührte Mischung von 100 mmol des 6-Amino-phenanthridin-Derivats, 130 mmol des 1,2-Halogenbenzols, 130 mmol Kaliumcarbonat, 100 g Glaskugeln (3 mm Durchmesser) 8 mmol Triphenylphosphin und 2 mmol Palladium(II)acetat in 300 ml o-Xylol wird 3 - 48 h unter Rückfluss erhitzt, bis das 6-Amino-phenanthridin-Derivat verbraucht ist. Nach Abkühlen auf 80 °C saugt man von den Salzen und den Glaskugeln über ein Celite-Bett ab, wäscht diese mit 500 heißem o-Xylol nach, engt das Filtrat im Vakuum zur Trockene ein und kristallisiert den Rückstand um oder chromatographiert an Kieselgel. Die so erhaltenen Feststoffe bzw. Öle werden durch fraktionierte Kugelrohrdestillation oder Sublimation (p ca. 10⁻⁴ - 10⁻⁵ mbar, T ca. 160 - 240 °C) von Leichtsiedern und nicht flüchtigen Nebenkomponenten befreit. Verbindungen mit aliphatischen Resten, die mehr als 6 C-Atome aufweisen, bzw. solche mit Aralkylgruppen, die mehr als 9 C-Atome aufweisen, werden typischerweise chromatographisch gereinigt und dann im Vakuum getrocknet, um Leichtsieder zu entfernen. Reinheit nach ¹H-NMR typischerweise > 99.5 % ig.

### Allgemeine Ligandensynthese Variante B:

### Aus 6-Halogen-phenanthridinen und 1-Amino-2-halogenbenzolen:

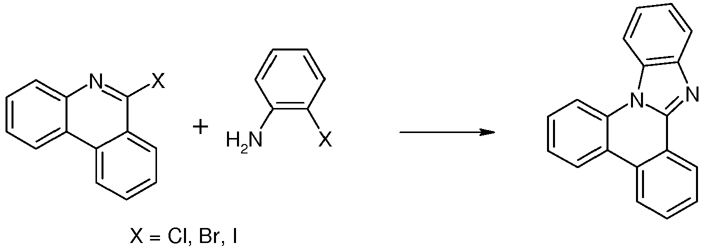

Darstellung analog zu 1) Liganden vom Benzo[4,5]imidazo[2,1-a]isochinolin-Typ.

### Beispiel L511:

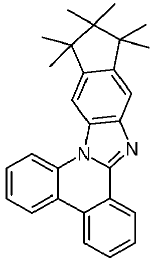

### Variante B:

Einsatz von 213.67 g (100 mmol) 6-Chlor-phenanthridin [15679-03-5] und 35.6 g (120 mmol) S6, Umkristallisation aus Dioxan/Ethanol, zweimalige fraktionierte Sublimation des Produktes bei T ca. 210 °C, p ca. 10⁻⁴ mbar. Ausbeute: 22.8 g (58 mmol), 58 %; Reinheit: ca. 99.5 % ig nach ¹H-NMR.

### Allgemeine Ligandensynthese Variante C:

### Aus 6-Amino-phenanthridinen und 1,2-Halogen-benzolen:

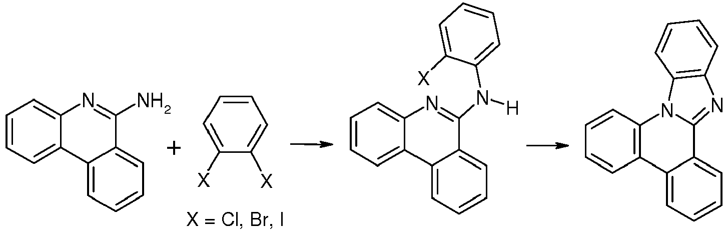

Darstellung analog 1) Variante C, Liganden vom Benzo[4,5]imidazo[2,1-a]isochinolin-Typ.

### Beispiel L511:

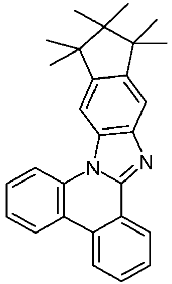

Einsatz von 36.0 g (100 mmol) 5,6-Dibrom-1,1,2,2,3,3-hexamethylindan, S24, Variante A, Stufe A, 23.3 g (120 mmol) 6-Amino-phenanthridin [832-68-8], 50.2 g (300 mmol) Lithium bis(trimethylsilyl)amid, 2.9 g (5 mmol) Xanthphos, 1.1 g (5 mmol) Palladium(II)acetat und dann 1.9 g (10 mmol) Kupfer(I)iodid, 1.75 g (20 mmol) N,N'-Ethylendiamin und 31.8 g (230 mmol) Kaliumcarbonat. Das Rohprodukt wird aus Dioxan (ca. 5 ml/g) umkristallisiert und im Vakuum sublimiert (p = 10⁻⁵ mbar, T = 240 °C). Ausbeute: 25.5 g (65 mmol), 65 %; Reinheit: ca. 99.5 % ig nach ¹H-NMR.

Analog können folgende Derivate dargestellt werden:

| **Bsp.** | **Phenanthridin** | **1,2-Dihalogen-bzw. 1-Amino-2-halogen-benzol** | **Produkt Variante** | **Aus-beute** |
|---|---|---|---|---|
| L512 | 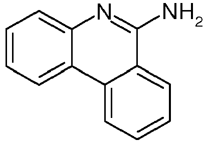 | 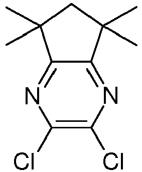 | 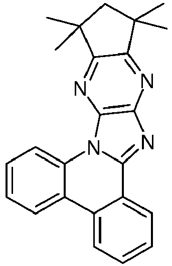 | 53 % |
| | 832-68-8 | 1311465-45-8 | A | |
| L513 | 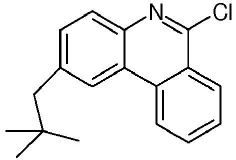 | 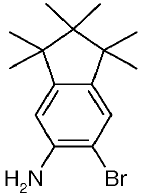 | 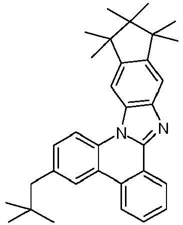 | 48 % |
| | 1089735-10-3 | S6 | B | |
| L514 | 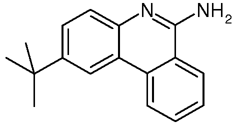 | 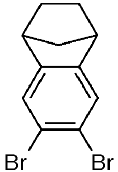 | 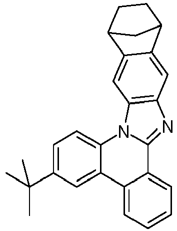 | 52 % |
| | 946147-22-4 | 42810-32-2 | A | |
| L515 | 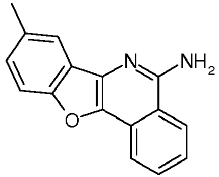 | 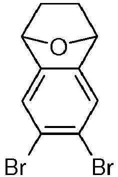 | 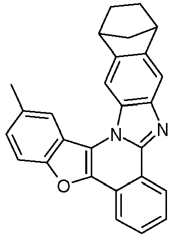 | 40 % |
| | 1293961-03-1 | 749859-07-2 | A | |
| L516 | 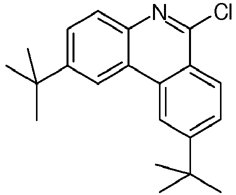 | 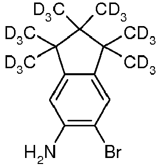 | 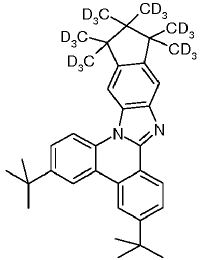 | 55 % |
| | 946147-36-0 | S7 | B | |
| L517 | 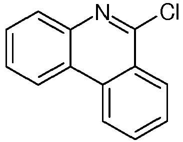 | 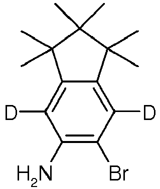 | 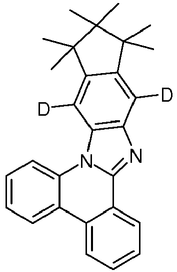 | 58 % |
| | 15679-03-5 | S8 | B | |
| L518 | 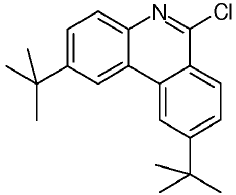 | 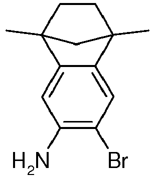 | 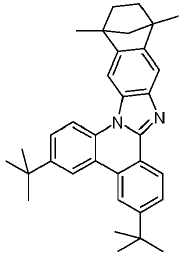 | 50 % |
| | 946147-36-0 | S11 | B | |

### 18)Tetradentate Liganden, L283

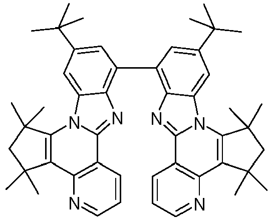

### A) L282-Br

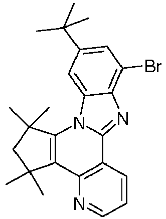

Eine auf 100 °C erwärmte Lösung von 37.2 g (100 mmol) L282 in 300 ml DMF wird portionsweise mit 19.6 g (110 mmol) NBS versetzt und anschließend 6 h nachgerührt. Man engt die Reaktionsmischung im Vakuum auf ca. 150 ml ein, rührt 2 h nach, saugt von den ausgefallenen Kristallen ab und wäscht diese abschließend zweimal mit je 50 ml Methanol und trocknet in Vakuum. Ausbeute: 35.6 g (79 mmol), 79 %. Reinheit: 97 % nach ¹H-NMR.

### B) L282-B

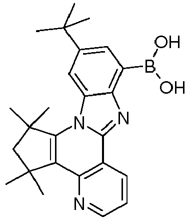

Eine auf -78 °C gekühlte Lösung von 22.5 g (50 mmol) L282-Br in 500 ml THF wird unter gutem Rühren tropfenweise mit 20 ml (50 mmol) n-Butyllithium (2.5 M in Hexan) versetzt und 30 min. nachgerührt. Zu dieser Lösung gibt man 5.6 ml (50 mmol) Trimethylborat auf ein Mal zu, rührt 1 h nach und lässt dann auf Raumtemperatur erwärmen. Man entfernt das Lösungsmittel im Vakuum und setzt den Rückstand in die Suzukikupplung C) ein.

### C) Suzukikupplung

Ein Gemisch aus 22.5 g (50 mmol) L282-Br und 20.7 g (50 mmol) L282-B - wie unter B) erhalten - 31.8 g (150 mmol) Trikaluimphosphat, 1.8 g (6 mmol) Tri-o-tolylphosphin, 224 mg (1 mmol) Palladium(II)acetat, 300 ml Toluol, 100 ml Dioxan und 300 ml Wasser wird 12 h unter Rückfluss erhitzt. Nach Erkalten saugt man vom ausgefallenen Feststoff ab, wäscht diesen zweimal mit je 50 ml Toluol und dreimal mit je 100 ml Ethanol. Das Rohprodukt wird aus Sulfolan (ca. 5 ml/g) umkristallisiert und im Vakuum sublimiert (p = 10⁻⁵ mbar, T = 300 °C). Ausbeute: 21.9 g (29 mmol),58 %; Reinheit: ca. 99.5 % ig nach ¹H-NMR.

### 19)Hexadentate Liganden, L284

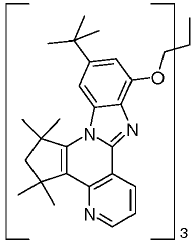

### A) L282-OH

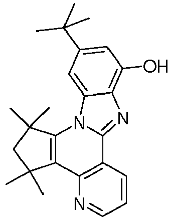

Eine auf -78 °C gekühlte Lösung von 22.5 g (50 mmol) L282-Br in 500 ml THF wird unter gutem Rühren tropfenweise mit 20 ml (50 mmol) n-Butyllithium (2.5 M in Hexan) versetzt und 30 min. nachgerührt. Zu dieser Lösung gibt man 5.6 ml (50 mmol) Trimethylborat auf ein Mal zu, rührt 1 h nach und lässt dann auf Raumtemperatur erwärmen. Man entfernt das Lösungsmittel im Vakuum, nimmt den Rückstand in 1000 ml Ethylacetat auf, kühlt die Lösung auf 5 °C ab, versetzt unter gut em Rühren mit 32 ml wässriger H₂O₂-Lösung (30 Gew.-%) und tropft dann eine Lösung von 1.4 g NaOH in 30 ml Wasser zu. Nach 3 h Rühren gibt man 500 ml gesättigte Ammoniumchloridlösung zu, trennt die organische Phase ab, wäscht diese zweimal mit je 200 ml Wasser, trocknet über Magnesiumsulfat und engt die organische Phase dann im Vakuum auf ein Volumen von ca. 50 ml ein. Man versetzt den Kristallbrei mit 200 ml Methanol, saugt ab, wäscht die Kristalle einmal mit 50 ml Methanol und trocknet im Vakuum. Ausbeute: 13.5 g (35 mmol), 70 %. Reinheit: 95 % nach ¹H-NMR.

### B) L284:

Eine gut gerührte Lösung von 13.5 g (35 mmol) L282-OH in 100 ml THF wird portionsweise mit 1.6 g (40 mmol) Natriumhydrid, 60 %ig Dispersion in Mineralöl versetzt (Achtung: Wasserstoffentwicklung, Schäumen). Nach beendeter Wasserstoffentwicklung fügt man 3.1 g (10 mmol) 1,1,1-Tris-(brommethyl)ethan [60111-68-4] zu und erhitzt die Reaktionsmischung 16 h unter Rückfluss. Nach Erkalten entfernt man das THF im Vakuum, nimmt den Rückstand in 500 ml Ethylacetat auf, wäscht dreimal mit je 200 ml Wasser und einmal mit 100 ml gesättigter Kochsalzlösung und trocknet die organische Phase über Natriumsulfat. Der nach Entfernen das Lösungsmittels erhaltene Rückstand wird an Kieselgel (Ethylacetat: n-Heptan, 5:1 vv) chromatographiert und dann im Vakuum getrocknet. Ausbeute: 7.1 g (5.8 mmol), 58 %. Reinheit: 99 % nach ¹H-NMR.

### C: Synthese der Metallkomplexe

### 1) Homoleptische tris-faciale Iridium-Komplexe:

### Variante A: Tris-acetylacetonato-iridium(III) als Iridium-Edukt

Ein Gemisch aus 10 mmol Tris-acetylacetonato-iridium(III) [15635-87-7] und 60 mmol des Liganden L und ein glasummantelter Magnetrührkern werden unter Vakuum (10⁻⁵ mbar) in eine dickwandige 50 ml Glasampulle abgeschmolzen. Die Ampulle wird für die angegebene Zeit bei der angegebenen Temperatur getempert, wobei das aufgeschmolzene Gemisch mit Hilfe eines Magnetrührers gerührt wird. Um eine Sublimation der Liganden an kältere Stellen der Ampulle zu vermeiden, muss die gesamte Ampulle die angegebene Temperatur besitzen. Alternativ kann die Synthese in einem Rührautoklaven mit Glaseinsatz erfolgen. Nach Erkalten (ACHTUNG: die Ampullen stehen meist unter Druck!) wird die Ampulle geöffnet, der Sinterkuchen wird mit 100 g Glaskugeln (3 mm Durchmessser) in 100 ml eines Suspensionsmittels (das Suspensionsmittel wird so gewählt, dass der Ligand gut, der Metallkomplex jedoch schlecht darin löslich ist, typische Suspensionsmittel sind Methanol, Ethanol, Dichlormethan, Aceton, THF, Ethylacetat, Toluol, etc.) 3 h gerührt und dabei mechanisch aufgeschlossen. Man dekantiert die feine Suspension von den Glaskugeln ab, saugt den Feststoff ab, wäscht mit 50 ml des Suspensionsmittels nach, und trocknet diesen im Vakuum. Der trockene Feststoff wird in einem kontinuierlichen Heißextraktor auf einem 3-5 cm hohen Alox-Bett (Alox, basisch Aktivitätsstufe 1) platziert und dann mit einem Extraktionsmittel (Vorlagemenge ca. 500 ml, das Extraktionsmittel wird so gewählt, dass der Komplex darin in der Hitze gut und in der Kälte schlecht löslich ist, besonders geeignete Extraktionsmittel sind Kohlenwasserstoffe wie Toluol, Xylole, Mesitylen, Naphthalin, o-Dichlorbenzol, halogenierte aliphatische Kohlenwasserstoffe sind in der Regel ungeeignet, da sie die Komplexe gegebenenfalls halogenieren oder zersetzen) extrahiert. Nach beendeter Extraktion wird das Extraktionsmittel im Vakuum auf ca. 100 ml eingeengt. Metallkomplexe, die im Extraktionsmittel eine zu gute Löslichkeit aufweisen, werden durch Zutropfen von 200 ml Methanol zur Kristallisation gebracht. Der Feststoff der so erhaltenen Suspensionen wird abgesaugt, einmal mit ca. 50 ml Methanol gewaschen und getrocknet. Nach Trocknen wird die Reinheit des Metall-Komplexes mittels NMR und / oder HPLC bestimmt. Liegt die Reinheit unter 99.5 % wird der Heißextraktionsschritt wiederholt, wobei ab der 2ten Extraktion das Alox-Bett weggelassen wird. Ist eine Reinheit von 99.5 - 99.9 % erreicht, wird der Metallkomplex getempert oder sublimiert. Das Tempern erfolgt im Hochvakuum (p ca. 10⁻⁶ mbar) im Temperaturbereich von ca. 200 - 300 °C . Die Sublimation erfolgt im H ochvakuum (p ca. 10⁻⁶ mbar) im Temperaturbereich von ca. 300 -400 °C, wobei die Sublimation bevorzugt in Form einer fraktionierten Sublimation durchgeführt wird. Werden Liganden der Punktgruppe C1 racemisch eingesetzt, fallen die abgeleiteten fac- Metallkomplexe als Diastereomerenmischung an. Das Enantiomerenpaar Λ,Δ der Punktgruppe C3 weist in der Regel eine deutlich geringere Löslichkeit im Extraktionsmittel auf als das der Punktgruppe C1, das sich folglich in der Mutterlauge anreichert. Eine Trennung der Diasteromeren auf diesem Wege ist häufig möglich. Daneben können die Diastereomeren auch chromatographisch getrennt werden. Werden Liganden der Punktgruppe C1 enantiomerenrein eingesetzt, entsteht das Enantiomerenpaar Λ,Δ der Punktgruppe C3.

### Variante B: Tris-(2,2,6,6-tetramethyl-3,5-heptandionato)iridium(III) als Iridium-Edukt

Durchführung analog zu Variante A, wobei anstelle von 10 mmol Tris-acetylacetonato-iridium(III) [15635-87-7] 10 mmol Tris-(2,2,6,6-tetramethyl-3,5-heptandionato)iridium [99581-86-9] eingesetzt werden. Die Verwendung dieses Edukts ist vorteilhaft, da die Reinheit der erhaltenen Rohprodukte häufig besser ist als bei Variante A. Außerdem ist der Druckaufbau in der Ampulle häufig nicht so ausgeprägt.

| **Bsp.** | **Ligand** L | **Ir-Komplex Diastereomer** | **Variante Reaktionstemp./ Reaktionszei**t **Suspensionsmittel Extraktionsmittel** | **Aus-beute** |
|---|---|---|---|---|
| Ir(L1)₃ | L1 | 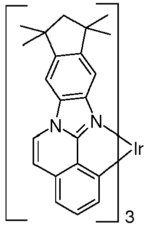 | A | 40 % |
| | | | 260 °C / 100 h | |
| | | | DCM | |
| | | | Mesitylen | |
| | | Ir(L1)₃ | | |
| Ir(L1)₃ | L1 | 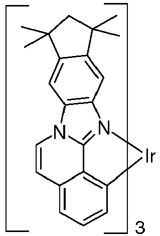 | B | 48 % |
| | | | 280 °C / 100 h | |
| | | | DCM | |
| | | | Mesitylen | |
| | | Ir(L1)₃ | | |
| Ir(L2)₃ | L2 | Ir(L2)₃ | B | 43 % |
| | | | 290 °C / 100h | |
| | | | DCM | |
| | | | Mesitylen | |
| Ir(L3)₃ | L3 | Ir(L3)₃ | wie Bsp. Ir(L1)₃ / B | 43 % |
| Ir(L4)₃ | L4 | Ir(L4)₃ | wie Bsp. Ir(L1)₃ / B | 45 % |
| Ir(L5)₃ | L5 | Ir(L5)₃ | wie Bsp. Ir(L1)₃ / B | 40 % |
| Ir(L6)₃ | L6 | Ir(L6)₃ | wie Bsp. Ir(L1)₃ / B | 47 % |
| Ir(L7)₃ | L7 | Ir(L7)₃ | wie Bsp. Ir(L1)₃ / B | 25 % |
| | | Λ,Δ-C3 | | |
| Ir(L8)₃ | L8 | Ir(L8)₃ | wie Bsp. Ir(L1)₃ / B | 25 % |
| | | Λ,Δ-C3 | | |
| Ir(L9)₃ | L9 | Ir(L9)₃ | B | 41 % |
| | | | 280 °C / 150 h | |
| | | | | |
| | | | Mesitylen | |
| Ir(L10)₃ | L10 | Ir(L10)₃ | wie Bsp. Ir(L1)₃ / B | 43 % |
| Ir(L11)₃ | L11 | Ir(L101₃ | wie Bsp. Ir(L1)₃ / B | 24 % |
| | | Λ,Δ-C3 | | |
| Ir(L12)₃ | L12 | Ir(L12)₃ | wie Bsp. Ir(L1)₃ / B | 7 % |
| | | Λ,Δ-C3 | | |
| Ir(L13)₃ | L13 | Ir(L13)₃ | wie Bsp. Ir(L1)₃ / B | 26 % |
| | | Λ,Δ-C3 | | |
| Ir(L14)₃ | L14 | Ir(L14)₃ | wie Bsp. Ir(L1)₃ / B | 45 % |
| Ir(L15)₃ | L15 | Ir(L15)₃ | wie Bsp. Ir(L1)₃ / B | 47 % |
| Ir(L16)₃ | L16 | Ir(L16)₃ | B | 43 % |
| | | | 280 °C / 180 h | |
| | | | DCM | |
| | | | Mesitylen | |
| Ir(L17)₃ | L17 | Ir(L17)₃ | wie Bsp. Ir(L16)₃ | 38 % |
| Ir(L18)₃ | L18 | Ir(L18)₃ | wie Bsp. Ir(L1)₃ / B | 45 % |
| Ir(L19)₃ | L19 | Ir(L19)₃ | wie Bsp. Ir(L1)₃ / B | 21 % |
| | | Λ,Δ-C3 | | |
| Ir(L20)₃ | L20 | Ir(L20)₃ | wie Bsp. Ir(L1)₃ / B | 45 % |
| Ir(L21)₃ | L21 | Ir(L21)₃ | wie Bsp. Ir(L1)₃ / B | 23 % |
| | | Λ,Δ-C3 | | |
| Ir(L22)₃ | L22 | Ir(L22)₃ | wie Bsp. Ir(L1)₃ / B | 23 % |
| Ir(L23)₃ | L23 | Ir(L23)₃ | wie Bsp. Ir(L16)₃ | 21 % |
| Ir(L24)₃ | L24 | Ir(L24)₃ | wie Bsp. Ir(L1)₃ / B | 26 % |
| | | Λ,Δ-C3 | | |
| Ir(L25)₃ | L25 | Ir(L25)₃ | wie Bsp. Ir(L16)₃ | 27 % |
| | | Λ,Δ-C3 | | |
| Ir(L26)₃ | L26 | Ir(L26)₃ | wie Bsp. Ir(L1)₃ / B | 44 % |
| Ir(L27)₃ | L27 | Ir(L27)₃ | wie Bsp. Ir(L1)₃ / B | 48 % |
| Ir(L28)₃ | L28 | Ir(L28)₃ | wie Bsp. Ir(L1)₃ / B | 47 % |
| Ir(L29)₃ | L29 | Ir(L29)₃ | wie Bsp. Ir(L16)₃ | 37 % |
| Ir(L30)₃ | L30 | Ir(L30)₃ | wie Bsp. Ir(L16)₃ | 35 % |
| Ir(L31)₃ | L31 | Ir(L31)₃ | wie Bsp. Ir(L2)₃ | 26 % |
| | | Λ,Δ-C3 | | |
| Ir(L32)₃ | L32 | Ir(L32)₃ | wie Bsp. Ir(L2)₃ | 50 % |
| Ir(L33)₃ | L33 | Ir(L33)₃ | wie Bsp. Ir(L2)₃ | 43 % |
| Ir(L34)₃ | L34 | Ir(L34)₃ | wie Bsp. Ir(L2)₃ | 51 % |
| Ir(L35)₃ | L35 | Ir(L35)₃ | wie Bsp. Ir(L1)₃ / B | 21 % |
| Ir(L36)₃ | L36 | Ir(L36)₃ | wie Bsp. Ir(L1)₃ / B | 19 % |
| Ir(L37)₃ | L37 | Ir(L37)₃ | wie Bsp. Ir(L16)₃ | 30 % |
| Ir(L38)₃ | L38 | Ir(L38)₃ | wie Bsp. Ir(L16)₃ | 17 % |
| Ir(L39)₃ | L39 | Ir(L39)₃ | wie Bsp. Ir(L1)₃ / B | 39 % |
| Ir(L40)₃ | L40 | Ir(L40)₃ | wie Bsp. Ir(L1)₃ / B | 37 % |
| Ir(L41)₃ | L41 | Ir(L41)₃ | wie Bsp. Ir(L1)₃ / B | 46 % |
| Ir(L42)₃ | L42 | Ir(L42)₃ | wie Bsp. Ir(L1)₃ / B | 36 % |
| Ir(L43)₃ | L43 | Ir(L43)₃ | wie Bsp. Ir(L1)₃ / B | 33 % |
| Ir(L44)₃ | L44 | Ir(L44)₃ | wie Bsp. Ir(L16)₃ | 4 % |
| Ir(L45)₃ | L45 | Ir(L45)₃ | wie Bsp. Ir(L2)₃ | 43 % |
| Ir(L46)₃ | L46 | Ir(L46)₃ | wie Bsp. Ir(L1)₃ / B | 45 % |
| | | Λ,Δ-C3 + C1 | | |
| Ir(L47)₃ | L47 | Ir(L47)₃ | wie Bsp. Ir(L1)₃ / B | 23 % |
| Ir(L48)₃ | L48 | Ir(L48)₃ | wie Bsp. Ir(L1)₃ / B | 36 % |
| Ir(L49)₃ | L49 | Ir(L49)₃ | wie Bsp. Ir(L1)₃ / B | 39 % |
| Ir(L50)₃ | L50 | Ir(L50)₃ | wie Bsp. Ir(L1)₃ / B | 38 % |
| Ir(L51)₃ | L51 | Ir(L51)₃ | wie Bsp. Ir(L1)₃ / B | 50 % |
| Ir(L52)₃ | L52 | Ir(L52)₃ | wie Bsp. Ir(L1)₃ / B | 48 % |
| Ir(L53)₃ | L53 | Ir(L53)₃ | wie Bsp. Ir(L1)₃ / B | 48 % |
| Ir(L54)₃ | L54 | Ir(L54)₃ | wie Bsp. Ir(L1)₃ / B | 34 % |
| Ir(L55)₃ | L55 | Ir(L55)₃ | wie Bsp. Ir(L1)₃ / B | 41 % |
| | | Λ,Δ-C3 + C1 | | |
| Ir(L56)₃ | L56 | Ir(L56)₃ | wie Bsp. Ir(L1)₃ / B | 40 % |
| Ir(L57)₃ | L57 | Ir(L57)₃ | wie Bsp. Ir(L1)₃ / B | 24 % |
| | | Λ,Δ-C3 | | |
| Ir(L58)₃ | L58 | Ir(L58)₃ | wie Bsp. Ir(L1)₃ / B | 17 % |
| | | Λ,Δ-C3 | | |
| Ir(L59)₃ | L59 | Ir(L59)₃ | wie Bsp. Ir(L16)₃ | 2 % |
| | | Λ,Δ-C3 | | |
| Ir(L60)₃ | L60 | Ir(L60)₃ | wie Bsp. Ir(L1)₃ / B | 23 % |
| | | Λ,Δ-C3 | | |
| Ir(L61)₃ | L61 | Ir(L61)₃ | wie Bsp. Ir(L1)₃ / B | 18 % |
| | | Λ,Δ-C3 | | |
| Ir(L62)₃ | L62 | Ir(L62)₃ | wie Bsp. Ir(L2)₃ | 40 % |
| Ir(L63)₃ | L63 | Ir(L63)₃ | wie Bsp. Ir(L1)₃ / B | 41 % |
| Ir(L64)₃ | L64 | Ir(L64)₃ | wie Bsp. Ir(L1)₃ / B | 34 % |
| Ir(L65)₃ | L65 | Ir(L65)₃ | wie Bsp. Ir(L1)₃ / B | 17 % |
| | | Λ,Δ-C3 | | |
| Ir(L66)₃ | L66 | Ir(L66)₃ | wie Bsp. Ir(L1)₃ / B | 23 % |
| | | Λ,Δ-C3 | | |
| Ir(L67)₃ | L67 | Ir(L67)₃ | wie Bsp. Ir(L1)₃ / B | 19 % |
| | | Λ,Δ-C3 | | |
| Ir(L68)₃ | L68 | Ir(L68)₃ | wie Bsp. Ir(L16)₃ | 12 % |
| | | Λ,Δ-C3 | | |
| Ir(L69)₃ | L69 | Ir(L69)₃ | wie Bsp. Ir(L16)₃ | 16 % |
| | | Λ,Δ-C3 | | |
| Ir(L70)₃ | L70 | Ir(L70)₃ | wie Bsp. Ir(L1)₃ / B | 21 % |
| | | Λ,Δ-C3 | | |
| Ir(L71)₃ | L71 | Ir(L71)₃ | wie Bsp. Ir(L1)₃ / B | 18 % |
| | | Λ,Δ-C3 | | |
| Ir(L72)₃ | L72 | Ir(L72)₃ | wie Bsp. Ir(L1)₃ / B | 9 % |
| | | Λ,Δ-C3 | | |
| Ir(L73)₃ | L73 | Ir(L73)₃ | wie Bsp. Ir(L1)₃ / B | 17 % |
| Ir(L74)₃ | L74 | Ir(L74)₃ | wie Bsp. Ir(L1)₃ / B | 46 % |
| Ir(L75)₃ | L75 | Ir(L75)₃ | wie Bsp. Ir(L1)₃ / B | 46 % |
| Ir(L76)₃ | L76 | Ir(L76)₃ | wie Bsp. Ir(L1)₃ / B | 48 % |
| Ir(L77)₃ | L77 | Ir(L77)₃ | wie Bsp. Ir(L1)₃ / B | 45 % |
| Ir(L78)₃ | L78 | Ir(L78)₃ | wie Bsp. Ir(L16)₃ | 33 % |
| Ir(L79)₃ | L79 | Ir(L79)₃ | wie Bsp. Ir(L16)₃ | 30 % |
| Ir(L80)₃ | L80 | Ir(L80)₃ | wie Bsp. Ir(L1)₃ / B | 44 % |
| Ir(L81)₃ | L81 | Ir(L81)₃ | wie Bsp. Ir(L1)₃ / B | 45 % |
| Ir(L82)₃ | L82 | Ir(L82)₃ | wie Bsp. Ir(L1)₃ / B | 22 % |
| | | Λ,Δ-C3 | | |
| Ir(L83)₃ | L83 | Ir(L83)₃ | wie Bsp. Ir(L1)₃ / B | 26 % |
| | | Λ,Δ-C3 | | |
| Ir(L84)₃ | L84 | Ir(L84)₃ | wie Bsp. Ir(L2)₃ | 51 % |
| Ir(L85)₃ | L85 | Ir(L85)₃ | wie Bsp. Ir(L16)₃ | 23 % |
| | | Λ,Δ-C3 | | |
| Ir(L85)₃ | L85 | Ir(L85)₃ | wie Bsp. Ir(L16)₃ | 9 % |
| | | | | |
| Ir(L86)₃ | L86 | Ir(L86)₃ | wie Bsp. Ir(L16)₃ | 28 % |
| | | Λ,Δ-C3 | | |
| Ir(L87)₃ | L87 | Ir(L87)₃ | wie Bsp. Ir(L16)₃ | 25 % |
| | | Λ,Δ-C3 | | |
| Ir(L88)₃ | L88 | Ir(L88)₃ | wie Bsp. Ir(L16)₃ | 27 % |
| | | Λ,Δ-C3 | | |
| Ir(L89)₃ | L89 | Ir(L89)₃ | wie Bsp. Ir(L16)₃ | 24 % |
| | | Λ,Δ-C3 | | |
| Ir(L90)₃ | L90 | Ir(L90)₃ | wie Bsp. Ir(L16)₃ | 26 % |
| | | Λ,Δ-C3 | | |
| Ir(L91)₃ | L91 | Ir(L91)₃ | wie Bsp. Ir(L16)₃ | 28 % |
| | | Λ,Δ-C3 | | |
| Ir(L92)₃ | L92 | Ir(L92)₃ | wie Bsp. Ir(L1)₃ / B | 42 % |
| Ir(L93)₃ | L93 | Ir(L93)₃ | wie Bsp. Ir(L1)₃ / B | 45 % |
| Ir(L94)₃ | L94 | Ir(L94)₃ | wie Bsp. Ir(L16)₃ | 29 % |
| | | Λ,Δ-C3 | | |
| Ir(L95)₃ | L95 | Ir(L95)₃ | wie Bsp. Ir(L16)₃ | 22 % |
| | | Λ,Δ-C3 | | |
| Ir(L276)₃ | L276 | Ir(L276)₃ | wie Bsp. Ir(L1)₃ / B | 40 % |
| Ir(L277)₃ | L277 | Ir(L277)₃ | wie Bsp. Ir(L1)₃ / B | 38 % |
| Ir(L278)₃ | L278 | Ir(L278)₃ | wie Bsp. Ir(L1)₃ / B | 22 % |
| | | Λ,Δ-C3 | | |
| Ir(L96)₃ | L96 | 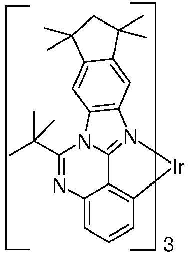 | B | 46 % |
| | | | 300 00 h | |
| | | | °C / 1 | |
| | | | Mesitylen | |
| | | Ir(L96)₃ | | |
| Ir(L97)₃ | L97 | Ir(L97)₃ | wie Bsp. Ir(L96)₃ | 40 % |
| Ir(L98)₃ | L98 | Ir(L98)₃ | B | 38 % |
| | | | 300 °C / 180 h | |
| | | | DCM | |
| | | | Mesitylen | |
| Ir(L99)₃ | L99 | Ir(L99)₃ | wie Bsp. Ir(L98)₃ | 31 % |
| Ir(L100)₃ | L100 | Ir(L100)₃ | wie Bsp. Ir(L96)₃ | 48 % |
| Ir(L101)₃ | L101 | Ir(L101)₃ | wie Bsp. Ir(L98)₃ | 32 % |
| Ir(L102)₃ | L102 | Ir(L102)₃ | wie Bsp. Ir(L96)₃ | 50 % |
| Ir(L103)₃ | L103 | Ir(L103)₃ | wie Bsp. Ir(L98)₃ | 19 % |
| | | Λ,Δ-C3 | | |
| Ir(L104)₃ | L104 | Ir(L104₎3 | wie Bsp. Ir(L96)₃ | 22 % |
| | | Λ,Δ-C3 | | |
| Ir(L105)₃ | L105 | Ir(L105)₃ | wie Bsp. Ir(L96)₃ | 36 % |
| | | Λ,Δ-C3 + C1 | | |
| Ir(L106)₃ | L106 | Ir(L106)₃ | wie Bsp. Ir(L98)₃ | 28 % |
| | | Λ,Δ-C3 | | |
| Ir(L107)₃ | L107 | Ir(L107)₃ | wie Bsp. Ir(L98)₃ | 31 % |
| Ir(L108)₃ | L108 | Ir(L108)₃ | wie Bsp. Ir(L96)₃ | 33 % |
| | | Λ,Δ-C3 + C1 | | |
| Ir(L109)₃ | L109 | Ir(L109)₃ | wie Bsp. Ir(L96)₃ | 49 % |
| Ir(L110)₃ | L110 | Ir(L110)₃ | wie Bsp. Ir(L98)₃ | 35 % |
| Ir(L111)₃ | L111 | Ir(L111)₃ | wie Bsp. Ir(L96)₃ | 44 % |
| Ir(L112)₃ | L112 | Ir(L112)3 | wie Bsp. Ir(L96)₃ | 43 % |
| Ir(L113)₃ | L113 | Ir(L113)₃ | wie Bsp. Ir(L96)₃ | 44 % |
| Ir(L114)₃ | L113 | Ir(L114)₃ | wie Bsp. Ir(L96)₃ | 39 % |
| Ir(L115)₃ | L113 | Ir(L115)₃ | wie Bsp. Ir(L96)₃ | 40 % |
| Ir(L116)₃ | L116 | Ir(L116)₃ | wie Bsp. Ir(L96)₃ | 37 % |
| Ir(L117)₃ | L117 | Ir(L117)₃ | wie Bsp. Ir(L98)₃ | 28 % |
| Ir(L118)₃ | L118 | Ir(L118)₃ | wie Bsp. Ir(L96)₃ | 40 % |
| Ir(L119)₃ | L119 | Ir(L119)₃ | wie Bsp. Ir(L98)₃ | 44 % |
| Ir(L120)₃ | L120 | Ir(L120)₃ | wie Bsp. Ir(L98)₃ | 45 % |
| Ir(L121)₃ | L121 | Ir(L121)₃ | wie Bsp. Ir(L98)₃ | 18 % |
| Ir(L122)₃ | L122 | Ir(L122)3 | wie Bsp. Ir(L96)₃ | 19 % |
| | | Λ,Δ-C3 | | |
| Ir(L123)₃ | L123 | Ir(L123)₃ | wie Bsp. Ir(L96)₃ | 36 % |
| Ir(L124)₃ | L124 | Ir(L124)₃ | wie Bsp. Ir(L96)₃ | 37 % |
| Ir(L125)₃ | L125 | Ir(L125)₃ | wie Bsp. Ir(L96)₃ | 41 % |
| Ir(L126)₃ | L126 | Ir(L126)₃ | wie Bsp. Ir(L96)₃ | 24 % |
| | | Λ,Δ-C3 | | |
| Ir(L127)₃ | L127 | Ir(L127)3 | wie Bsp. Ir(L98)₃ | 19 % |
| | | Λ,Δ-C3 | | |
| Ir(L128)₃ | L128 | Ir(L128)₃ | wie Bsp. Ir(L98)₃ | 17 % |
| | | Λ,Δ-C3 | | |
| Ir(L129)₃ | L129 | Ir(L129)₃ | wie Bsp. Ir(L96)₃ | 23 % |
| | | Λ,Δ-C3 | | |
| Ir(L130)₃ | L130 | Ir(L130)₃ | wie Bsp. Ir(L96)₃ | 22 % |
| | | Λ,Δ-C3 | | |
| Ir(L131)₃ | L131 | Ir(L131)₃ | wie Bsp. Ir(L98)₃ | 22 % |
| | | Λ,Δ-C3 | | |
| Ir(L132)₃ | L132 | Ir(L132)₃ | wie Bsp. Ir(L96)₃ | 24 % |
| | | Λ,Δ-C3 | | |
| Ir(L133)₃ | L133 | Ir(L133)₃ | wie Bsp. Ir(L96)₃ | 25 % |
| | | Λ,Δ-C3 | | |
| Ir(L134)₃ | L134 | Ir(L134)₃ | wie Bsp. Ir(L98)₃ | 21 % |
| | | Λ,Δ-C3 | | |
| Ir(L135)₃ | L135 | Ir(L135)₃ | wie Bsp. Ir(L96)₃ | 21 % |
| | | Λ,Δ-C3 | | |
| Ir(L136)₃ | L136 | Ir(L136)₃ | wie Bsp. Ir(L96)₃ | 24 % |
| | | Λ,Δ-C3 | | |
| Ir(L137)₃ | L137 | Ir(L137)₃ | wie Bsp. Ir(L96)₃ | 17 % |
| | | Λ,Δ-C3 | | |
| Ir(L138)₃ | L138 | Ir(L138)₃ | wie Bsp. Ir(L98)₃ | 22 % |
| | | Λ,Δ-C3 | | |
| Ir(L139)₃ | L139 | Ir(L139)₃ | wie Bsp. Ir(L96)₃ | 21 % |
| | | Λ,Δ-C3 | | |
| Ir(L140)₃ | L140 | Ir(L140)₃ | wie Bsp. Ir(L96)₃ | 14 % |
| | | Λ,Δ-C3 | | |
| Ir(L141)₃ | L141 | Ir(L141)₃ | wie Bsp. Ir(L98)₃ | 23 % |
| | | Λ,Δ-C3 | | |
| Ir(L142)₃ | L142 | Ir(L142)₃ | wie Bsp. Ir(L96)₃ | 39 % |
| Ir(L143)₃ | L143 | Ir(L143)₃ | wie Bsp. Ir(L96)₃ | 45 % |
| Ir(L144)₃ | L144 | Ir(L144)₃ | wie Bsp. Ir(L96)₃ | 36 % |
| Ir(L145)₃ | L145 | Ir(L145)₃ | wie Bsp. Ir(L96)₃ | 18 % |
| | | Λ,Δ-C3 | | |
| Ir(L146)₃ | L146 | Ir(L146)₃ | wie Bsp. Ir(L98)₃ | 24 % |
| | | Λ,Δ-C3 | | |
| Ir(L147)₃ | L147 | Ir(L147)₃ | wie Bsp. Ir(L96)₃ | 18 % |
| | | Λ,Δ-C3 | | |
| Ir(L148)₃ | L148 | Ir(L148)₃ | wie Bsp. Ir(L98)₃ | 19 % |
| | | Λ,Δ-C3 | | |
| Ir(L149)₃ | L149 | Ir(L149)₃ | wie Bsp. Ir(L98)₃ | 15 % |
| | | Λ,Δ-C3 | | |
| Ir(L150)₃ | L150 | Ir(L150)₃ | wie Bsp. Ir(L98)₃ | 6 % |
| | | Λ,Δ-C3 | | |
| Ir(L151)₃ | L151 | Ir(L151)₃ | wie Bsp. Ir(L96)₃ | 16 % |
| | | Λ,Δ-C3 | | |
| Ir(L152)₃ | L152 | Ir(L152)₃ | wie Bsp. Ir(L96)₃ | 46 % |
| | | Λ,Δ-C3 | | |
| Ir(L153)₃ | L153 | Ir(L153)₃ | wie Bsp. Ir(L98)₃ | 10 % |
| | | Λ,Δ-C3 | | |
| Ir(L154)₃ | L154 | Ir(L154)₃ | wie Bsp. Ir(L96)₃ | 8 % |
| | | Λ,Δ-C3 | | |
| Ir(L155)₃ | L155 | Ir(L155)₃ | wie Bsp. Ir(L96)₃ | 23 % |
| Ir(L156)₃ | L156 | Ir(L156)₃ | wie Bsp. Ir(L96)₃ | 14 % |
| Ir(L157)₃ | L157 | Ir(L157)₃ | wie Bsp. Ir(L96)₃ | 48 % |
| Ir(L158)₃ | L158 | Ir(L158)₃ | wie Bsp. Ir(L96)₃ | 46 % |
| Ir(L159)₃ | L159 | Ir(L159)₃ | wie Bsp. Ir(L96)₃ | 47 % |
| Ir(L160)₃ | L160 | Ir(L160)₃ | wie Bsp. Ir(L96)₃ | 47 % |
| Ir(L161)₃ | L161 | Ir(L161)₃ | wie Bsp. Ir(L96)₃ | 44 % |
| Ir(L162)₃ | L162 | Ir(L162)₃ | wie Bsp. Ir(L96)₃ | 40 % |
| Ir(L163)₃ | L163 | Ir(L163)₃ | wie Bsp. Ir(L96)₃ | 26 % |
| | | Λ,Δ-C3 | | |
| Ir(L164)₃ | L164 | Ir(L164)₃ | wie Bsp. Ir(L96)₃ | 25 % |
| | | Λ,Δ-C3 | | |
| Ir(L165)₃ | L165 | Ir(L165)₃ | wie Bsp. Ir(L96)₃ | 43 % |
| Ir(L166)₃ | L166 | Ir(L166)₃ | wie Bsp. Ir(L96)₃ | 48 % |
| Ir(L167)₃ | L167 | 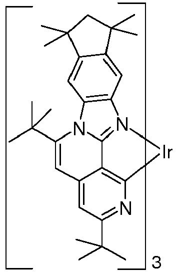 | B | 44 % |
| | | | 310 °C/ 180 h | |
| | | | DCM | |
| | | | Mesitylen | |
| | | Ir(L167)₃ | | |
| Ir(L168)₃ | L168 | Ir(L168)₃ | B | 38 % |
| | | | 310 °C / 200 h | |
| | | | DCM | |
| | | | Mesitylen | |
| Ir(L169)₃ | L169 | Ir(L169)₃ | wie Bsp. Ir(L167)₃ | 46 % |
| Ir(L170)₃ | L170 | Ir(L170)₃ | wie Bsp. Ir(L167)₃ | 44 % |
| Ir(L171)₃ | L171 | Ir(L171)₃ | wie Bsp. Ir(L167)₃ | 43 % |
| Ir(L172)₃ | L172 | Ir(L172)₃ | wie Bsp. Ir(L167)₃ | 20 % |
| | | Λ,Δ-C3 | | |
| Ir(L173)₃ | L173 | Ir(L173)₃ | wie Bsp. Ir(L167)₃ | 25 % |
| | | Λ,Δ-C3 | | |
| Ir(L174)₃ | L174 | Ir(L174)₃ | wie Bsp. Ir(L167)₃ | 45 % |
| Ir(L175)₃ | L175 | Ir(L175)₃ | wie Bsp. Ir(L167)₃ | 23 % |
| | | Λ,Δ-C3 | | |
| Ir(L176)₃ | L176 | Ir(L176₃ | wie Bsp. Ir(L168)₃ | 18 % |
| | | Λ,Δ-C3 | | |
| Ir(L177)₃ | L177 | Ir(L177)₃ | wie Bsp. Ir(L167)₃ | 21 % |
| Ir(L178)₃ | L178 | Ir(L178)₃ | wie Bsp. Ir(L167)₃ | 42 % |
| Ir(L179)₃ | L179 | Ir(L179)₃ | wie Bsp. Ir(L167)₃ | 43 % |
| Ir(L180)₃ | L180 | Ir(L180)₃ | wie Bsp. Ir(L167)₃ | 45 % |
| | | Λ,Δ-C3 + C1 | | |
| Ir(L181)₃ | L181 | Ir(L181)₃ | wie Bsp. Ir(L167)₃ | 26 % |
| | | Λ,Δ-C3 | | |
| Ir(L182)₃ | L182 | Ir(L182)₃ | wie Bsp. Ir(L167)₃ | 20 % |
| | | Λ,Δ-C3 | | |
| Ir(L183)₃ | L183 | Ir(L183)₃ | wie Bsp. Ir(L167)₃ | 36 % |
| Ir(L184)₃ | L184 | Ir(L184)₃ | wie Bsp. Ir(L167)₃ | 18 % |
| | | Λ,Δ-C3 | | |
| Ir(L185)₃ | L185 | Ir(L185)₃ | wie Bsp. Ir(L167)₃ | 12 % |
| Ir(L186)₃ | L186 | Ir(L186)₃ | wie Bsp. Ir(L167)₃ | 45 % |
| Ir(L187)₃ | L187 | Ir(L187)₃ | wie Bsp. Ir(L167)₃ | 45 % |
| Ir(L188)₃ | L188 | Ir(L188)₃ | wie Bsp. Ir(L167)₃ | 47 % |
| Ir(L189)₃ | L189 | Ir(L189)₃ | wie Bsp. Ir(L167)₃ | 41 % |
| Ir(L190)₃ | L190 | Ir(L190)₃ | wie Bsp. Ir(L167)₃ | 23 % |
| | | Λ,Δ-C3 | | |
| Ir(L191)₃ | L191 | Ir(L191)₃ | wie Bsp. Ir(L167)₃ | 44 % |
| Ir(L192)₃ | L192 | 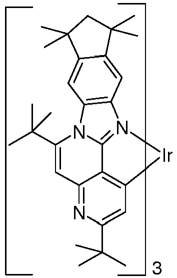 | B | 46 % |
| | | | 305 °C / 180 h | |
| | | | Aceton | |
| | | | Mesitylen | |
| | | Ir(L192)₃ | | |
| Ir(L193)₃ | L193 | Ir(L193)₃ | B | 36 % |
| | | | 310 °C/ 210 h | |
| | | | Aceton | |
| | | | Mesitylen | |
| Ir(L194)₃ | L194 | Ir(L194)₃ | wie Bsp. Ir(L192)₃ | 46 % |
| Ir(L195)₃ | L195 | Ir(L195)₃ | wie Bsp. Ir(L193)₃ | 3.5 % |
| Ir(L196)₃ | L196 | Ir(L196)₃ | wie Bsp. Ir(L192)₃ | 44 % |
| Ir(L197)₃ | L197 | Ir(L197)₃ | wie Bsp. Ir(L192)₃ | 45 % |
| Ir(L198)₃ | L198 | Ir(L198)₃ | wie Bsp. Ir(L192)₃ | 27 % |
| | | Λ,Δ-C3 | | |
| Ir(L199)₃ | L199 | Ir(L199)₃ | wie Bsp. Ir(L193)₃ | 26 % |
| | | Λ,Δ-C3 | | |
| Ir(L200)₃ | L200 | Ir(L200)₃ | wie Bsp. Ir(L192)₃ | 21 % |
| | | Λ,Δ-C3 | | |
| Ir(L201)₃ | L201 | Ir(L201)₃ | wie Bsp. Ir(L192)₃ | 42 % |
| Ir(L202)₃ | L202 | Ir(L202)₃ | wie Bsp. Ir(L192)₃ | 26 % |
| | | Λ,Δ-C3 | | |
| Ir(L203)₃ | L203 | Ir(L203)₃ | wie Bsp. Ir(L192)₃ | 28% |
| Ir(L204)₃ | L204 | Ir(L204)₃ | wie Bsp. Ir(L192)₃ | 45 % |
| Ir(L205)₃ | L205 | Ir(L205)₃ | wie Bsp. Ir(L192)₃ | 45 % |
| | | Λ,Δ-C3 + C1 | | |
| Ir(L206)₃ | L206 | Ir(L206)₃ | wie Bsp. Ir(L192)₃ | 20 % |
| | | Λ,Δ-C3 | | |
| Ir(L207)₃ | L207 | Ir(L207)₃ | wie Bsp. Ir(L192)₃ | 23 % |
| | | Λ,Δ-C3 | | |
| Ir(L208)₃ | L208 | Ir(L208)₃ | wie Bsp. Ir(L192)₃ | 25 % |
| | | Λ,Δ-C3 | | |
| Ir(L209)₃ | L209 | Ir(L209)₃ | wie Bsp. Ir(L192)₃ | 44 % |
| Ir(L210)₃ | L210 | Ir(L210)₃ | wie Bsp. Ir(L192)₃ | 43 % |
| Ir(L211)₃ | L211 | Ir(L211)₃ | wie Bsp. Ir(L192)₃ | 46 % |
| Ir(L212)₃ | L212 | Ir(L212)₃ | wie Bsp. Ir(L192)₃ | 46 % |
| Ir(L213)₃ | L213 | Ir(L213)₃ | wie Bsp. Ir(L192)₃ | 21 % |
| | | Λ,Δ-C3 | | |
| Ir(L214)₃ | L214 | Ir(L214)₃ | wie Bsp. Ir(L192)₃ | 37 % |
| Ir(L279)₃ | L279 | Ir(L279)₃ | wie Bsp. Ir(L192)₃ | 40 % |
| Ir(L280)₃ | L280 | Ir(L280)₃ | wie Bsp. Ir(L192)₃ | 37 % |
| Ir(L281)₃ | L281 | Ir(L281)₃ | wie Bsp. Ir(L192)₃ | 19 % |
| | | Λ,Δ-C3 | | |
| Ir(L282)₃ | L282 | Ir(L282)₃ | wie Bsp. Ir(L192)₃ | 33 % |
| Ir(L284) | L284 | Ir(L284) | wie Bsp. Ir(L192)₃ | 16 % |
| | 10 mmol | | Zusatz von 1 ml Tridecan | |
| Ir(L215)₃ | L215 | 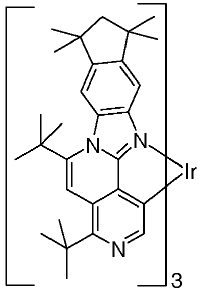 | B | 45 % |
| | | | 305 °C / 180 h | |
| | | | Aceton | |
| | | | Mesitylen | |
| | | Ir(L215)₃ | | |
| Ir(L216)₃ | L216 | Ir(L216)₃ | B | 34 % |
| | | | 310 °C / 210 h | |
| | | | Aceton | |
| | | | Mesitylen | |
| Ir(L217)₃ | L217 | Ir(L217)₃ | wie Bsp. Ir(L215)₃ | 44 % |
| Ir(L218)₃ | L218 | Ir(L218)₃ | wie Bsp. Ir(L215)₃ | 46 % |
| Ir(L219)₃ | L219 | Ir(L219)₃ | wie Bsp. Ir(L215)₃ | 22 % |
| | | Λ,Δ-C3 | | |
| Ir(L220)₃ | L220 | Ir(L220)₃ | wie Bsp. Ir(L215)₃ | 45 % |
| Ir(L221)₃ | L221 | Ir(L221)₃ | wie Bsp. Ir(L215)₃ | 44 % |
| | | Λ,Δ-C3 + C1 | | |
| Ir(L222)₃ | L222 | Ir(L222)₃ | wie Bsp. Ir(L215)₃ | 21 % |
| | | Λ,Δ-C3 | | |
| Ir(L223)₃ | L223 | Ir(L223)₃ | wie Bsp. Ir(L215)₃ | 41 % |
| Ir(L224)₃ | L224 | Ir(L224)₃ | wie Bsp. Ir(L215)₃ | 20 % |
| | | Λ,Δ-C3 | | |
| Ir(L225)₃ | L225 | Ir(L225)₃ | wie Bsp. Ir(L215)₃ | 35 % |
| Ir(L226)₃ | L226 | 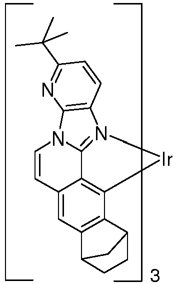 | B | 22 % |
| | | | 300 °C / 200 h | |
| | | | Aceton | |
| | | | Mesitylen | |
| | | Ir(L226)₃ Λ,Δ-C3 | | |
| Ir(L227)₃ | L227 | 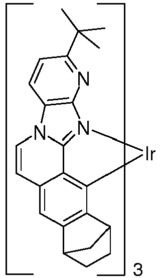 | wie Bsp. Ir(L226)₃ | 24 % |
| | | Ir(L227)₃ | | |
| | | Λ,Δ-C3 | | |
| Ir(L228)₃ | L228 | Ir(L228)₃ | wie Bsp. Ir(L226)₃ | 19 % |
| | | Λ,Δ-C3 | | |
| Ir(L229)₃ | L229 | Ir(L229)₃ | wie Bsp. Ir(L226)₃ | 17 % |
| | | Λ,Δ-C3 | | |
| Ir(L230)₃ | L230 | | B | 23 % |
| | | 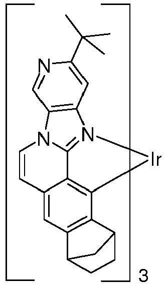 | 300 °C / 200 h | |
| | | | Aceton | |
| | | | Mesitylen | |
| | | Ir(L230)₃ | | |
| | | Λ,Δ-C3 | | |
| Ir(L231)₃ | L231 | 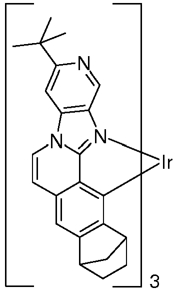 | wie Bsp. Ir(L230)₃ | 21 % |
| | | Ir(L231)₃ | | |
| | | Λ,Δ-C3 | | |
| Ir(L232)₃ | L232 | Ir(L232)₃ | wie Bsp. Ir(L230)₃ | 22 % |
| | | Λ,Δ-C3 | | |
| Ir(L233)₃ | L233 | Ir(L233)₃ | wie Bsp. Ir(L230)₃ | 24 % |
| | | Λ,Δ-C3 | | |
| Ir(L234)₃ | L234 | 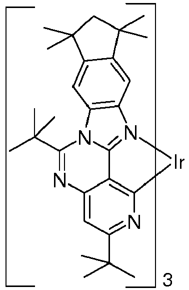 | B | 40 % |
| | | | 310 °C / 180 h | |
| | | | Aceton | |
| | | | Mesitylen | |
| | | Ir(L234)₃ | | |
| Ir(L235)₃ | L235 | Ir(L235)₃ | B | 30 % |
| | | | 310 °C / 210 h | |
| | | | Aceton | |
| | | | Mesitylen | |
| Ir(L236)₃ | L236 | Ir(L236)₃ | wie Bsp. Ir(L234)₃ | 38 % |
| Ir(L237)₃ | L237 | Ir(L237)₃ | wie Bsp. Ir(L234)₃ | 37 % |
| Ir(L238)₃ | L238 | Ir(L238)₃ | wie Bsp. Ir(L234)₃ | 18 % |
| | | Λ,Δ-C3 | | |
| Ir(L239)₃ | L230 | Ir(L239)₃ | wie Bsp. Ir(L234)₃ | 33 % |
| Ir(L240)₃ | L240 | Ir(L240)₃ | wie Bsp. Ir(L234)₃ | 21 % |
| | | Λ,Δ-C3 | | |
| Ir(L241)₃ | L241 | Ir(L241)₃ | wie Bsp. Ir(L234)₃ | 16 % |
| | | Λ,Δ-C3 | | |
| Ir(L242)₃ | L242 | Ir(L242)₃ | wie Bsp. Ir(L234)₃ | 31 % |
| Ir(L243)₃ | L243 | 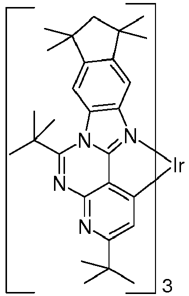 | B | 16 % |
| | | | 310 h | |
| | | | °C / 210 | |
| | | | Mesitylen | |
| | | Ir(L243)₃ | | |
| Ir(L244)₃ | L244 | Ir(L244)₃ | B | 14 % |
| | | | 315 °C / 210 h | |
| | | | Aceton | |
| | | | Mesitylen | |
| Ir(L245)₃ | L245 | Ir(L245)₃ | wie Bsp. Ir(L243)₃ | 18 % |
| Ir(L246)₃ | L246 | Ir(L246)₃ | wie Bsp. Ir(L243)₃ | 9 % |
| | | Λ,Δ-C3 | | |
| Ir(L247)₃ | L247 | Ir(L247)₃ | wie Bsp. Ir(L243)₃ | 7 % |
| Ir(L248)₃ | L248 | 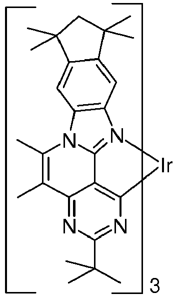 | B | 16 % |
| | | | 310 °C / 210 h | |
| | | | Aceton | |
| | | | Mesitylen | |
| | | Ir(L248)₃ | | |
| Ir(L249)₃ | L249 | Ir(L249)₃ | B | 8 % |
| | | | 315 °C / 240 h | |
| | | | Aceton | |
| | | | Mesitylen | |
| Ir(L250)₃ | L250 | Ir(L250)₃ | wie Bsp. Ir(L248)₃ | 17 % |
| Ir(L251)₃ | L251 | Ir(L251)₃ | wie Bsp. Ir(L248)₃ | 6 % |
| | | Λ,Δ-C3 | | |
| Ir(L252)₃ | L252 | 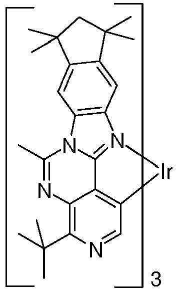 | B | 35 % |
| | | | 310 °C / 210 h | |
| | | | Aceton | |
| | | | Mesitylen | |
| | | Ir(L252)₃ | | |
| Ir(L253)₃ | L253 | Ir(L253)₃ | wie Bsp. Ir(L252)₃ | 20 % |
| Ir(L254)₃ | L254 | Ir(L254)₃ | wie Bsp. Ir(L252)₃ | 36 % |
| Ir(L255)₃ | L255 | Ir(L255)₃ | wie Bsp. Ir(L252)₃ | 34 % |
| Ir(L256)₃ | L256 | Ir(L256)₃ | wie Bsp. Ir(L252)₃ | 17 % |
| | | Λ,Δ-C3 | | |
| Ir(L257)₃ | L257 | Ir(L257)₃ | wie Bsp. Ir(L252)₃ | 18 % |
| | | Λ,Δ-C3 | | |
| Ir(L258)₃ | L258 | Ir(L258)₃ | wie Bsp. Ir(L252)₃ | 24 % |
| Ir(L259)₃ | L259 | 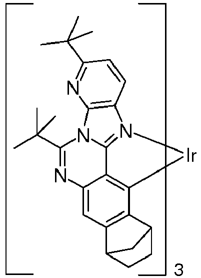 | B | 21 % |
| | | | 310 °C/ 200 h | |
| | | | Aceton | |
| | | | Mesitylen | |
| | | Ir(L259)₃ | | |
| | | Λ,Δ-C3 | | |
| Ir(L260)₃ | L260 | 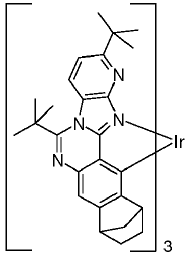 | wie Bsp. Ir(L259)₃ | 19 % |
| | | Ir(L260)₃ | | |
| | | Λ,Δ-C3 | | |
| Ir(L261)₃ | L261 | Ir(L261)₃ | wie Bsp. Ir(L259)₃ | 18 % |
| | | Λ,Δ-C3 | | |
| Ir(L262)₃ | L262 | Ir(L262)₃ | wie Bsp. Ir(L259)₃ | 16 % |
| | | Λ,Δ-C3 | | |
| Ir(L263)₃ | L263 | 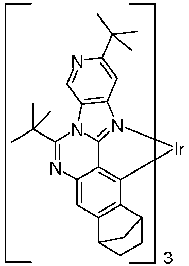 | B | 20 % |
| | | | 310 °C / 200 h | |
| | | | Aceton | |
| | | | Mesitylen | |
| | | Ir(L263)₃ | | |
| | | Λ,Δ-C3 | | |
| Ir(L264)₃ | L264 | 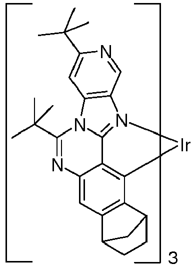 | wie Bsp. Ir(L263)₃ | 18 % |
| | | Ir(L264)₃ | | |
| | | Λ,Δ-C3 | | |
| Ir(L265)₃ | L265 | Ir(L265)₃ | wie Bsp. Ir(L263)₃ | 16 % |
| | | Λ,Δ-C3 | | |
| Ir(L266)₃ | L266 | Ir(L266)₃ | wie Bsp. Ir(L263)₃ | 15 % |
| | | Λ,Δ-C3 | | |
| Ir(L267)₃ | L267 | 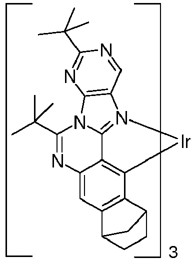 | B | 15 % |
| | | | 310 °C / 220 h | |
| | | | Aceton | |
| | | | Mesitylen | |
| | | Ir(L267)₃ | | |
| | | Λ,Δ-C3 | | |
| Ir(L268)₃ | L268 | 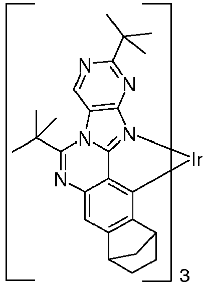 | B | 13 % |
| | | | 310 °C/ 220 h | |
| | | | Aceton | |
| | | | Mesitylen | |
| | | Ir(L268)₃ | | |
| | | Λ,Δ-C3 | | |
| Ir(L269)₃ | L269 | Ir(L269)₃ | wie Bsp. Ir(L267)₃ | 16 % |
| | | Λ,Δ-C3 | | |
| Ir(L270)₃ | L270 | Ir(L270)₃ | wie Bsp. Ir(L267)₃ | 16 % |
| | | Λ,Δ-C3 | | |
| Ir(L271)₃ | L271 | 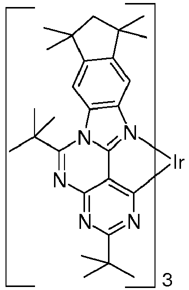 | B | 8 % |
| | | | 320 °C / 200 h | |
| | | | Aceton | |
| | | | Mesitylen | |
| | | Ir(L271)₃ | | |
| Ir(L272)₃ | L272 | Ir(L272)₃ | wie Bsp. Ir(L271)₃ | 2 % |
| Ir(L273)₃ | L273 | Ir(L273)₃ | wie Bsp. Ir(L271)₃ | 6 % |
| Ir(L274)₃ | L274 | Ir(L274)₃ | wie Bsp. Ir(L271)₃ | 6 % |
| | | Λ,Δ-C3 + C1 | | |
| Ir(L275)₃ | L275 | Ir(L275)₃ | wie Bsp. Ir(L271)₃ | 4 % |
| Ir(L500)₃ | L500 | 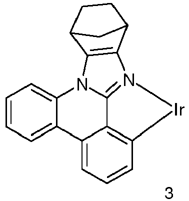 | B | 44 % |
| | | | 270 h | |
| | | | °C / 100 | |
| | | | Mesitylen | |
| | | Ir(L500)₃ | | |
| | | Λ,Δ-C3 | | |
| Ir(L501)₃ | L502 | Ir(L501)₃ | wie Bsp. Ir(L500)₃ | 21 % |
| | | Λ,Δ-C3 | | |
| Ir(L502)₃ | L503 | Ir(L502)₃ | wie Bsp. Ir(L500)₃ | 20 % |
| | | Λ,Δ-C3 | | |
| Ir(L503)₃ | L504 | Ir(L503)₃ | wie Bsp. Ir(L500)₃ | 23 % |
| | | Λ,Δ-C3 | | |
| Ir(L504)₃ | L505 | Ir(L504)₃ | wie Bsp. Ir(L500)₃ | 25 % |
| | | Λ,Δ-C3 | | |
| Ir(L505)₃ | L506 | Ir(L505)₃ | wie Bsp. Ir(L500)₃ | 19 % |
| | | Λ,Δ-C3 | | |
| Ir(L506)₃ | L507 | Ir(L506)₃ | wie Bsp. Ir(L500)₃ | 20 % |
| | | Λ,Δ-C3 | | |
| Ir(L507)₃ | L508 | Ir(L507)₃ | wie Bsp. Ir(L500)₃ | 22 % |
| | | Λ,Δ-C3 | | |
| Ir(L508)₃ | L509 | Ir(L508)₃ | wie Bsp. Ir(L500)₃ | 22 % |
| | | Λ,Δ-C3 | | |
| Ir(L509)₃ | L509 | Ir(L509)₃ | wie Bsp. Ir(L500)₃ | 26 % |
| | | Λ,Δ-C3 | | |
| Ir(L510)₃ | L510 | Ir(L510)₃ | wie Bsp. Ir(L500)₃ | 11 % |
| | | Λ,Δ-C3 | | |
| Ir(L511)₃ | L511 | 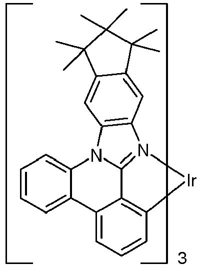 | B | 56 % |
| | | | 280 °C/150h | |
| | | | Aceton | |
| | | | Mesitylen | |
| | | Ir(L511)₃ | | |
| Ir(L512)₃ | L512 | Ir(L512)₃ | B | 45 % |
| | | | 300 150 h DCM | |
| | | | °C/ | |
| | | | Mesitylen | |
| Ir(L513)₃ | L513 | Ir(L513)₃ | wie Bsp. Ir(L511)₃ | 49 % |
| Ir(L514)₃ | L514 | Ir(L514)₃ | wie Bsp. Ir(L511)₃ | 23 % |
| | | Λ,Δ-C3 | | |
| Ir(L515)₃ | L515 | Ir(L515)₃ | wie Bsp. Ir(L511)₃ | 19 % |
| | | Λ,Δ-C3 | | |
| Ir(L516)₃ | L516 | Ir(L516)₃ | wie Bsp. Ir(L511)₃ | 51 % |
| Ir(L517)₃ | L517 | Ir(L517)₃ | wie Bsp. Ir(L511)₃ | 50 % |
| Ir(L518)₃ | L518 | Ir(L518)₃ | wie Bsp. Ir(L511)₃ | 21 % |
| | | Λ,Δ-C3 | | |

### 2) Heteroleptische Iridium-Komplexe:

### Variante A:

### Schritt 1:

Ein Gemisch aus 10 mmol Natrium-bis-acetylacetonato-dichloro-iridat(III) [770720-50-8] und 24 mmol des Liganden L und ein glasummantelter Magnetrührkern werden unter Vakuum (10⁻⁵ mbar) in eine dickwandige 50 ml Glasampulle abgeschmolzen. Die Ampulle wird für die angegebene Zeit bei der angegebenen Temperatur getempert, wobei das aufgeschmolzene Gemisch mit Hilfe eines Magnetrührers gerührt wird. Nach Erkalten - ACHTUNG: die Ampullen stehen meist unter Druck! - wird die Ampulle geöffnet, der Sinterkuchen wird mit 100 g Glaskugeln (3 mm Durchmessser) in 100 ml des angegebenen Suspensionsmittels (das Suspensionsmittel wird so gewählt, dass der Ligand gut, das Chloro-Dimer der Formel [Ir(L)₂Cl]₂ jedoch schlecht darin löslich ist, typische Suspensionsmittel sind DCM, Aceton, Ethylacetat, Toluol, etc.) 3 h gerührt und dabei mechanisch aufgeschlossen. Man dekantiert die feine Suspension von den Glaskugeln ab, saugt den Feststoff (Ir(L)₂Cl]₂, das noch ca. 2 eq NaCl enthält, nachfolgend das rohe Chloro-Dimer genannt) ab und trocknet diesen im Vakuum.

### Schritt 2:

Das so erhaltene rohe Chloro-Dimer der Formel [Ir(L)₂Cl]₂ wird in einem Gemisch aus 75 ml 2-Ethoxyethanol und 25 ml Wasser suspendiert, mit 13 mmol des Co-Liganden CL bzw. der Co-Liganden-Verbindung CL und 15 mmol Natriumcarbonat versetzt. Nach 20 h unter Rückfluss gibt man weitere 75 ml Wasser tropfenweise zu, saugt nach Erkalten vom Feststoff ab, wäscht diesen dreimal mit je 50 ml Wasser und dreimal mit je 50 ml Methanol und trocknet diesen im Vakuum. Der trockene Feststoff wird in einem kontinuierlichen Heißextraktor auf einem 3-5 cm hohen Alox-Bett (Alox, basisch Aktivitätsstufe 1) platziert und dann mit dem angegebenen Extraktionsmittel (Vorlagemenge ca. 500 ml, das Extraktionsmittel wird so gewählt, dass der Komplex darin in der Hitze gut und in der Kälte schlecht löslich ist, besonders geeignete Extraktionsmittel sind Kohlenwasserstoffe wie Toluol, Xylole, Mesitylen, Naphthalin, o-Dichlorbenzol, halogenierte aliphatische Kohlenwasserstoffe sind in der Regel ungeeignet, da sie die Komplexe gegebenenfalls halogenieren oder zersetzen) extrahiert. Nach beendeter Extraktion wird das Extraktionsmittel im Vakuum auf ca. 100 ml eingeengt. Metallkomplexe, die im Extraktionsmittel eine zu gute Löslichkeit aufweisen, werden durch Zutropfen von 200 ml Methanol zur Kristallisation gebracht. Der Feststoff der so erhaltenen Suspensionen wird abgesaugt, einmal mit ca. 50 ml Methanol gewaschen und getrocknet. Nach Trocknen wird die Reinheit des Metall-Komplexes mittels NMR und / oder HPLC bestimmt. Liegt die Reinheit unter 99.5 % wird der Heißextraktionsschritt wiederholt, ist eine Reinheit von 99.5 - 99.9 % erreicht, wird der Metallkomplex getempert oder sublimiert. Neben dem Heißextraktionsverfahren zur Reinigung kann die Reinigung auch chromatographisch an Kieselgel oder Alox erfolgen. Das Tempern erfolgt im Hochvakuum (p ca. 10⁻⁶ mbar) im Temperaturbereich von ca. 200 - 300 °C. Die Sublimation erfolgt im Hochvakuum (p ca. 10⁻⁶ mbar) im Temperaturbereich von ca. 300 - 400 °C, wobei die Sublim ation bevorzugt in Form einer fraktionierten Sublimation durchgeführt wird.

| **Bsp.** | **Ligand L** | **Co-Ligand CL** | **Ir-Komplex Schritt 1: Reaktionstemp. / Reaktionszeit / Suspensionsmittel Schritt 2: Extraktionsmittel** | **Aus-beute** |
|---|---|---|---|---|
| Ir(L3)₂(CL1) | L3 | 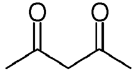 | 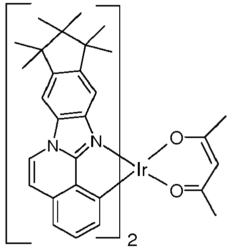 | 48% |
| | | 123-54-6 | | |
| | | CL1 | 260 °C / 60 h / Aceton Xylol | |
| Ir(L16)₂(CL1) | L16 | CL1 | 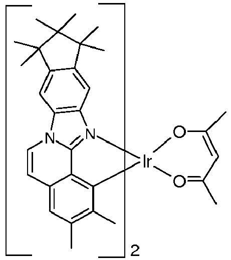 | 45% |
| | | | 270 °C / 80 h / Aceton Xylol | |
| Ir(L32)₂(CL1) | L32 | CL1 | 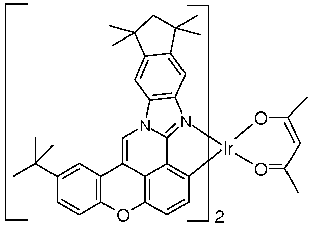 | 56% |
| | | | wie Bsp. Ir(L16)₂(CL1) | |
| Ir(L79)₂(CL2) | L79 | 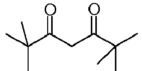 | 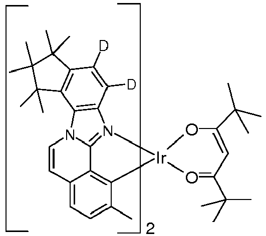 | 39% |
| | | 1118-71-4 | | |
| | | CL2 | 280 °C / 80 h / Ethylacetat Xylol | |
| Ir(L98)₂(CL2) | L98 | CL2 | 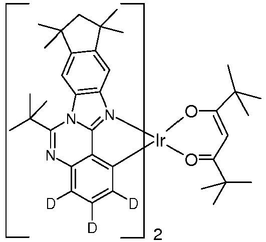 | 54% |
| | | | 290 °C / 60 h / Aceton Xylol | |
| Ir(L113)₂(CL2) | L113 | CL2 | 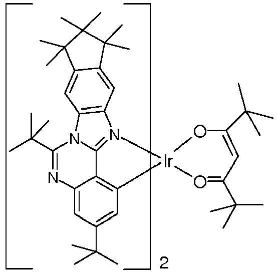 | 60% |
| | | | wie Bsp. Ir(L98)₂(CL2) | |
| Ir(L127)₂(CL3) | L127 | 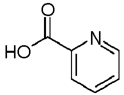 | 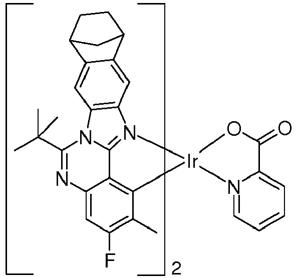 | 47% |
| | | 98-98-6 | | |
| | | CL3 | wie Bsp. Ir(L98)₂(CL2) Diasteromerengemisch | |
| Ir(L158)₂(CL4) | L158 | 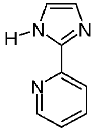 | 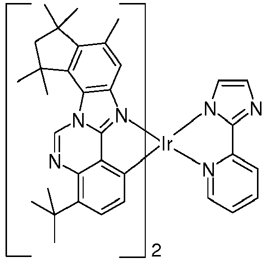 | 44% |
| | | 18653-75-3 | | |
| | | CL4 | wie Bsp. Ir(L98)₂(CL2) | |
| Ir(L169)₂(CL5) | L169 | 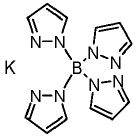 | 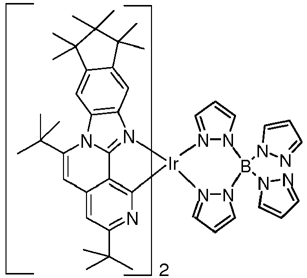 | 50% |
| | | 14782-58-2 | | |
| | | CL5 | 295 °C / 100 h / Aceton Mesitylen | |
| Ir(L195)₂(CL7) | L195 | 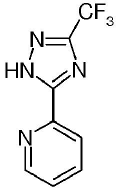 | 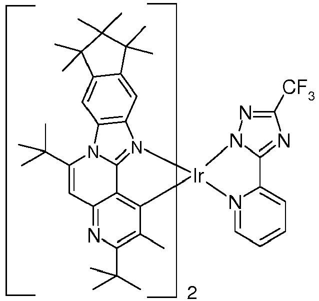 | 47% |
| | | 219508-27-7 | | |
| | | CL6 | 300 °C / 120 h / Aceton Mesitylen | |

### Variante B:

### Schritt 1:

Siehe Variante A, Schritt 1.

### Schritt 2:

Das so erhaltene rohe Chloro-Dimer der Formel [Ir(L)₂Cl]₂ wird in 200 ml THF suspendiert, die Suspension wird mit 20 mmol des Co-Liganden CL, 20 mmol Silber(I)trifluoracetat und 30 mmol Kaliumcarbonat versetzt und 24 h unter Rückfluss erhitzt. Nach Erkalten wird das THF im Vakuum entfernt. Der Rückstand wird in 200 ml eines Gemischs aus Ethanol und konz. Ammoniak-Lösung (1:1, vv) aufgenommen. Die Suspension wird 1 h bei Raumtemperatur gerührt, der Feststoff wird abgesaugt, zweimal mit je 50 ml eines Gemischs aus Ethanol und konz. Ammoniak-Lösung (1:1, vv) und zweimal mit je 50 ml Ethanol gewaschen und dann im Vakuum getrocknet. Heißextraktion und Sublimation wie in Variante A.

| **Bsp.** | **Ligand L** | **Co-Ligand CL** | **Ir-Komplex Schritt 1: Reaktionstemp. / Reaktionszeit / Suspensionsmittel Schritt 2: Extraktionsmittel** | **Ausbeute** |
|---|---|---|---|---|
| Ir(L99)₂(CL7) | L99 | 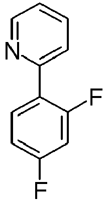 | 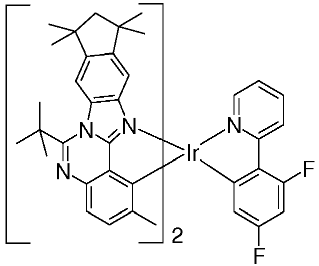 | 39% |
| | | 391604-55-0 | | |
| | | CL7 | wie Bsp. Ir(L98)₂(CL2) | |
| Ir(L110)₂(CL8) | L110 | 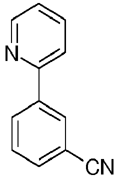 | 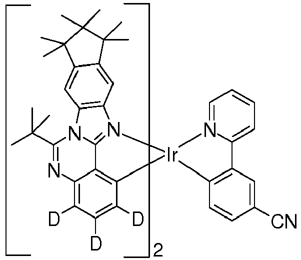 | 31 % |
| | | 4350-51-0 | | |
| | | CL8 | wie Bsp. Ir(L98)₂(CL2) | |
| Ir(L158)₂(CL8) | L158 | 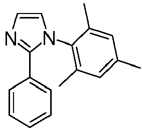 | 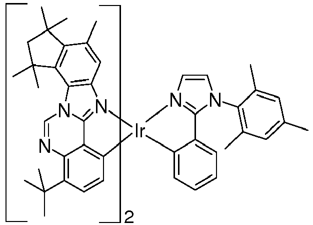 | 39% |
| | | 1093072-00-4 | 290 °C / 70 h / Aceton | |
| | | CL9 | Xylol | |
| Ir(L195)₂(CL10) | L195 | 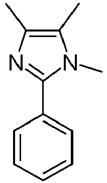 | 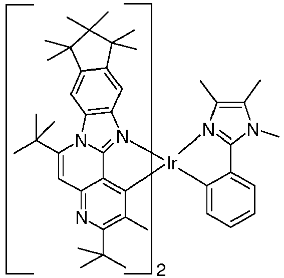 | 38% |
| | | 152536-39-5 | | |
| | | CL10 | 300 °C / 80 h / Aceton Xylol | |

### Variante C:

### Schritt 1:

Siehe Variante A, Schritt 1.

### Schritt 2:

Das so erhaltene rohe Chloro-Dimer der Formel [Ir(L)₂Cl]₂ wird in 1000 ml Dichlormethan und 150 ml Ethanol suspendiert, die Suspension wird mit 20 mmol Silber(I)trifluormethansulfonat versetzt und 24 h bei Raumtemperatur gerührt. Man saugt vom ausgefallen Feststoff (AgCl) über eine kurzes Celite-Bett ab und engt das Filtrat im Vakuum zur Trockene ein. Der so erhaltene Feststoff wird in 100 ml Ethylenglykol aufgenommen, mit 20 mmol des Co-Liganden CL versetzt und dann 30 h bei 130 °C gerührt. Nach Erkalten saugt man vom Feststoff ab, wäscht diesen zweimal mit je 50 ml Ethanol und trocknet im Vakuum. Heißextraktion und Sublimation wie in Variante A.

| **Bsp.** | **Ligand L** | **Co-Ligand CL** | **Ir-Komplex Schritt 1: Reaktionstemp. / Reaktionszeit / Suspensionsmittel Schritt 2: Extraktionsmittel** | **Ausbeute** |
|---|---|---|---|---|
| Ir(L191)₂(CL11) | L191 | 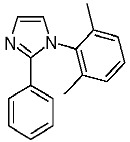 | 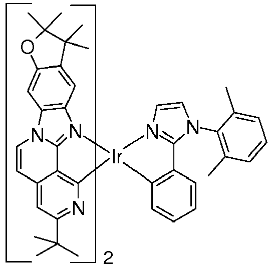 | 46% |
| | | 914306-48-2 | | |
| | | CL11 | 290 °C / 80 h / Aceton Xylol | |
| Ir(L201)₂(CL11) | L201 | CL11 | 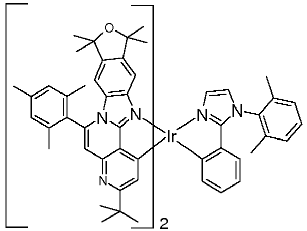 | 39% |
| | | | 290 °C / 80 h / Aceton Xylol | |
| Ir(L220)₂(CL12) | L220 | 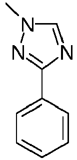 | 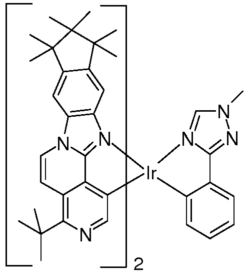 | 44% |
| | | 39696-58-7 | | |
| | | CL12 | 300 °C / 80 h / Aceton Xylol | |

### Variante E:

Ein Gemisch aus 10 mmol des Ir-Komplexes Ir(L)₂(CL1 oder CL2) und 20 mmol des Liganden L' und ein glasummantelter Magnetrührkern werden unter Vakuum (10⁻⁵ mbar) in eine 50 ml Glasampulle abgeschmolzen. Die Ampulle wird für die angegebene Zeit bei der angegebenen Temperatur getempert, wobei das aufgeschmolzene Gemisch mit Hilfe eines Magnetrührers gerührt wird. Weitere Aufarbeitung, Reinigung und Sublimation wie unter 1) Homoleptische tris-faciale Iridium-Komplexe beschrieben.

| **Bsp.** | **Ir-Komplex Ir(L)₂(CL)** | **Ligand L** | **Ir-Komplex Schritt 1: Reaktionstemp. / Reaktionszeit / Suspensionsmittel Schritt 2: Extraktionsmittel** | **Ausbeute** |
|---|---|---|---|---|
| Ir(L3)₂(L9) | Ir(L3)₂(CL1) | L9 | 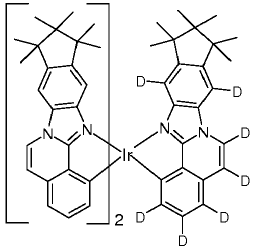 | 39% |
| | | | 280 °C / 80 h / DCM Mesitylen | |
| Ir(L98)₂(L53) | Ir(L98)₂(CL2) | L53 | 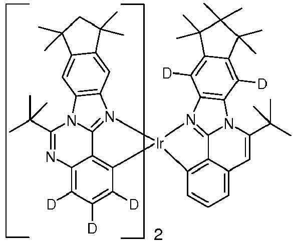 | 43% |
| | | | 300 °C / 70 h / DCM Mesitylen | |
| Ir(L113)₂(L109) | Ir(L113)₂(CL2) | L109 | 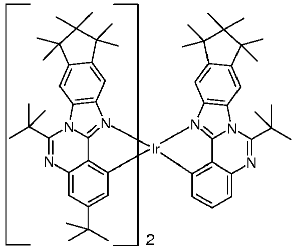 | 44% |
| | | | 300 °C / 70 h / DCM Mesitylen | |
| Ir(L113)₂(L204) | Ir(L113)₂(CL2) | L204 | 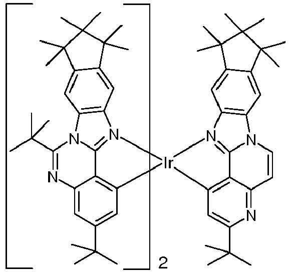 | 36% |
| | | | 305 °C / 70 h / DCM Mesitylen | |

### 3) Heteroleptische Platin-Komplexe:

Ein Gemisch aus 10 mmol Platin(II)chlorid und 12 mmol des Liganden L und ein glasummantelter Magnetrührkern werden unter Vakuum (10⁻⁵ mbar) in eine dickwandige 50 ml Glasampulle abgeschmolzen. Die Ampulle wird für die angegebene Zeit bei der angegebenen Temperatur getempert, wobei das aufgeschmolzene Gemisch mit Hilfe eines Magnetrührers gerührt wird. Nach Erkalten - ACHTUNG: die Ampullen stehen meist unter Druck! - wird die Ampulle geöffnet, der Sinterkuchen wird mit 100 g Glaskugeln (3 mm Durchmessser) in 100 ml des angegebenen Suspensionsmittels (das Suspensionsmittel wird so gewählt, dass der Ligand gut, das Chloro-Dimer der Formel [Pt(L)Cl]₂ jedoch schlecht darin löslich ist, typische Suspensionsmittel sind DCM, Aceton, THF, Ethylacetat, Toluol, etc.) 3 h gerührt und dabei mechanisch aufgeschlossen. Man dekantiert die feine Suspension von den Glaskugeln ab, saugt den Feststoff ab und trocknet diesen im Vakuum. Das so erhaltene rohe Chloro-Dimer der Formel [Pt(L)Cl]₂ wird in einem Gemisch aus 60 ml 2-Ethoxyethanol und 20 ml Wasser suspendiert und mit 20 mmol des Co-Liganden CL bzw. der Co-Liganden-Verbindung CL und 20 mmol Natriumcarbonat versetzt. Nach 20 h unter Rückfluss gibt man weitere 100 ml Wasser tropfenweise zu, saugt nach Erkalten vom Feststoff ab, wäscht diesen dreimal mit je 50 ml Wasser und dreimal mit je 50 ml Methanol und trocknet diesen im Vakuum. Der so erhaltene Feststoff wird in einem Heißextraktor auf einem 3-5 cm hohen Celite-Bett platziert und dann mit dem angegebenen Extraktionsmittel (Vorlagemenge ca. 500 ml) extrahiert. Nach beendeter Extraktion wird das Extraktionsmittel im Vakuum auf ca. 100 ml eingeengt. Metallkomplexe, die im Extraktionmittel eine zu gute Löslichkeit aufweisen, werden durch Zutropfen von 200 ml Methanol zur Kristallisation gebracht. Der Feststoff der so erhaltenen Suspensionen wird abgesaugt, einmal mit ca. 50 ml Methanol gewaschen und getrocknet. Nach Trocknen wird die Reinheit des Metall-Komplexes mittels NMR und / oder HPLC bestimmt. Liegt die Reinheit unter 99.5 % wird der Heißextraktionsschritt wiederholt, ist eine Reinheit von 99.5 - 99.9 % erreicht, wird der Metallkomplex getempert oder sublimiert. Das Tempern erfolgt im Hochvakuum (p ca. 10⁻⁶ mbar) im Temperaturbereich von ca. 200 - 300 °C. Die Sublimation erfolgt im Hochvakuum (p ca. 10⁻⁶ mbar) im Temperaturbereich von ca. 250 - 350 °C, wobei die Sublimation bevorzugt in Form einer fraktionierten Sublimation durchgeführt wird.

| **Bsp.** | **Ligand L** | **Co-Ligand CL** | **Pt-Komplex** | **Ausbeute** |
|---|---|---|---|---|
| Pt(L3)(CL1) | L3 | CL1 | 270 °C / 60 h / Aceton Xylol | 20 % |
| Pt(L126)(CL2) | L126 | CL2 | 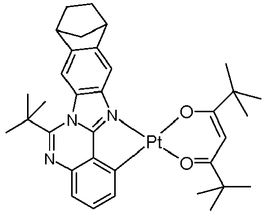 | 23 % |
| | | | 280 °C / 50 h / Aceton Xylol | |

### 4) Platin-Komplexe tetradentater Liganden:

Ein Gemisch aus 10 mmol Bis(benzonitril)-dichloro-platin(II) und 10 mmol des Liganden L in 100 ml Benzonitril wird 24 h unter Rückfluss erhitzt. Nach tropfenweiser Zugabe von 100 ml Methanol zur erkalteten Reaktionsmischung wird vom Feststoff abgesaugt, dieser wird fünfmal mit je 25 ml Methanol gewaschen und im Vakuum getrocknet. Der so erhaltene Feststoff wird in einem Heißextraktor auf einem 3 cm hohen Celite-Bett (Alox, basisch Aktivitätsstufe 1) platziert und dann mit dem angegebenen Extraktionsmittel (Vorlagemenge ca. 300 ml) extrahiert. Nach beendeter Extraktion wird das Extraktionsmittel im Vakuum auf ca. 100 ml eingeengt. Metallkomplexe, die im Extraktionsmittel eine zu gute Löslichkeit aufweisen, werden durch Zutropfen von 200 ml Methanol zur Kristallisation gebracht. Der Feststoff der so erhaltenen Suspensionen wird abgesaugt, einmal mit ca. 50 ml Methanol gewaschen und getrocknet. Nach Trocknen wird die Reinheit des Metall-Komplexes mittels NMR und / oder HPLC bestimmt. Liegt die Reinheit unter 99.5 % wird der Heißextraktionsschritt wiederholt, ist eine Reinheit von 99.5 - 99.9 % erreicht, wird der Pt-Komplex sublimiert. Die Sublimation erfolgt im Hochvakuum (p ca. 10⁻⁶ mbar) im Temperaturbereich von ca. 350 bis ca. 390 °C, wobei die Sublimation bevorzugt in Form einer fr aktionierten Sublimation durchgeführt wird.

### Pt(L283):

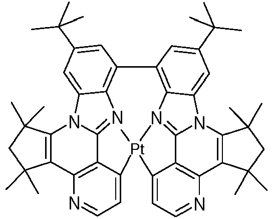

Einsatz von 4.74 g 10 mmol Bis(benzonitril)-dichloro-platin(II) und 7.55 g (10 mmol) L283. Extraktionsmittel: Mesitylen. Ausbeute: 3.22 g (3.4 mmol) 34 %; Reinheit: ca. 99.8 %ig nach NMR.

### E: Derivatisierung der Metallkomplexe

### 1) Halogenierung der Iridium-Komplexe:

Eine Lösung bzw. Suspension von 10 mmol eines Komplexes, der in para-Position zum Iridium A x C-H-Gruppen(mit A = 1, 2 oder 3) trägt, in 3000 ml Dichlormethan wird unter Licht- und Luftausschluss bei 30 °C mit A x 11 mmol N-Halogensuccinimid (Halogen: Cl, Br, I) versetzt und 20 h gerührt. In DCM schlecht lösliche Komplexe können auch in anderen Lösungsmitteln (TCE, THF, DMF, etc.) und bei erhöhter Temperatur umgesetzt werden. Anschließend wird das Lösungsmittel im Vakuum weitgehend entfernt. Der Rückstand wird mit 100 ml MeOH ausgekocht, der Feststoff wird abgesaugt, dreimal mit 30 ml Methanol gewaschen und dann im Vakuum getrocknet.

### Synthese von Ir(L1-Br)₃:

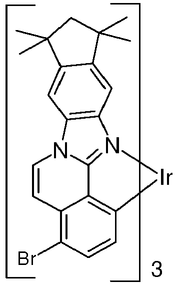

Eine bei 30 °C gerührte Suspension von 11.3 g (10 mmol) Ir(L1)₃ in 3000 ml DCM wird auf ein Mal mit 5.9 g (33 mmol) N-Bromsuccinimid versetzt und dann weitere 20 h gerührt. Nach Entfernen von ca. 2900 ml das DCMs im Vakuum wird die zitronengelbe Suspension mit 100 ml Methanol versetzt, der Feststoff wird abgesaugt, dreimal mit ca. 30 ml Methanol gewaschen und dann im Vakuum getrocknet. Ausbeute: 13.8 g (9.7 mmol) 97 %; Reinheit: ca. 99.5 %ig nach NMR.

Analog können folgende Verbindungen dargestellt werden:

| Bsp. | Komplex | Bromierter Komplex | Ausbeute |
|---|---|---|---|
| Ir(L3-Br)₃ | 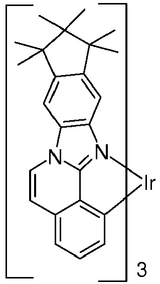 | 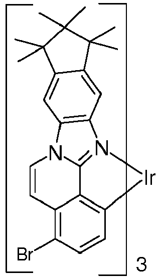 | 95 % |
| | Ir(L3)₃ | Ir(L3-Br)₃ | |
| Ir(L8-Br)₃ | 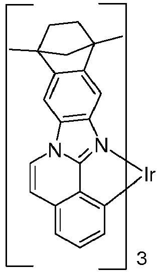 | 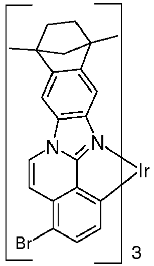 | 96 % |
| | Ir(L8)₃ | Ir(L8-Br)₃ | |
| | Λ,Δ-C3 | Λ,Δ-C3 | |
| Ir(L10-Br)₃ | 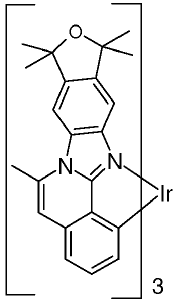 | 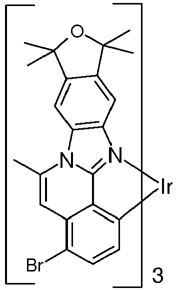 | 95 % |
| | Ir(L10)₃ | Ir(L10-Br)₃ | |
| Ir(L55-Br)₃ | 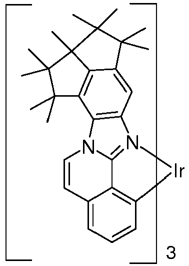 | 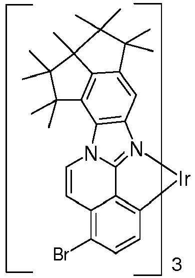 | 91 % |
| | Ir(L55)₃ Diastereomerenmischung | Ir(L55-Br)₃ Diastereomerenmischung | |
| Ir(L96-Br)₃ | 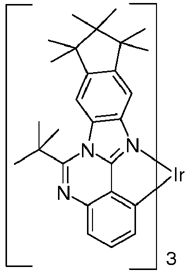 | 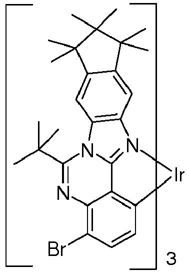 | 98 % |
| | Ir(L96)₃ | Ir(L96-Br)₃ | |
| Ir(L120-Br)₃ | 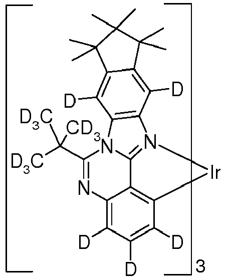 | 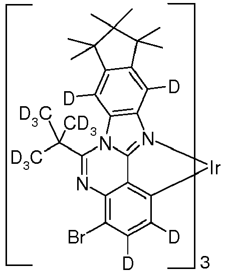 | 96 % |
| | Ir(L120)₃ | Ir(L120-Br)₃ | |
| Ir(L126-Br)₃ | 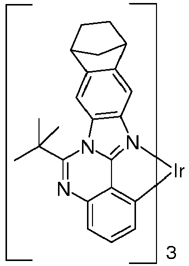 | 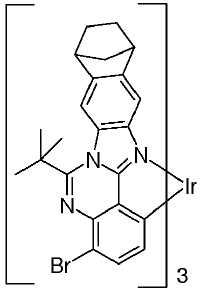 | 97 % |
| | Λ,Δ-C3-Ir(L126)₃ | Λ,Δ-C3-Ir(L126-Br)₃ | |
| Ir(L113)₂(L109-Br) | 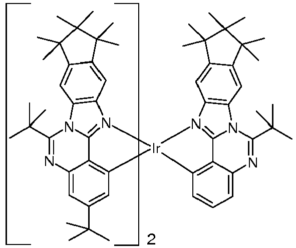 | 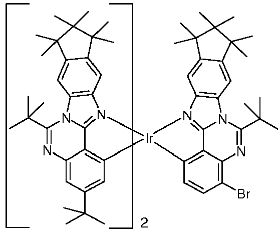 | 95 % |
| | Ir(L113)₂(L109) 1.1 eq NBS | Ir(L113)₂(L109-Br) | |

### 2) Suzuki-Kupplung an den Iridium-Komplexen: Variante A, zweiphasige Reaktionsmischung:

Eine Suspension von 10 mmol eines bromierten Komplexes, 40 - 80 mmol der Boronsäure bzw. des Boronsäureesters und 80 mmol Trikaliumphosphat in einem Gemisch aus 300 ml Toluol, 100 ml Dioxan und 300 ml Wasser wird mit 0.6 mmol Tri-o-Tolylphosphin und dann mit 0.1 mmol Palladium(II)acetat versetzt und 16 h unter Rückfluss erhitzt. Nach Erkalten gibt man 500 ml Wasser und 200 ml Toluol zu, trennt die wässrige Phase ab, wäscht die org. Phase dreimal mit 200 ml Wasser, einmal mit 200 ml ges. Kochsalzlösung und trocknet über Magnesiumsulfat. Man filtriert über ein Celite-Bett ab, wäscht dieses mit Toluol nach, entfernt das Toluol fast vollständig im Vakuum, gibt 300 ml Ethanol zu, saugt vom ausgefallenen Rohprodukt ab, wäscht dieses dreimal mit je 100 ml EtOH und trocknet im Vakuum. Das Rohprodukt wird mit Toluol zweimal an Kieselgel gesäult. Der Metallkomplex wird abschließend getempert oder sublimiert. Das Tempern erfolgt im Hochvakuum (p ca. 10⁻⁶ mbar) im Temperaturbereich von ca. 200 - 300 °C. Die Sublimation erfolgt im Hochvakuum (p ca. 10⁻⁶ mbar) im Temperaturbereich von ca. 300 - 400 °C, wobei die Sublim ation bevorzugt in Form einer fraktionierten Sublimation durchgeführt wird.

### Variante B, einphasige Reaktionsmischung:

Eine Suspension von 10 mmol eines bromierten Komplexes, 40 - 80 mmol der Boronsäure bzw. des Boronsäureesters und 60 - 100 mmol der Base (Kaliumfluorid, Trikaliumphosphat, Kaliumcarbonat, Cäsiumcarbonat etc. jeweils wasserfrei) und 100 g Glaskugeln (3 mm Durchmesser) in 100 ml - 500 ml eines aprotischen Lösungsmittels (THF, Dioxan, Xylol, Mesitylen, Dimethylacetamid, NMP, DMSO, etc.) wird mit 0.6 mmol Tri-o-Tolylphosphin und dann mit 0.1 mmol Palladium(II)acetat versetzt und 1 - 24 h unter Rückfluss erhitzt. Alternativ können andere Phosphine wir Tri-tert-butylphosphin, Di-tert-butylphosphin, S-Phos, Xanthphos, etc. eingesetzt werden, wobei bei diesen Phosphinen das bevorzugte Phosphin : Palladium Verhältnis 2:1 bis 1.2 : 1 beträgt. Man entfernt das Lösungsmittel im Vakuum, nimmt das Produkt in einem geeigneten Lösungsmittel (Toluol, Dichlormethan, Ethylacetat, etc.) auf und reinigt wie unter A beschrieben.

### Synthese von Ir(L276)₃:

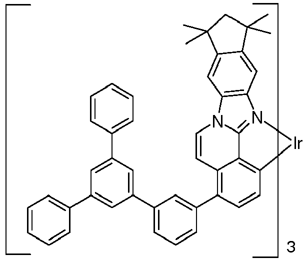

### Variante B:

Einsatz von 14.5 g (10 mmol) Ir(L1-Br)₃ und 14.0 g (40 mmol) Quaterphenylboronsäure [1233200-59-3], Cäsiumcarbonat, Tri-ortho-tolylphosphin, NMP, 180 °C, 1 h . Ausbeute: 12.1 g (5. 9 mmol) 59 %; Reinheit: ca. 99.8 %ig nach HPLC.

Analog können folgende Verbindungen dargestellt werden:

| Bsp. | Produkt | Ausbeute |
|---|---|---|
| | Variante | |
| Ir(L277)₃ | 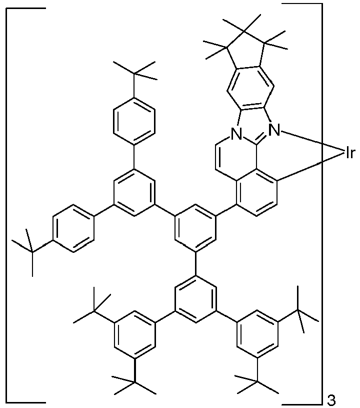 | 47 % |
| | Ir(L3-Br)₃ + [952583-08-3] > Ir(277)₃ | |
| | B, wie Ir(L276)₃ | |
| Ir(L278)₃ | 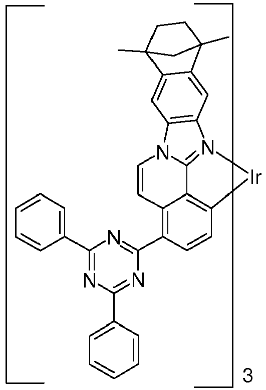 | 55 % |
| | Ir(L8-Br)₃ + [1251825-65-6] > Ir(278)₃ | |
| | A | |
| Ir(L279)₃ | | 46 % |
| | 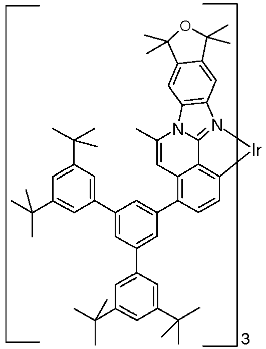 | |
| | Ir(10-Br)₃ + [1071924-15-6] > Ir(278)₃ | |
| | B, wie Ir(L276)₃ | |
| Ir(L280)₃ | 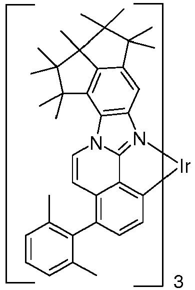 | 52 % |
| | Ir(L55-Br)₃ + [100379-00-8] > Ir(L280)₃ Diasteroemerenmischung | |
| | B, wie Ir(L276)₃, Dioxan statt NMP | |
| Ir(L281)₃ | 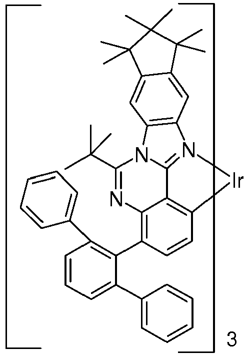 | 23 % |
| | Ir(L96-Br)₃ + [1065663-52-6] > Ir(L281)₃ | |
| | B, wie Ir(L276)₃ | |
| Ir(L282)₃ | 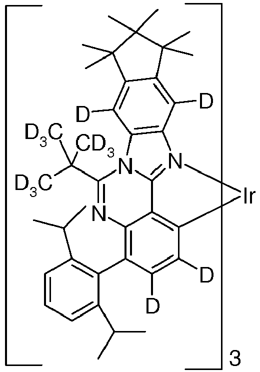 | 19 % |
| | Ir(L120-Br)₃+ [363166-79-4] > Ir(L282)₃ | |
| | B, Cs(CO₃)₂, NMP, S-Phos | |
| Ir(L283)₃ | 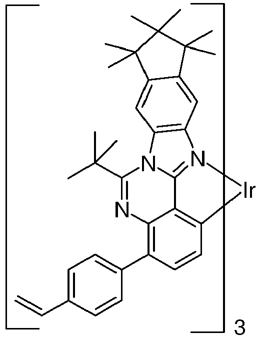 | 25 % |
| | Ir(L96-Br)₃ + [2156-04-9] > Ir(L283)₃ | |
| | B, wie Ir(L276)₃, DMAC statt NMP | |
| Ir(L284)₃ | 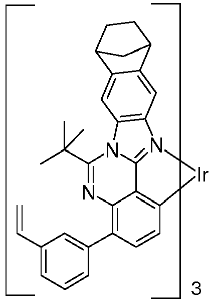 | 27% |
| | Λ,Δ-C3-Ir(L126-Br) ₃ + [15016-43-0] > Λ,A-C3-Ir(L284)₃ | |
| | B, wie Ir(L276)₃, DMAC statt NMP | |
| Ir(L285)₃ | 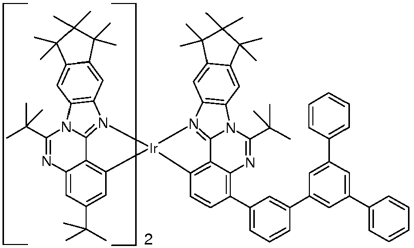 | 67% |
| | Ir(L113)₂(L109-Br)+ [1233200-59-3] > Ir(L285)₃ | |
| | A | |

### 3) Buchwald-Kupplung an den Iridium-Komplexen:

Ein Gemisch aus 10 mmol des bromierten Komplexe, 40 mmol des Diarylamins oder Carbazols, 45 mmol Natrium-tert-butylat bei den Aminen bzw. 80 mmol Trikaliumphosphat, wasserfrei bei Carbazolen, 100 g Glaskugeln (3 mm Durchmesser) und 300 - 500 ml o-Xylol oder Mesitylen wird mit 0.4 mmol Tri-tert-butylphosphin und dann mit 0.3 mmol Palladium(II)acetat versetzt und unter gutem Rühren 16 h unter Rückfluss erhitzt. Nach Erkalten trennt man die wässrige Phase ab, wäscht zweimal mit 200 ml Wasser, einmal mit 200 ml ges. Kochsalzlösung und trocknet über Magnesiumsulfat. Man filtriert über ein Celite-Bett ab, wäscht dieses mit o-Xylol oder Mesitylen nach, entfernt das Lösungsmittel fast vollständig im Vakuum, gibt 300 ml Ethanol zu, saugt vom ausgefallenen Rohprodukt ab, wäscht dieses dreimal mit je 100 ml EtOH und trocknet im Vakuum. Das Rohprodukt wird mit Toluol zweimal an Kieselgel gesäult. Der Metallkomplex wird abschließend getempert oder sublimiert. Das Tempern erfolgt im Hochvakuum (p ca. 10⁻⁶ mbar) im Temperaturbereich von ca. 200 - 300 °C. Die Sublimation erfolgt im Hochvakuum (p ca. 10⁻⁶ mbar) im Temperaturbereich von ca. 300 - 400 °C, wobei die Sublim ation bevorzugt in Form einer fraktionierten Sublimation durchgeführt wird.

### Synthese von Ir(L286)₃:

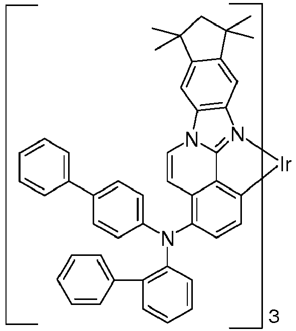

Einsatz von 14.5 g (10 mmol) Ir(L1-Br)₃ und 12.9 g (40 mmol) p-Biphenyl-o-biphenyl-amin [1372775-52-4] , Mesitylen. Ausbeute: 9.8 g (4.7 mmol) 47 %; Reinheit: ca. 99.8 %ig nach HPLC.

Analog können folgende Verbindungen dargestellt werden:

| Bsp. | Produkt | Ausbeute |
|---|---|---|
| Ir(L287)₃ | 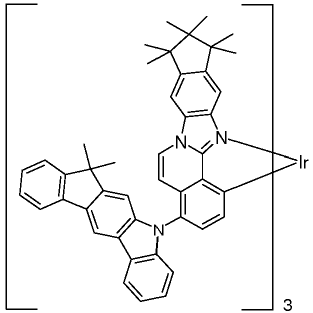 | 49 % |
| | Ir(L3-Br)₃ + [1257220-47-5] > Ir(287)₃ | |
| Ir(L288)₃ | 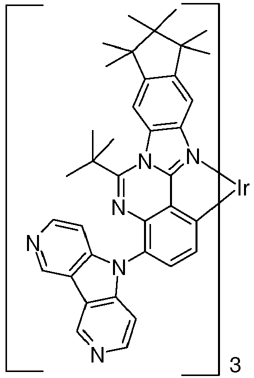 | 53 % |
| | Ir(L96-Br)₃ + [244-78-0] > Ir(L288)₃ | |

### 4) Cyanierung der Iridium-Komplexe:

Ein Gemisch aus 10 mmol des bromierten Komplexes, 1.3 mmol Kupfer(I)cyanid pro Brom-Funktion und 300 ml NMP wird 20 h bei 200 °C gerührt. Nach Erkalten entfernt man das Lösungsmittel im Vakuum, nimmt den den Rückstand in 500 ml Dichlormethan auf, filtriert über Celite von den Kupfersalzen ab, engt das Dichlormethan im Vakuum fast bis zur Trockene ein, gibt 100 ml Ethanol zu, saugt vom ausgefallenen Feststoff ab, wäscht diesen zweimal mit je 50 ml Ethanol und trocknet im Vakuum. Heißextraktion und Sublimation wie in 1) Variante A. Das Rohprodukt kann alternativ an Kieselgel mit Dichlormethan, gegebenenfalls unter Zusatz von Ethylacetat, chromatographiert und dann sublimiert werden.

### Synthese von Ir(L289)₃:

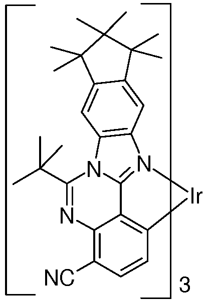

Einsatz von 16.2 g (10 mmol) Ir(L96-Br)₃ und 3.5 g (39) mmol) Kupfer(I)cyanid. Ausbeute: 7.7 g (5.2 mmol) 52 %; Reinheit: ca. 99.8 %ig nach HPLC.

Analog können folgende Verbindungen dargestellt werden:

| Bsp. | Produkt | Ausbeute |
|---|---|---|
| Ir(L290)₃ | 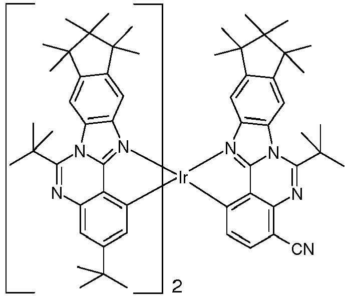 | 69 % |
| | Ir(L113)₂(L109-Br)+ CuCN > Ir(L290)₃ | |

### 5) Borylierung der Iridium-Komplexe:

Ein Gemisch von 10 mmol des bromierten Komplexes, 12 mmol Bis-(pinacolato)diboran [73183-34-3] pro Brom-Funktion, 30 mmol Kaliumacetat, wasserfrei pro Bromfunktion, 0.2 mmol Tricyclohexylphosphin und 0.1 mmol Palladium(II)acetat und 300 ml Lösungsmittel (Dioxan, DMSO, NMP, etc.) wird 4 - 16 h bei 80 °- 160 °C gerührt. N ach Entfernen des Lösungsmittels im Vakuum wird der Rückstand in 300 ml Dichlormethan, THF oder Ethylacetat aufgenommen, über ein Celite-Bett filtriert, das Filtrat wird bis zur beginnenden Kristallisation im Vakuum eingeengt und abschießend noch tropfenweise mit ca. 100 ml Methanol versetzt, um die Kristallisation zu vervollständigen. Die Verbindungen können aus Dichlormethan, Ethylacetat oder THF unter Zusatz von Methanol oder alternativ Cyclohexan umkristallisiert werden.

### Synthese von Ir(L1-B)₃:

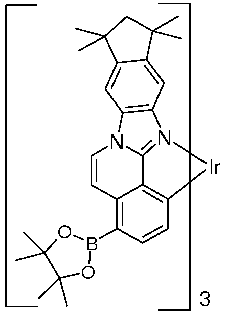

Einsatz von 14.5 g (10 mmol) Ir(L1-Br)₃ und 9.1 g (36 mmol) Bis(pinacolato)-diboran [73183-34-3], DMSO, 140 °C, 6 h, THF, Umkrist allisation aus THF:Methanol. Ausbeute: 7.1 g (4.7 mmol) 47 %; Reinheit: ca. 99.7 %ig nach HPLC.

### E: Polymere enthaltend die Metallkomplexe:

### 1) Allgemeine Polymerisationsvorschrift für die Styrylgruppe als polymerisierbare Gruppe

Die Monomere werden in der angegebenen Zusammensetzung in einer Gesamtkonzentration von ca. 1 mol/L in Toluol 80 °C gelöst. Anschließend werden 60 mg AIBN zugegeben und weitere 2 h bei 80 °C gerührt. Nach Erkalten auf Raumtemperatur wird das Polymer durch Fällung (tropfenweise Zugabe) in 100 mL Methanol erhalten. Der Niederschlag wird abgesaugt und anschließend wiederum in wenig Toluol gelöst und erneut in Methanol gefällt, abgesaugt und im Vakuum getrocknet. Der Umfällvorgang wird weitere dreimal durchgeführt.

### Monomere:

| | |
|---|---|
| 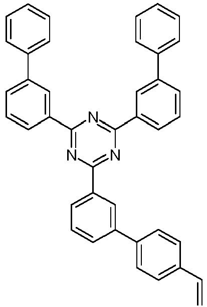 | 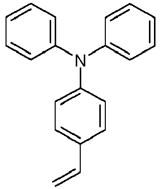 |
| 1354469-30-9 | 25069-74-3 |
| M1 | M2 |
| 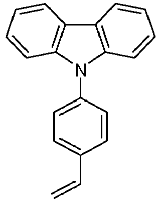 | 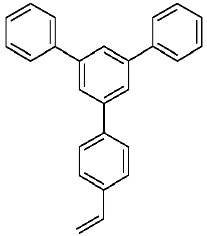 |
| 52913-19-6 | 1354469-27-4 |
| M3 | M4 |

### Polymere:

Zusammensetzung der Polymere, mol %:

| Polymer | M1 [%] | M2 [%] | M3 [%] | M4 [%] | Ir-Komplex / [%] |
|---|---|---|---|---|---|
| P1 | 80 | --- | --- | --- | Ir(L283)₃ / 20 |
| P2 | 60 | --- | 30 | --- | Ir(L283)₃ / 10 |
| P3 | 50 | 10 | 30 | --- | Ir(L283)₃ / 10 |
| P4 | 50 | 10 | 20 | 10 | Ir(L283)₃ / 10 |
| P5 | 60 | --- | 30 | --- | Ir(L284)₃ / 10 |

Molekulargewichte und Ausbeute der erfindungsgemäßen Polymere

| Polymer | Mn [gmol⁻¹] | Mw [gmol⁻¹] | Ausbeute |
|---|---|---|---|
| P1 | 77.000 | 17.100 | 53 % |
| P2 | 133.000 | 50.600 | 64 % |
| P3 | 127.000 | 72.000 | 60 % |
| P4 | 121.000 | 51.400 | 57 % |
| P5 | 76.800 | 23.260 | 49 % |

### 2) Allgemeine Polymerisationsvorschrift für die Bromide bzw. Boronsäure-Derivate als polymerisierbare Gruppe, Suzuki-Polymerisation

### Variante A- Zweiphasiges Reaktionsgemisch:

Die Monomere (Bromide und Boronsäuren bzw. Boronsäureester, Reinheit nach HPLC > 99.8 % ig) werden in der in Tabelle angegebenen Zusammensetzung in einer Gesamtkonzentration von ca. 100 mmol/L in einem Gemisch aus 2 Volumenteilen Toluol : 6 Volumenteilen Dioxan : 1 Volumenteil Wasser gelöst bzw. suspendiert. Dann gibt man 2 mol Äquivalente Trikalium-phosphat pro eingesetzter Br-Funktionalität zu, rührt 5 min. nach, fügt dann 0.03 bis 0.003 mol Äquivalente Tri-ortho-tolylphosphin und dann 0.005 bis 0.0005 mol Äquivalente Palladium(II)acetat (Verhältnis Phosphin zu Pd bevorzugt 6:1) pro eingesetzter Br-Funktionalität zu, und erhitzt dann unter sehr gutem Rühren 2-3 h unter Rückfluss. Falls die Viskosität der Mischung zu stark ansteigt kann mit einem Gemisch aus 2 Volumenteilen Toluol : 3 Volumenteilen Dioxan verdünnt werden. Nach insgesamt 4-6 h Reaktionszeit fügt man zum end-capping 0.05 mol Aquivalente pro eingesetzter Boronsäure-Funktionalität eines Monobromaromaten und dann 30 min. danach 0.05 mol Äquivalente pro eingesetzter Br-Funktionalität einer Monoboronsäure bzw. eines Monoboronsäureesters zu und kocht weiter 1 h nach. Nach Erkalten verdünnt man mit 300 ml Toluol. Trennt die wässrige Phase ab, wäscht die organische Phase zweimal mit je 300 ml Wasser, trocknet über Magnesiumsulfat, filtriert über ein Celite-Bett ab, um Palladium zu entfernen und engt dann zur Trockene ein. Man löst das Rohpolymer in THF (Konzentration ca. 10 - 30 g/L) und lässt die Lösung unter sehr gutem Rühren langsam in das doppelte Volumen Methanol einlaufen. Das Polymer wird abgesagt und dreimal mit Methanol gewaschen. Der Umfällvorgang wird dreimal wiederholt, danach wird das Polymer im Vakuum bis zur Gewichtskonstanz bei 30 - 50 °C getrocknet.

### Variante B - Einphasiges Reaktionsgemisch:

Die Monomere (Bromide und Boronsäuren bzw. Boronsäureester, Reinheit nach HPLC > 99.8 % ig) werden in der in Tabelle angegebenen Zusammensetzung in einer Gesamtkonzentration von ca. 100 mmol/L in einem Lösemittel (THF, Dioxan, Xyylol, Mesitylen, Dimethylacetamid, NMP, DMSO, etc.) gelöst bzw. suspendiert. Dann gibt man 3 mol Äquivalente Base (Kaliumfluorid, Trikaliumphosphat, Kaliumcarbonat, Cäsiumcarbonat etc. jeweils wasserfrei) pro Br-Funktionalität und das Gewichtsäquivalent Glaskugeln (3 mm Durchmesser) zu, rührt 5 min. nach, fügt dann 0.03 bis 0.003 mol Äquivalente Tri-orthotolylphosphin und dann 0.005 bis 0.0005 mol

Äquivalente Palladium(II)acetat (Verhältnis Phosphin zu Pd bevorzugt 6:1)pro Br-Funktionalität zu, und erhitzt dann unter sehr gutem Rühren 2-3 h unter Rückfluss. Alternativ können andere Phosphine wir Tri-tert-butylphosphin, Di-tert-butylphosphin, S-Phos, Xanthphos, etc. eingesetzt werden, wobei bei diesen Phosphinen das bevorzugte Phosphin : Palladium Verhältnis 2:1 bis 1.3:1 beträgt. Nach insgesamt 4-12 h Reaktionszeit fügt man zum end-capping 0.05 mol Aquivalente eines Monobromaromaten und dann 30 min. danach 0.05 mol Äquivalente einer Monoboronsäure bzw. eines Monoboronsäureesters zu und kocht weiter 1 h nach. Man entfernt das Lösungsmittel weitgehend im Vakuum, nimmt den Rückstand in Toluol auf, und reinigt das Polymer wie unter Variante A beschrieben.

### Monomere / Endcapper:

| | |
|---|---|
| 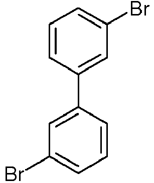 | 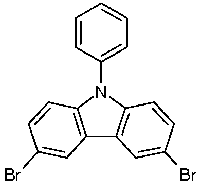 |
| 16400-51-4 | 57103-20-5 |
| M1 | M2 |
| 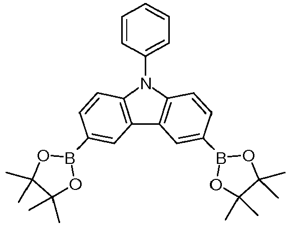 | 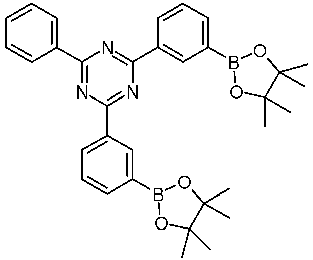 |
| 618442-57-2 | 1238752-26-5 |
| M3 | M4 |
| 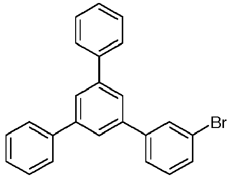 | 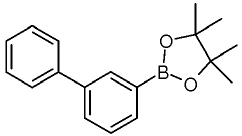 |
| 1233200-57-1 | 912844-88-3 |
| E1 | E2 |

### Polymere:

Zusammensetzung der Polymere, mol %:

| Polymer | M1 [%] | M2 [%] | M3 [%] | M4 [%] | Ir-Komplex / [%] |
|---|---|---|---|---|---|
| P6 | --- | 30 | --- | 45 | Ir(L1-Br)₃ / 10 |
| P7 | 10 | 10 | --- | 35 | Ir(L96-Br)₃ / 10 |
| P8 | 50 | --- | 20 | 45 | Ir(L96-Br)₃ / 10 |
| P9 | 30 | 30 | --- | 67.5 | Ir(L126-Br)₃ / 5 |

Molekulargewichte und Ausbeute der erfindungsgemäßen Polymere

| Polymer | Mn [gmol⁻¹] | Polydispersität | Ausbeute |
|---|---|---|---|
| P6 | 167.000 | 4.6 | 60 % |
| P7 | 153.000 | 5.1 | 57 % |
| P8 | 177.000 | 6.0 | 63 % |
| P9 | 224.000 | 3.7 | 67 % |

### Herstellung der OLEDs

### 1) Vakuum-prozessierte Devices:

Die Herstellung von erfindungsgemäßen OLEDs sowie OLEDs nach dem Stand der Technik erfolgt nach einem allgemeinen Verfahren gemäß WO 2004/058911, das auf die hier beschriebenen Gegebenheiten (Schichtdickenvariation, verwendete Materialien) angepasst wird.

In den folgenden Beispielen werden die Ergebnisse verschiedener OLEDs vorgestellt. Glasplättchen, mit strukturiertem ITO (Indium Zinn Oxid) bilden die Substrate, auf welche die OLEDs aufgebracht werden. Die OLEDs haben prinzipiell folgenden Schichtaufbau: Substrat / Lochtransportschicht 1 (HTL1) bestehend aus HTM dotiert mit 3 % NDP-9 (kommerziell erhältlich von der Fa. Novaled), 20 nm / Lochtransportschicht 2 (HTL2) / Elektronenblockerschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Zunächst werden vakuumprozessierte OLEDs beschrieben. Hierfür werden alle Materialien in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie M3:M2:Ir(L1)₃ (55%:35%:10%) bedeutet hierbei, dass das Material M3 in einem Volumenanteil von 55%, M2 in einem Anteil von 35% und Ir(L1)₃ in einem Anteil von 10% in der Schicht vorliegt. Analog kann auch die Elektronentransportschicht aus einer Mischung zweier Materialien bestehen. Der genaue Aufbau der OLEDs ist Tabelle 1 zu entnehmen. Die zur Herstellung der OLEDs verwendeten Materialien sind in Tabelle 6 gezeigt.

Die OLEDs werden standardmäßig charakterisiert. Hierfür werden die Elektrolumineszenzspektren, die Stromeffizienz (gemessen in cd/A) und die Spannung (gemessen bei 1000 cd/m² in V) bestimmt aus Strom-Spannungs-Helligkeits-Kennlinien (IUL-Kennlinien). Für ausgewählte Versuche wird die Lebensdauer bestimmt. Als Lebensdauer wird die Zeit definiert, nach der die Leuchtdichte von einer bestimmten Startleuchtdichte aus auf einen gewissen Anteil abgesunken ist. Die Angabe LD50 bedeutet, dass es sich bei der genannten Lebensdauer um die Zeit handelt, bei der die Leuchtdichte auf 50% der Startleuchtdichte abgefallen ist, also von z.B. 1000 cd/m² auf 500 cd/m². Je nach Emissionsfarbe wurden unterschiedliche Starthelligkeiten gewählt. Die Werte für die Lebensdauer können mit Hilfe dem Fachmann bekannten Umrechnungsformeln auf eine Angabe für andere Startleuchtdichten umgerechnet werden. Hierbei ist die Lebensdauer für eine Startleuchtdichte von 1000 cd/m² eine übliche Angabe.

### Verwendung von erfindungsgemäßen Verbindungen als Emittermaterialien in phosphoreszierenden OLEDs

Die erfindungsgemäßen Verbindungen lassen sich unter anderem als phosphoreszierende Emittermaterialien in der Emissionsschicht in OLEDs einsetzen. Als Vergleich gemäß dem Stand der Technik werden die Verbindungen Ir(Ref)₃ verwendet. Die Ergebnisse der OLEDs sind in Tabelle 2 zusammengefasst.

**Tabelle 1: Aufbau der OLED**

| **Bsp.** | **HTL2** | **EBL** | **EML** | **HBL** | **ETL** |
|---|---|---|---|---|---|
| | **Dicke** | **Dicke** | **Dicke** | **Dicke** | **Dicke** |
| D-Ir(Ref1)₃ | | | M1 :M4:Ir(Ref1)₃ | | ETM1:ETM2 |
| | HTM | EBM | (65%:30%:5%) | HBM | (50%:50%) |
| | 180 nm | 20 nm | 25 nm | 10 nm | 20 nm |
| D-Ir(Ref2)₃ | | | M1:M3:Ir(Ref2)₃ | | ETM1:ETM2 |
| | HTM | EBM | (65%:30%:5%) | HBM | (50%:50%) |
| | 180 nm | 20 nm | 25 nm | 10 nm | 20 nm |
| D-Ir(Ref3)₃ | | | M1:M4:Ir(Ref3)₃ | | ETM1:ETM2 |
| | HTM | EBM | (65%:30%:5%) | HBM | (50%:50%) |
| | 180 nm | 20 nm | 25 nm | 10 nm | 20 nm |
| D-Ir(Ref4)₃ | | | M1 :M4:Ir(Ref4)₃ | | ETM1:ETM2 |
| | HTM | EBM | (65%:30%:5%) | HBM | (50%:50%) |
| | 180 nm | 20 nm | 25 nm | 10 nm | 20 nm |
| D-Ir(Ref5)₃ | | | M1:M4:Ir(Ref5)₃ | | ETM1:ETM2 |
| | HTM | EBM | (65%:30%:5%) | HBM | (50%:50%) |
| | 180 nm | 20 nm | 25 nm | 10 nm | 20 nm |
| D-Ir(Ref6)₃ | | | M1:M4:Ir(Ref6)₃ | | ETM1:ETM2 |
| | HTM | EBM | (65%:30%:5%) | HBM | (50%:50%) |
| | 180 nm | 20 nm | 25 nm | 10 nm | 20 nm |
| D-Ir(Ref7)₃ | | | M1:M4:Ir(Ref7)₃ | | ETM1:ETM2 |
| | HTM | EBM | (65%:30%:5%) | HBM | (50%:50%) |
| | 180 nm | 20 nm | 25 nm | 10 nm | 20 nm |
| D-Ir(Ref8)₃ | | | M1 :M4:Ir(Ref8)₃ | | ETM1:ETM2 |
| | HTM | EBM | (65%:30%:5%) | HBM | (50%:50%) |
| | 180 nm | 20 nm | 25 nm | 10 nm | 20 nm |
| D-Ir(Ref9)₃ | | | M1 :M4:Ir(Ref9)₃ | | ETM1:ETM2 |
| | HTM | EBM | (65%:30%:5%) | HBM | (50%:50%) |
| | 180 nm | 20 nm | 25 nm | 10 nm | 20 nm |
| D-Ir(Ref10)₃ | | | M1:M4:Ir(Ref10)₃ | | ETM1:ETM2 |
| | HTM | EBM | (65%:30%:5%) | HBM | (50%:50%) |
| | 180 nm | 20 nm | 25 nm | 10 nm | 20 nm |
| D-Ir(L1)₃ | | | M1:M4:Ir(L1)₃ | | ETM1:ETM2 |
| | HTM | EBM | (65%:30%:5%) | HBM | (50%:50%) |
| | 180 nm | 20 nm | 25 nm | 10 nm | 20 nm |
| D-Ir(L96)₃ | | | M1:M4:Ir(L96)₃ | | ETM1:ETM2 |
| | HTM | EBM | (65%:30%:5%) | HBM | (50%:50%) |
| | 180 nm | 20 nm | 25 nm | 10 nm | 20 nm |
| D-Ir(L96)₃-2 | | | M2:M3:Ir(L96)₃ | | ETM1:ETM2 |
| | HTM | EBM | (65%:30%:5%) | HBM | (50%:50%) |
| | 180 nm | 20 nm | 25 nm | 10 nm | 20 nm |
| D-Ir(L167)₃ | | | M1:M4:Ir(L96)₃ | | ETM1:ETM2 |
| | HTM | EBM | (65%:30%:5%) | HBM | (50%:50%) |
| | 180 nm | 20 nm | 25 nm | 10 nm | 20 nm |
| D-Ir(192)₃ | | | M1:M4:Ir(L192)₃ | | ETM1:ETM2 |
| | HTM | EBM | (65%:30%:5%) | HBM | (50%:50%) |
| | 180 nm | | 25 nm | 10 nm | 20 nm |
| D-Ir(L215)₃ | | | M1:M4:Ir(215)₃ | | ETM1:ETM2 |
| | HTM | EBM | (65%:30%:5%) | HBM | (50%:50%) |
| | 180 nm | 20 nm | 25 nm | 10 nm | 20 nm |
| D-Ir(L226)₃ | | | M1:M4:Ir(L226)₃ | | ETM1:ETM2 |
| | HTM | EBM | (65%:30%:5%) | HBM | (50%:50%) |
| | 180 nm | 20 nm | 25 nm | 10 nm | 20 nm |
| D-Ir(L227)₃ | | | M1:M4:Ir(L227)₃ | | ETM1:ETM2 |
| | HTM | EBM | (65%:30%:5%) | HBM | (50%:50%) |
| | 180 nm | | 25 nm | 10 nm | 20 nm |
| D-Ir(L230)₃ | | | M1:M4:Ir(L230)₃ | | ETM1:ETM2 |
| | HTM | EBM | (65%:30%:5%) | HBM | (50%:50%) |
| | 180 nm | 20 nm | 25 nm | 10 nm | 20 nm |
| D-Ir(L231)₃ | | | M1 :M4:Ir(L231)₃ | | ETM1:ETM2 |
| | HTM | EBM | (65%:30%:5%) | HBM | (50%:50%) |
| | 180 nm | 20 nm | 25 nm | 10 nm | 20 nm |
| D-Ir(L234)₃ | | | M1:M4:Ir(L234)₃ | | ETM1:ETM2 |
| | HTM | EBM | (65%:30%:5%) | 10nm | (50%:50%) |
| | 180 nm | 20 nm | 25 nm | HBM | 20 nm |
| D-Ir(L243)₃ | | | M1:M4:Ir(L243)₃ | | ETM1:ETM2 |
| | HTM | EBM | (65%:30%:5%) | HBM | (50%:50%) |
| | 180 nm | 20 nm | 25 nm | 10 nm | 20 nm |
| D-Ir(L248)₃ | | | M1:M4:Ir(L248)₃ | | ETM1:ETM2 |
| | HTM | EBM | (65%:30%:5%) | HBM | (50%:50%) |
| | 180 nm | 20nm | 25 nm | 10 nm | 20 nm |
| D-Ir(L252)₃ | | | M1:M4:Ir(L252)₃ | | ETM1:ETM2 |
| | HTM | EBM | (65%:30%:5%) | HBM | (50%:50%) |
| | 180 nm | 20 nm | 25 nm | 10 nm | 20 nm |
| D-Ir(L259)₃ | | | M1:M4:Ir(L259)₃ | | ETM1:ETM2 |
| | HTM | EBM | (65%:30%:5%) | HBM | (50%:50%) |
| | 180 nm | 20 nm | 25 nm | 10 nm | 20 nm |
| D-Ir(L260)₃ | | | M1:M4:Ir(L260)₃ | | ETM1:ETM2 |
| | HTM | EBM | (65%:30%:5%) | HBM | (50%:50%) |
| | 180 nm | 20nm | 25 nm | 10 nm | 20 nm |
| D-Ir(L263)₃ | | | M1:M4:Ir(L263)₃ | | ETM1:ETM2 |
| | HTM | EBM | (65%:30%:5%) | HBM | (50%:50%) |
| | 180 nm | 20 nm | 25 nm | 10 nm | 20 nm |
| D-Ir(L264)₃ | | | M1:M4:Ir(L264)₃ | | ETM1:ETM2 |
| | HTM | EBM | (65%:30%:5%) | HBM | (50%:50%) |
| | 180 nm | 20 nm | 25 nm | 10 nm | 20 nm |
| D-Ir(L267)₃ | | | M1:M4:Ir(L267)₃ | | ETM1:ETM2 |
| | HTM | EBM | (65%:30%:5%) | HBM | (50%:50%) |
| | 180 nm | 20nm | 25 nm | 10 nm | 20 nm |
| D-Ir(L268)₃ | | | M1:M4:Ir(L268)₃ | | ETM1:ETM2 |
| | HTM | EBM | (65%:30%:5%) | HBM | (50%:50%) |
| | 180 nm | 20 nm | 25 nm | 10 nm | 20 nm |
| D-Ir(L271)₃ | | | M1 :M4:Ir(L271)₃ | | ETM1:ETM2 |
| | HTM | EBM | (65%:30%:5%) | HBM | (50%:50%) |
| | 180 nm | 20 nm | 25 nm | 10 nm | 20 nm |
| D-Ir(L279)₃ | | | M1:M4:Ir(L279)₃ | | ETM1:ETM2 |
| | HTM | EBM | (65%:30%:5%) | HBM | (50%:50%) |
| | 180 nm | 20 nm | 25 nm | 10nm | 20 nm |
| D-Ir(L284) | | | M1:M4:Ir(L284) | | ETM1:ETM2 |
| | HTM | EBM | (65%:30%:5%) | HBM | (50%:50%) |
| | 180 nm | 20 nm | 25 nm | 10 nm | 20 nm |
| D-Ir(L3)₂(CL1) | | | M1:M4:Ir(L3)₂(CL 1) | | ETM1:ETM2 |
| | HTM | EBM | (65%:30%:5%) | HBM | (50%:50%) |
| | 180 nm | 20nm | 25 nm | 10 nm | 20 nm |
| D-Ir(L158)₂(CL8) | | | M1:M4:Ir(L158)₂(CL8) | | ETM1:ETM2 |
| | HTM | EBM | (65%:30%:5%) | HBM | (50%:50%) |
| | 180 nm | 20 nm | 25 nm | 10 nm | 20 nm |
| D-Ir(L113)₂(L109) | | | M1:M4:Ir(L113)₂(L109) | | ETM1:ETM2 |
| | HTM | EBM | (65%:30%:5%) | HBM | (50%:50%) |
| | 180 nm | 20 nm | 25 nm | 10 nm | 20 nm |
| Pt(L3)(CL1) | | | M1:M4:Pt(L3)(CL1) | | ETM1:ETM2 |
| | HTM | EBM | (55%:40%:5%) | HBM | (50%:50%) |
| | 180 nm | 20 nm | 25 nm | 10 nm | 20 nm |
| Pt(L283) | | | M1:M4:Pt(L283) | | ETM1:ETM2 |
| | HTM | EBM | (60%:35%:5%) | HBM | (50%:50%) |
| | 230 nm | 20 nm | 25 nm | 10 nm | 20 nm |

**Tabelle 2: Ergebnisse der Vakuum-prozessierten OLEDs**

| **Bsp.** | **EQE (%) 1000 cd/m²** | **Spannung (V) 1000 cd/m²** | **CIE x/y 1000 cd/m²** | **LD50 (h) 1000 cd/m²** |
|---|---|---|---|---|
| D-Ir(Ref1)₃ | 10.0 | 4.1 | 0.16/0.48 | 1700 |
| D-Ir(Ref2)₃ | 13.2 | 4.7 | 0.15/0.31 | 600 |
| D-Ir(Ref3)₃ | 11.8 | 4.2 | 0.16/0.33 | 1100 |
| D-Ir(Ref4)₃ | 10.3 | 4.4 | 0.15/0.24 | 600 |
| D-Ir(Ref5)₃ | 4.6 | 4.3 | 0.16/0.29 | --- |
| D-Ir(Ref6)₃ | 4.7 | 4.5 | 0.15/0.26 | --- |
| D-Ir(Ref7)₃ | 9.0 | 4.7 | 0.15/0.22 | 200 |
| D-Ir(Ref8)₃ | 6.3 | 4.7 | 0.15/0.17 | --- |
| D-Ir(Ref9)₃ | 10.8 | 4.5 | 0.18/0.34 | 150 |
| D-Ir(Ref10)₃ | 7.9 | 4.3 | 0.25/0.61 | --- |
| D-Ir(L1)₃ | 22.8 | 4.0 | 0.15/0.39 | 1400 |
| D-Ir(L96)₃ | 23.0 | 4.0 | 0.14/0.34 | 1200 |
| D-Ir(L96)₃ | 21.7 | 4.8 | 0.14/0.34 | 1000 |
| D-Ir(L167)₃ | 21.3 | 4.1 | 0.15/0.35 | 1600 |
| D-Ir(L192)₃ | 20.8 | 4.5 | 0.15/0.25 | 900 |
| D-Ir(L215)₃ | 23.7 | 4.1 | 0.22/0.71 | --- |
| D-Ir(L226)₃ | 21.5 | 4.2 | 0.25/0.69 | 36000 |
| D-Ir(L227)₃ | 21.9 | 3.9 | 0.29/0.67 | --- |
| D-Ir(L230)₃ | 9.7 | 4.3 | 0.14/0.30 | --- |
| D-Ir(L231)₃ | 13.8 | 4.5 | 0.15/0.40 | --- |
| D-Ir(L234)₃ | 19.9 | 4.5 | 0.14/0.28 | 600 |
| D-Ir(L243)₃ | 18.7 | 4.6 | 0.14/0.24 | 350 |
| D-Ir(L248)₃ | 19.2 | 4.6 | 0.14/0.21 | 300 |
| D-Ir(L252)₃ | 25.2 | 4.3 | 0.23/0.70 | --- |
| D-Ir(L259)₃ | 22.9 | 4.2 | 0.23/0.69 | --- |
| D-Ir(L260)₃ | 23.2 | 4.2 | 0.23/0.70 | --- |
| D-Ir(L263)₃ | 18.7 | 4.3 | 0.15/0.26 | 600 |
| D-Ir(L264)₃ | 18.4 | 4.3 | 0.14/0.34 | 1400 |
| D-Ir(L267)₃ | 17.3 | 4.6 | 0.15/0.33 | --- |
| D-Ir(L268)₃ | 17.7 | 4.6 | 0.15/0.26 | --- |
| D-Ir(L271)₃ | 19.8 | 4.8 | 0.14/0.22 | --- |
| D-Ir(L279)₃ | 16.7 | 4.3 | 0.15/0.22 | --- |
| D-Ir(L284) | 17.0 | 4.4 | 0,15/0.24 | --- |
| D-Ir(L3)₂(CL1) | 21.8 | 4.0 | 0.15/0.38 | --- |
| D-Ir(L158)₂(CL8) | 23.1 | 4.3 | 0.15/0.25 | 1200 |
| D-Ir(L113)₂(L109) | 23.9 | 4.0 | 0.14/0.33 | 1300 |
| D-Ir(I500)₃ | 20.1 | 4.4 | 0.16/0.36 | 500 |
| D-Ir(I511)₃ | 22.7 | 4.1 | 0.24/0.65 | 41000 |
| Pt(L3)(CL1) | 17.4 | 4.6 | 0.16/0.38 | --- |
| Pt(L283) | 21.4 | 4.5 | 0.26/0.63 | --- |

### 2) Lösungs-prozessierte Devices:

### A: Aus löslichen Funktionsmaterialien

Die erfindungsgemäßen Iridium-Komplexe können auch aus Lösung verarbeitet werden und führen dort zu prozesstechnisch wesentlich einfacheren OLEDs, im Vergleich zu den vakuumprozessierten OLEDs, mit dennoch guten Eigenschaften. Die Herstellung solcher Bauteile lehnt sich an die Herstellung polymerer Leuchtdioden (PLEDs) an, die in der Literatur bereits vielfach beschrieben ist (z.B. in der WO 2004/037887).
Der Aufbau setzt sich aus Substrat / ITO / PEDOT (80 nm) / Interlayer (80 nm) / Emissionsschicht (80 nm) / Kathode zusammen. Dazu werden Substrate der Firma Technoprint (Sodalimeglas) verwendet, auf welche die ITO-Struktur (Indium-Zinn-Oxid, eine transparente, leitfähige Anode) aufgebracht wird. Die Substrate werden im Reinraum mit DI Wasser und einem Detergens (Deconex 15 PF) gereinigt und dann durch eine UV/Ozon-Plasmabehandlung aktiviert. Danach wird ebenfalls im Reinraum als Pufferschicht eine 80 nm Schicht PEDOT (PEDOT ist ein Polythiophen-Derivat (Baytron P VAI 4083sp.) von H. C. Starck, Goslar, das als wässrige Dispersion geliefert wird) durch Spin-Coating aufgebracht. Die benötigte Spinrate hängt vom Verdünnungsgrad und der spezifischen Spin-Coater-Geometrie ab (typisch für 80 nm: 4500 rpm). Um Restwasser aus der Schicht zu entfernen, werden die Substrate für 10 Minuten bei 180 °C auf einer Heizplatte ausgeheizt. Die verwendete Interlayer dient der Lochinjektion, in diesem Fall wird HIL-012 von Merck verwendet. Die Interlayer kann alternativ auch durch eine oder mehrere Schichten ersetzt werden, die lediglich die Bedingung erfüllen müssen, durch den nachgelagerten Prozessierungsschritt der EML-Abscheidung aus Lösung nicht wieder abgelöst zu werden. Zur Herstellung der Emissionsschicht werden die erfindungsgemäßen Emitter zusammen mit den Matrixmaterialien in Toluol gelöst. Der typische Feststoffgehalt solcher Lösungen liegt zwischen 16 und 25 g/L, wenn, wie hier, die für eine Device typische Schichtdicke von 80 nm mittels Spincoating erzielt werden soll. Die lösungsprozessierten Devices enthalten eine Emissionsschicht aus (Polystyrol):M5:M6:Ir(L)₃ (25%:25%:40%:10%). Die Emissionsschicht wird in einer Inertgasatmosphäre, im vorliegenden Fall Argon, aufgeschleudert und 30 min bei 130 °C ausgeheizt. Zuletzt wird eine Katho de aus Barium (5 nm) und dann Aluminium (100 nm) (hochreine Metalle von Aldrich, besonders Barium 99.99 % (Best-Nr. 474711); Aufdampfanlagen von Lesker o.a., typischer Aufdampfdruck 5 x 10⁻⁶ mbar) aufgedampft. Optional kann zunächst eine Lockblockierschicht und dann eine Eletronentransportschicht und dann erst die Kathode (z.B. Al oder LiF/Al) im Vakuum aufgedampft werden. Um das Device vor Luft und Luftfeuchtigkeit zu schützen, wird die Vorrichtung abschließend verkapselt und dann charakterisiert. Die genannten OLED-Beispiele sind noch nicht optimiert, Tabelle 3 fasst die erhaltenen Daten zusammen.

### B: Aus Polymeren Ir-Komplexen

Die Herstellung einer polymeren organischen Leuchtdiode (PLED) ist in der Literatur bereits vielfach beschrieben (z. B. WO 2004/037887).

Die Substrate werden - wie unter A: Aus löslichen Funktionsmaterialien - beschrieben vorbereitet, danach werden unter Inertgasatmosphäre (Stickstoff bzw. Argon) zunächst 20 nm einer Interlayer (typischerweise ein lochdominiertes Polymer, hier HIL-012 von Merck) und dann 65 nm der Polymerschichten aus Toluollösung (Konzentration Interlayer 5 g/l) aufgebracht. Beide Schichten werden bei 180 °C mindeste ns 10 Minuten ausgeheizt. Danach wird die Kathode aus Barium (5 nm) und dann Aluminium (100 nm) aufgedampft. Um das Device vor Luft und Luftfeuchtigkeit zu schützen, wird die Vorrichtung abschließend verkapselt und dann charakterisiert. Die genannten OLED-Beispiele sind noch nicht optimiert, Tabelle 3 fasst die erhaltenen Daten zusammen.

**Tabelle 3: Ergebnisse mit aus Lösung prozessierten Materialien**

| **Bsp.** | **Ir(L)₃ oder Ir-Polymer** | **EQE (%) 1000 cd/m²** | **Spannung (V) 1000 cd/m²** | **CIE x/y 1000 cd/m²** |
|---|---|---|---|---|
| D-Ir(L276)₃ | Ir(L276)₃ | 22.6 | 4.4 | 0.16/0.40 |
| D-Ir(L281)₃ | Ir(L281)₃ | 23.2 | 4.5 | 0.15/0.36 |
| D-Ir(L285)₃ | Ir(L285)₃ | 23.0 | 4.4 | 0.15/0.35 |
| D-Ir(L287)₃ | Ir(L287)₃ | 20.5 | 4.3 | 0.22/0.67 |
| D-P1 | P1 | 22.8 | 4.8 | 0.15/0.37 |
| D-P5 | P5 | 22.5 | 4.5 | 0.15/0.36 |
| D-P6 | P6 | 22.4 | 4.5 | 0.16/0.40 |
| D-P7 | P7 | 22.8 | 4.4 | 0.15/0.36 |
| D-P9 | P9 | 21.2 | 4.6 | 0.15/0.36 |

### 3) Weiß emittierende OLEDs

Gemäß den allgemeinen Verfahren aus 1) wird eine weiß emittierende OLED mit folgendem Schichtaufbau hergestellt:

**Tabelle 4: Aufbau der weißen OLEDs**

| **Bsp.** | **HTL2** | **EML** | **EML** | **EML** | **HBL** | **ETL** |
|---|---|---|---|---|---|---|
| | | **Rot** | **Blau** | **Grün** | | |
| | **Dicke** | **Dicke** | **Dicke** | **Dicke** | **Dicke** | **Dicke** |
| D-W1 | HTM | EBM:Ir-R | M1:M3:Ir(L109)₃ | M3:Ir-G | M3 | ETM1:ETM2 |
| | | (97%:3%) | (45%:50%:5%) | (90%:10%) | | (50%:50%) |
| | 230 nm | 9 nm | 8 nm | 7 nm | 10 nm | 30 nm |

**Tabelle 5: Deviceergebnisse**

| **Bsp.** | **EQE (%)** | **Spannung (V) 1000 cd/m²** | **CIE x/y 1000 cd/m²** | **LD50 (h)** |
|---|---|---|---|---|
| | **1000 cd/m²** | | **CRI** | **1000 cd/m²** |
| D-W1 | 23.0 | 6.3 | 0.45/0.44 | 3000 |
| | | | 80 | |

**Tabelle 6: Strukturformeln der verwendeten Materialien**

| | |
|---|---|
| 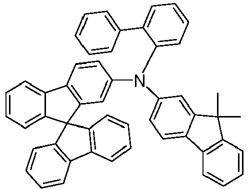 | 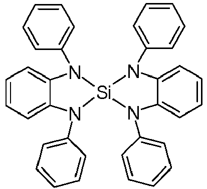 |
| HTM | EBM |
| 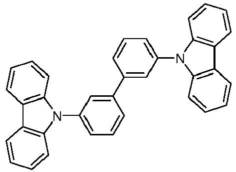 | 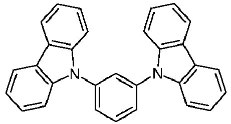 |
| M1 | M2 |
| 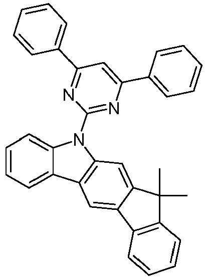 | 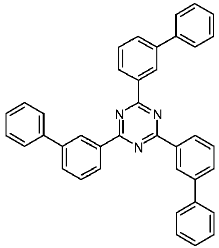 |
| M3 | M4 = HBM |
| 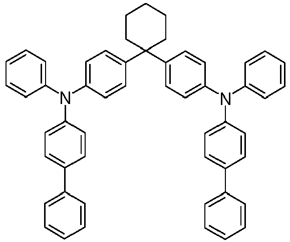 | 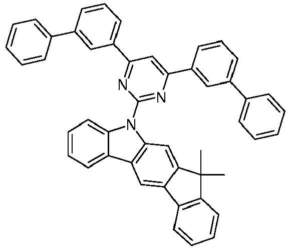 |
| M5 | M6 |
| 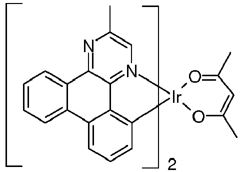 | 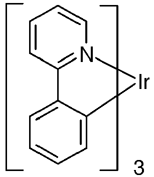 |
| Ir-R | Ir-G |
| 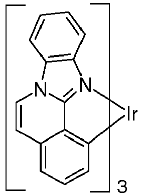 | 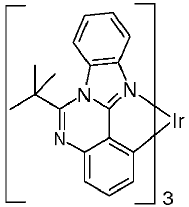 |
| WO2010086089 | WO2011157339 |
| Ir(Ref-1)₃ | Ir(Ref-2)₃ |
| 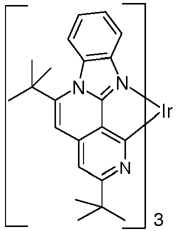 | 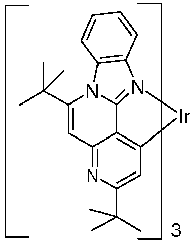 |
| WO2011157339 | WO2011157339 |
| Ir(Ref-3)₃ | Ir(Ref-4)₃ |
| 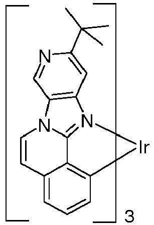 | 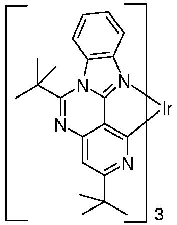 |
| WO2011157339 | WO2011157339 |
| Ir(Ref-5)₃ | Ir(Ref-6)₃ |
| 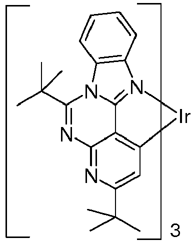 | 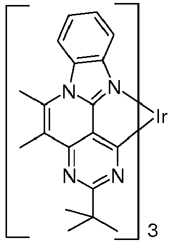 |
| WO2011157339 | WO2011157339 |
| Ir(Ref-7)₃ | Ir(Ref-8)₃ |
| 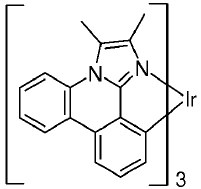 | 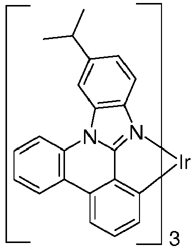 |
| WO2008/156879 | WO2008/156879 |
| Ir(Ref-9)₃ | Ir(Ref-10)₃ |
| 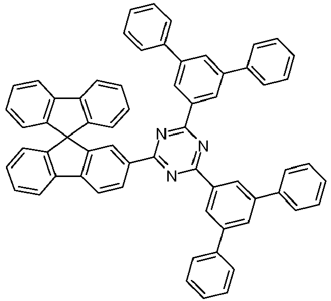 | 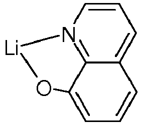 |
| ETM1 | ETM2 |

## Patentansprüche

1. Verbindung gemäß Formel (1),
M(L)ₙ(L')ₘ Formel (1)
enthaltend eine Teilstruktur M(L)ₙ der Formel (2), Formel (3) oder Formel (4): 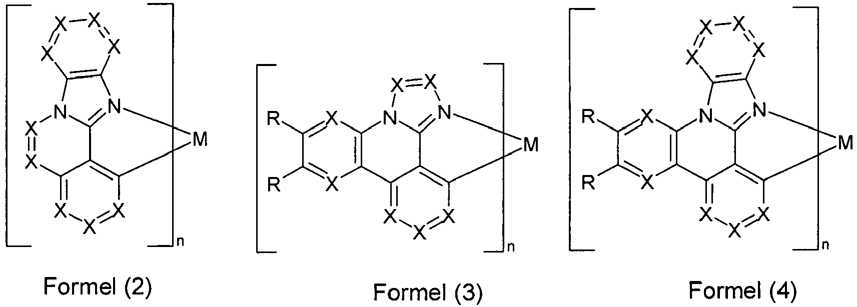 wobei für die verwendeten Symbole und Indizes gilt:
M ist Iridium oder Platin;
X ist bei jedem Auftreten gleich oder verschieden ausgewählt aus der Gruppe bestehend aus CR und N;
R ist bei jedem Auftreten gleich oder verschieden H, D, F, Cl, Br, I, N(R¹)₂, CN, NO₂, OH, COOH, C(=O)N(R¹)₂, Si(R¹)₃, B(OR¹)₂, C(=O)R¹, P(=O)(R¹)₂, S(=O)R¹, S(=O)₂R¹, OSO₂R¹, eine geradkettige Alkyl-, Alkoxy- oder Thioalkoxygruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkoxygruppe mit 3 bis 20 C-Atomen, die jeweils mit einem oder mehreren Resten R¹ substituiert sein kann, wobei eine oder mehrere nicht benachbarte CH₂-Gruppen durch R¹C=CR¹, C≡C, Si(R¹)₂, C=O, NR¹, O, S oder CONR¹ ersetzt sein können und wobei ein oder mehrere H-Atome durch D, F, Cl, Br, I oder CN ersetzt sein können, oder ein aromatisches oder heteroaromatisches Ringsystem mit 5 bis 60 aromatischen Ringatomen, das jeweils durch einen oder mehrere Reste R¹ substituiert sein kann, oder eine Aryloxy- oder Heteroaryloxygruppe mit 5 bis 40 aromatischen Ringatomen, die durch einen oder mehrere Reste R¹ substituiert sein kann, oder eine Aralkyl- oder Heteroaralkylgruppe mit 5 bis 40 aromatischen Ringatomen, die durch einen oder mehrere Reste R¹ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R¹ substituiert sein kann; dabei können zwei benachbarte Reste R auch miteinander ein mono- oder polycyclisches, aliphatisches, aromatisches oder heteroaromatisches Ringsystem bilden;
R¹ ist bei jedem Auftreten gleich oder verschieden H, D, F, Cl, Br, I, N(R²)₂, CN, NO₂, Si(R²)₃, B(OR²)₂, C(=O)R², P(=O)(R²)₂, S(=O)R², S(=O)₂R², OSO₂R², eine geradkettige Alkyl-, Alkoxy- oder Thioalkoxygruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkoxygruppe mit 3 bis 20 C-Atomen, die jeweils mit einem oder mehreren Resten R² substituiert sein kann, wobei eine oder mehrere nicht benachbarte CH₂-Gruppen durch R²C=CR², C≡C, Si(R²)₂, C=O, NR², O, S oder CONR² ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 40 aromatischen Ringatomen, die durch einen oder mehrere Reste R² substituiert sein kann, oder eine Aralkyl- oder Heteroaralkylgruppe mit 5 bis 40 aromatischen Ringatomen, die durch einen oder mehrere Reste R² substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R² substituiert sein kann; dabei können zwei oder mehrere benachbarte Reste R¹ miteinander oder R¹ mit R ein mono- oder polycyclisches, aliphatisches, aromatisches oder heteroaromatisches Ringsystem bilden;
R² ist bei jedem Auftreten gleich oder verschieden H, D, F oder ein aliphatischer, aromatischer und/oder heteroaromatischer Kohlenwasserstoffrest mit 1 bis 20 C-Atomen, in dem auch ein oder mehrere H-Atome durch F ersetzt sein können; dabei können zwei oder mehrere Substituenten R² auch miteinander ein mono- oder polycyclisches, aliphatisches Ringsystem bilden;
L' ist gleich oder verschieden bei jedem Auftreten ein beliebiger Coligand;
n ist 1, 2 oder 3;
m ist 0, 1, 2, 3 oder 4;
dabei können auch mehrere Liganden L miteinander oder L mit L' über eine Einfachbindung oder eine bivalente oder trivalente Brücke verknüpft sein und so ein tridentates, tetradentates, pentadentates oder hexadentates Ligandensystem aufspannen;
dabei kann auch ein Substituent R zusätzlich an das Metall koordinieren;
**dadurch gekennzeichnet, dass** zwei benachbarte Gruppen X für CR stehen und die jeweiligen Reste R zusammen mit den C-Atomen einen Ring der folgenden Formel (5) oder Formel (6) aufspannen, 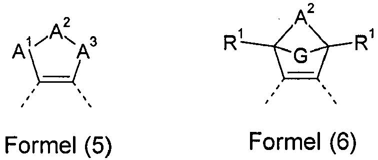 wobei R¹ und R² die oben genannten Bedeutungen aufweisen, die gestrichelten Bindungen die Verknüpfung der beiden Kohlenstoffatome im Liganden andeuten und weiterhin gilt:
A¹, A³ ist gleich oder verschieden bei jedem Auftreten C(R³)₂, O, S, NR³ oder C(=O);
A² ist C(R¹)₂, O, S, NR³ oder C(=O);
G ist eine Alkylengruppe mit 1, 2 oder 3 C-Atomen, welche mit einem oder mehreren Resten R² substituiert sein kann, -CR²=CR²- oder eine ortho-verknüpfte Arylen- oder Heteroarylengruppe mit 5 bis 14 aromatischen Ringatomen, welche durch einen oder mehrere Reste R² substituiert sein kann;
R³ ist gleich oder verschieden bei jedem Auftreten F, eine geradkettige Alkyl- oder Alkoxygruppe mit 1 bis 20 C-Atomen, eine verzweigte oder cyclische Alkyl- oder Alkoxygruppe mit 3 bis 20 C-Atomen, die jeweils mit einem oder mehreren Resten R² substituiert sein kann, wobei eine oder mehrere nicht benachbarte CH₂-Gruppen durch R²C=CR², C≡C, Si(R²)₂, C=O, NR², O, S oder CONR² ersetzt sein können und wobei ein oder mehrere H-Atome durch D oder F ersetzt sein können, oder ein aromatisches oder heteroaromatisches Ringsystem mit 5 bis 24 aromatischen Ringatomen, das jeweils durch einen oder mehrere Reste R² substituiert sein kann, oder eine Aryloxy- oder Heteroaryloxygruppe mit 5 bis 24 aromatischen Ringatomen, die durch einen oder mehrere Reste R² substituiert sein kann, oder eine Aralkyl- oder Heteroaralkylgruppe mit 5 bis 24 aromatischen Ringatomen, die durch einen oder mehrere Reste R² substituiert sein kann; dabei können zwei Reste R³, welche an dasselbe Kohlenstoffatom gebunden sind, miteinander ein aliphatisches oder aromatisches Ringsystem bilden und so ein Spirosystem aufspannen; weiterhin kann R³ mit einem benachbarten Rest R oder R¹ ein aliphatisches Ringsystem bilden;
mit der Maßgabe, dass in A¹-A²-A³ nicht zwei Heteroatome direkt aneinander gebunden sind.

2. Verbindung nach Anspruch 1, **dadurch gekennzeichnet, dass** die Teilstrukturen der Formel (2) ausgewählt sind aus den Formeln (2-A) bis (2-Q), dass die Teilstrukturen der Formel (3) ausgewählt sind aus den Formeln (3-A) bis (3-F) und dass die Teilstrukturen der Formel (4) ausgewählt sind aus den Formeln (4-A) bis (4-F), 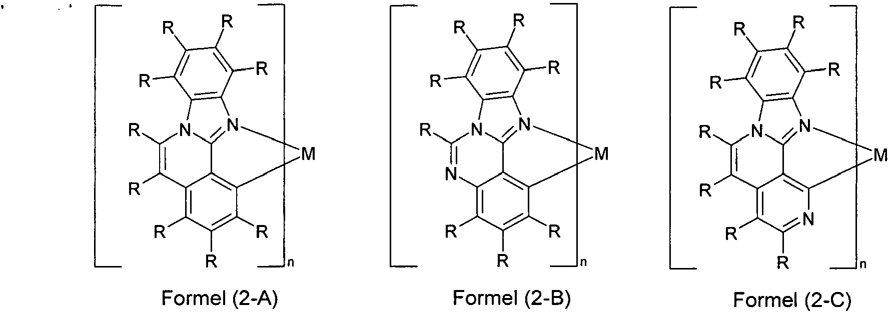 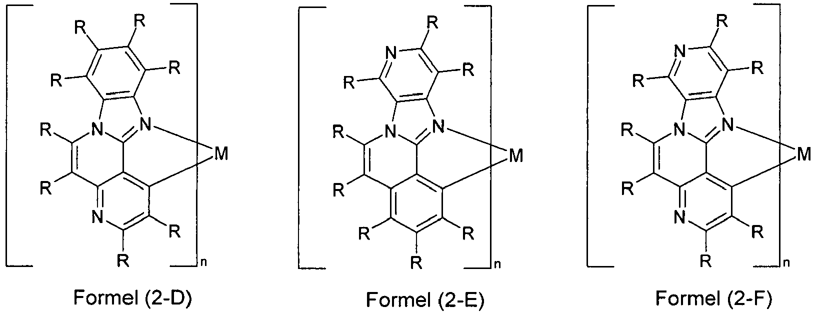 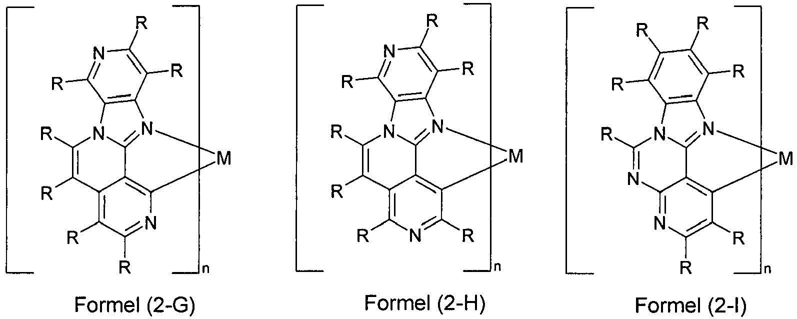 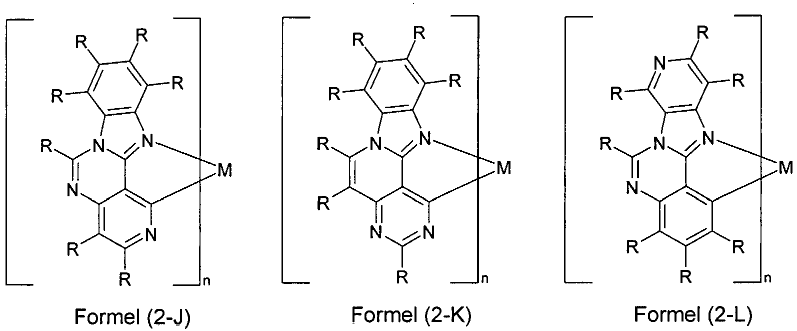 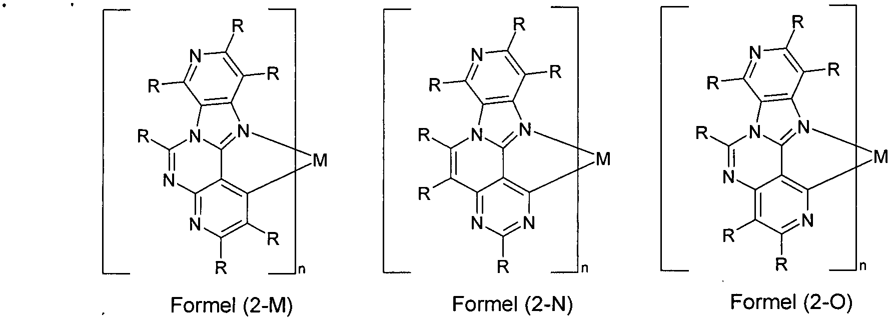 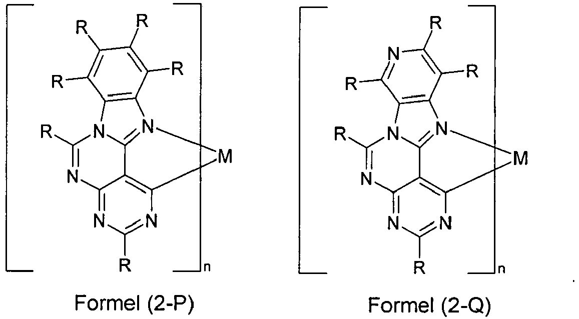 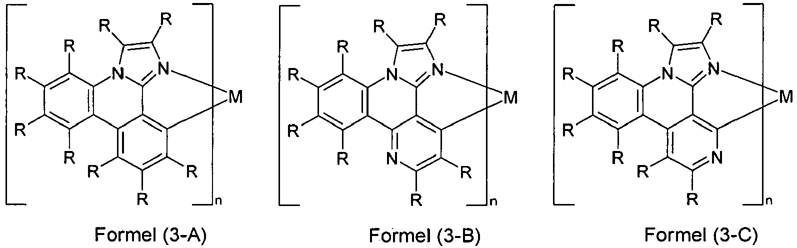 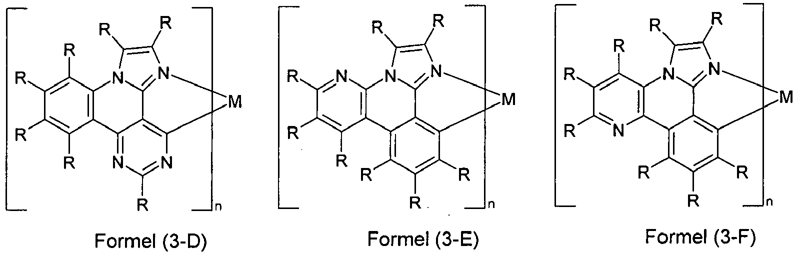 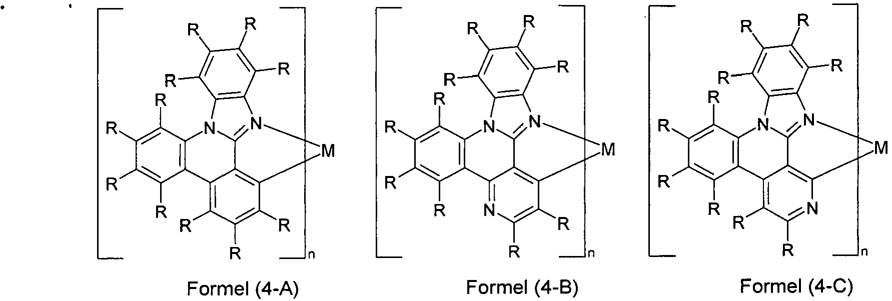 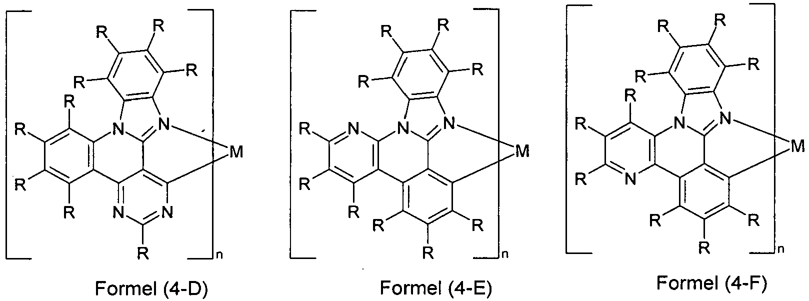 wobei die verwendeten Symbole und Indizes die in Anspruch 1 genannten Bedeutungen aufweisen.

3. Verbindung nach Anspruch 1 oder 2, **dadurch gekennzeichnet, dass** die Teilstrukturen der Formel (2) ausgewählt sind aus den Formeln (2-1) bis (2-5), die Teilstrukturen der Formel (3) ausgewählt sind aus den Formeln (3-1) bis (3-3) und die Teilstrukturen der Formel (4) ausgewählt sind aus den Formeln (4-1) bis (4-4), 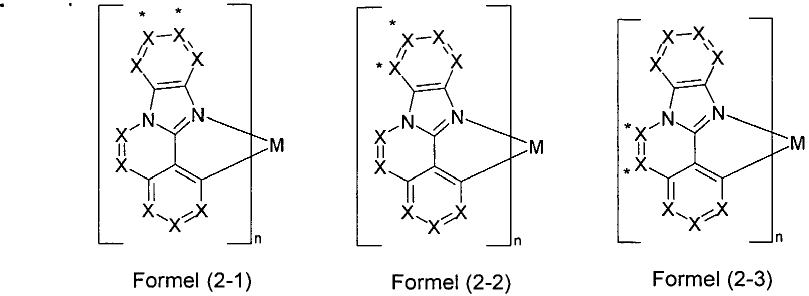 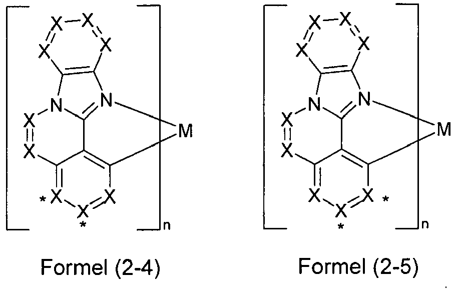 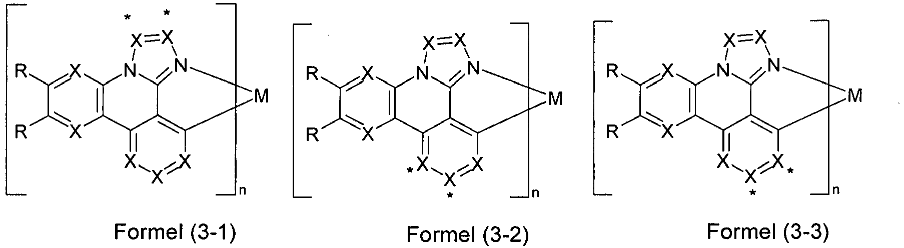 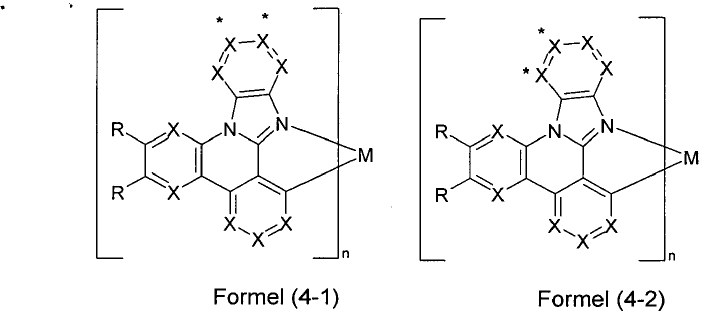 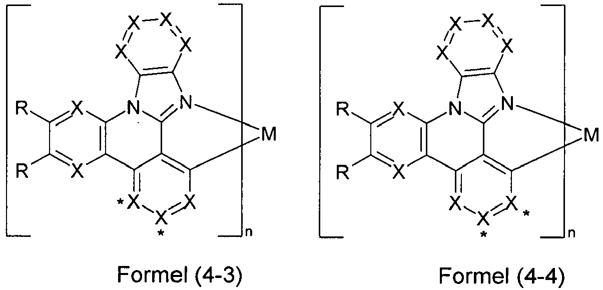 wobei die verwendeten Symbole und Indizes die in Anspruch 1 genannten Bedeutungen aufweisen und * jeweils die Positionen kennzeichnet, die für CR stehen, wobei die jeweiligen Reste R zusammen mit den C-Atomen, an die sie gebunden sind, einen Ring der oben genannten Formel (5) oder (6) aufspannen.

4. Verbindung nach einem oder mehreren der Ansprüche 1 bis 3, **dadurch gekennzeichnet, dass** die Struktur der Formel (5) ausgewählt ist aus den Formeln (5-A), (5-B), (5-C) und (5-D) und die Struktur der Formel (6) ausgewählt ist aus den Formeln (6-A) und (6-B), 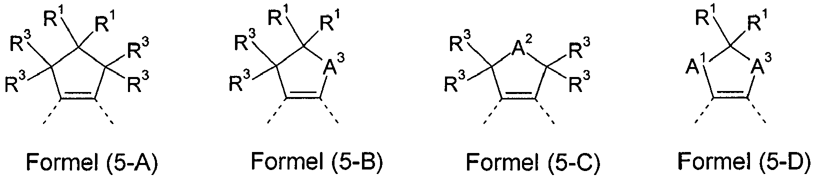 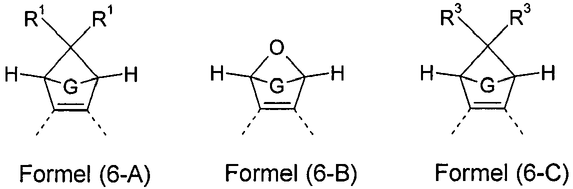 wobei R¹ und R³ die in Anspruch 1 genannten Bedeutungen aufweisen und A¹, A² und A³ gleich oder verschieden bei jedem Auftreten für O oder NR³ steht.

5. Verbindung nach einem oder mehreren der Ansprüche 1 bis 4, **dadurch gekennzeichnet, dass** die Gruppe G für eine Ethylengruppe, welche mit einem oder mehreren Resten R² substituiert sein kann, wobei R² bevorzugt gleich oder verschieden bei jedem Auftreten für H oder eine Alkylgruppe mit 1 bis 4 C-Atomen steht, oder für eine ortho-Arylengruppe mit 6 bis 10 C-Atomen, welche mit einem oder mehreren Resten R² substituiert sein kann, bevorzugt aber unsubstituiert ist, steht.

6. Verbindung nach einem oder mehreren der Ansprüche 1 bis 5, **dadurch gekennzeichnet, dass**, wenn eine oder mehrere Gruppen X für Stickstoff stehen, benachbart zu diesem Stickstoffatom eine Gruppe R als Substituent gebunden ist, welche ausgewählt ist aus der Gruppe bestehend aus CF₃, OCF₃, einer Alkyl- oder Alkoxygruppe mit 1 bis 10 C-Atomen, einem aromatischen oder heteroaromatischen Ringsystem oder einer Aralkyl- bzw. Heteroaralkylgruppe oder dass dieser Rest R mit einem benachbarten Rest R einen Cyclus der Formel (5) oder (6) bildet.

7. Verbindung nach einem oder mehreren der Ansprüche 1 bis 6, **dadurch gekennzeichnet, dass** der Substituent R, der in der ortho-Position zur Metallkoordination gebunden ist, eine Gruppe darstellt, die ebenfalls an das Metall M koordiniert, die ausgewählt ist aus Aryl- bzw. Heteroarylgruppen, Aryl- oder Alkylcyaniden, Aryl- oder Alkylisocyaniden, Aminen, Amiden, Alkoholen, Alkoholaten, Thioalkoholen, Thioalkoholaten, Phosphinen, Phosphiten, Carbonylfunktionen, Carboxylaten, Carbamiden oder Aryl- oder Alkylacetyliden.

8. Verbindung nach einem oder mehreren der Ansprüche 1 bis 7, ausgewählt aus den Strukturen der Formeln (13) bis (18), 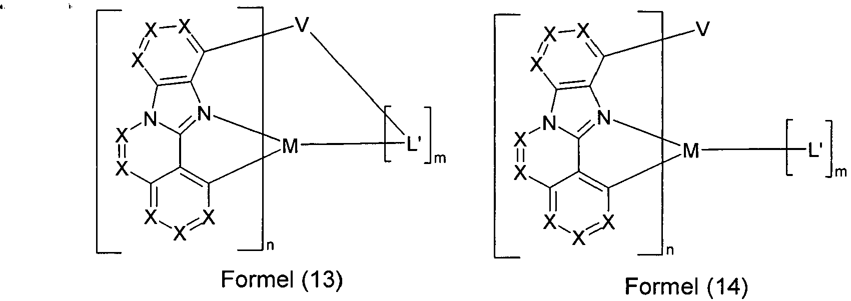 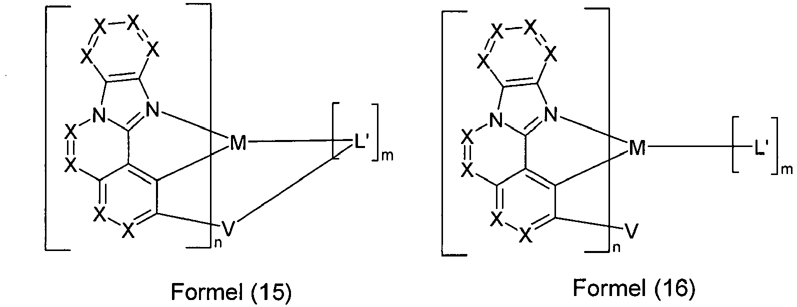 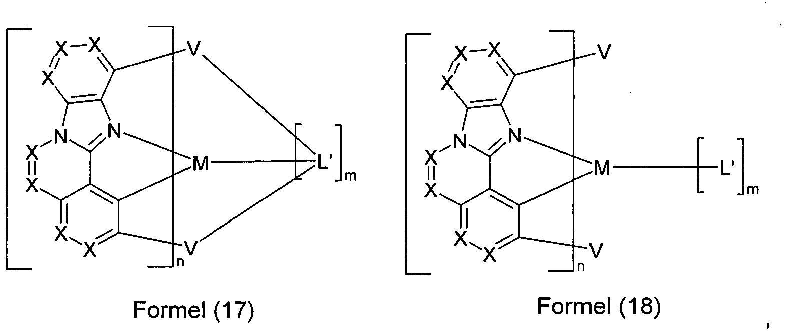 wobei die verwendeten Symbole und Indizes die in Anspruch 1 genannten Bedeutungen aufweisen und V eine Einfachbindung oder eine verbrückende Einheit darstell, enthaltend 1 bis 80 Atome aus der dritten, vierten, fünften und/oder sechsten Hauptgruppe (Gruppe 13, 14, 15 oder 16 gemäß IUPAC) oder einen 3- bis 6-gliedrigen Homo- oder Heterocyclus darstellt und die Teilliganden L miteinander oder L mit L' miteinander kovalent verbindet.

9. Verbindung nach einem oder mehreren der Ansprüche 1 bis 8, **dadurch gekennzeichnet, dass** L' ausgewählt ist aus der Gruppe bestehend aus Kohlenmonoxid, Stickstoffmonoxid, Alkylcyaniden, Arylcyaniden, Alkylisocyaniden, Arylisocyaniden, Aminen, Phosphinen, Phosphiten, Arsinen, Stibinen, stickstoffhaltigen Heterocyclen, Carbenen, Hydrid, Deuterid, den Halogeniden F⁻, Cl⁻, Br⁻ und I⁻, Alkylacetyliden, Arylacetyliden, Cyanid, Cyanat, Isocyanat, Thiocyanat, Isothiocyanat, aliphatischen oder aromatischen Alkoholaten, aliphatischen oder aromatischen Thioalkoholaten, Amiden, Carboxylaten, Arylgruppen, O²⁻, S²⁻, Carbide, welche zu einer Koordination der Form R-C=M führen, Nitrene, welche zu einer Koordination der Form R-N=M führen, wobei R allgemein für einen Substituenten steht, N³⁻, Diaminen, Iminen, Diiminen, Heterocyclen enthaltend zwei Stickstoffatome, Diphosphinen, 1,3-Diketonaten abgeleitet von 1,3-Diketonen, 3-Ketonaten abgeleitet von 3-Ketoestern, Carboxylaten abgeleitet von Aminocarbonsäuren, Salicyliminaten abgeleitet von Salicyliminen, Dialkoholaten, Dithiolaten, 3-(2-Pyridyl)-diazolen, 3-(2-Pyridyl)-triazolen, Boraten stickstoffhaltiger Heterocyclen und bidentaten monoanionischen, neutralen oder dianionischen Liganden L', welche mit dem Metall einen cyclometallierten Fünfring oder Sechsring mit mindestens einer Metall-Kohlenstoff-Bindung aufweisen.

10. Verfahren zur Herstellung einer Verbindung nach einem oder mehreren der Ansprüche 1 bis 9 durch Umsetzung des entsprechenden freien Liganden mit Metallalkoholaten der Formel (71), mit Metallketoketonaten der Formel (72), mit Metallhalogeniden der Formel (73), mit dimeren Metallkomplexen der Formel (74) oder mit Metallkomplexen der Formel (75), 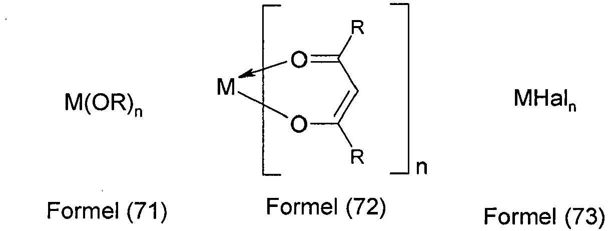 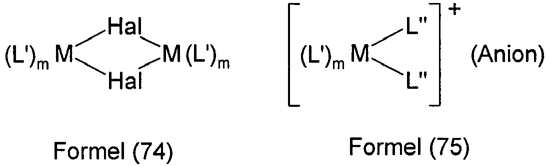 wobei die Symbole M, m, n und R die in Anspruch 1 angegebenen Bedeutungen haben, Hal = F, Cl, Br oder I ist, L" für einen Alkohol oder ein Nitril steht und (Anion) ein nicht-koordinierendes Anion ist, wobei auch Metallverbindungen eingesetzt werden können, die sowohl Alkoholat- und/oder Halogenid- und/oder Hydroxy- wie auch Ketoketonatreste tragen.

11. Polymer enthaltend eine oder mehrere der Verbindungen nach einem oder mehreren der Ansprüche 1 bis 9, wobei ein oder mehrere Bindungen der Verbindung zum Polymer vorhanden sind.

12. Formulierung, enthaltend mindestens eine Verbindung nach einem oder mehreren der Ansprüche 1 bis 9 oder ein Polymer nach Anspruch 11 und mindestens eine weitere Verbindung.

13. Verwendung einer Verbindung nach einem oder mehreren der Ansprüche 1 bis 9 oder eines Polymers nach Anspruch 11 in einer elektronischen Vorrichtung, insbesondere ausgewählt aus der Gruppe bestehend aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, organischen optischen Detektoren, organischen Photorezeptoren, organischen Feld-Quench-Devices, lichtemittierenden elektrochemischen Zellen oder organischen Laserdioden, oder Verwendung zur Erzeugung von Singulett-Sauerstoff oder in der Photokatalyse.

14. Elektronische Vorrichtung enthaltend eine oder mehrere Verbindungen nach einem oder mehreren der Ansprüche 1 bis 9 und/oder ein oder mehrere Polymere nach Anspruch 11.

15. Elektronische Vorrichtung nach Anspruch 14, wobei es sich um eine organische Elektrolumineszenzvorrichtung handelt, **dadurch gekennzeichnet, dass** die Verbindung nach einem oder mehreren der Ansprüche 1 bis 9 als emittierende Verbindung in einer oder mehreren emittierenden Schichten eingesetzt wird.

16. Elektronische Vorrichtung nach Anspruch 15, **dadurch gekennzeichnet, dass** als Matrixmaterial in mindestens einer der emittierenden Schichten ein Keton, Phosphinoxid, Sulfoxid, Sulfon, Triarylamin, Carbazolderivat, Indolocarbazolderivat, Indenocarbazolderivat, Azacarbazol, bipolares Matrixmaterial, Silan, Azaborol, Boronester, Diazasilolderivat, Diazaphospholderivat, Triazinderivat, Zinkkomplex, Dibenzofuranderivat oder verbrücktes Carbazolderivat oder eine Mischung aus zwei oder mehr dieser Materialien eingesetzt wird.

## Claims

1. Compound of the formula (1),
M(L)ₙ(L')ₘ formula (1)
containing a moiety M(L)ₙ of the formula (2), formula (3) or formula (4): 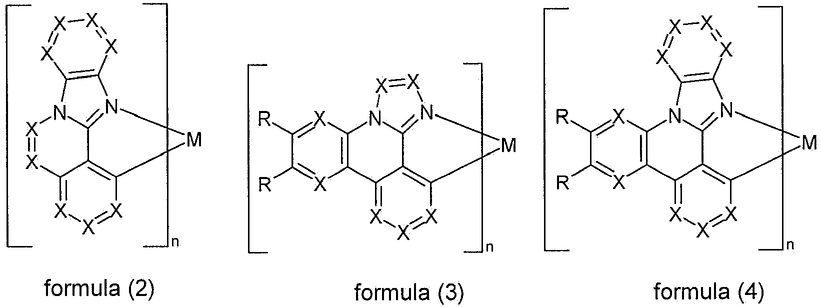 where the following applies to the symbols and indices used:
M is iridium or platinum;
X is selected on each occurrence, identically or differently, from the group consisting of CR and N;
R is on each occurrence, identically or differently, H, D, F, Cl, Br, I, N(R¹)₂, CN, NO₂, OH, COOH, C(=O)N(R¹)₂, Si(R¹)₃, B(OR¹)₂, C(=O)R¹, P(=O)(R¹)₂, S(=O)R¹, S(=O)₂R¹, OSO₂R¹, a straightchain alkyl, alkoxy or thioalkoxy group having 1 to 20 C atoms or an alkenyl or alkynyl group having 2 to 20 C atoms or a branched or cyclic alkyl, alkoxy or thioalkoxy group having 3 to 20 C atoms, which may in each case be substituted by one or more radicals R¹, where one or more non-adjacent CH₂ groups may be replaced by R¹C=CR¹, C≡C, Si(R¹)₂, C=O, NR¹, O, S or CONR¹ and where one or more H atoms may be replaced by D, F, Cl, Br, I or CN, or an aromatic or heteroaromatic ring system having 5 to 60 aromatic ring atoms, which may in each case be substituted by one or more radicals R¹, or an aryloxy or heteroaryloxy group having 5 to 40 aromatic ring atoms, which may be substituted by one or more radicals R¹, or an aralkyl or heteroaralkyl group having 5 to 40 aromatic ring atoms, which may be substituted by one or more radicals R¹, or a diarylamino group, diheteroarylamino group or arylheteroarylamino group having 10 to 40 aromatic ring atoms, which may be substituted by one or more radicals R¹; two adjacent radicals R may also form a mono- or polycyclic, aliphatic, aromatic or heteroaromatic ring system with one another;
R¹ is on each occurrence, identically or differently, H, D, F, Cl, Br, I, N(R²)₂, CN, NO₂, Si(R²)_{3,} B(OR²)₂, C(=O)R², P(=O)(R²)₂, S(=O)R², S(=O)₂R², OSO₂R², a straight-chain alkyl, alkoxy or thioalkoxy group having 1 to 20 C atoms or an alkenyl or alkynyl group having 2 to 20 C atoms or a branched or cyclic alkyl, alkoxy or thioalkoxy group having 3 to 20 C atoms, which may in each case be substituted by one or more radicals R², where one or more non-adjacent CH₂ groups may be replaced by R²C=CR², C≡C, Si(R²)₂, C=O, NR², O, S or CONR² and where one or more H atoms may be replaced by D, F, Cl, Br, I, CN or NO₂, or an aromatic or heteroaromatic ring system having 5 to 60 aromatic ring atoms, which may in each case be substituted by one or more radicals R², or an aryloxy or heteroaryloxy group having 5 to 40 aromatic ring atoms, which may be substituted by one or more radicals R², or an aralkyl or heteroaralkyl group having 5 to 40 aromatic ring atoms, which may be substituted by one or more radicals R², or a diarylamino group, diheteroarylamino group or arylheteroarylamino group having 10 to 40 aromatic ring atoms, which may be substituted by one or more radicals R²; two or more adjacent radicals R¹ with one another or R¹ with R may form a mono- or polycyclic, aliphatic, aromatic or heteroaromatic ring system;
R² is on each occurrence, identically or differently, H, D, F or an aliphatic, aromatic and/or heteroaromatic hydrocarbon radical having 1 to 20 C atoms, in which, in addition, one or more H atoms may be replaced by F; two or more substituents R² may also form a mono- or polycyclic, aliphatic ring system with one another;
L' is, identically or differently on each occurrence, any desired co-ligand;
n is 1, 2 or 3;
m is 0, 1, 2, 3 or 4;
a plurality of ligands L may also be linked to one another or L may be linked to L' via a single bond or a divalent or trivalent bridge and thus form a tridentate, tetradentate, pentadentate or hexadentate ligand system;
a substituent R may also additionally coordinate to the metal;
**characterised in that** two adjacent groups X stand for CR and the respective radicals R, together with the C atoms, form a ring of the following formula (5) or formula (6), 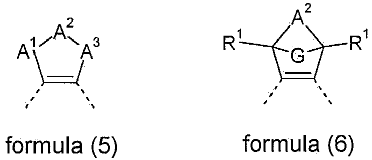 where R¹ and R² have the above-mentioned meanings, the dashed bonds indicate the linking of the two carbon atoms in the ligand, and furthermore:
A¹, A³ are, identically or differently on each occurrence, C(R³)₂, O, S, NR³ or C(=O);
A² is C(R¹)₂, O, S, NR³ or C(=O);
G is an alkylene group having 1, 2 or 3 C atoms, which may be substituted by one or more radicals R², or is -CR²=CR²- or an ortholinked arylene or heteroarylene group having 5 to 14 aromatic ring atoms, which may be substituted by one or more radicals R²;
R³ is, identically or differently on each occurrence, F, a straight-chain alkyl or alkoxy group having 1 to 20 C atoms, a branched or cyclic alkyl or alkoxy group having 3 to 20 C atoms, which may in each case be substituted by one or more radicals R², where one or more non-adjacent CH₂ groups may be replaced by R²C=CR², C≡C, Si(R²)₂, C=O, NR², O, S or CONR² and where one or more H atoms may be replaced by D or F, or an aromatic or heteroaromatic ring system having 5 to 24 aromatic ring atoms, which may in each case be substituted by one or more radicals R², or an aryloxy or heteroaryloxy group having 5 to 24 aromatic ring atoms, which may be substituted by one or more radicals R², or an aralkyl or heteroaralkyl group having 5 to 24 aromatic ring atoms, which may be substituted by one or more radicals R²; two radicals R³ which are bonded to the same carbon atom may form an aliphatic or aromatic ring system with one another and thus form a spiro system; furthermore, R³ may form an aliphatic ring system with an adjacent radical R or R¹;
with the proviso that two heteroatoms in A¹-A²-A3 are not bonded directly to one another.

2. Compound according to Claim 1, **characterised in that** the moieties of the formula (2) are selected from the formulae (2-A) to (2-Q), **in that** the moieties of the formula (3) are selected from the formulae (3-A) to (3-F) and **in that** the moieties of the formula (4) are selected from the formulae (4-A) to (4-F), 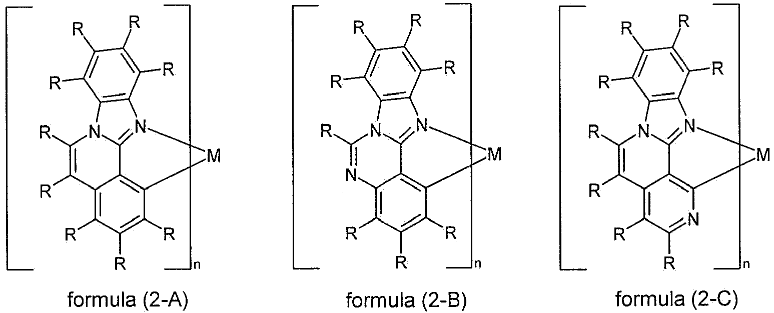 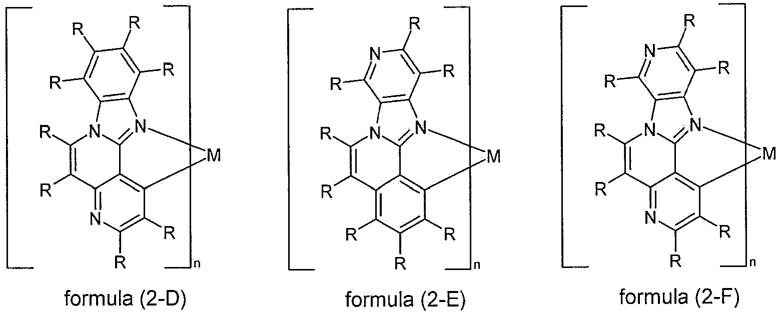 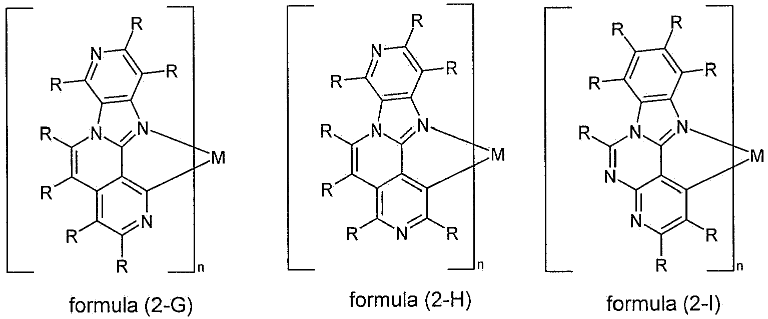 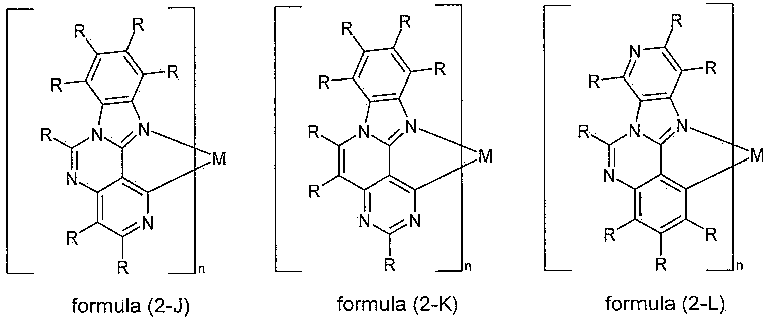 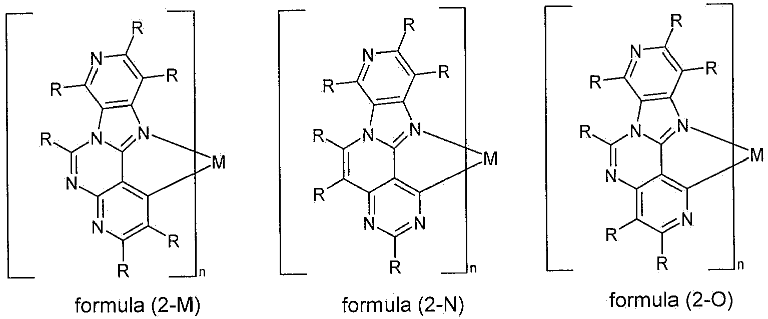 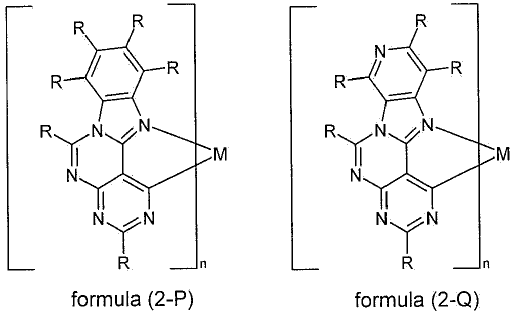 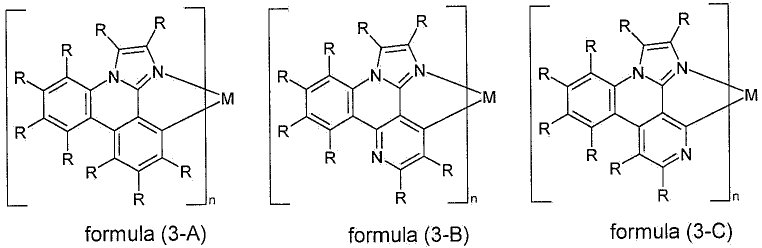 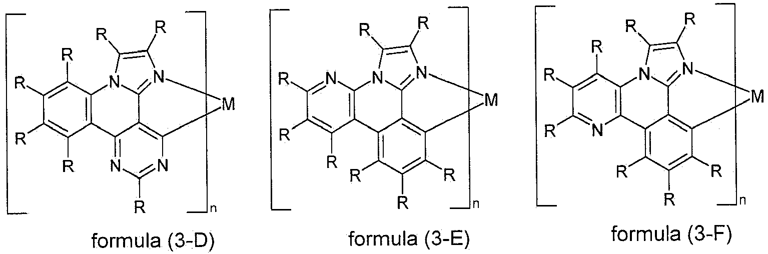 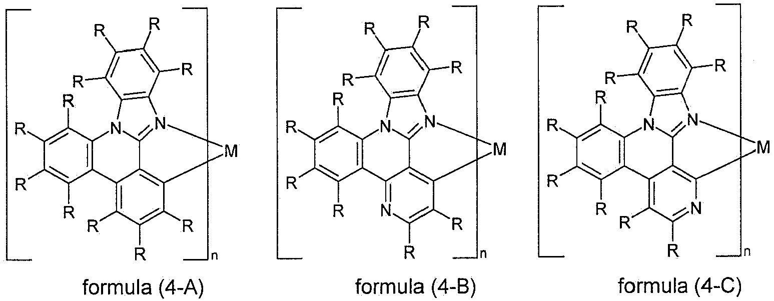 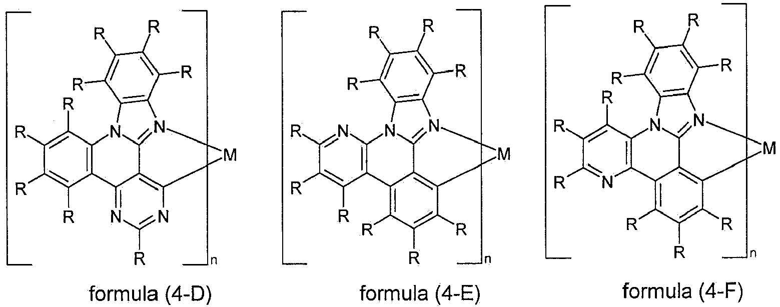 where the symbols and indices used have the meanings given in Claim 1.

3. Compound according to Claim 1 or 2, **characterised in that** the moieties of the formula (2) are selected from the formulae (2-1) to (2-5), the moieties of the formula (3) are selected from the formulae (3-1) to (3-3) and the moieties of the formula (4) are selected from the formulae (4-1) to (4-4), 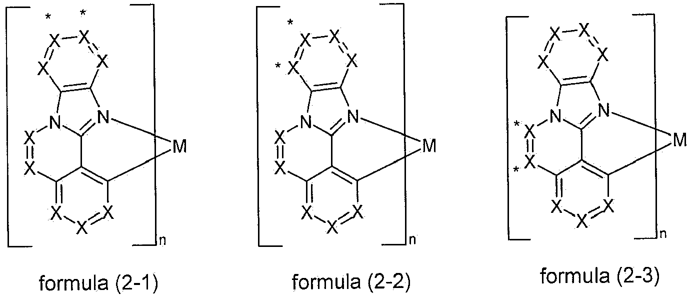 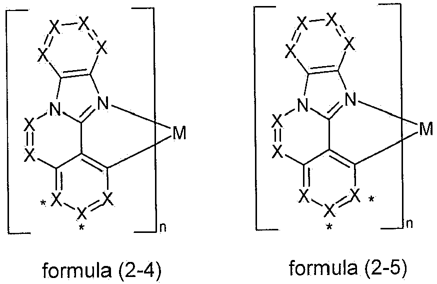 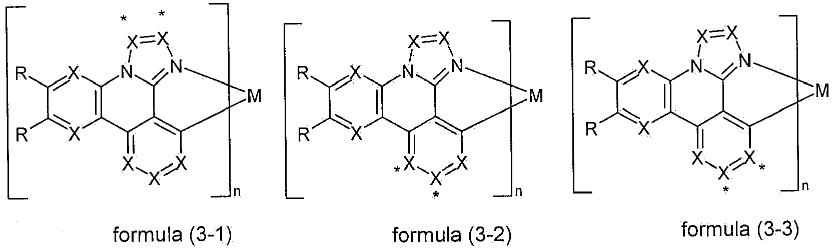 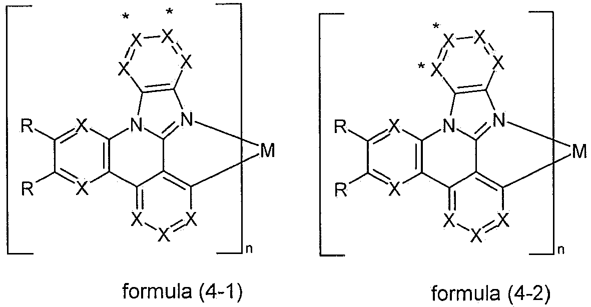 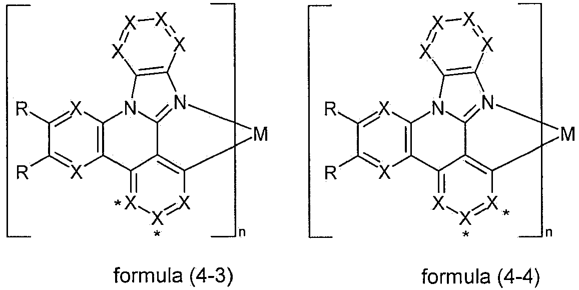 where the symbols and indices used have the meanings given in Claim 1 and * in each case denotes the positions which stand for CR, where the respective radicals R, together with the C atoms to which they are bonded, form a ring of the above-mentioned formula (5) or (6).

4. Compound according to one or more of Claims 1 to 3, **characterised in that** the structure of the formula (5) is selected from the formulae (5-A), (5-B), (5-C) and (5-D) and the structure of the formula (6) is selected from the formulae (6-A) and (6-B), 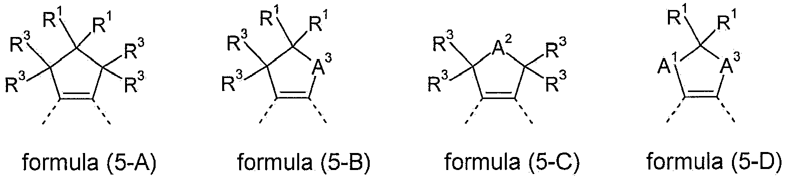 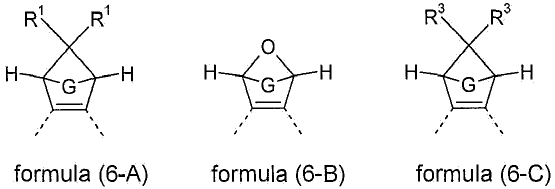 where R¹ and R³ have the meanings given in Claim 1 and A¹, A² and A³ stand, identically or differently on each occurrence, for O or NR³.

5. Compound according to one or more of Claims 1 to 4, **characterised in that** the group G stands for an ethylene group, which may be substituted by one or more radicals R², where R² preferably stands, identically or differently on each occurrence, for H or an alkyl group having 1 to 4 C atoms, or for an ortho-arylene group having 6 to 10 C atoms, which may be substituted by one or more radicals R², but is preferably unsubstituted.

6. Compound according to one or more of Claims 1 to 5, **characterised in that**, if one or more groups X stand for nitrogen, a group R which is selected from the group consisting of CF₃, OCF₃, an alkyl or alkoxy group having 1 to 10 C atoms, an aromatic or heteroaromatic ring system or an aralkyl or heteroaralkyl group is bonded as substituent adjacent to this nitrogen atom or **in that** this radical R forms a ring of the formula (5) or (6) with an adjacent radical R.

7. Compound according to one or more of Claims 1 to 6, **characterised in that** the substituent R which is bonded in the ortho-position to the metal coordination represents a group, likewise coordinated to the metal M, which is selected from aryl or heteroaryl groups, aryl or alkyl cyanides, aryl or alkyl isocyanides, amines, amides, alcohols, alcoholates, thioalcohols, thioalcoholates, phosphines, phosphites, carbonyl functions, carboxylates, carbamides or aryl- or alkylacetylides.

8. Compound according to one or more of Claims 1 to 7, selected from the structures of the formulae (13) to (18), 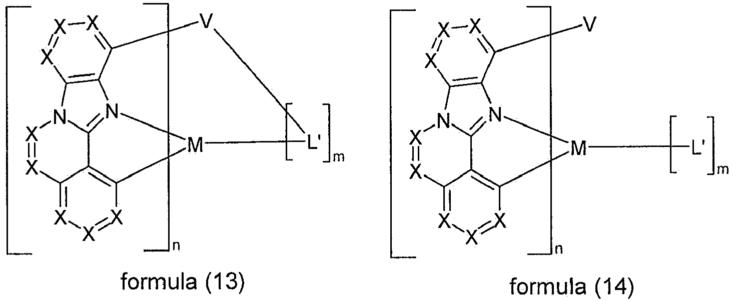 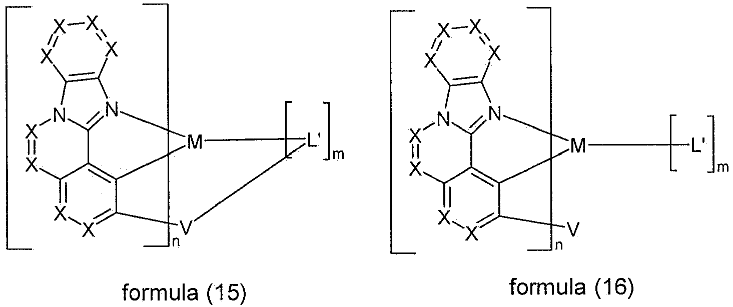 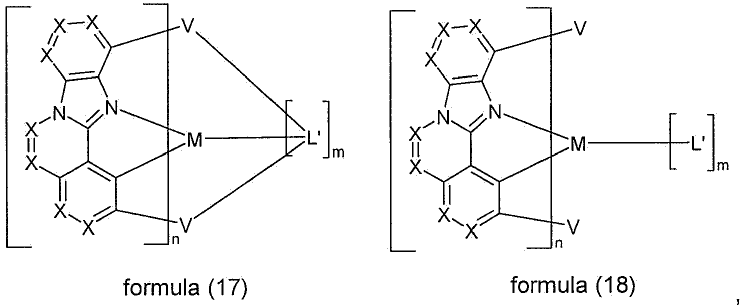 where the symbols and indices used have the meanings given in Claim 1 and V represents a single bond or a bridging unit containing 1 to 80 atoms from the third, fourth, fifth and/or sixth main group (group 13, 14, 15 or 16 in accordance with IUPAC) or represents a 3- to 6-membered homo- or heterocycle and the part-ligands L are covalently bonded to one another or to L'.

9. Compound according to one or more of Claims 1 to 8, **characterised in that** L' is selected from the group consisting of carbon monoxide, nitrogen monoxide, alkyl cyanides, aryl cyanides, alkyl isocyanides, aryl isocyanides, amines, phosphines, phosphites, arsines, stibines, nitrogen-containing heterocycles, carbenes, hydride, deuteride, the halides F-, Cl-, Br and I-, alkylacetylides, arylacetylides, cyanide, cyanate, isocyanate, thiocyanate, isothiocyanate, aliphatic or aromatic alcoholates, aliphatic or aromatic thioalcoholates, amides, carboxylates, aryl groups, O²⁻, S²⁻, carbides, which result in coordination in the form R-C≡M, nitrenes, which result in coordination in the form R-N=M, where R generally stands for a substituent, N³⁻, diamines, imines, diimines, heterocycles containing two nitrogen atoms, diphosphines, 1,3-diketonates derived from 1,3-diketones, 3-ketonates derived from 3-ketoesters, carboxylates derived from aminocarboxylic acids, salicyliminates derived from salicylimines, dialcoholates, dithiolates, 3-(2-pyridyl)diazoles, 3-(2-pyridyl)triazoles, borates of nitrogen-containing heterocycles and bidentate monoanionic, neutral or dianionic ligands L', which, with the metal, form a cyclometallated five-membered ring or six-membered ring having at least one metal-carbon bond.

10. Process for the preparation of a compound according to one or more of Claims 1 to 9 by reaction of the corresponding free ligand with metal alcoholates of the formula (71), with metal ketoketonates of the formula (72), with metal halides of the formula (73), with dimeric metal complexes of the formula (74) or with metal complexes of the formula (75), 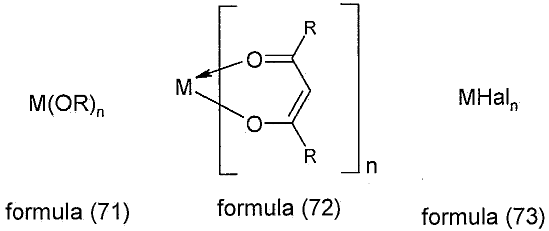 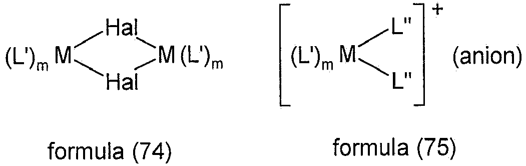 where the symbols M, m, n and R have the meanings indicated in Claim 1, Hal = F, Cl, Br or I, L" stands for an alcohol or a nitrile and (anion) is a non-coordinating anion, where it is also possible to employ metal compounds which carry both alcoholate and/or halide and/or hydroxyl and also ketoketonate radicals.

11. Polymer containing one or more of the compounds according to one or more of Claims 1 to 9, where one or more bonds are present from the compound to the polymer.

12. Formulation comprising at least one compound according to one or more of Claims 1 to 9 or a polymer according to Claim 11 and at least one further compound.

13. Use of a compound according to one or more of Claims 1 to 9 or a polymer according to Claim 11 in an electronic device, in particular selected from the group consisting of organic electroluminescent devices, organic integrated circuits, organic field-effect transistors, organic thin-film transistors, organic light-emitting transistors, organic solar cells, organic optical detectors, organic photoreceptors, organic field-quench devices, light-emitting electrochemical cells or organic laser diodes, or use for the generation of singlet oxygen or in photocatalysis.

14. Electronic device containing one or more compounds according to one or more of Claims 1 to 9 and/or one or more polymers according to Claim 11.

15. Electronic device according to Claim 14, which is an organic electroluminescent device, **characterised in that** the compound according to one or more of Claims 1 to 9 is employed as emitting compound in one or more emitting layers.

16. Electronic device according to Claim 15, **characterised in that** the matrix material employed in at least one of the emitting layers is a ketone, phosphine oxide, sulfoxide, sulfone, triarylamine, carbazole derivative, indolocarbazole derivative, indenocarbazole derivative, azacarbazole, bipolar matrix material, silane, azaborole, boronic ester, diazasilole derivative, diazaphosphole derivative, triazine derivative, zinc complex, dibenzofuran derivative or bridged carbazole derivative or a mixture of two or more of these materials.

## Revendications

1. Composé de la formule (1):
M(L)ₙ(L')ₘ formule (1)
contenant une moitié M(L)ₙ de la formule (2), de la formule (3) ou de la formule (4) : 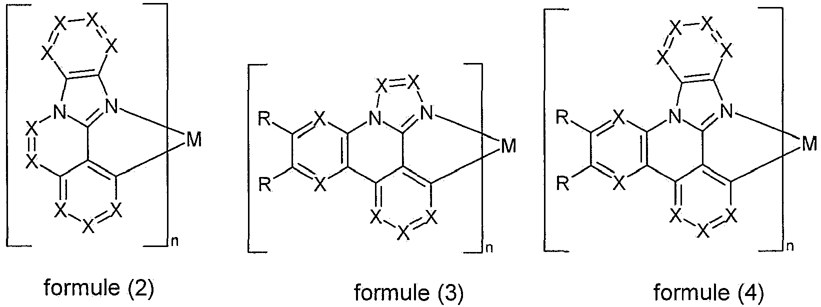 dans lesquelles ce qui suit s'applique aux symboles et aux indices qui sont utilisés :
M est iridium ou platine ;
X est sélectionné pour chaque occurrence, de manière identique ou différente, parmi le groupe qui est constitué par CR et N ;
R est pour chaque occurrence, de manière identique ou différente, H, D, F, Cl, Br, I, N(R¹)₂, CN, NO₂, OH, COOH, C(=O)N(R¹)₂, Si(R¹)₃, B(OR¹)₂, C(=O)R¹, P(=O)(R¹)₂, S(=O)R¹, S(=O)₂R¹, OSO₂R¹, un groupe alkyle, alcoxy ou thioalcoxy en chaîne droite qui comporte de 1 à 20 atome(s) de C ou un groupe alkényle ou alkynyle qui comporte de 2 à 20 atomes de C ou un groupe alkyle, alcoxy ou thioalcoxy ramifié ou cyclique qui comporte de 3 à 20 atomes de C, lequel peut dans chaque cas être substitué par un radical ou par plusieurs radicaux R¹, où un ou plusieurs groupe(s) CH₂ non adjacents peut/peuvent être remplacé(s) par R¹C=CR¹, C=C, Si(R¹)₂, C=O, NR¹, O, S ou CONR¹ et où un ou plusieurs atome(s) de H peut/peuvent être remplacé(s) par D, F, Cl, Br, I ou CN, ou un système de cycle aromatique ou hétéroaromatique qui comporte de 5 à 60 atomes de cycle aromatique, lequel peut dans chaque cas être substitué par un radical ou par plusieurs radicaux R¹, ou un groupe aryloxy ou hétéroaryloxy qui comporte de 5 à 40 atomes de cycle aromatique, lequel peut être substitué par un radical ou par plusieurs radicaux R¹, ou un groupe aralkyle ou hétéroaralkyle qui comporte de 5 à 40 atomes de cycle aromatique, lequel peut être substitué par un radical ou par plusieurs radicaux R¹, ou un groupe diarylamino, un groupe dihétéroarylamino ou un groupe arylhétéroarylamino qui comporte de 10 à 40 atomes de cycle aromatique, lequel peut être substitué par un radical ou par plusieurs radicaux R¹ ; deux radicaux R adjacents peuvent également former un système de cycle aliphatique, aromatique ou hétéroaromatique mono- ou polycyclique l'un avec l'autre ;
R¹ est pour chaque occurrence, de manière identique ou différente, H, D, F, Cl, Br, I, N(R²)₂, CN, NO₂, Si(R²)₃, B(OR²)₂, C(=O)R², P(=O)(R²)₂, S(=O)R², S(=O)₂R², OSO₂R², un groupe alkyle, alcoxy ou thioalkoxy en chaîne droite qui comporte de 1 à 20 atome(s) de C ou un groupe alkényle ou alkynyle qui comporte de 2 à 20 atomes de C ou un groupe alkyle, alcoxy ou thioalcoxy ramifié ou cyclique qui comporte de 3 à 20 atomes de C, lequel peut dans chaque cas être substitué par un radical ou par plusieurs radicaux R², où un ou plusieurs groupe(s) CH₂ non adjacents peut/peuvent être remplacés par R²C=CR², C=C, Si(R²)₂, C=O, NR², O, S ou CONR² et où un ou plusieurs atome(s) de H peut/peuvent être remplacé(s) par D, F, Cl, Br, I, CN ou NO₂, ou un système de cycle aromatique ou hétéroaromatique qui comporte de 5 à 60 atomes de cycle aromatique, lequel peut dans chaque cas être substitué par un radical ou par plusieurs radicaux R², ou un groupe aryloxy ou hétéroaryloxy qui comporte de 5 à 40 atomes de cycle aromatique, lequel peut être substitué par un radical ou par plusieurs radicaux R², ou un groupe aralkyle ou hétéroaralkyle qui comporte de 5 à 40 atomes de cycle aromatique, lequel peut être substitué par un radical ou par plusieurs radicaux R², ou un groupe diarylamino, un groupe dihétéroarylamino ou un groupe arylhétéroarylamino qui comporte de 10 à 40 atomes de cycle aromatique, lequel peut être substitué par un radical ou par plusieurs radicaux R² ; deux radicaux R¹ adjacents ou plus peuvent former l'un avec l'autre ou les uns avec les autres ou R¹ avec R un système de cycle aliphatique, aromatique ou hétéroaromatique mono- ou polycyclique ;
R² est pour chaque occurrence, de manière identique ou différente, H, D, F ou un radical hydrocarbone aliphatique, aromatique et/ou hétéroaromatique qui comporte de 1 à 20 atome(s) de C, où, en outre, un ou plusieurs atome(s) de H peut/peuvent être remplacé(s) par F ; deux substituants R² ou plus peuvent également former un système de cycle aliphatique mono- ou polycyclique l'un avec l'autre ou les uns avec les autres ;
L' est, de manière identique ou différente pour chaque occurrence, un quelconque co-ligand souhaité ;
n est 1, 2 ou 3 ;
m est 0, 1, 2, 3 ou 4 ;
une pluralité de ligands L peuvent également être liés les uns aux autres ou L peut être lié à L' via une liaison simple ou un pont divalent ou trivalent et peuvent ainsi former un système de ligand tridenté, tétradenté, pentadenté ou hexadenté ;
un substituant R peut également de façon additionnelle être coordonné au métal ;
**caractérisé en ce que** deux groupes X adjacents représentent CR et les radicaux respectifs R, en association avec les atomes de C, forment un cycle de la formule (5) ou de la formule (6) qui suit : 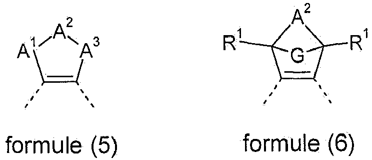 dans lesquelles R¹ et R² présentent les significations qui ont été mentionnées ci-avant, les liens en pointillés indiquent la liaison des deux atomes de carbone dans le ligand, et en outre :
A¹, A³ sont, de manière identique ou différente pour chaque occurrence, C(R³)₂, O, S, NR³ ou C(=O) ;
A² est C(R¹)₂, O, S, NR³ ou C(=O) ;
G est un groupe alkylène qui comporte 1, 2 ou 3 atome(s) de C, lequel peut être substitué par un radical ou par plusieurs radicaux R², ou est -CR²=CR²- ou un groupe arylène ou hétéroarylène ortho-lié qui comporte de 5 à 14 atomes de cycle aromatique, lequel peut être substitué par un radical ou par plusieurs radicaux R² ;
R³ est, de manière identique ou différente pour chaque occurrence, F, un groupe alkyle ou alcoxy en chaîne droite qui comporte de 1 à 20 atome(s) de C, un groupe alkyle ou alcoxy ramifié ou cyclique qui comporte de 3 à 20 atomes de C, lequel peut dans chaque cas être substitué par un radical ou par plusieurs radicaux R², où un ou plusieurs groupe(s) CH₂ non adjacents peut/peuvent être remplacé(s) par R²C=CR², C≡C, Si(R²)₂, C=O, NR², O, S ou CONR² et où un ou plusieurs atome(s) de H peut/peuvent être remplacé(s) par D ou F, ou un système de cycle aromatique ou hétéroaromatique qui comporte de 5 à 24 atomes de cycle aromatique, lequel peut dans chaque cas être substitué par un radical ou par plusieurs radicaux R², ou un groupe aryloxy ou hétéroaryloxy qui comporte de 5 à 24 atomes de cycle aromatique, lequel peut être substitué par un radical ou par plusieurs radicaux R², ou un groupe aralkyle ou hétéroaralkyle qui comporte de 5 à 24 atomes de cycle aromatique, lequel peut être substitué par un radical ou par plusieurs radicaux R² ; deux radicaux R³ qui sont liés au même atome de carbone peuvent former un système de cycle aliphatique ou aromatique l'un avec l'autre et peuvent ainsi former un système spiro ; en outre, R³ peut former un système de cycle aliphatique avec un radical adjacent R ou R¹ ;
étant entendu que deux hétéroatomes dans A¹-A²-A3 ne sont pas liés directement l'un à l'autre.

2. Composé selon la revendication 1, **caractérisé en ce que** les moitiés de la formule (2) sont sélectionnées parmi les formules (2-A) à (2-Q), **en ce que** les moitiés de la formule (3) sont sélectionnées parmi les formules (3-A) à (3-F) et **en ce que** les moitiés de la formule (4) sont sélectionnées parmi les formules (4-A) à (4-F) : 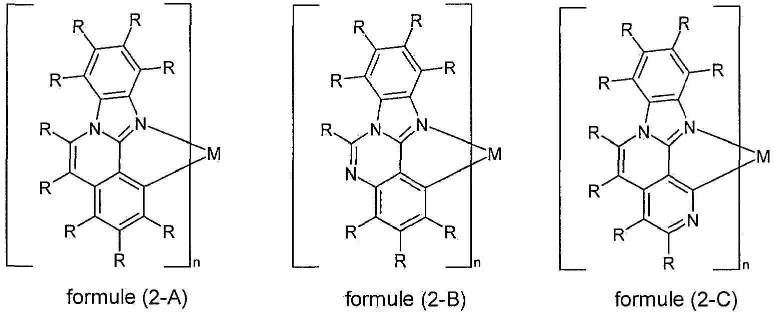 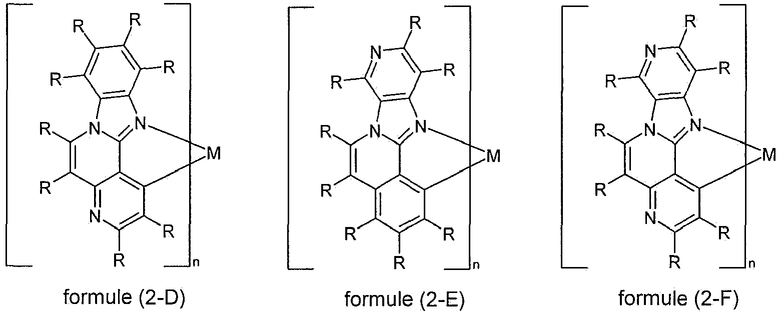 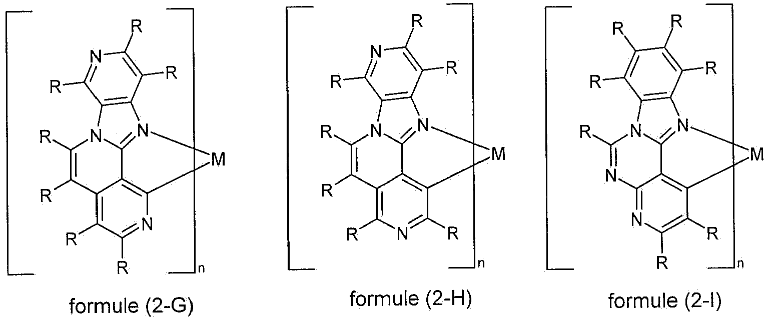 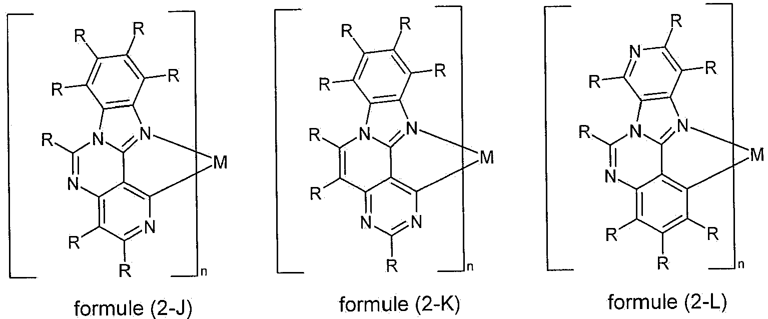 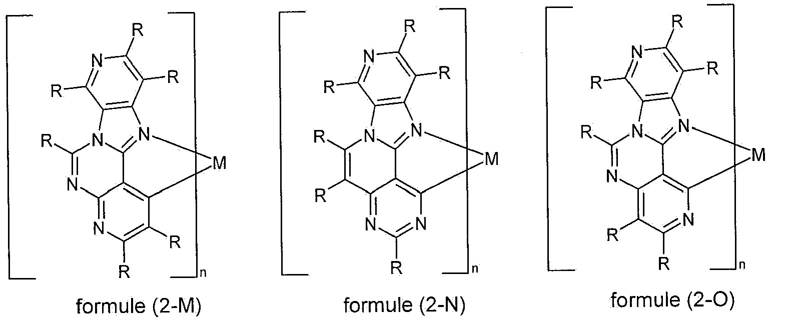 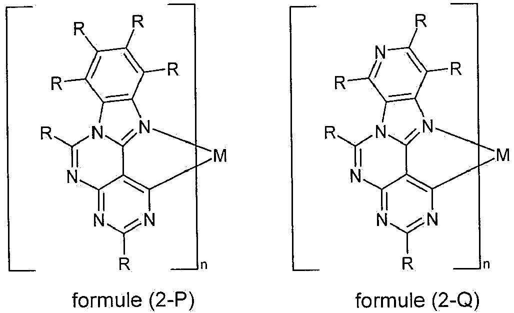 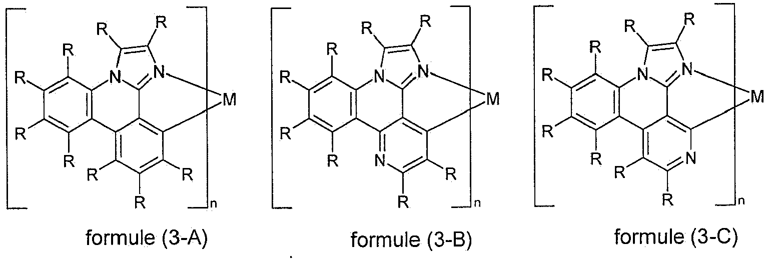 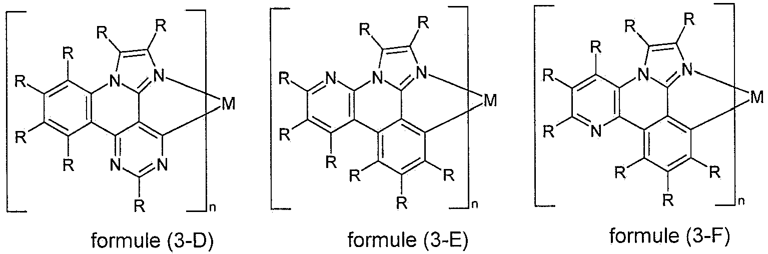 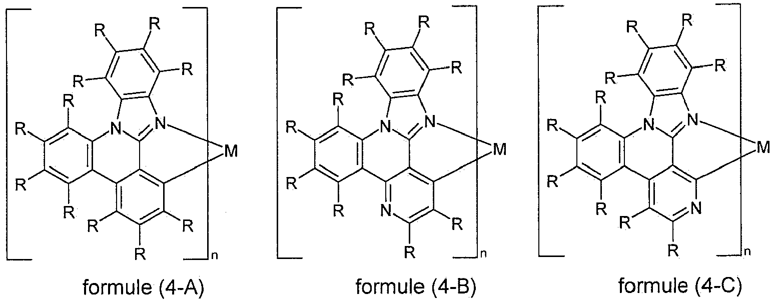 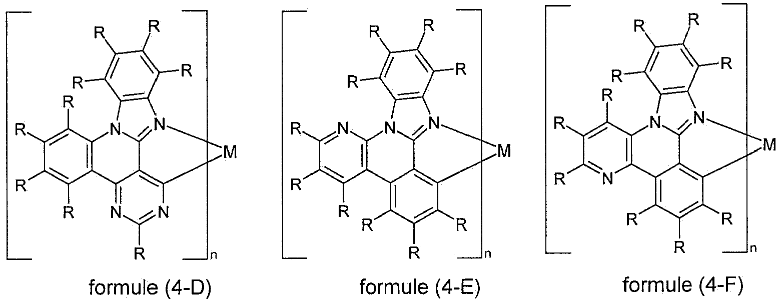 dans lesquelles les symboles et les indices qui sont utilisés présentent les significations qui ont été données selon la revendication 1.

3. Composé selon la revendication 1 ou 2, **caractérisé en ce que** les moitiés de la formule (2) sont sélectionnées parmi les formules (2-1) à (2-5), les moitiés de la formule (3) sont sélectionnées parmi les formules (3-1) à (3-3) et les moitiés de la formule (4) sont sélectionnées parmi les formules (4-1) à (4-4) : 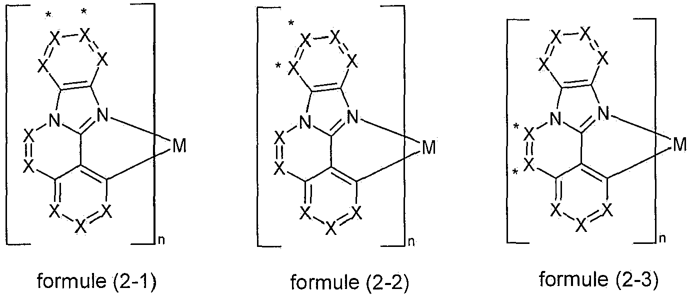 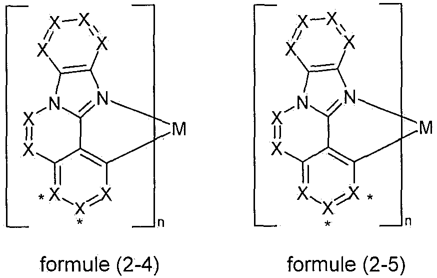 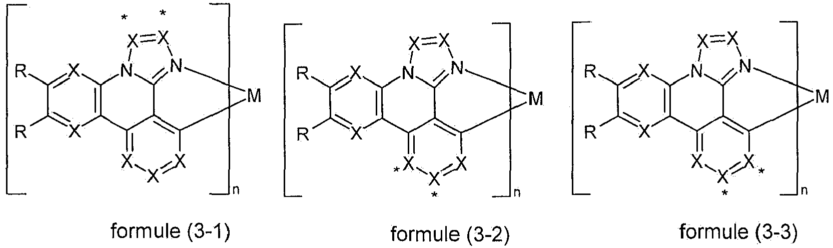 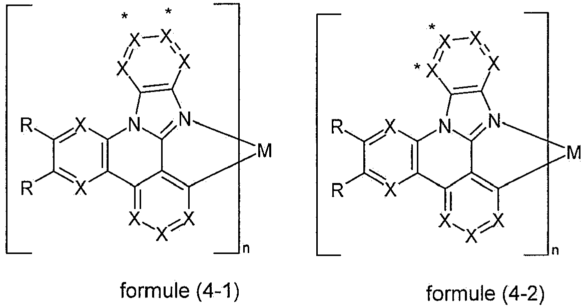 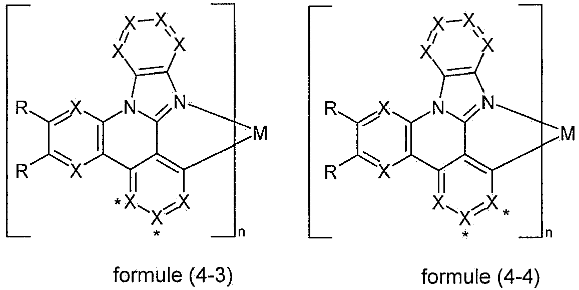 dans lesquelles les symboles et les indices qui sont utilisés présentent les significations qui ont été données selon la revendication 1 et * représente dans chaque cas les positions qui représentent CR, où les radicaux R respectifs, en association avec les atomes de C auxquels ils sont liés, forment un cycle de la formule (5) ou (6) qui a été mentionnée ci-avant.

4. Composé selon une ou plusieurs des revendications 1 à 3, **caractérisé en ce que** la structure de la formule (5) est sélectionnée parmi les formules (5-A), (5-B), (5-C) et (5-D) et la structure de la formule (6) est sélectionnée parmi les formules (6-A) et (6-B) : 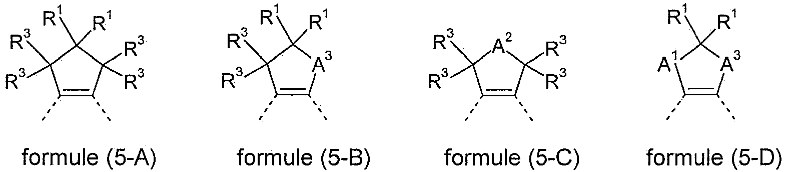 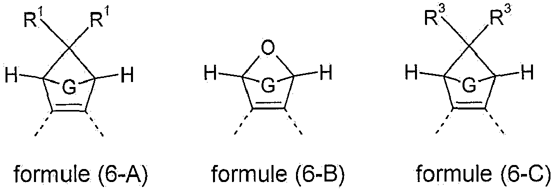 dans lesquelles R¹ et R³ présentent les significations qui ont été données selon la revendication 1 et A¹, A² et A³ représentent, de manière identique ou différente pour chaque occurrence, O ou NR³.

5. Composé selon une ou plusieurs des revendications 1 à 4, **caractérisé en ce que** le groupe G représente un groupe éthylène, lequel peut être substitué par un radical ou par plusieurs radicaux R², où R² représente de préférence, de manière identique ou différente pour chaque occurrence, H ou un groupe alkyle qui comporte de 1 à 4 atome(s) de C, ou un groupe ortho-arylène qui comporte de 6 à 10 atomes de C, lequel peut être substitué par un radical ou par plusieurs radicaux R², mais est de préférence non substitué.

6. Composé selon une ou plusieurs des revendications 1 à 5, **caractérisé en ce que**, si un ou plusieurs groupe(s) X représente(nt) azote, un groupe R qui est sélectionné parmi le groupe qui est constitué par CF₃, OCF₃, un groupe alkyle ou alcoxy qui comporte de 1 à 10 atome(s) de C, un système de cycle aromatique ou hétéroaromatique ou un groupe aralkyle ou hétéroaralkyle est lié en tant que substituant adjacent à cet atome d'azote ou **en ce que** ce radical R forme un cycle de la formule (5) ou (6) avec un radical R adjacent.

7. Composé selon une ou plusieurs des revendications 1 à 6, **caractérisé en ce que** le substituant R qui est lié au niveau de la position ortho à la coordination de métal représente un groupe, pareillement coordonné au métal M, qui est sélectionné parmi les groupes aryle ou hétéroaryle, les cyanures d'aryle ou d'alkyle, les isocyanures d'aryle ou d'alkyle, les aminés, les amides, les alcools, les alcoolates, les thioalcools, les thioalcoolates, les phosphines, les phosphites, les fonctions carbonyle, les carboxylates, les carbamides ou les aryl- ou alkylacétylures.

8. Composé selon une ou plusieurs des revendications 1 à 7, sélectionné parmi les structures des formules (13) à (18) : 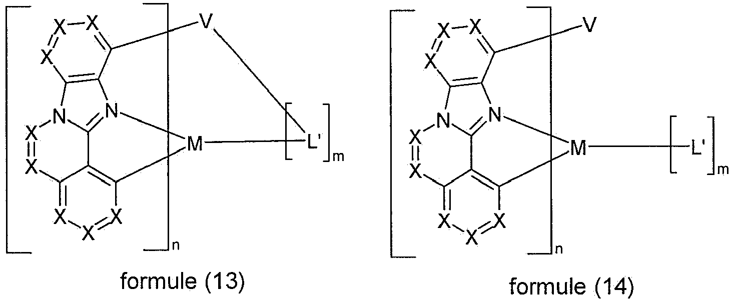 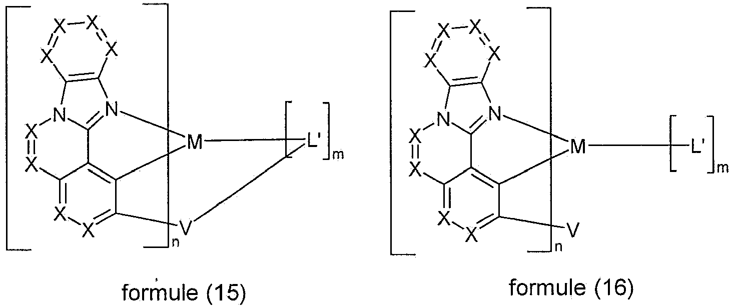 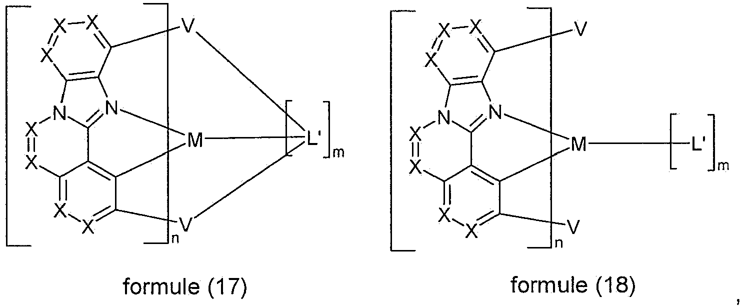 dans lesquelles les symboles et les indices qui sont utilisés présentent les significations qui ont été données selon la revendication 1 et V représente une liaison simple ou une unité de pontage qui contient de 1 à 80 atome(s) pris parmi les troisième, quatrième, cinquième et/ou sixième groupes principaux (le groupe 13, 14, 15 ou 16 conformément à IUPAC) ou représente un homo- ou hétérocycle à 3 à 6 éléments et les ligands partiels L sont liés de façon covalente les uns aux autres ou sont liés à L'.

9. Composé selon une ou plusieurs des revendications 1 à 8, **caractérisé en ce que** L' est sélectionné parmi le groupe qui est constitué par le monoxyde de carbone, le monoxyde d'azote, les cyanures d'alkyle, les cyanures d'aryle, les isocyanures d'alkyle, les isocyanures d'aryle, les aminés, les phosphines, les phosphites, les arsines, les stibines, les hétérocycles contenant de l'azote, les carbènes, l'hydrure, le deutérure, les halogénures F⁻, Cl⁻, Br et I⁻, les alkylacétylures, les arylacétylures, le cyanure, le cyanate, l'isocyanate, le thiocyanate, l'isothiocyanate, les alcoolates aliphatiques ou aromatiques, les thioalcoolates aliphatiques ou aromatiques, les amides, les carboxylates, les groupes aryle, O²⁻, S²⁻, les carbures, lesquels conduisent à une coordination sous la forme R-C≡M, les nitrènes, lesquels conduisent à une coordination sous la forme R-N=M, où R représente de façon générale un substituant, N³⁻, les diamines, les imines, les diimines, les hétérocycles qui contiennent deux atomes d'azote, les diphosphines, les 1,3-dicétonates qui sont dérivés à partir des 1,3-dicétones, les 3-cétonates qui sont dérivés à partir des 3-cétoesters, les carboxylates qui sont dérivés à partir des acides aminocarboxyliques, les salicyliminates qui sont dérivés à partir des salicylimines, les dialcoolates, les dithiolates, les 3-(2-pyridyl)diazoles, les 3-(2-pyridyl)triazoles, les borates d'hétérocycles qui contiennent de l'azote et les ligands monoanioniques, neutres ou dianioniques bidentés L', lesquels, avec le métal, forment un cycle à 5 éléments ou un cycle à 6 éléments cyclométallaté qui comporte au moins une liaison métal-carbone.

10. Procédé pour la préparation d'un composé selon une ou plusieurs des revendications 1 à 9 par réaction du ligand libre correspondant avec des alcoolates de métal/métaux de la formule (71), avec des cétocétonates de métal/métaux de la formule (72), avec des halogénures de métal/métaux de la formule (73), avec des complexes de métal/métaux dimériques de la formule (74) ou avec des complexes de métal/métaux de la formule (75) : 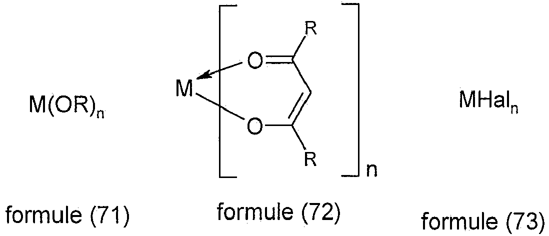 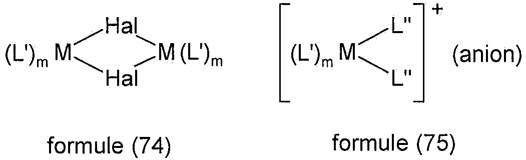 dans lesquelles les symboles M, m, n et R présentent les significations qui ont été indiquées selon la revendication 1, Hal = F, Cl, Br ou I, L" représente un alcool ou un nitrile et (anion) est un anion de non coordination, où il est également possible d'utiliser des composés de métal/ métaux qui portent à la fois des radicaux alcoolate et/ou halogénure et/ou hydroxyle et également un cétocétonate.

11. Polymère contenant un ou plusieurs des composés selon une ou plusieurs des revendications 1 à 9, où une ou plusieurs liaison(s) est/sont présente(s) depuis le composé jusqu'au polymère.

12. Formulation comprenant au moins un composé selon une ou plusieurs des revendications 1 à 9 ou un polymère selon la revendication 11 et au moins un autre composé.

13. Utilisation d'un composé selon une ou plusieurs des revendications 1 à 9 ou d'un polymère selon la revendication 11 dans un dispositif électronique, en particulier qui est sélectionné parmi le groupe qui est constitué par les dispositifs électroluminescents organiques, les circuits intégrés organiques, les transistors à effet de champ organiques, les transistors à film mince organiques, les transistors à émission de lumière organiques, les cellules solaires organiques, les détecteurs optiques organiques, les photorécepteurs organiques, les dispositifs à extinction de champ organiques, les cellules électrochimiques à émission de lumière ou les diodes laser organiques, ou utilisation pour la génération d'oxygène singlet ou lors d'une photocatalyse.

14. Dispositif électronique contenant un ou plusieurs composé(s) selon une ou plusieurs des revendications 1 à 9 et/ou un ou plusieurs polymère(s) selon la revendication 11.

15. Dispositif électronique selon la revendication 14, lequel est un dispositif électroluminescent organique, **caractérisé en ce que** le composé selon une ou plusieurs des revendications 1 à 9 est utilisé en tant que composé d'émission dans une ou plusieurs couche(s) d'émission.

16. Dispositif électronique selon la revendication 15, **caractérisé en ce que** le matériau de matrice qui est utilisé dans au moins l'une des couches d'émission est un cétone, de l'oxyde de phosphine, du sulfoxyde, du sulfone, du triarylamine, un dérivé de carbazole, un dérivé d'indolocarbazole, un dérivé d'indénocarbazole, de l'azacarbazole, un matériau de matrice bipolaire, du silane, de l'azaborole, de l'ester boronique, un dérivé de diazasilole, un dérivé de diazaphosphole, un dérivé de triazine, un complexe de zinc, un dérivé de dibenzofurane ou un dérivé de carbazole ponté ou un mélange de deux ou plus de ces matériaux.
